# EUROPEAN PATENT APPLICATION

(11) **EP 4 603 587 A1**
(43) Date of publication of application: **20.08.2025**
(21) Application number: 23877309.7
(22) Date of filing: 11.10.2023
(51) Int. Cl.: C12N 15/62, C07K 14/71, C07K 14/765, C07K 16/00, C07K 19/00, C12N 5/10, C12N 15/13, C12N 15/14, C12N 15/19, C12N 15/63, C12P 21/02

(54) **FUSION PROTEIN OF SERUM ALBUMIN AND PHYSIOLOGICALLY-ACTIVE PROTEIN**

(30) Priority: 11.10.2022 JP 2022163605
(71) Applicant: JCR Pharmaceuticals Co., Ltd., Ashiya-shi, Hyogo 659-0021 (JP)
(72) Inventor: FUJIYAMA, Saki, Kobe-shi, Hyogo 651-2241 (JP); TAKAHASHI, Kenichi, Kobe-shi, Hyogo 651-2241 (JP); SUGANO, Yasunori, Kobe-shi, Hyogo 651-2241 (JP); ONOUCHI, Takashi, Kobe-shi, Hyogo 651-2241 (JP)
(74) Representative: Maiwald GmbH
(86) International application number: PCT/JP2023/036887
(87) International publication number: WO 2024/080306

(57) **Abstract**

According to one embodiment of the present invention, it is possible to efficiently produce a highly-active recombinant protein by expressing, in the form of a fusion protein with serum albumin (SA), a physiologically-active protein having a low expression level and/or low activity when the physiologically-active protein is expressed as a recombinant protein by generally using host cells, such as CHO cells. The fusion protein may be one obtained by binding the physiologically-active protein either to the amino terminal side of the SA or to the carboxyl terminal. Also, the fusion protein may be a conjugate with a ligand or an antibody.

## Description

### Technical Field

The present invention relates to a fusion protein obtained by binding serum albumin (SA) and a protein having physiological activity (physiologically active protein) and a method for producing the fusion protein. The fusion protein refers to a protein obtained by binding, for example, the C terminal of SA and the N terminal of a physiologically active protein. Some physiologically active proteins exhibit low expression level and/or low activity when a gene encoding the physiologically active protein is introduced in a host cell such as a mammalian cell and expressed as a recombinant protein, particularly expressed as a recombinant protein so as to be secreted from the cell and accumulated in a culture solution. The present invention relates to a fusion protein of such SA and a physiologically active protein that can be efficiently produced as a highly active recombinant protein, and a method for producing the fusion protein. Note that the physiologically active protein to be fused with SA is not particularly limited and any physiologically active protein can be fused with SA to obtain a fusion protein.

The present invention particularly relates to a fusion protein obtained by binding serum albumin (SA) and a lysosome enzyme, for example, a fusion protein obtained by binding the C terminal of SA and the N terminal of a lysosome enzyme, or the C terminal of a lysosome enzyme and the N terminal of SA, directly or via a linker. Some lysosome enzymes exhibit low expression level and/or low activity when a gene encoding the lysosome enzyme is introduced in a host cell such as a mammalian cell and expressed as a recombinant protein, particularly expressed as a recombinant protein so as to be secreted from the cell and accumulated in a culture solution. The present invention relates to a fusion protein of such a lysosome enzyme and SA that can be efficiently produced as a highly active recombinant protein, and a method for producing the fusion protein. Note that the physiologically active protein to be fused with a lysosome enzyme is not particularly limited and any lysosome enzyme can be fused with SA to obtain a fusion protein.

The present invention also relates particularly to a fusion protein obtained by binding serum albumin (SA) and galactosylceramidase (GALC), for example, a fusion protein obtained by binding the C terminal of SA and the N terminal of GALC, or the C terminal of GALC and the N terminal of SA, directly or via a linker. GALC may exhibit expression level and/or low activity when a gene encoding the GALC is introduced in a host cell such as a mammalian cell and expressed as a recombinant protein, particularly expressed as a recombinant protein so as to be secreted from the cell and accumulated in a culture solution. The present invention relates to a fusion protein of such GALC and SA that can be efficiently produced as a highly active recombinant protein, and a method for producing the fusion protein.

The present invention also relates particularly to a fusion protein obtained by binding serum albumin (SA) and glucocerebrosidase (GBA), for example, a fusion protein obtained by binding the C terminal of SA and the N terminal of GBA, or the C terminal of GBA and the N terminal of SA, directly or via a linker. GBA may exhibit low expression level and/or low activity when a gene encoding the GBA is introduced in a host cell such as a mammalian cell and expressed as a recombinant protein, particularly expressed as a recombinant protein so as to be secreted from the cell and accumulated in a culture solution. The present invention relates to a fusion protein of such GBA and SA that can be efficiently produced as a highly active recombinant protein, and a method for producing the fusion protein.

The present invention relates to a fusion protein obtained by binding serum albumin (SA) and a cytokine, for example, a fusion protein obtained by binding the C terminal of SA and the N terminal of a cytokine, or the C terminal of a cytokine and the N terminal of SA, directly or via a linker. Some cytokines exhibit low expression level and/or low activity when a gene encoding the cytokine is introduced in a host cell such as a mammalian cell and expressed as a recombinant protein, particularly expressed as a recombinant protein so as to be secreted from the cell and accumulated in a culture solution. The present invention relates to a fusion protein of such a cytokine and SA that can be efficiently produced as a highly active recombinant protein, and a method for producing the fusion protein. Note that the cytokine to be fused with a lysosome enzyme is not particularly limited and any cytokine can be fused with SA to obtain a fusion protein.

The present invention relates particularly to a fusion protein obtained by binding serum albumin (SA) and an interleukin, for example, a fusion protein obtained by binding the C terminal of SA and the N terminal of an interleukin, or the C terminal of an interleukin and the N terminal of SA, directly or via a linker. Some interleukins exhibit low expression level and/or low activity when a gene encoding the interleukin is introduced in a host cell such as a mammalian cell and expressed as a recombinant protein, particularly expressed as a recombinant protein so as to be secreted from the cell and accumulated in a culture solution. The present invention relates to a fusion protein of such an interleukin and SA that can be efficiently produced as a highly active recombinant protein, and a method for producing the fusion protein. Note that the physiologically active protein to be fused with an interleukin is not particularly limited and any interleukin can be fused with SA to obtain a fusion protein.

The present invention relates particularly to a fusion protein obtained by binding serum albumin (SA) and interleukin 10 (IL-10), for example, a fusion protein obtained by binding the C terminal of SA and the N terminal of IL-10, or the C terminal of IL-10 and the N terminal of SA, directly or via a linker. IL-10 may exhibit low expression level and/or low activity when a gene encoding IL-10 is introduced in a host cell such as a mammalian cell and expressed as a recombinant protein, particularly expressed as a recombinant protein so as to be secreted from the cell and accumulated in a culture solution. The present invention relates to a fusion protein of such IL-10 and SA that can be efficiently produced as a highly active recombinant protein, and a method for producing the fusion protein.

The present invention relates to a fusion protein obtained by binding serum albumin (SA) and a neurotrophic factor, for example, a fusion protein obtained by binding the C terminal of SA and the N terminal of a neurotrophic factor, or the C terminal of a neurotrophic factor and the N terminal of SA, directly or via a linker. Some neurotrophic factors exhibit low expression level and/or low activity when a gene encoding the neurotrophic factor is introduced in a host cell such as a mammalian cell and expressed as a recombinant protein, particularly expressed as a recombinant protein so as to be secreted from the cell and accumulated in a culture solution. The present invention relates to a fusion protein of such a neurotrophic factor and SA that can be efficiently produced as a highly active recombinant protein, and a method for producing the fusion protein. Note that the physiologically active protein to be fused with a neurotrophic factor is not particularly limited and any neurotrophic factor can be fused with SA to obtain a fusion protein.

The present invention relates particularly to a fusion protein obtained by binding serum albumin (SA) and a brain-derived neurotrophic factor (BDNF), for example, a fusion protein obtained by binding the C terminal of SA and the N terminal of a BDNF, or the C terminal of a BDNF and the N terminal of SA, directly or via a linker. A BDNF may exhibit low expression level and/or low activity when a gene encoding the BDNF is introduced in a host cell such as a mammalian cell and expressed as a recombinant protein, particularly expressed as a recombinant protein so as to be secreted from the cell and accumulated in a culture solution. The present invention relates to a fusion protein of such a BDNF and SA that can be efficiently produced as a highly active recombinant protein, and a method for producing the fusion protein.

The present invention also relates particularly to a fusion protein obtained by binding serum albumin (SA) and a nerve growth factor (NGF), for example, a fusion protein obtained by binding the C terminal of SA and the N terminal of an NGF, or the C terminal of an NGF and the N terminal of SA, directly or via a linker. An NGF may exhibit low expression level and/or low activity when a gene encoding the NGF is introduced in a host cell such as a mammalian cell and expressed as a recombinant protein, particularly expressed as a recombinant protein so as to be secreted from the cell and accumulated in a culture solution. The present invention relates to a fusion protein of such NGF and SA that can be efficiently produced as a highly active recombinant protein, and a method for producing the fusion protein.

The present invention also relates particularly to a fusion protein obtained by binding serum albumin (SA) and neurotrophin 3 (NT-3), for example, a fusion protein obtained by binding the C terminal of SA and the N terminal of NT-3, or the C terminal of NT-3 and the N terminal of SA, directly or via a linker. NT-3 may exhibit low expression level and/or low activity when a gene encoding NT-3 is introduced in a host cell such as a mammalian cell and expressed as a recombinant protein, particularly expressed as a recombinant protein so as to be secreted from the cell and accumulated in a culture solution. The present invention relates to a fusion protein of such NT-3 and SA that can be efficiently produced as a highly active recombinant protein, and a method for producing the fusion protein.

The present invention also relates particularly to a fusion protein obtained by binding serum albumin (SA) and neurotrophin 4 (NT-4), for example, a fusion protein obtained by binding the C terminal of SA and the N terminal of NT-4, or the C terminal of NT-4 and the N terminal of SA, directly or via a linker. NT-4 may exhibit low expression level and/or low activity when a gene encoding NT-4 is introduced in a host cell such as a mammalian cell and expressed as a recombinant protein, particularly expressed as a recombinant protein so as to be secreted from the cell and accumulated in a culture solution. The present invention relates to a fusion protein of such NT-4 and SA that can be efficiently produced as a highly active recombinant protein, and a method for producing the fusion protein.

### Background Art

Krabbe disease, a type of lysosomal disease, is also known as galactosylceramide lipidosis or globoid-cell leukodystrophy, is a genetic disease caused by a reduction in the activity of, or a defect in, galactosylceramidase (galactocerebrosidase, GALC) required for the decomposition of sphingolipid in a lysosome due to genetic abnormality. The galactosylceramidase (GALC) uses, e.g., galactosylsphingosine, or galactocerebroside, as a substrate and catalyzes a hydrolytic reaction of a galactose-ester bond in a molecule of the substrate. In Krabbe disease patients, since GALC is defective, a substrate such as galactosylsphingosine is accumulated in the bodies. Galactosylsphingosine is known to be highly cytotoxic, and when galactosylsphingosine is accumulated, demyelination occurs, destroying the myelin sheath or myelin of the central nervous/peripheral nervous. Krabbe disease is a progressive disease. In severe cases, intellectual disability, paralysis, blindness, hearing loss, and pseudobulbar palsy are observed. Based on the onset period, Krabbe disease is classified into the following types: infantile-onset type, which develops about 3 to 6 months after birth, shows symptoms such as irritability and regression, and results in death mostly in 2 to 3 years; late infantile-onset type, which develops about 6 months to 3 years after birth and shows symptoms such as irritability, psychomotor developmental delay, and regression; juvenile type, which develops about 3 to 10 years old, shows symptoms such as visual impairment, gait disorder, and ataxia, and gradually progresses; and adult type, which develops about 10 years old and later and shows, e.g., psychiatric symptoms.

Gaucher's disease, a type of lysosomal disease, is a genetic disease caused by a reduction in the activity of, or a defect in, glucocerebrosidase (β-glucosidase, GBA) required for the decomposition of a living-body glycolipid, i.e., glucocerebroside, in a lysosome due to genetic abnormality. Glucocerebrosidase (GBA) uses glucocerebroside as a substrate and catalyzes a hydrolytic reaction of a dehydration-condensation site of a sugar and a lipid of a molecule of the substrate. In Gaucher's disease patients, since GBA is defective, a substrate such as glucocerebroside is accumulated in the bodies. Glucocerebroside accumulates in macrophages particularly in e.g., liver, spleen, and bone, causing anemia and thrombocytopenia associated with a reduction in splenic function or, e.g., hepatosplenomegaly, bone pain, broken bone, and central nervous system damage. It is considered that a central nervous system damage is caused by accumulation of a lysophospholipid of glucocerebroside, i.e., glucosylsphingosine, in the brain. Based on the presence/absence of a neurological symptom and the severity thereof, Gaucher's disease is classified into the following types: type I (non-neuropathic), the mildest severity, developed from infants to adults, not associated with a neurological symptom and associated with hypertrophy of the liver/spleen, anemia, thrombocytopenia, and broken bone, whose symptoms gradually proceed; type II (acute-neuropathic), the severest one developed during infancy, having symptoms of type I, in addition neurological symptoms such as psychomotor developmental delay, convulsions and nuchal retroflexion, whose symptoms rapidly progress and result in death due to oxygen deficiency until 2 years old; and type III (subacute-neuropathic), gradually developing during infancy and childhood and more slowly progressing compared to type II.

Lysosomal diseases other than Krabbe disease and Gaucher's disease are also caused by a genetic defect of a lysosome enzyme. Of the lysosomal diseases, e.g., Fabry's disease and Hunter syndrome are treated by an enzyme replacement therapy in which a genetically deficient enzyme is produced as a recombinant enzyme by gene recombination technology and administered to patients.

A gene encoding a human GALC (hGALC) was isolated in 1993 (Non Patent Literature 1). However, there are no medicaments containing a recombinant human GALC (rhGALC) produced using the gene as an active ingredient and used as an enzyme replacement therapy for Krabbe disease.

A gene encoding human GBA (hGBA) was isolated in 1986 (Non Patent Literature 2). However, there are no medicaments containing a recombinant human GBA (rhGBA) produced using the gene as an active ingredient and used as an enzyme replacement therapy for Gaucher's disease.

IL-10, a type of cytokine, is an anti-inflammatory cytokine produced in Th2 cell and capable of inhibiting production of a cytokine by Th1 cell has a function to inhibit immune response. Owing to the anti-inflammatory effect, IL-10 is expected to produce an effect on many inflammatory diseases, more specifically, neuropathic pain, multiple sclerosis, spinal cord injury, ALS, neuroinflammation, arthritis, symptoms associated with other diseases of the joint and autoimmune diseases. Other than an immunosuppressive effect, it has been reported that IL-10 may have an anti-cancer effect (Non Patent Literature 3). A gene encoding human IL-10 was isolated in 1991 (Non Patent Literature 4). However, there are no medicaments containing recombinant human IL-10 (rhIL-10) produced using the gene as an active ingredient and used as a therapeutic agent for inflammatory diseases or cancer.

BDNF, a type of neurotrophic factor, is a liquid protein of the nervous system binding to a specific receptor, TrkB, present on the surface of a target cell and having a function to regulate the growth of nerve cells, such as survival/growth of nerve cells and synaptic hyperfunction. Owing to neurodevelopment action, it has been expected that BDNF is developed as therapeutic agents for various diseases including neurodegenerative diseases such as Alzheimer's disease, Parkinson's disease, and Huntington's disease; spinal degenerative diseases such as amyotrophic lateral sclerosis; developmental impairments such as diabetic neuropathy, ischemic cerebral disease and Rett syndrome; schizophrenia, depression and Rett syndrome. A gene encoding human BDNF was isolated in 1993 (Non Patent Literatures 5 and 6). However, there are no medicaments containing a recombinant human BDNF (rhBDNF) produced using the gene as an active ingredient and used as a therapeutic agent for, e.g., a neurodegenerative disease.

The NGF, a type of neurotrophic factor, is a protein promoting survival and growth of sympathetic nerve cells and spinal sensory neurons in the peripheral nervous system and having a function to promote survival and differentiation of cholinergic nerve cells in the central nervous system, particularly in the basal forebrain. Particularly due to the action to prevent functional decline of dendrites, it is expected that the NGF is developed as therapeutic agents for neurodegenerative diseases such as Alzheimer's disease, Parkinson's disease, and Huntington's disease, and also expected to produce an effect on prevention of aging/degeneration of brain functions, improvement of brain functions, prevention and treatment for dementia, construction of neural network to improve memory learning ability, and enhancement of the function of neurotransmission substances. A gene encoding at least β subunit of the three subunits, α, β, and γ constituting a human NGF was isolated in 1990 (Non Patent Literature 7). However, there are no medicaments containing a recombinant human NGF (rhNGF) produced using the gene encoding human NGF as an active ingredient and used as a therapeutic agent for, e.g., a neurodegenerative disease.

NT-3, a type of neurotrophic factor, is known to perform signal transduction through a Trk receptor, particularly, TrkC, and be involved in the promotion of survival and growth of nerve cells and glial cells and neurogenesis, and an action of NT-3 to promote neurotransmission and repairment of nerve, particularly an action to promote differentiation and regeneration of photoreceptors has attracted attention. Accordingly, it is expected that NT-3 is developed as a therapeutic agent for a neurodegenerative disease. A gene encoding human NT-3 was isolated in 1991 (Non Patent Literature 8). However, there are no medicaments containing recombinant human NT-3 (rhNT-3) produced using the gene encoding human NT-3 as an active ingredient and used as a therapeutic agent for, e.g., a neurodegenerative disease.

NT-4, a type of neurotrophic factor, performs signal transduction through a Trk receptor, particularly TrkB, and promotes the growth and survival of neurons of the peripheral nervous system and central nervous system, similarly to NT-3. Accordingly, it is expected that NT-4 is developed as a therapeutic agent for a neurodegenerative disease. A gene encoding human NT-4 was isolated in 1992 (Non Patent Literature 9). However, there are no medicaments containing recombinant human NT-4 (rhNT-4) produced using the gene encoding human NT-4 as an active ingredient and used as a therapeutic agent for, e.g., a neurodegenerative disease.

A method for producing a fusion protein of growth hormone, which dissolves and loses its activity immediately after administration *in vivo,* with serum albumin is known (Non Patent Literature 10). The growth hormone fused with serum albumin is increased in stability *in vivo.* Accordingly, growth hormone should be subcutaneously administered every day as usual, but if it is formed into a fusion protein with serum albumin, the frequency of administration thereof can be reduced.

### Citation List

### Non Patent Literature

Non Patent Literature 1: Chen YQ. Hum Mol Genet. 2 (11). 1841-5 (1993)
Non Patent Literature 2: Tsuji S. J Biol Chem. 261 (1). 50-3 (1986)
Non Patent Literature 3: Mumm JB. Cancer Cell. 20 (6). 781-96 (2011)
Non Patent Literature 4: Vieira P. Proc Natl Acad Sci USA. 88 (4). 1172-6 (1991)
Non Patent Literature 5: Metsis M. Proc Natl Acad Sci USA. 90 (19). 8802-8806 (1993)
Non Patent Literature 6: Timmusk T. Neuron.10 (3). 475-89 (1993)
Non Patent Literature 7: Borsani G. Nucleic Acids Res.18 (13). 4020 (1990)
Non Patent Literature 8: Maisonpierre PC. Genomics. 10 (3). 558-68 (1991)
Non Patent Literature 9: Ip NY. Proc Natl Acad Sci USA. 89 (7). 3060-4 (1992)
Non Patent Literature 10: Poznansky MJ. FEBS. 239. 18-22 (1988)

### Summary of Invention

### Technical Problem

An object of the present invention is to provide a physiologically active substance in the form of a fusion protein with serum albumin (SA), which otherwise exhibits low expression level and/or low activity when usually expressed as a recombinant protein using a host cell such as a CHO cell. Such a fusion protein can be efficiently produced as a highly active recombinant protein. Also, a method for producing the fusion protein is provided.

Another object of the present invention is to provide a lysosome enzyme in the form of a fusion protein with serum albumin, which otherwise exhibits low expression level and/or low activity when usually expressed as a recombinant protein using a host cell such as a CHO cell, particularly expressed as a recombinant protein so as to be secreted from the cell and accumulated in a culture solution. Such a fusion protein can be efficiently produced as a highly active recombinant protein. Also, a method for producing the fusion protein is provided. The lysosome enzyme herein is, for example, hGALC or hGBA.

Note that, when a recombinant protein is expressed so as to be secreted from a cell and accumulated in a culture solution, a DNA sequence encoding a leader peptide is disposed in frame at the 5' side of a gene encoding the recombinant protein. By this manipulation, the recombinant protein expressed is secreted from a cell. Such a leader peptide is preferably, at the N terminal of a recombinant protein, a leader peptide of SA when SA is positioned; a leader peptide of a lysosome enzyme when the lysosome enzyme is positioned; a leader peptide of a cytokine when the cytokine is positioned; and a leader peptide of a neurotrophic factor when the neurotrophic factor is positioned. Note that, in place of these, the leader peptide can be a leader peptide of a heterologous protein such as a leader peptide of a growth hormone or an artificial leader peptide.

Another object of the present invention is to provide a cytokine in the form of a fusion protein with serum albumin, which otherwise exhibits low expression level and/or low activity when usually expressed as a recombinant protein using a host cell such as a CHO cell, particularly expressed as a recombinant protein so as to be secreted from the cell and accumulated in a culture solution. Such a fusion protein can be efficiently produced as a highly active recombinant protein. Also, a method for producing the fusion protein is provided. The cytokine herein is, for example, interleukin, in particular, hIL-10.

Another object of the present invention is to provide a neurotrophic factor in the form of a fusion protein with serum albumin, which otherwise exhibits low expression level and/or low activity when usually expressed as a recombinant protein using a host cell such as a CHO cell, particularly expressed as a recombinant protein so as to be secreted from the cell and accumulated in a culture solution. Such a fusion protein can be efficiently produced as a highly active recombinant protein. Also, a method for producing the fusion protein is provided. The neurotrophic factor herein is, for example, hBDNF, hNGF, hNT-3, or hNT-4.

### Solution to Problem

In the research directed to the above objects, the present inventors have conducted intensive studies. As a result, they have found that a fusion protein in which HSA is bound to the N terminal or C terminal of a human lysosome enzyme, i.e., hGALC or hGBA, directly or via a linker, as specifically described herein, when expressed as a recombinant fusion protein by culturing a host cell to which an expression vector integrating a gene encoding the fusion protein is introduced, provides a remarkably increased expression level as the recombinant fusion protein (converted to the expression level of a moiety corresponding to a wild-type human lysosome enzyme of the recombinant fusion protein), compared to the expression level of the recombinant wild-type human lysosome enzyme when expressed by culturing a host cell to which an expression vector integrating a gene encoding the wild-type human lysosome enzyme is introduced. Based on the finding, the present invention has been accomplished. Also in the research directed to the above objects, the present inventors have conducted intensive studies. As a result, they have found that a fusion protein in which HSA is bound to the N terminal or C terminal of a human cytokine, hIL-10, directly or via a linker, as specifically described herein, when expressed as a recombinant fusion protein by culturing a host cell to which an expression vector integrating a gene encoding the fusion protein is introduced, provides a remarkably increased activity as the recombinant fusion protein, compared to the activity of the recombinant wild-type hIL-10 when expressed by culturing a host cell to which an expression vector integrating a gene encoding the wild-type hIL-10 is introduced. Based on the finding, the present invention has been accomplished. Also in the research directed to the above objects, the present inventors have conducted intensive studies. As a result, they have found that a fusion protein in which HSA is bound to the N terminal or C terminal of a human neurotrophic factor, hBDNF, hNGF, hNT-3, or hNT-4, directly or via a linker, as specifically described herein, when expressed as a recombinant fusion protein by culturing a host cell to which an expression vector integrating a gene encoding the fusion protein is introduced, provides a remarkably increased activity as the recombinant fusion protein, compared to the activity of the recombinant wild-type human neurotrophic factor when expressed by culturing a host cell to which an expression vector integrating a gene encoding the wild-type human neurotrophic factor is introduced. Based on the finding, the present invention has been accomplished. More specifically, the present invention includes the following items.
1. A fusion protein comprising a neurotrophic factor and serum albumin (SA).
2. The fusion protein according to 1, wherein the neurotrophic factor is a human neurotrophic factor.
3. The fusion protein according to 1 or 2, wherein the SA is human serum albumin (HSA).
4. The fusion protein according to any one of 1 to 3, wherein the neurotrophic factor is a human brain-derived neurotrophic factor (hBDNF) having an identity of 80% or more to wild-type human brain-derived neurotrophic factor having an amino acid sequence represented by SEQ ID NO: 60, and the SA is a human serum albumin (HSA) having an identity of 80% or more to wild-type human serum albumin having an amino acid sequence represented by SEQ ID NO: 3.
5. The fusion protein according to 4, wherein the hBDNF has an identity of 90% or more to the wild-type hBDNF having the amino acid sequence represented by SEQ ID NO: 60, and the HSA has an identity of 90% or more to the wild-type HSA having the amino acid sequence represented by SEQ ID NO: 3.
6. The fusion protein according to 4, wherein the hBDNF comprises an amino acid sequence having a substitution, deletion or/and addition of 1 to 10 amino acids in the amino acid sequence of the wild-type hBDNF represented by SEQ ID NO: 60.
7. The fusion protein according to 4, wherein the hBDNF comprises an amino acid sequence having a substitution, deletion or/and addition of 1 to 5 amino acids in the amino acid sequence of the wild-type hBDNF represented by SEQ ID NO: 60.
8. The fusion protein according to 4, wherein the hBDNF comprises an amino acid sequence having a substitution, deletion or/and addition of 1 to 3 amino acids in the amino acid sequence of the wild-type hBDNF represented by SEQ ID NO: 60.
9. The fusion protein according to 4, wherein the hBDNF comprises an amino acid sequence having a single amino acid substitution in the amino acid sequence of the wild-type hBDNF represented by SEQ ID NO: 60.
10. The fusion protein according to 9, wherein the amino acid substitution is a substitution within a family of amino acids having a side chain to be possibly hydroxylated.
11. The fusion protein according to any one of 4 to 10, wherein the HSA comprises an amino acid sequence having a substitution, deletion or/and addition of 1 to 10 amino acids in the amino acid sequence of the wild-type HSA represented by SEQ ID NO: 3.
12. The fusion protein according to any one of 4 to 10, wherein the HSA comprises an amino acid sequence having a substitution, deletion or/and addition of 1 to 5 amino acids in the amino acid sequence of the wild-type HSA represented by SEQ ID NO: 3.
13. The fusion protein according to any one of 4 to 10, wherein the HSA comprises an amino acid sequence having a substitution, deletion or/and addition of 1 to 3 amino acids in the amino acid sequence of the wild-type HSA represented by SEQ ID NO: 3.
14. The fusion protein according to 4, wherein the hBDNF comprises the amino acid sequence of the wild-type hBDNF represented by SEQ ID NO: 60, and the HSA comprises the amino acid sequence of the wild-type human serum albumin represented by SEQ ID NO: 3.
15. The fusion protein according to 4, wherein the hBDNF comprises the amino acid sequence of the wild-type hBDNF represented by SEQ ID NO: 60, and the HSA comprises an amino acid sequence of a wild-type human serum albumin represented by SEQ ID NO: 12.
16. The fusion protein according to 4, wherein the hBDNF comprises the amino acid sequence of the wild-type hBDNF represented by SEQ ID NO: 60, and the HSA comprises an amino acid sequence of a wild-type human serum albumin represented by SEQ ID NO: 13.
17. The fusion protein according to any one of 4 to 16, wherein the HSA is bound to a C terminal of the hBDNF directly or via a linker.
18. The fusion protein according to any one of 4 to 16, wherein the hBDNF is bound to a C terminal of the HSA directly or via a linker.
19. The fusion protein according to 17 or 18,
   wherein the linker is a peptide chain consisting of 1 to 150 amino acids.
20. The fusion protein according to 19, wherein the linker consists of an amino acid sequence selected from the group consisting of the following (a) to (g):
   (a) Gly;
   (b) Ser;
   (c) Gly Ser;
   (d) Gly Gly Ser;
   (e) an amino acid sequence represented by SEQ ID NO: 9;
   (f) an amino acid sequence represented by SEQ ID NO: 10; and
   (g) an amino acid sequence represented by SEQ ID NO: 11.
21. The fusion protein according to 19, wherein the linker consists of 2 to 10 repeats of an amino acid sequence selected from the group consisting of the following (a) to (g):
   (a) Gly;
   (b) Ser;
   (c) Gly Ser;
   (d) Gly Gly Ser;
   (e) the amino acid sequence represented by SEQ ID NO: 9;
   (f) the amino acid sequence represented by SEQ ID NO: 10; and
   (g) the amino acid sequence represented by SEQ ID NO: 11.
22. The fusion protein according to 19, wherein the linker consists of 2 to 6 repeats of an amino acid sequence selected from the group consisting of the following (a) to (g):
   (a) Gly;
   (b) Ser;
   (c) Gly Ser;
   (d) Gly Gly Ser;
   (e) the amino acid sequence represented by SEQ ID NO: 9;
   (f) the amino acid sequence represented by SEQ ID NO: 10; and
   (g) the amino acid sequence represented by SEQ ID NO: 11.
23. The fusion protein according to 19, wherein the linker consists of 3 to 5 repeats of an amino acid sequence selected from the group consisting of the following (a) to (g):
   (a) Gly;
   (b) Ser;
   (c) Gly Ser;
   (d) Gly Gly Ser;
   (e) the amino acid sequence represented by SEQ ID NO: 9;
   (f) the amino acid sequence represented by SEQ ID NO: 10; and
   (g) the amino acid sequence represented by SEQ ID NO: 11.
24. The fusion protein according to 19, wherein the linker consists of the amino acid sequence represented by Gly Ser.
25. The fusion protein according to 18, wherein the fusion protein comprises an amino acid sequence having an identity of 80% or more to an amino acid sequence represented by SEQ ID NO: 85.
26. The fusion protein according to 18, wherein the fusion protein comprises an amino acid sequence having an identity of 90% or more to an amino acid sequence represented by SEQ ID NO: 85.
27. The fusion protein according to 26, wherein the fusion protein comprises an amino acid sequence having a substitution, deletion or/and addition of 1 to 10 amino acids in the amino acid sequence represented by SEQ ID NO: 85.
28. The fusion protein according to 26, wherein the fusion protein comprises an amino acid sequence having a substitution, deletion or/and addition of 1 to 5 amino acids in the amino acid sequence represented by SEQ ID NO: 85.
29. The fusion protein according to 26, wherein the fusion protein comprises an amino acid sequence having a substitution, deletion or/and addition of 1 to 3 amino acids in the amino acid sequence represented by SEQ ID NO: 85.
30. The fusion protein according to 18, wherein the fusion protein comprises an amino acid sequence represented by SEQ ID NO: 85.
31. The fusion protein according to 17, wherein the fusion protein comprises an amino acid sequence having an identity of 80% or more to an amino acid sequence represented by SEQ ID NO: 76.
32. The fusion protein according to 17, wherein the fusion protein comprises an amino acid sequence having an identity of 90% or more to an amino acid sequence represented by SEQ ID NO: 76.
33. The fusion protein according to 32, wherein the fusion protein comprises an amino acid sequence having a substitution, deletion or/and addition of 1 to 10 amino acids in the amino acid sequence represented by SEQ ID NO: 76.
34. The fusion protein according to 32, wherein the fusion protein comprises an amino acid sequence having a substitution, deletion or/and addition of 1 to 5 amino acids in the amino acid sequence represented by SEQ ID NO: 76.
35. The fusion protein according to 32, wherein the fusion protein comprises an amino acid sequence having a substitution, deletion or/and addition of 1 to 3 amino acids in the amino acid sequence represented by SEQ ID NO: 76.
36. The fusion protein according to 17, wherein the fusion protein comprises an amino acid sequence represented by SEQ ID NO: 76.
37. The fusion protein according to any one of 4 to 36, wherein the fusion protein has a specific activity of 10% or more compared to a specific activity of a normal wild-type hBDNF.
38. The fusion protein according to any one of 1 to 3, wherein the neurotrophic factor is a human nerve growth factor (hNGF) having an identity of 80% or more to wild-type human nerve growth factor having an amino acid sequence represented by SEQ ID NO: 62, and the SA is a human serum albumin (HSA) having an identity of 80% or more to wild-type human serum albumin having an amino acid sequence represented by SEQ ID NO: 3.
39. The fusion protein according to 38, wherein the hNGF has an identity of 90% or more to the wild-type hNGF having the amino acid sequence represented by SEQ ID NO: 62, and the HSA has an identity of 90% or more to the wild-type HSA having the amino acid sequence represented by SEQ ID NO: 3.
40. The fusion protein according to 38, wherein the hNGF comprises an amino acid sequence having a substitution, deletion or/and addition of 1 to 10 amino acids in the amino acid sequence of the wild-type hNGF represented by SEQ ID NO: 62.
41. The fusion protein according to 38, wherein the hNGF comprises an amino acid sequence having a substitution, deletion or/and addition of 1 to 5 amino acids in the amino acid sequence of the wild-type hNGF represented by SEQ ID NO: 62.
42. The fusion protein according to 38, wherein the hNGF comprises an amino acid sequence having a substitution, deletion or/and addition of 1 to 3 amino acids in the amino acid sequence of the wild-type hNGF represented by SEQ ID NO: 62.
43. The fusion protein according to 38, wherein the hNGF comprises an amino acid sequence having a single amino acid substitution in the amino acid sequence of the wild-type hNGF represented by SEQ ID NO: 62.
44. The fusion protein according to 43, wherein the amino acid substitution is a substitution within a family of amino acids having a side chain to be possibly hydroxylated.
45. The fusion protein according to any one of 38 to 44, wherein the HSA comprises an amino acid sequence having a substitution, deletion or/and addition of 1 to 10 amino acids in the amino acid sequence of the wild-type HSA represented by SEQ ID NO: 3.
46. The fusion protein according to any one of 38 to 44, wherein the HSA comprises an amino acid sequence having a substitution, deletion or/and addition of 1 to 5 amino acids in the amino acid sequence of the wild-type HSA represented by SEQ ID NO: 3.
47. The fusion protein according to any one of 38 to 44, wherein the HSA comprises an amino acid sequence having a substitution, deletion or/and addition of 1 to 3 amino acids in the amino acid sequence of the wild-type HSA represented by SEQ ID NO: 3.
48. The fusion protein according to 38, wherein the hNGF comprises the amino acid sequence of the wild-type hNGF represented by SEQ ID NO: 62, and the HSA comprises the amino acid sequence of the wild-type HSA represented by SEQ ID NO: 3.
49. The fusion protein according to 38, wherein the hNGF comprises the amino acid sequence of the wild-type hNGF represented by SEQ ID NO: 62, and the HSA comprises the amino acid sequence of the wild-type HSA represented by SEQ ID NO: 12.
50. The fusion protein according to 38, wherein the hNGF comprises the amino acid sequence of the wild-type hNGF represented by SEQ ID NO: 62, and the HSA comprises the amino acid sequence of the wild-type HSA represented by SEQ ID NO: 13.
51. The fusion protein according to any one of 38 to 50, wherein the HSA is bound to a C terminal of the hNGF directly or via a linker.
52. The fusion protein according to any one of 38 to 50, wherein the hNGF is bound to the C terminal of the HSA directly or via a linker.
53. The fusion protein according to 51 or 52,
   wherein the linker is a peptide chain consisting of 1 to 150 amino acids.
54. The fusion protein according to 53, wherein the linker consists of an amino acid sequence selected from the group consisting of the following (a) to (g):
   (a) Gly;
   (b) Ser;
   (c) Gly Ser;
   (d) Gly Gly Ser;
   (e) an amino acid sequence represented by SEQ ID NO: 9;
   (f) an amino acid sequence represented by SEQ ID NO: 10; and
   (g) an amino acid sequence represented by SEQ ID NO: 11.
55. The fusion protein according to 53, wherein the linker consists of 2 to 10 repeats of an amino acid sequence selected from the group consisting of the following (a) to (g):
   (a) Gly;
   (b) Ser;
   (c) Gly Ser;
   (d) Gly Gly Ser;
   (e) the amino acid sequence represented by SEQ ID NO: 9;
   (f) the amino acid sequence represented by SEQ ID NO: 10; and
   (g) the amino acid sequence represented by SEQ ID NO: 11.
56. The fusion protein according to 53, wherein the linker consists of 2 to 6 repeats of an amino acid sequence selected from the group consisting of the following (a) to (g):
   (a) Gly;
   (b) Ser;
   (c) Gly Ser;
   (d) Gly Gly Ser;
   (e) the amino acid sequence represented by SEQ ID NO: 9;
   (f) the amino acid sequence represented by SEQ ID NO: 10; and
   (g) the amino acid sequence represented by SEQ ID NO: 11.
57. The fusion protein according to 53, wherein the linker consists of 3 to 5 repeats of an amino acid sequence selected from the group consisting of the following (a) to (g):
   (a) Gly;
   (b) Ser;
   (c) Gly Ser;
   (d) Gly Gly Ser;
   (e) the amino acid sequence represented by SEQ ID NO: 9;
   (f) the amino acid sequence represented by SEQ ID NO: 10; and
   (g) the amino acid sequence represented by SEQ ID NO: 11.
58. The fusion protein according to 53, wherein the linker consists of the amino acid sequence represented by Gly Ser.
59. The fusion protein according to 52, wherein the fusion protein comprises an amino acid sequence having an identity of 80% or more to an amino acid sequence represented by SEQ ID NO: 88.
60. The fusion protein according to 52, wherein the fusion protein comprises an amino acid sequence having an identity of 90% or more to an amino acid sequence represented by SEQ ID NO: 88.
61. The fusion protein according to 60, wherein the fusion protein comprises an amino acid sequence having a substitution, deletion or/and addition of 1 to 10 amino acids in the amino acid sequence represented by SEQ ID NO: 88.
62. The fusion protein according to 60, wherein the fusion protein comprises an amino acid sequence having a substitution, deletion or/and addition of 1 to 5 amino acids in the amino acid sequence represented by SEQ ID NO: 88.
63. The fusion protein according to 60, wherein the fusion protein comprises an amino acid sequence having a substitution, deletion or/and addition of 1 to 3 amino acids in the amino acid sequence represented by SEQ ID NO: 88.
64. The fusion protein according to 51, wherein the fusion protein comprises an amino acid sequence represented by SEQ ID NO: 88.
65. The fusion protein according to 51, wherein the fusion protein comprises an amino acid sequence having an identity of 80% or more to an amino acid sequence represented by SEQ ID NO: 78.
66. The fusion protein according to 51, wherein the fusion protein comprises an amino acid sequence having an identity of 90% or more to an amino acid sequence represented by SEQ ID NO: 78.
67. The fusion protein according to 66, wherein the fusion protein comprises an amino acid sequence having a substitution, deletion or/and addition of 1 to 10 amino acids in the amino acid sequence represented by SEQ ID NO: 78.
68. The fusion protein according to 66, wherein the fusion protein comprises an amino acid sequence having a substitution, deletion or/and addition of 1 to 5 amino acids in the amino acid sequence represented by SEQ ID NO: 78.
69. The fusion protein according to 66, wherein the fusion protein comprises an amino acid sequence having a substitution, deletion or/and addition of 1 to 3 amino acids in the amino acid sequence represented by SEQ ID NO: 78.
70. The fusion protein according to 51, comprising the amino acid sequence represented by SEQ ID NO: 78.
71. The fusion protein according to any one of 38 to 70, having a specific activity of 10% or more compared to a specific activity of a normal wild-type hNGF.
72. The fusion protein according to any one of 1 to 3, wherein the neurotrophic factor is human neurotrophin-3 (hNT-3) having an identity of 80% or more to wild-type human neurotrophin-3 having an amino acid sequence represented by SEQ ID NO: 64, and the SA is human serum albumin (HSA) having an identity of 80% or more to wild-type human serum albumin having an amino acid sequence represented by SEQ ID NO: 3.
73. The fusion protein according to 72, wherein the hNT-3 has an identity of 90% or more to wild-type hNT-3 having the amino acid sequence represented by SEQ ID NO: 64, and the HSA has an identity of 90% or more to the wild-type HSA having the amino acid sequence represented by SEQ ID NO: 3.
74. The fusion protein according to 72, wherein the hNT-3 comprises an amino acid sequence having a substitution, deletion or/and addition of 1 to 10 amino acids in the amino acid sequence of the wild-type hNT-3 represented by SEQ ID NO: 64.
75. The fusion protein according to 72, wherein the hNT-3 comprises an amino acid sequence having a substitution, deletion or/and addition of 1 to 5 amino acids in the amino acid sequence of the wild-type hNT-3 represented by SEQ ID NO: 64.
76. The fusion protein according to 72, wherein the hNT-3 comprises an amino acid sequence having a substitution, deletion or/and addition of 1 to 3 amino acids in the amino acid sequence of the wild-type hNT-3 represented by SEQ ID NO: 64.
77. The fusion protein according to 72, wherein the hNT-3 comprises an amino acid sequence having a single amino acid substitution in the amino acid sequence of the wild-type hNT-3 represented by SEQ ID NO: 64.
78. The fusion protein according to 77, wherein the amino acid substitution is a substitution within a family of amino acids having a side chain to be possibly hydroxylated.
79. The fusion protein according to any one of 72 to 78, wherein the HSA comprises an amino acid sequence having a substitution, deletion or/and addition of 1 to 10 amino acids in the amino acid sequence of the wild-type HSA represented by SEQ ID NO: 3.
80. The fusion protein according to any one of 72 to 78, wherein the HSA comprises an amino acid sequence having a substitution, deletion or/and addition of 1 to 5 amino acids in the amino acid sequence of the wild-type HSA represented by SEQ ID NO: 3.
81. The fusion protein according to any one of 72 to 78, wherein the HSA comprises an amino acid sequence having a substitution, deletion or/and addition of 1 to 3 amino acids in the amino acid sequence of the wild-type HSA represented by SEQ ID NO: 3.
82. The fusion protein according to 72, wherein the hNT-3 comprises the amino acid sequence of the wild-type hNT-3 represented by SEQ ID NO: 64, and the HSA comprises the amino acid sequence of the wild-type human serum albumin represented by SEQ ID NO: 3.
83. The fusion protein according to 72, wherein the hNT-3 comprises the amino acid sequence of the wild-type hNT-3 represented by SEQ ID NO: 64, and the HSA comprises the amino acid sequence of the wild-type human serum albumin represented by SEQ ID NO: 12.
84. The fusion protein according to 72, wherein the hNT-3 comprises the amino acid sequence of the wild-type hNT-3 represented by SEQ ID NO: 64 and the HSA comprises the amino acid sequence of the wild-type human serum albumin represented by SEQ ID NO: 13.
85. The fusion protein according to any one of 72 to 84, wherein the HSA is bound to a C terminal of the hNT-3 directly or via a linker.
86. The fusion protein according to any one of 72 to 84, wherein the hNT-3 is bound to the C terminal of the HSA directly or via a linker.
87. The fusion protein according to 85 or 86,
   wherein the linker is a peptide chain consisting of 1 to 150 amino acids.
88. The fusion protein according to 87, wherein the linker consists of an amino acid sequence selected from the group consisting of the following (a) to (g):
   (a) Gly;
   (b) Ser;
   (c) Gly Ser;
   (d) Gly Gly Ser;
   (e) an amino acid sequence represented by SEQ ID NO: 9;
   (f) an amino acid sequence represented by SEQ ID NO: 10; and
   (g) an amino acid sequence represented by SEQ ID NO: 11.
89. The fusion protein according to 87, wherein the linker consists of 2 to 10 repeats of an amino acid sequence selected from the group consisting of the following (a) to (g):
   (a) Gly;
   (b) Ser;
   (c) Gly Ser;
   (d) Gly Gly Ser;
   (e) the amino acid sequence represented by SEQ ID NO: 9;
   (f) the amino acid sequence represented by SEQ ID NO: 10; and
   (g) the amino acid sequence represented by SEQ ID NO: 11.
90. The fusion protein according to 87, wherein the linker consists of 2 to 6 repeats of an amino acid sequence selected from the group consisting of the following (a) to (g):
   (a) Gly;
   (b) Ser;
   (c) Gly Ser;
   (d) Gly Gly Ser;
   (e) the amino acid sequence represented by SEQ ID NO: 9;
   (f) the amino acid sequence represented by SEQ ID NO: 10; and
   (g) the amino acid sequence represented by SEQ ID NO: 11.
91. The fusion protein according to 87, wherein the linker consists of 3 to 5 repeats of an amino acid sequence selected from the group consisting of the following (a) to (g):
   (a) Gly;
   (b) Ser;
   (c) Gly Ser;
   (d) Gly Gly Ser;
   (e) the amino acid sequence represented by SEQ ID NO: 9;
   (f) the amino acid sequence represented by SEQ ID NO: 10; and
   (g) the amino acid sequence represented by SEQ ID NO: 11.
92. The fusion protein according to 87, wherein the linker consists of the amino acid sequence represented by Gly Ser.
93. The fusion protein according to 86, wherein the fusion protein comprises an amino acid sequence having an identity of 80% or more to an amino acid sequence represented by SEQ ID NO: 91.
94. The fusion protein according to 86, wherein the fusion protein comprises an amino acid sequence having an identity of 90% or more to an amino acid sequence represented by SEQ ID NO: 91.
95. The fusion protein according to 94, wherein the fusion protein comprises an amino acid sequence having a substitution, deletion or/and addition of 1 to 10 amino acids in the amino acid sequence represented by SEQ ID NO: 91.
96. The fusion protein according to 94, wherein the fusion protein comprises an amino acid sequence having a substitution, deletion or/and addition of 1 to 5 amino acids in the amino acid sequence represented by SEQ ID NO: 91.
97. The fusion protein according to 94, wherein the fusion protein comprises an amino acid sequence having a substitution, deletion or/and addition of 1 to 3 amino acids in the amino acid sequence represented by SEQ ID NO: 91.
98. The fusion protein according to 86, wherein the fusion protein comprises an amino acid sequence represented by SEQ ID NO: 91.
99. The fusion protein according to 85, wherein the fusion protein comprises an amino acid sequence having an identity of 80% or more to an amino acid sequence represented by SEQ ID NO: 80.
100. The fusion protein according to 85, wherein the fusion protein comprises an amino acid sequence having an identity of 90% or more to an amino acid sequence represented by SEQ ID NO: 80.
101. The fusion protein according to 100, wherein the fusion protein comprises an amino acid sequence having a substitution, deletion or/and addition of 1 to 10 amino acids in the amino acid sequence represented by SEQ ID NO: 80.
102. The fusion protein according to 100, wherein the fusion protein comprises an amino acid sequence having a substitution, deletion or/and addition of 1 to 5 amino acids in the amino acid sequence represented by SEQ ID NO: 80.
103. The fusion protein according to 100, wherein the fusion protein comprises an amino acid sequence having a substitution, deletion or/and addition of 1 to 3 amino acids in the amino acid sequence represented by SEQ ID NO: 80.
104. The fusion protein according to 85, wherein the fusion protein comprises an amino acid sequence represented by SEQ ID NO: 80.
105. The fusion protein according to any one of 72 to 104, wherein the fusion protein has a specific activity of 10% or more compared to a specific activity of a normal wild-type hNT-3.
106. The fusion protein according to any one of 1 to 3, wherein the neurotrophic factor is human neurotrophin-4 (hNT-4) having an identity of 80% or more to wild-type human neurotrophin-4 having an amino acid sequence represented by SEQ ID NO: 66, and the SA is human serum albumin (HSA) having an identity of 80% or more to wild-type human serum albumin having an amino acid sequence represented by SEQ ID NO: 3.
107. The fusion protein according to 106, wherein the hNT-4 has an identity of 90% or more to wild-type hNT-4 having an amino acid sequence represented by SEQ ID NO: 66, and the HSA has an identity of 90% or more to the wild-type HSA having the amino acid sequence represented by SEQ ID NO: 3.
108. The fusion protein according to 106, wherein the hNT-4 comprises an amino acid sequence having a substitution, deletion or/and addition of 1 to 10 amino acids in the amino acid sequence of the wild-type hNT-4 represented by SEQ ID NO: 66.
109. The fusion protein according to 106, wherein the hNT-4 comprises an amino acid sequence having a substitution, deletion or/and addition of 1 to 5 amino acids in the amino acid sequence of the wild-type hNT-4 represented by SEQ ID NO: 66.
110. The fusion protein according to 106, wherein the hNT-4 comprises an amino acid sequence having a substitution, deletion or/and addition of 1 to 3 amino acids in the amino acid sequence of the wild-type hNT-4 represented by SEQ ID NO: 66.
111. The fusion protein according to 106, wherein the hNT-4 comprises an amino acid sequence having a single amino acid substitution in the amino acid sequence of the wild-type hNT-4 represented by SEQ ID NO: 66.
112. The fusion protein according to 111, wherein the amino acid substitution is a substitution within a family of amino acids having a side chain to be possibly hydroxylated.
113. The fusion protein according to any one of 106 to 112, wherein the HSA comprises an amino acid sequence having a substitution, deletion or/and addition of 1 to 10 amino acids in the amino acid sequence of the wild-type HSA represented by SEQ ID NO: 3.
114. The fusion protein according to any one of 106 to 112, wherein the HSA comprises an amino acid sequence having a substitution, deletion or/and addition of 1 to 5 amino acids in the amino acid sequence of the wild-type HSA represented by SEQ ID NO: 3.
115. The fusion protein according to any one of 106 to 112, wherein the HSA comprises an amino acid sequence having a substitution, deletion or/and addition of 1 to 3 amino acids in the amino acid sequence of the wild-type HSA represented by SEQ ID NO: 3.
116. The fusion protein according to 106, wherein the hNT-4 comprises the amino acid sequence of the wild-type hNT-4 represented by SEQ ID NO: 66, and the HSA comprises the amino acid sequence of the wild-type HSA represented by SEQ ID NO: 3.
117. The fusion protein according to 106, wherein the hNT-4 comprises the amino acid sequence of the wild-type hNT-4 represented by SEQ ID NO: 66, and the HSA comprises the amino acid sequence of the wild-type HSA represented by SEQ ID NO: 12.
118. The fusion protein according to 106, wherein the hNT-4 comprises the amino acid sequence of the wild-type hNT-4 represented by SEQ ID NO: 66, and the HSA comprises the amino acid sequence of the wild-type HSA represented by SEQ ID NO: 13.
119. The fusion protein according to any one of 106 to 118, wherein the HSA is bound to a C terminal of the hNT-4 directly or via a linker.
120. The fusion protein according to any one of 106 to 118, wherein the hNT-4 is bound to the C terminal of the HSA directly or via a linker.
121. The fusion protein according to 119 or 120, wherein the linker is a peptide chain consisting of 1 to 150 amino acids.
122. The fusion protein according to 121, wherein the linker consists of an amino acid sequence selected from the group consisting of the following (a) to (g):
   (a) Gly;
   (b) Ser;
   (c) Gly Ser;
   (d) Gly Gly Ser;
   (e) an amino acid sequence represented by SEQ ID NO: 9;
   (f) an amino acid sequence represented by SEQ ID NO: 10; and
   (g) an amino acid sequence represented by SEQ ID NO: 11.
123. The fusion protein according to 121, wherein the linker consists of 2 to 10 repeats of an amino acid sequence selected from the group consisting of the following (a) to (g):
   (a) Gly;
   (b) Ser;
   (c) Gly Ser;
   (d) Gly Gly Ser;
   (e) the amino acid sequence represented by SEQ ID NO: 9;
   (f) the amino acid sequence represented by SEQ ID NO: 10; and
   (g) the amino acid sequence represented by SEQ ID NO: 11.
124. The fusion protein according to 121, wherein the linker consists of 2 to 6 repeats of an amino acid sequence selected from the group consisting of the following (a) to (g):
   (a) Gly;
   (b) Ser;
   (c) Gly Ser;
   (d) Gly Gly Ser;
   (e) the amino acid sequence represented by SEQ ID NO: 9;
   (f) the amino acid sequence represented by SEQ ID NO: 10; and
   (g) the amino acid sequence represented by SEQ ID NO: 11.
125. The fusion protein according to 121, wherein the linker consists of 3 to 5 repeats of an amino acid sequence selected from the group consisting of the following (a) to (g):
   (a) Gly;
   (b) Ser;
   (c) Gly Ser;
   (d) Gly Gly Ser;
   (e) the amino acid sequence represented by SEQ ID NO: 9;
   (f) the amino acid sequence represented by SEQ ID NO: 10; and
   (g) the amino acid sequence represented by SEQ ID NO: 11.
126. The fusion protein according to 121, wherein the linker consists of the amino acid sequence represented by Gly Ser.
127. The fusion protein according to 120, wherein the fusion protein comprises an amino acid sequence having an identity of 80% or more to an amino acid sequence represented by SEQ ID NO: 94.
128. The fusion protein according to 120, wherein the fusion protein comprises an amino acid sequence having an identity of 90% or more to an amino acid sequence represented by SEQ ID NO: 94.
129. The fusion protein according to 128, wherein the fusion protein comprises an amino acid sequence having a substitution, deletion or/and addition of 1 to 10 amino acids in the amino acid sequence represented by SEQ ID NO: 94.
130. The fusion protein according to 128, wherein the fusion protein comprises an amino acid sequence having a substitution, deletion or/and addition of 1 to 5 amino acids in the amino acid sequence represented by SEQ ID NO: 94.
131. The fusion protein according to 128, wherein the fusion protein comprises an amino acid sequence having a substitution, deletion or/and addition of 1 to 3 amino acids in the amino acid sequence represented by SEQ ID NO: 94.
132. The fusion protein according to 119, wherein the fusion protein comprises an amino acid sequence represented by SEQ ID NO: 94.
133. The fusion protein according to 119, wherein the fusion protein comprises an amino acid sequence having an identity of 80% or more to an amino acid sequence represented by SEQ ID NO: 82.
134. The fusion protein according to 119, wherein the fusion protein comprises an amino acid sequence having an identity of 90% or more to an amino acid sequence represented by SEQ ID NO: 82.
135. The fusion protein according to 134, wherein the fusion protein comprises an amino acid sequence having a substitution, deletion or/and addition of 1 to 10 amino acids in the amino acid sequence represented by SEQ ID NO: 82.
136. The fusion protein according to 134, wherein the fusion protein comprises an amino acid sequence having a substitution, deletion or/and addition of 1 to 5 amino acids in the amino acid sequence represented by SEQ ID NO: 82.
137. The fusion protein according to 134, wherein the fusion protein comprises an amino acid sequence having a substitution, deletion or/and addition of 1 to 3 amino acids in the amino acid sequence represented by SEQ ID NO: 82.
138. The fusion protein according to 134, wherein the fusion protein comprises an amino acid sequence represented by SEQ ID NO: 82.
139. The fusion protein according to any one of 106 to 138, wherein the fusion protein has a specific activity of 10% or more compared to a specific activity of a normal wild-type hNT-4.
140. The fusion protein according to 1 or 2, wherein the neurotrophic factor is hCDNF or hMANF and the SA is HSA.
141. The fusion protein according to 140, wherein the HSA is bound to the C terminal of the hCDNF or the hMANF, directly or via a linker.
142. The fusion protein according to 140, wherein the hBDNF or the hMANF is bound to the C terminal of the HSA, directly or via a linker.
143. The fusion protein according to 141 or 142, wherein the linker is a peptide chain consisting of 1 to 150 amino acids.
144. The fusion protein according to 143, wherein the linker consists of the amino acid sequence selected from the group consisting of the following (a) to (g):
   (a) Gly;
   (b) Ser;
   (c) Gly Ser;
   (d) Gly Gly Ser;
   (e) the amino acid sequence represented by SEQ ID NO: 9;
   (f) the amino acid sequence represented by SEQ ID NO: 10; and
   (g) the amino acid sequence represented by SEQ ID NO: 11.
145. The fusion protein according to any one of 140 to 144, wherein the fusion protein has a specific activity of 10% or more compared to a specific activity of a normal wild-type hCDNF or hMANF.
146. The fusion protein according to 1 or 2, wherein the neurotrophic factor is hGDNF and the SA is HSA.
147. The fusion protein according to 146, wherein the HSA is bound to the C terminal of the hGDNF directly or via a linker.
148. The fusion protein according to 146, wherein the hGDNF is bound to the C terminal of the HSA directly or via a linker.
149. The fusion protein according to 147 or 148, wherein the linker is a peptide chain consisting of 1 to 150 amino acids.
150. The fusion protein according to 149, wherein the linker consists of the amino acid sequence selected from the group consisting of the following (a) to (g):
   (a) Gly;
   (b) Ser;
   (c) Gly Ser;
   (d) Gly Gly Ser;
   (e) the amino acid sequence represented by SEQ ID NO: 9;
   (f) the amino acid sequence represented by SEQ ID NO: 10; and
   (g) the amino acid sequence represented by SEQ ID NO: 11.
151. The fusion protein according to any one of 146 to 150, wherein the fusion protein has a specific activity of 10% or more compared to a specific activity of a normal wild-type hGDNF.
152. The fusion protein according to 1 or 2, wherein the neurotrophic factor is hNRTN and the SA is HSA.
153. The fusion protein according to 152, wherein the HSA is bound to the C terminal of the hNRTN directly or via a linker.
154. The fusion protein according to 152, wherein the hNRTN is bound to the C terminal of the HSA directly or via a linker.
155. The fusion protein according to 153 or 154, wherein the linker is a peptide chain consisting of 1 to 150 amino acids.
156. The fusion protein according to 155, wherein the linker consists of the amino acid sequence selected from the group consisting of the following (a) to (g):
   (a) Gly;
   (b) Ser;
   (c) Gly Ser;
   (d) Gly Gly Ser;
   (e) the amino acid sequence represented by SEQ ID NO: 9;
   (f) the amino acid sequence represented by SEQ ID NO: 10; and
   (g) the amino acid sequence represented by SEQ ID NO: 11.
157. The fusion protein according to any one of 152 to 156, wherein the fusion protein has a specific activity of 10% or more compared to a specific activity of a normal wild-type hNRTN.
158. The fusion protein according to 1 or 2, wherein the neurotrophic factor is hARTN and the SA is HSA.
159. The fusion protein according to 158, wherein the HSA is bound to the C terminal of the hARTN directly or via a linker.
160. The fusion protein according to 158, wherein the hARTN is bound to the C terminal of the HSA directly or via a linker.
161. The fusion protein according to 159 or 160,
   wherein the linker is a peptide chain consisting of 1 to 150 amino acids.
162. The fusion protein according to 161, wherein the linker consists of the amino acid sequence selected from the group consisting of the following (a) to (g):
   (a) Gly;
   (b) Ser;
   (c) Gly Ser;
   (d) Gly Gly Ser;
   (e) the amino acid sequence represented by SEQ ID NO: 9;
   (f) the amino acid sequence represented by SEQ ID NO: 10; and
   (g) the amino acid sequence represented by SEQ ID NO: 11.
163. The fusion protein according to any one of 158 to 162, wherein the fusion protein has a specific activity of 10% or more compared to a specific activity of a normal wild-type hARTN.
164. The fusion protein according to 1 or 2, wherein the neurotrophic factor is hPSPN and the SA is HSA.
165. The fusion protein according to 164, wherein the HSA is bound to the C terminal of the hPSPN, directly or via a linker.
166. The fusion protein according to 164, wherein the hPSPN is bound to the C terminal of the HSA, directly or via a linker.
167. The fusion protein according to 165 or 166,
   wherein the linker is a peptide chain consisting of 1 to 150 amino acids.
168. The fusion protein according to 167, wherein the linker consists of the amino acid sequence selected from the group consisting of the following (a) to (g):
   (a) Gly;
   (b) Ser;
   (c) Gly Ser;
   (d) Gly Gly Ser;
   (e) the amino acid sequence represented by SEQ ID NO: 9;
   (f) the amino acid sequence represented by SEQ ID NO: 10; and
   (g) the amino acid sequence represented by SEQ ID NO: 11.
169. The fusion protein according to any one of 164 to 168, wherein the fusion protein has a specific activity of 10% or more compared to a specific activity of a normal wild-type hPSPN.
170. A DNA fragment including a gene encoding the fusion protein according to any one of 1 to 169.
171. An expression vector comprising the DNA fragment according to 170.
172. A mammalian cell transformed with the expression vector according to 171.
173. A method for producing a fusion protein, comprising a step of culturing the mammalian cell according to 172 in a serum-free medium.
174. A conjugate of the fusion protein according to any one of 1 to 139 with an antibody.
175. A conjugate of a neurotrophic factor, serum albumin and an antibody.
176. The conjugate according to 175, selected from the following (1) to (6):
   (1) a conjugate in which the serum albumin is bound to the C terminal of the neurotrophic factor directly or via a linker, and the antibody is bound to the C terminal thereof directly or via a linker;
   (2) a conjugate in which the antibody is bound to the C terminal of the neurotrophic factor directly or via a linker, and the serum albumin is bound to the C terminal thereof directly or via a linker;
   (3) a conjugate in which the neurotrophic factor is bound to the C terminal of the serum albumin directly or via a linker, and the antibody is bound to the C terminal thereof directly or via a linker;
   (4) a conjugate in which the antibody is bound to the C terminal of the serum albumin directly or via a linker, and the neurotrophic factor is bound to the C terminal thereof directly or via a linker;
   (5) a conjugate in which the neurotrophic factor is bound to the C terminal of the antibody directly or via a linker, and the serum albumin is bound to the C terminal thereof directly or via a linker; and
   (6) a conjugate in which the serum albumin is bound to the C terminal of the antibody directly or via a linker, and the neurotrophic factor is bound to the C terminal thereof directly or via a linker.
177. The conjugate according to any one of 174 to 177, wherein the antibody is an antibody to a receptor expressed on a vascular endothelial cell.
178. The conjugate according to 177, wherein the receptor on the vascular endothelial cell is selected from the group consisting of an insulin receptor, a transferrin receptor, a leptin receptor, a lipoprotein receptor, and an IGF receptor.
179. The conjugate according to 177, wherein the receptor on the vascular endothelial cell is a transferrin receptor.
180. The conjugate according to any one of 174 to 179, wherein the antibody is any one of a Fab antibody, a F(ab')₂ antibody, a F(ab') antibody, a single domain antibody, a single chain antibody, VHH, or an Fc antibody.
181. The conjugate according to any one of 174 to 180, wherein the fusion protein is bound to either a C terminal side or N terminal side of a light chain of the antibody.
182. The conjugate according to any one of 174 to 180, wherein the fusion protein is bound to the C terminal side or N terminal side of a heavy chain of the antibody.
183. The conjugate according to any one of 174 to 180, wherein the fusion protein is bound to the C terminal side or N terminal side of a light chain of the antibody or the C terminal side or N terminal side of a heavy-chain via a linker sequence.
184. The conjugate according to any one of 176 to 183, wherein the linker sequence consists of 1 to 50 amino acid residues.
185. The conjugate according to 183, wherein the linker sequence includes a single glycine, a single serine or an amino acid sequence selected from the group consisting of amino acid sequence Gly-Ser, amino acid sequence Ser-Ser, amino acid sequence Gly-Gly-Ser, the amino acid sequence of SEQ ID NO: 9, the amino acid sequence of SEQ ID NO: 10, the amino acid sequence of SEQ ID NO: 11, and an amino acid sequence formed by sequentially connecting 1 to 10 of these amino acid sequences.
186. DNA comprising a gene encoding the conjugate according to any one of 174 to 185.
187. An expression vector comprising the DNA according to 186.
188. A mammalian cell transformed with the expression vector according to 187.
189. A method for producing a conjugate of a fusion protein of a protein having physiological activity and SA with an antibody, comprising a step of culturing the mammalian cell according to 188 in a serum-free medium.

### Advantageous Effects of Invention

According to the present invention, it is possible to provide, for example, a human lysosome enzyme in the form of a fusion protein with HSA, which is otherwise relatively difficult to be expressed as an active recombinant protein. Since such a fusion protein can be efficiently produced as a highly active recombinant protein, it can be stably supplied to medical institutions as a drug for an enzyme replacement therapy to patients with lysosomal diseases defective in the lysosome enzyme. Also, according to the present invention, it is possible to provide, for example, a cytokine in the form of a fusion protein with HSA, which is otherwise relatively difficult to be expressed as an active recombinant protein. Since such a fusion protein can be efficiently produced as a recombinant protein, it can be stably supplied to medical institutions as a drug. Also, according to the present invention, it is possible to provide, for example, a human neurotrophic factor in the form of a fusion protein with HSA, which is otherwise relatively difficult to be expressed as an active recombinant protein. Since such a fusion protein can be efficiently produced as a recombinant protein, it can be stably supplied to medical institutions as a drug. Note that, the effects of the present invention are not limited to these.

### Brief Description of Drawings

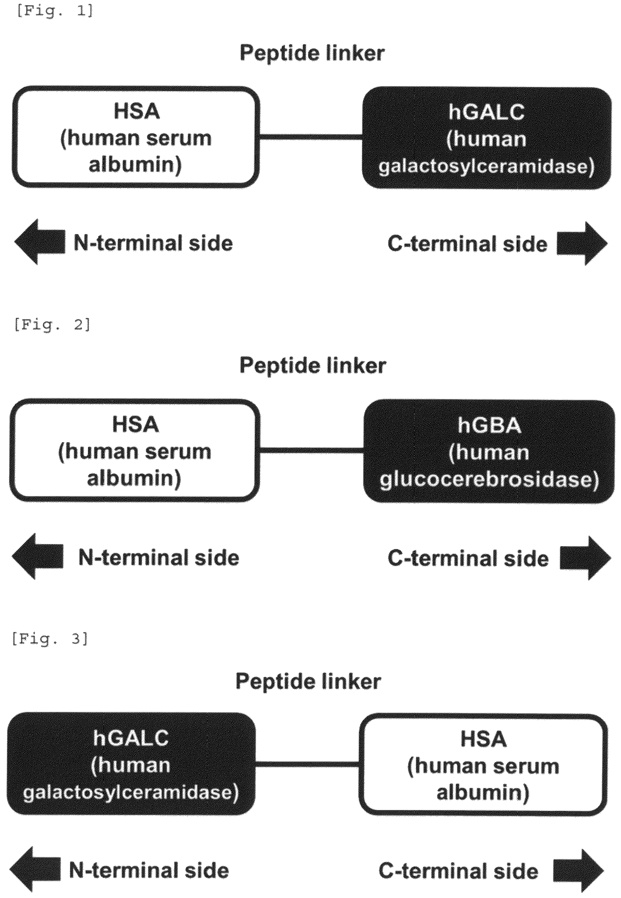

[Figure 1] The figure schematically shows a fusion protein of a single-chain polypeptide having HSA, a linker and hGALC in this order from the N terminal side. The linker is a peptide linker, and the fusion protein is a fusion protein in which the C terminal of HSA and the N terminal of the linker are bound by a peptide bond, and the C terminal of the linker and the N terminal of hGALC are bound by a peptide bond.
[Figure 2] The figure schematically shows a fusion protein of a single-chain polypeptide having HSA, a linker and hGBA in this order from the N terminal side. The linker is a peptide linker, and the fusion protein is a fusion protein in which the C terminal of HSA and the N terminal of the linker are bound by a peptide bond, and the C terminal of the linker and the N terminal of hGBA are bound by a peptide bond.
[Figure 3] The figure schematically shows a fusion protein of a single-chain polypeptide having hGALC, a linker and HSA in this order from the N terminal side. The linker is a peptide linker, and the fusion protein is a fusion protein in which the C terminal of hGALC and the N terminal of the linker are bound by a peptide bond, and the C terminal of the linker and the N terminal of HSA are bound by a peptide bond.

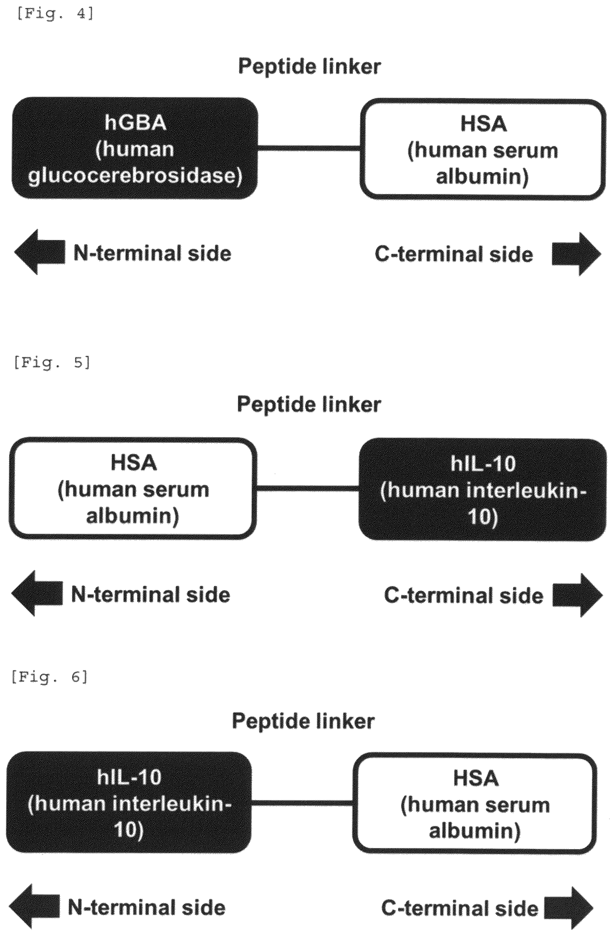

[Figure 4] The figure schematically shows a fusion protein of a single-chain polypeptide having hGBA, a linker and HSA and in this order from the N terminal side. The linker is a peptide linker, and the fusion protein is a fusion protein in which the C terminal of hGBA and the N terminal of the linker are bound by a peptide bond, and the C terminal of the linker and the N terminal of HSA are bound by a peptide bond.
[Figure 5] The figure schematically shows a fusion protein of a single-chain polypeptide having HSA, a linker and hIL-10 in this order from the N terminal side. The linker is a peptide linker, and the fusion protein is a fusion protein in which the C terminal of HSA and the N terminal of the linker are bound by a peptide bond, and the C terminal of the linker and the N terminal of hIL-10 are bound by peptide bond.
[Figure 6] The figure schematically shows a fusion protein of a single-chain polypeptide having hIL-10, a linker and HSA in this order from the N terminal side. The linker is a peptide linker, and the fusion protein is a fusion protein in which the C terminal of hIL-10 and the N terminal of the linker are bound by a peptide bond, and the C terminal of the linker and the N terminal of HSA are bound by a peptide bond.

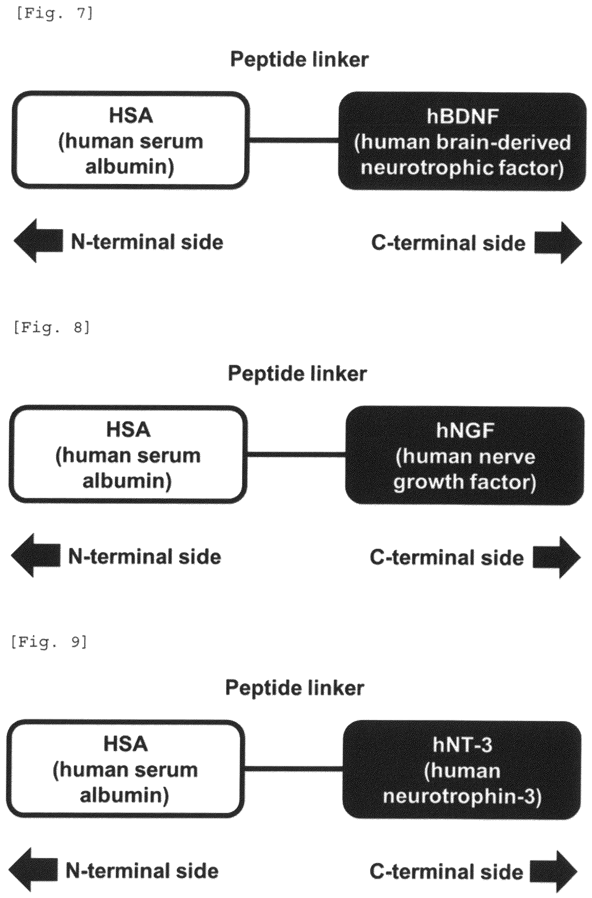

[Figure 7] The figure schematically shows a fusion protein of a single-chain polypeptide having HSA, a linker and hBDNF in this order from the N terminal side. The linker is a peptide linker, and the fusion protein is a fusion protein in which the C terminal of HSA and the N terminal of the linker are bound by a peptide bond, and the C terminal of the linker and the N terminal of hBDNF are bound by a peptide bond.
[Figure 8] The figure schematically shows a fusion protein of a single-chain polypeptide having HSA, a linker and hNGF in this order from the N terminal side. The linker is a peptide linker, and the fusion protein is a fusion protein in which the C terminal of HSA and the N terminal of the linker are bound by a peptide bond, and the C terminal of the linker and the N terminal of hNGF are bound by a peptide bond.
[Figure 9] The figure schematically shows a fusion protein of a single-chain polypeptide having HSA, a linker and hNT-3 in this order from the N terminal side. The linker is a peptide linker, and the fusion protein is a fusion protein in which the C terminal of HSA and the N terminal of the linker are bound by a peptide bond, and the C terminal of the linker and the N terminal of hNT-3 are bound by a peptide bond.

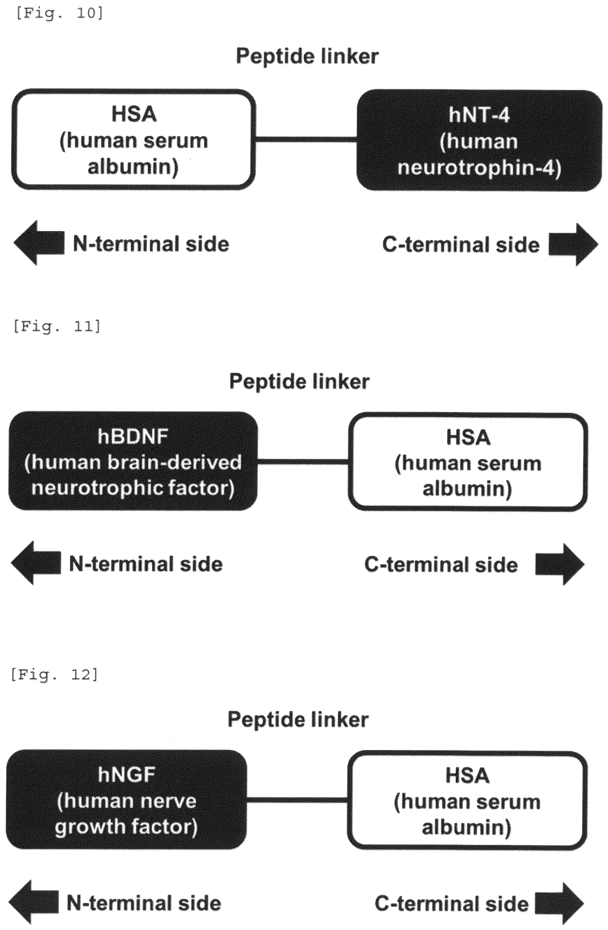

[Figure 10] The figure schematically shows a fusion protein of a single-chain polypeptide having HSA, a linker and hNT-4 in this order from the N terminal side. The linker is a peptide linker, and the fusion protein is a fusion protein in which the C terminal of HSA and the N terminal of the linker are bound by a peptide bond, and the C terminal of the linker and the N terminal of hNT-4 are bound by a peptide bond.
[Figure 11] The figure schematically shows a fusion protein of a single-chain polypeptide having hBDNF, a linker and HSA in this order from the N terminal side. The linker is a peptide linker, and the fusion protein is a fusion protein in which the C terminal of hBDNF and the N terminal of the linker are bound by a peptide bond, and the C terminal of the linker and the N terminal of HSA are bound by a peptide bond.
[Figure 12] The figure schematically shows a fusion protein of a single-chain polypeptide having hNGF, a linker and HSA in this order from the N terminal side. The linker is a peptide linker, and the fusion protein is a fusion protein in which the C terminal of hNGF and the N terminal of the linker are bound by a peptide bond, and the C terminal of the linker and the N terminal of HSA are bound by a peptide bond.

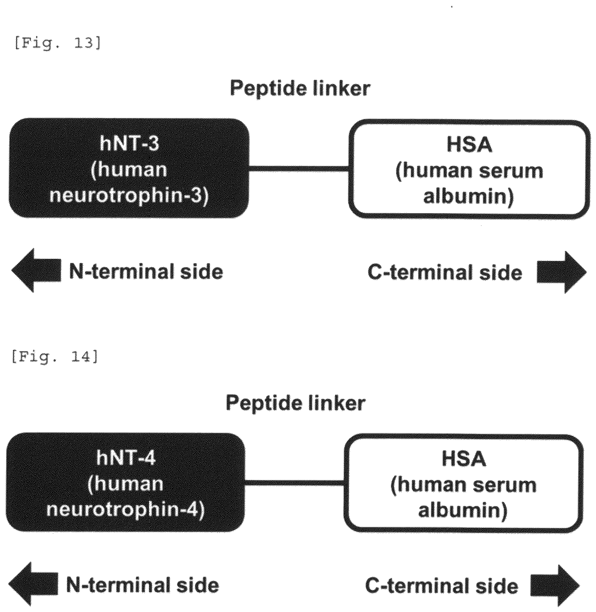

[Figure 13] The figure schematically shows a fusion protein of a single-chain polypeptide having hNT-3, a linker and HSA in this order from the N terminal side. The linker is a peptide linker, and the fusion protein is a fusion protein in which the C terminal of hNT-3 and the N terminal of the linker are bound by a peptide bond, and the C terminal of the linker and the N terminal of HSA are bound by a peptide bond.
[Figure 14] The figure schematically shows a fusion protein of a single-chain polypeptide having hNT-4, a linker and HSA in this order from the N terminal side. The linker is a peptide linker, and the fusion protein is a fusion protein in which the C terminal of hNT-4 and the N terminal of the linker are bound by a peptide bond, and the C terminal of the linker and the N terminal of HSA are bound by a peptide bond.

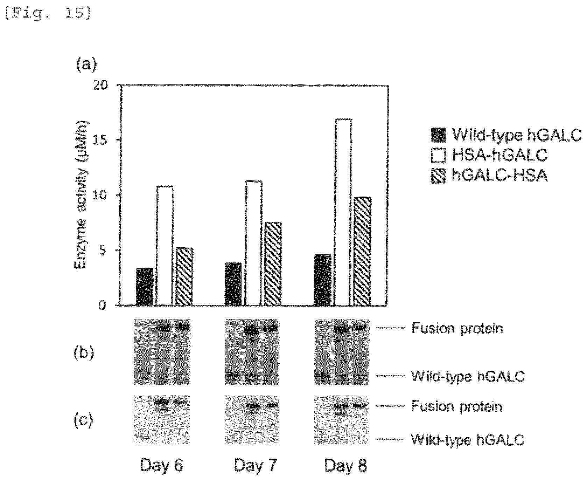

[Figure 15] The figure shows results of an experiment to confirm expression levels of wild-type hGALC, HSA-hGALC, and hGALC-HSA by transient expression. The vertical axis of the bar graph in the upper part (a) indicates an expression level of an enzyme contained in a culture supernatant in terms of enzyme activity (µM/h). The black bar indicates enzyme activity of wild-type hGALC, the white bar indicates enzyme activity of HSA-hGALC, and the diagonal striped bar indicates enzyme activity of hGALC-HSA. The middle part (b) shows results of SDS-page analysis of the culture supernatant, and the lower part (c) shows results of Western blotting analysis of the culture supernatant, each of which depicts positions of bands corresponding to wild-type hGALC and a fusion protein of HSA and hGALC. The analysis results 6, 7, and 8 days after the start of culture in transient expression are shown from left to right.

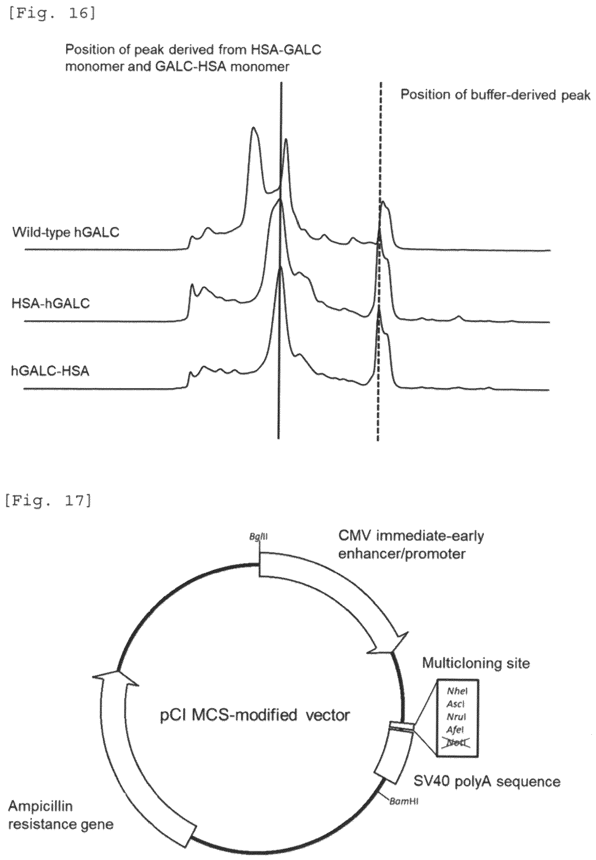

[Figure 16] The figure shows elution profiles of wild-type hGALC, HSA-hGALC, and hGALC-HSA by transient expression in SE-HPLC analysis. The dashed vertical line indicates the position of a buffer-derived peak, and the straight vertical line indicates the position of a peak corresponding to monomers of HSA-hGALC and hGALC-HSA.
[Figure 17] The figure schematically shows a structure of a pCI MCS-modified vector (plasmid).

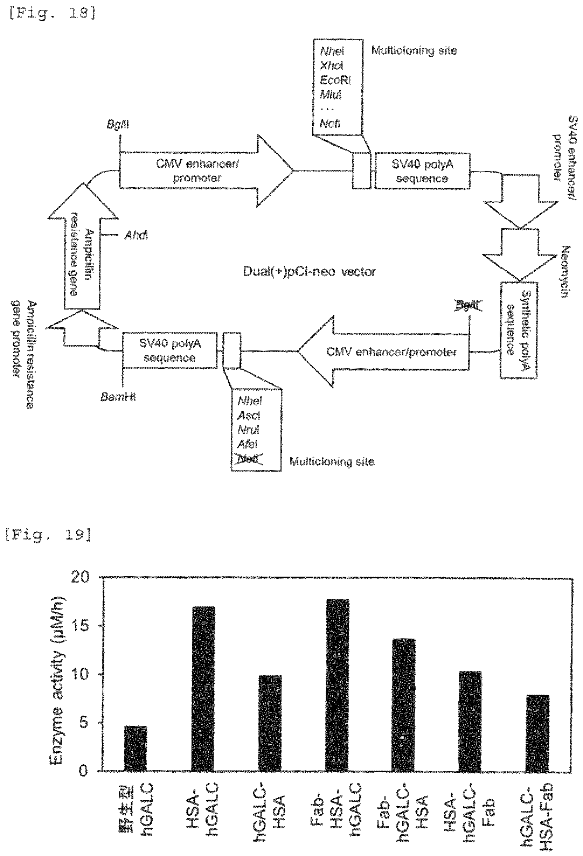

[Figure 18] The figure schematically shows a structure of a Dual(+)pCI-neo vector (plasmid).
[Figure 19] The figure shows results of an experiment to confirm expression levels of wild-type hGALC, Fab-HSA-hGALC, HSA-hGALC-Fab, Fab-hGALC-HSA, and hGALC-HSA-Fab by transient expression. The vertical axis indicates an expression level of an enzyme contained in a culture supernatant in terms of enzyme activity (µM/h).

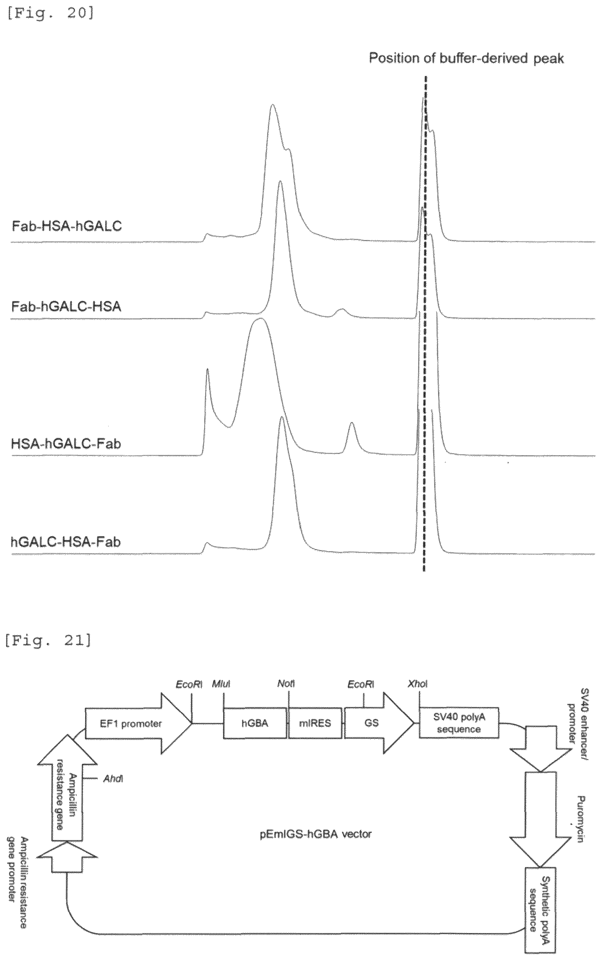

[Figure 20] The figure shows elution profiles of Fab-HSA-hGALC, HSA-hGALC-Fab, Fab-hGALC-HSA, and hGALC-HSA-Fab by transient expression in SE-HPLC analysis. The dashed vertical line indicates the position of a buffer-derived peak.
[Figure 21] The figure schematically shows a structure of a pEmIGS-hGBA vector (plasmid).

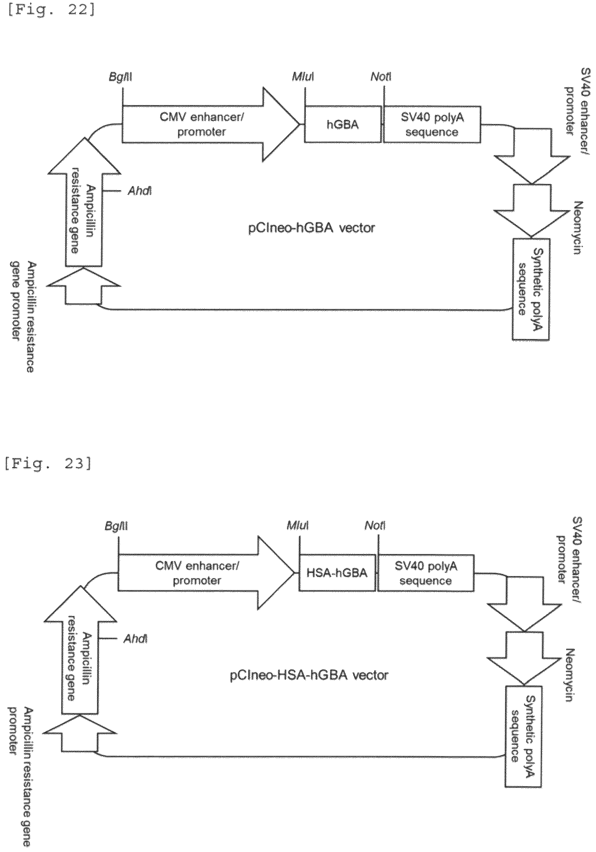

[Figure 22] The figure schematically shows a structure of a pCIneo-hGBA vector (plasmid).
[Figure 23] The figure schematically shows a structure of a pCI-neo-HSA-hGBA vector (plasmid).

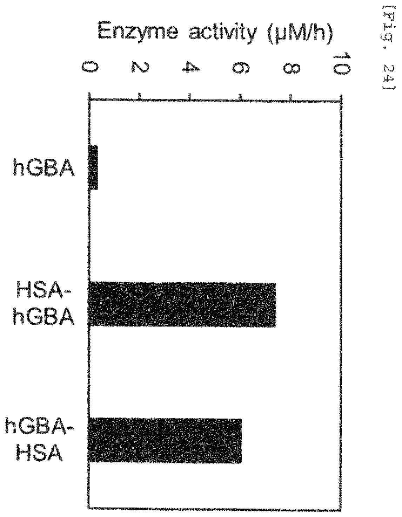

[Figure 24] The figure shows results of an experiment (activity measurement) to confirm expression levels of wild-type hGBA, HSA-hGBA, and hGBA-HSA by transient expression. The vertical axis indicates an expression level of an enzyme contained in a culture supernatant in terms of enzyme activity (µM/h).

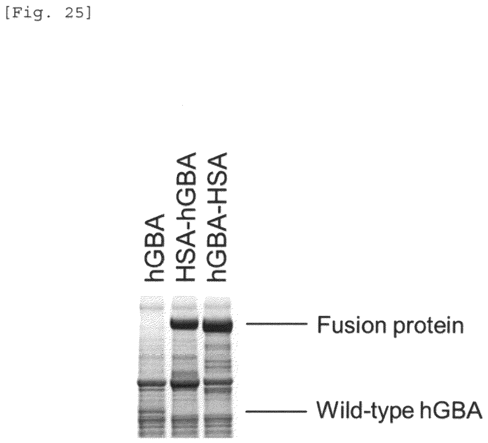

[Figure 25] The figure shows results of an experiment (SDS-PAGE) to confirm expression levels of wild-type hGBA, HSA-hGBA, and hGBA-HSA by transient expression. The positions of bands corresponding to wild-type hGBA and a fusion protein of HSA and hGBA are each shown.

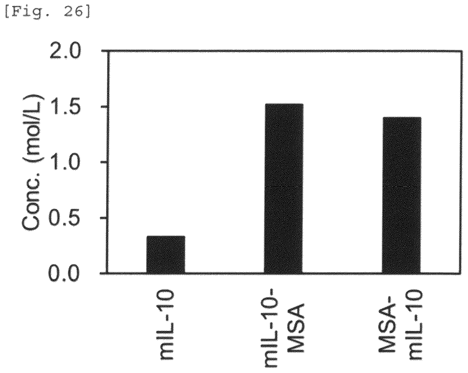

[Figure 26] The figure shows results of an experiment (ELISA) to confirm expression levels of wild-type mIL-10, mIL-10-MSA, and MSA-mIL-10 by transient expression. The vertical axis indicates an expression level of each protein contained in a culture supernatant in terms of concentration (mol/L).

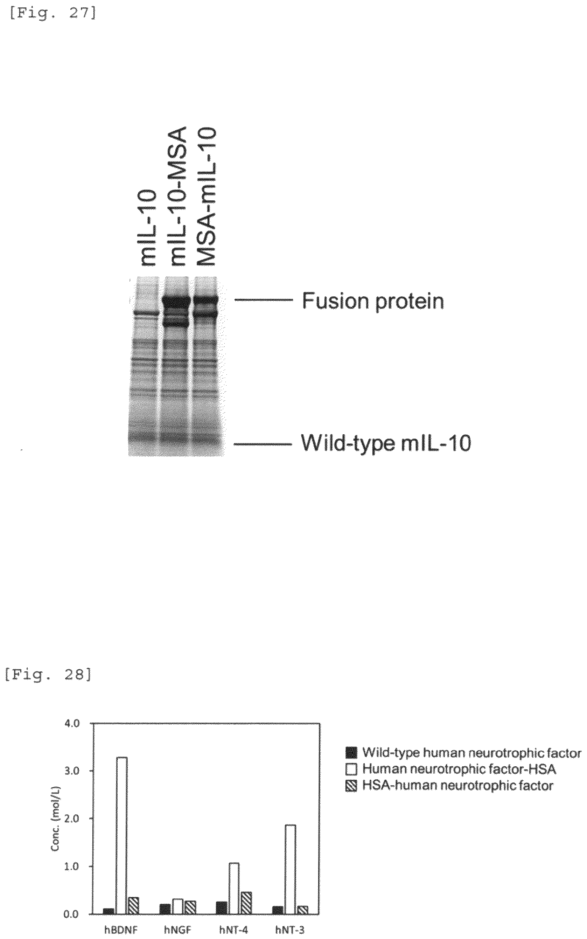

[Figure 27] The figure shows results of an experiment (SDS- PAGE) to confirm expression levels of wild-type mIL-10, mIL-10-MSA, and MSA-mIL-10 by transient expression. The positions of bands corresponding to wild-type mIL-10 and a fusion protein of MSA and mIL-10 are each shown.
[Figure 28] The figure shows results of an experiment (ELISA) to confirm expression levels of a wild-type human neurotrophic factor and a fusion protein of HSA and a human neurotrophic factor by transient expression. The vertical axis indicates an expression level of each protein contained in a culture supernatant in terms of concentration (mol/L). The black bar indicates the concentration of each wild-type human neurotrophic factor, the white bar indicates the concentration of each human neurotrophic factor-HSA fusion protein, and the diagonal striped bar indicates the concentration of each HSA-human neurotrophic factor fusion protein. There are four types of human neurotrophic factors: hBDNF, hNGF, hNT-4, and hNT-4, the results of which are shown from the left in the figure.

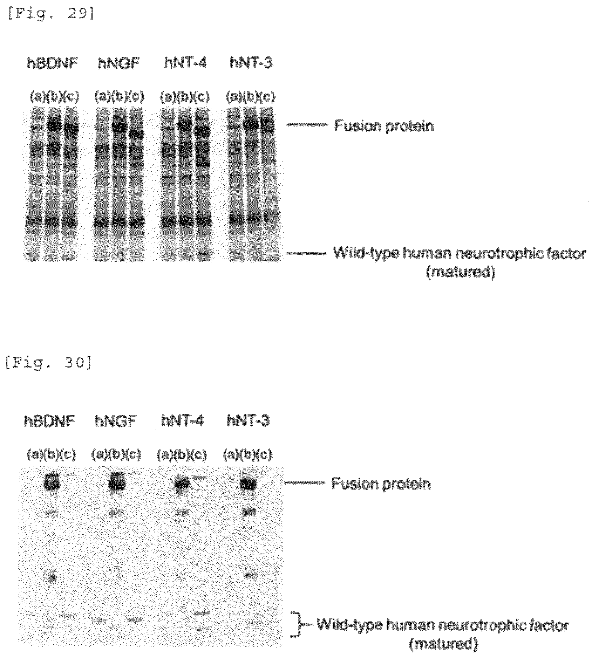

[Figure 29] The figure shows results of an experiment (SDS-PAGE) to confirm expression levels of a wild-type human neurotrophic factor and a fusion protein of HSA and a human neurotrophic factor by transient expression. A lane (a) corresponds to the wild-type human neurotrophic factor, a lane (b) corresponds to a human neurotrophic factor-HSA fusion protein, and a lane (c) corresponds to an HSA-human neurotrophic factor fusion protein. The positions of bands corresponding to the wild-type human neurotrophic factor and the fusion protein of HSA and a human neurotrophic factor are each shown. There are four types of human neurotrophic factors: hBDNF, hNGF, hNT-4, and hNT-4, the results of which are shown from the left in the figure.
[Figure 30] The figure shows results of an experiment (Western blotting) to confirm expression levels of a wild-type human neurotrophic factor and a fusion protein of HSA and a human neurotrophic factor by transient expression. A lane (a) corresponds to the wild-type human neurotrophic factor, a lane (b) corresponds to a human neurotrophic factor-HSA fusion protein, and a lane (c) corresponds to an HSA-human neurotrophic factor fusion protein. The positions of bands corresponding to the wild-type human neurotrophic factor and the fusion protein of HSA and a human neurotrophic factor are each shown. There are four types of human neurotrophic factors: hBDNF, hNGF, hNT-4, and hNT-4, the results of which are shown from the left in the figure.

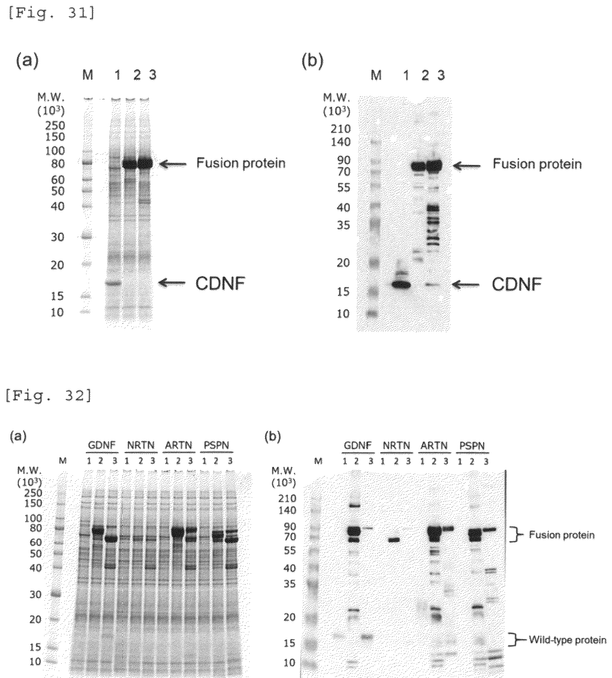

[Figure 31] The figure shows results of an experiment to confirm expression levels of wild-type human CDNF and a fusion protein of HSA and human CDNF by transient expression. Figure 31(a) shows results of gel staining in SDS-PAGE, and Figure 31(b) shows results of Western blotting. Lanes 1 to 3 correspond to wild-type human CDNF, human CDNF-HSA, and HSA-CDNF, respectively. The positions of bands corresponding to the wild-type human CDNF and the fusion protein of HSA and human CDNF are each shown.
[Figure 32] The figure shows results of an experiment to confirm expression levels of wild-type human GFL and a fusion protein of HSA and human GFL by transient expression. Figure 32(a) shows results of gel staining in SDS-PAGE, and Figure 32(b) shows results of Western blotting. Lanes 1 to 3 correspond to wild-type human GFL, human GFL-HSA, and HSA-GFL, respectively. There are four types of human GFLs: hGDNF, hNRTN, hARTN, and hPSPN, the results of which are shown from the left in the figure.

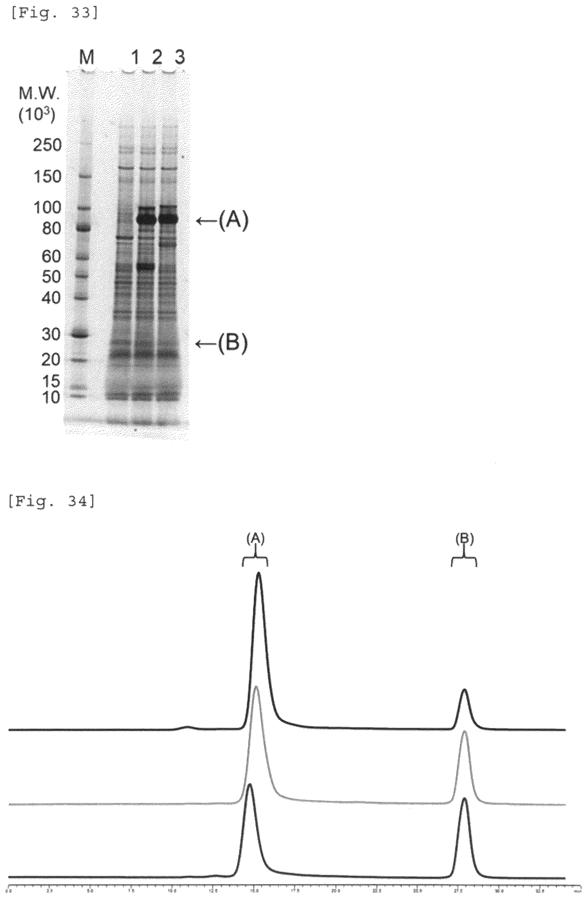

[Figure 33] The figure shows results of an experiment to confirm expression levels of fusion proteins of hBDNF and VHH, and hBDNF, VHH, and HSA by transient expression. Lanes 1 to 3 correspond to hBDNF-VHH, hBDNF-HSA-VHH, and hBDNF-VHH-HSA, respectively. (A) indicates the position corresponding to molecular weight of hBDNF-HSA-VHH and hBDNF-VHH-HSA, and (B) indicates the position corresponding to molecular weight of hBDNF-VHH.
[Figure 34] The figure shows elution profiles of hBDNF-VHH, hBDNF-HSA-VHH, and hBDNF-VHH-HSA by transient expression in SE-HPLC analysis. (A) indicates the position of a peak corresponding to monomers of hBDNF-VHH, hBDNF-HSA-VHH, and hBDNF-VHH-HSA, and (B) indicates the position of a buffer-derived peak.

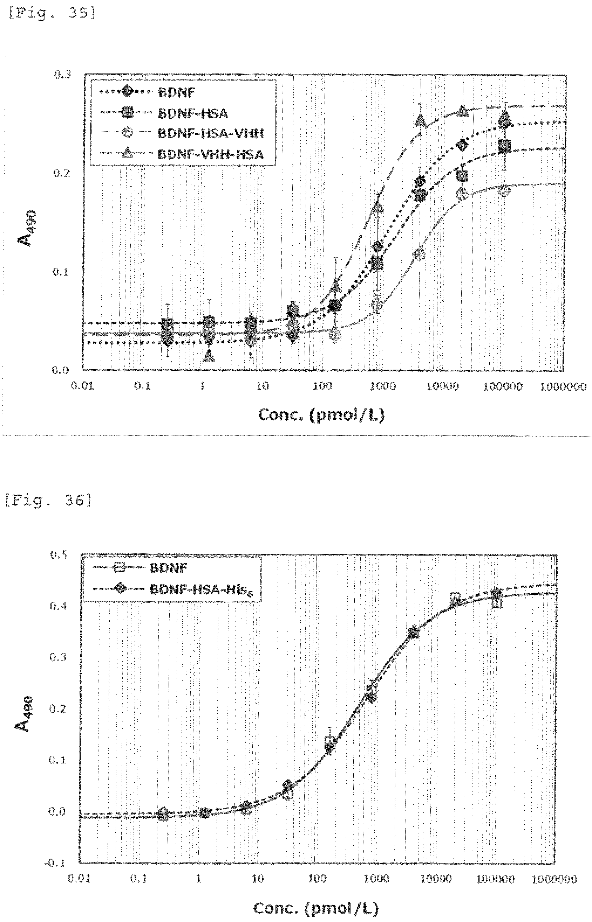

[Figure 35] The figure shows results of measuring BDNF activity of hBDNF-HSA, hBDNF-HSA-VHH, and hBDNF-VHH-HSA using BaF/TrkB·c-Mpl chimera cells. The vertical axis indicates absorbance at 490 nm, and the horizontal axis indicates the concentration of each protein in a medium.
[Figure 36] The figure shows results of measuring BDNF activity of hBDNF-HSA using BaF/TrkB·c-Mpl chimera cells. The vertical axis indicates absorbance at 490 nm, and the horizontal axis indicates the concentration of each protein in a medium.

### Description of Embodiments

In the present invention, the type of protein to be fused with serum albumin (SA) is not particularly limited, and the protein exhibits low expression level and/or low activity when expressed as a recombinant protein by integrating a gene encoding the protein into a host cell. Examples of the protein include a lysosome enzyme, a cytokine, an interleukin, and a neurotrophic factor, or fusion proteins of these and an antibody. Note that, an interleukin belongs to a group of cytokines, and is particularly a term collectively referring to cytokines secreted from a helper T cell.

The lysosome enzyme includes particularly galactosylceramidase (GALC) and glucocerebrosidase (GBA), or fusion proteins of these with an antibody or a ligand. However, the lysosome enzyme is not limited to GALC and GBA. Other lysosome enzymes that can exhibit increased expression level and/or increased activity when bound to SA and expressed as a recombinant protein, particularly expressed as a recombinant protein so as to be secreted from the cell and accumulated in a culture solution, are also included in the lysosome enzyme to be bound to SA. The same applies to fusion proteins of these other lysosome enzymes with an antibody or a ligand. Furthermore, the present invention can be applied to lysosome enzymes that can be easily produced as a recombinant protein. More specifically, examples of the lysosome enzyme may include, but are not particularly limited to, iduronate 2-sulfatase, α-L-iduronidase, β-galactosidase, GM2 activator protein, β-hexosaminidase A, β-hexosaminidase B, N-acetylglucosamine 1-phosphotransferase, α-mannosidase, β-mannosidase, saposin C, arylsulfatase A, α-L-fucosidase, aspartylglucosaminidase, α-N-acetylgalactosaminidase, acid sphingomyelinase, α-galactosidase A, β-glucuronidase, heparan N-sulfatase, α-N-acetylglucosaminidase, acetyl-CoA α-glucosaminide N-acetyltransferase, N-acetylglucosamine 6-sulfatase sulfate, acid ceramidase, amylo-1,6-glucosidase, sialidase, palmitoyl protein thioesterase-l, tripeptidylpeptidase-1, hyaluronidase-1, acid α-glucosidase, CLN1 and CLN2.

The cytokine includes particularly an interleukin, for example, IL-10, and a fusion protein of IL-10 with an antibody or a ligand. However, the cytokine is not limited to IL-10. Other cytokines that can exhibit increased expression level and/or increased activity when bound to SA and expressed as a recombinant protein, particularly expressed as a recombinant protein so as to be secreted from the cell and accumulated in a culture solution, are also included in the cytokine to be bound to SA. The same applies to fusion proteins of these other cytokines with an antibody or a ligand. Note that, the present invention can be applied to cytokines that can be easily produced as a recombinant protein. More specifically, examples of the fusion protein may include a fusion protein of a cytokine except IL-10 and SA and fusion proteins of these and an antibody. Examples of the cytokine may include, but are not particularly limited to, IL-1, IL-2, IL-3, IL-4, IL-5, IL-6, IL-7, IL-8, IL-9, IL-11, IL-12, IL-13, IL-14, IL-15, IL-16, IL-17, IL-18 and IL-19 to IL-36.

The neurotrophic factor includes particularly BDNF, NGF, NT-3, and NT-4 and fusion proteins of these and an antibody. However, the neurotrophic factor is not limited to these. Other neurotrophic factors that can exhibit increased expression level and/or increased activity when bound to SA and expressed as a recombinant protein, particularly expressed as a recombinant protein so as to be secreted from the cell and accumulated in a culture solution, are also included in the neurotrophic factor to be bound to SA. The same applies to fusion proteins of these neurotrophic factors with an antibody. Furthermore, the present invention can be applied to neurotrophic factors that can be easily produced as a recombinant protein. More specifically, examples of the neurotrophic factor may include, but are not particularly limited to, glial cell line neurotrophic factor (GDNF) and NT-5.

The organism from which the protein to be fused with SA is derived is not particularly limited and is preferably a human. Examples of the protein include a human lysosome enzyme, a human cytokine and a human growth trophic factor.

A fusion protein of serum albumin (SA) and a lysosome enzyme can be used as a therapeutic agent for an enzyme replacement therapy for a lysosomal disease. For example, glucocerebrosidase (GBA) fused with SA can be used as a therapeutic agent for Gaucher's disease, and galactosylceramidase (GALC) is used as a therapeutic agent for Krabbe disease.

The term "human lysosome enzyme" as used herein simply includes indistinguishably not only a normal wild-type human lysosome enzyme but also human lysosome enzyme mutants, which correspond to human lysosome enzymes having a substitution, deletion, and/or addition of one or more amino acid residues ("addition" of an amino acid residue herein means adding the residue to a terminal of a sequence or in the sequence) in the amino acid sequence of a wild-type human lysosome enzyme, as long as the mutants have a function as the human lysosome enzyme, such as an enzyme activity depending on the type of human lysosome enzyme. The same applies to lysosome enzymes of non-human animal species.

Note that, the phrase that a human lysosome enzyme has a function as the human lysosome enzyme herein means that the human lysosome enzyme has a specific activity of preferably 10% or more, more preferably 20% or more, further preferably 50% or more and further more preferably 80% or more when the specific activity of a normal wild-type human lysosome enzyme is regarded as 100%. The specific activity herein refers to enzyme activity per mass of the protein. Note that, the specific activity of a fusion protein of a human lysosome enzyme and a protein is obtained as an enzyme activity per mass of the moiety corresponding to the human lysosome enzyme of the fusion protein. The same applies to lysosome enzymes of non-human animal species. Note that, the specific activity of a human lysosome enzyme in a fusion protein herein is computationally obtained by multiplying enzyme activity (µM/h/mg protein) of the human lysosome enzyme per unit mass of the fusion protein by (the molecular weight of the fusion protein/the molecular weight of the moiety corresponding to the human lysosome enzyme in the fusion protein).

When an amino acid residue in the amino acid sequence of a wild-type human lysosome enzyme is substituted with another amino acid residue, the number of amino acid residues to be substituted is 1 to 10, 1 to 5, or 1 to 3, for example, one or two. When an amino acid residue in the amino acid sequence of a wild-type human lysosome enzyme is deleted, the number of amino acid residues to be deleted is 1 to 10, 1 to 5, or 1 to 3, for example, one or two. Mutants such as a human lysosome enzyme mutant consisting of an amino acid sequence obtained by deleting a single amino acid residue at the N terminal or C terminal of a wild-type human lysosome enzyme, and a human lysosome enzyme mutant consisting of an amino acid sequence obtained by deleting two amino acid residues at the N terminal or C terminal of the wild-type human lysosome enzyme are also included in human lysosome enzymes. Also, a mutation having these substitution and deletion of amino acid residues in combination can be introduced to the amino acid sequence of the wild-type human lysosome enzyme. The same applies to lysosome enzymes of non-human animal species.

In a case where an amino acid residue is added to the amino acid sequence of a wild-type human lysosome enzyme, one or more amino acid residues are added in the amino acid sequence of a human lysosome enzyme or to the N terminal or C terminal of the amino acid sequence thereof. The number of amino acid residues to be added herein is 1 to 10, 1 to 5, or 1 to 3, for example, one or two. A mutation having the addition of amino acid residues and the substitution mentioned above in combination can be introduced to the amino acid sequence of a wild-type human lysosome enzyme, and a mutation having the addition of amino acid residues and the deletion mentioned above in combination can be introduced to the amino acid sequence of a wild-type human lysosome enzyme. The same applies to lysosome enzymes of non-human animal species.

Further, a combination of three types of mutations, i.e., substitution, deletion and addition of the amino acid residues, can be introduced to the amino acid sequence of a wild-type human lysosome enzyme. For example, amino acid sequences obtained by deleting 1 to 10 amino acid residues from the amino acid sequence of a wild-type human lysosome enzyme, substituting 1 to 10 amino acid residues thereof with different amino acid residues and adding 1 to 10 amino acid residues thereto are regarded as human lysosome enzymes; amino acid sequences obtained by deleting 1 to 5 amino acid residues from the amino acid sequence of a wild-type human lysosome enzyme, substituting 1 to 5 amino acid residues thereof with different amino acid residues and adding 1 to 5 amino acid residues thereto are also regarded as human lysosome enzymes; amino acid sequences obtained by deleting 1 to 3 amino acid residues from the amino acid sequence of a wild-type human lysosome enzyme, substituting 1 to 3 amino acid residues thereof with different amino acid residues and adding 1 to 3 amino acid residues thereto are also regarded as human lysosome enzymes; amino acid sequences obtained by deleting 1 or 2 amino acid residues from the amino acid sequence of a wild-type human lysosome enzyme, substituting 1 or 2 amino acid residues thereof with different amino acid residues and adding 1 or 2 amino acid residues thereto are also regarded as human lysosome enzymes; and amino acid sequences obtained by deleting a single amino acid residue from the amino acid sequence of a wild-type human lysosome enzyme, substituting a single amino acid residue thereof with a different amino acid residue and adding a single amino acid residue thereto are also regarded as human lysosome enzymes. The same applies to lysosome enzymes of non-human animal species.

The sites and types (deletion, substitution, and addition) of individual mutations in a human lysosome enzyme mutant compared to a normal wild-type human lysosome enzyme can be easily identified by alignment of amino acid sequences of these human lysosome enzymes. The same applies to lysosome enzymes of non-human animal species.

The amino acid sequence of a human lysosome enzyme mutant exhibits an identity of preferably 80% or more, 85% or more, 90% or more, or 95% or more, for example, an identity of 98% or more or 99% or more, to the amino acid sequence of a normal wild-type human lysosome enzyme. The same applies to lysosome enzyme mutants of non-human animal species.

The identity between the amino acid sequence of a wild-type human lysosome enzyme and the amino acid sequence of a human lysosome enzyme mutant can be easily calculated using homology calculation algorithm commonly known. Examples of the algorithm include BLAST (Altschul SF. J Mol. Biol. 215. 403-10, (1990)), Pearson and Lipman's similarity search method (Proc. Natl. Acad. Sci. USA. 85. 2444 (1988)), and Smith and Waterman's local homology algorithm (Adv. Appl. Math. 2. 482-9 (1981)). The same applies to lysosome enzymes of non-human animal species, GALC, GBA, MSA and SA of non-human animal species. These algorithms can be applied to the calculation of homologies between wild-type amino acid sequences of other proteins and amino acid sequences of mutants of the other proteins throughout the specification.

The term "human galactosylceramidase" and "human galactocerebrosidase", or "hGALC" as used herein simply includes indistinguishably not only a normal wild-type hGALC consisting of 643 amino acid residues represented by SEQ ID NO: 1 but also hGALC mutants, which correspond to hGALCs having a substitution, deletion, and/or addition of one or more amino acid residues ("addition" of an amino acid residue herein means adding the residue to a terminal of a sequence or in the sequence) in the amino acid sequence represented by SEQ ID NO: 1, as long as the mutants have a function as the hGALC, such as an enzyme activity to decompose a sphingolipid such as a galactocerebroside and/or a galactosylsphingosine. The wild-type hGALC is encoded by, for example, a gene having the nucleotide sequence represented by SEQ ID NO: 2.

The term "mouse galactosylceramidase", "mouse galactocerebrosidase", or "mGALC" as used herein simply includes indistinguishably not only a normal wild-type mGALC having the amino acid sequence represented by SEQ ID NO: 14 but also mGALC mutants, which correspond to mGALCs having a substitution, deletion, and/or addition of one or more amino acid residues ("addition" of an amino acid residue herein means adding the residue to a terminal of a sequence or in the sequence) in the amino acid sequence represented by SEQ ID NO: 14, as long as the mutants have a function as the mGALC, such as an enzyme activity to decompose a sphingolipid such as a galactocerebroside and/or a galactosylsphingosine. The wild-type mGALC is encoded by, for example, a gene having the nucleotide sequence represented by SEQ ID NO: 15.

Note that, the phrase that an hGALC has a function as the hGALC herein means that the hGALC has a specific activity of preferably 10% or more, more preferably 20% or more, further preferably 50% or more and further more preferably 80% or more when the specific activity of a normal wild-type hGALC is regarded as 100%. The specific activity herein refers to enzyme activity per mass of the protein. Note that, the specific activity of a fusion protein of an hGALC and a protein is obtained as an enzyme activity per mass of the moiety corresponding to the hGALC in the fusion protein. The same applies to mGALC. Note that, the specific activity of an hGALC in the fusion protein herein is computationally obtained by multiplying enzyme activity (µM/h/mg protein) of the hGALC per unit mass of the fusion protein by (the molecular weight of the fusion protein/the molecular weight of the moiety corresponding to the hGALC in the fusion protein).

When an amino acid residue in the amino acid sequence of a wild-type hGALC is substituted with another amino acid residue, the number of amino acid residues to be substituted is 1 to 10, 1 to 5, or 1 to 3, for example, one or two. When an amino acid residue in the amino acid sequence of a wild-type hGALC is deleted, the number of amino acid residues to be deleted is 1 to 10, 1 to 5, or 1 to 3, for example, one or two. Mutants such as an hGALC mutant consisting of 642 amino acid residues obtained by deleing a single amino acid residue from the N terminal or C terminal of a wild-type hGALC and an hGALC mutant consisting of 641 amino acid residues obtained by deleing two amino acid residues from the N terminal or C terminal of a wild-type hGALC are also regarded as hGALCs. Also, a mutation having the substitution and deletion of amino acid residues in combination can be introduced to the amino acid sequence of a wild-type hGALC. The same applies to GALCs of non-human animal species.

When an amino acid residue is added to the amino acid sequence of a wild-type hGALC, one or more amino acid residues are added in the amino acid sequence of an hGALC or added to the N terminal or C terminal of the amino acid sequence thereof. The number of amino acid residues to be added herein is 1 to 10, 1 to 5, or 1 to 3, for example, one or two. A mutation having the addition of amino acid residues and the substitution mentioned above in combination can be introduced to the amino acid sequence of a wild-type hGALC, and a mutation having the addition of amino acid residues and the deletion mentioned above in combination can be introduced to the amino acid sequence of a wild-type hGALC. The same applies to the GALCs of non-human animal species.

Further, a combination of three types of mutations, i.e., substitution, deletion and addition of the amino acid residues, can be introduced to the amino acid sequence of a wild-type hGALC. For example, amino acid sequences obtained by deleting 1 to 10 amino acid residues from the amino acid sequence of a wild-type hGALC represented by SEQ ID NO: 1, substituting 1 to 10 amino acid residues thereof with different amino acid residues and adding 1 to 10 amino acid residues thereto are regarded as hGALCs; amino acid sequences obtained by deleting 1 to 5 amino acid residues from the amino acid sequence of a wild-type hGALC represented by SEQ ID NO: 1, substituting 1 to 5 amino acid residues thereof with different amino acid residues and adding 1 to 5 amino acid residues thereto are also regarded as hGALCs; amino acid sequences obtained by deleting 1 to 3 amino acid residues from the amino acid sequence of a wild-type hGALC represented by SEQ ID NO: 1, substituting 1 to 3 amino acid residues thereof with different amino acid residues and adding 1 to 3 amino acid residues thereto are also regarded as hGALCs; amino acid sequences obtained by deleting 1 or 2 amino acid residues from the amino acid sequence of a wild-type hGALC represented by SEQ ID NO: 1, substituting 1 or 2 amino acid residues thereof with different amino acid residues and adding 1 or 2 amino acid residues thereto are also regarded as hGALCs; and amino acid sequences obtained by deleting a single amino acid residue from the amino acid sequence of a wild-type hGALC represented by SEQ ID NO: 1, substituting a single amino acid residue thereof with a different amino acid residue and adding a single amino acid residue thereto are also regarded as hGALCs. The same applies to GALCs of non-human animal species.

The sites and types (deletion, substitution, and addition) of individual mutations in an hGALC mutant compared to a normal wild-type hGALC can be easily identified by alignment of the amino acid sequences of these hGALCs. The same applies to GALCs of non-human animal species.

The amino acid sequence of an hGALC mutant exhibits an identity of preferably 80% or more, 85% or more, 90% or more, or 95% or more, for example, an identity of 98% or more or 99% or more, to the amino acid sequence of a normal wild-type hGALC represented by SEQ ID NO: 1. The same applies to GALCs of non-human animal species.

The term "human glucocerebrosidase", "human β-glucosidase", or "hGBA" as used herein simply includes indistinguishably not only a normal wild-type hGBA consisting of 497 amino acid residues represented by SEQ ID NO: 37 but also hGBA mutants, which correspond to hGBAs having a substitution, deletion, and/or addition of one or more amino acid residues ("addition" of an amino acid residue herein means adding the residue to a terminal of a sequence or in the sequence) in the amino acid sequence represented by SEQ ID NO: 37, as long as the mutants have a function as the hGBA, such as an enzyme activity to decompose a glycolipid such as a galactocerebroside and/or a galactosylsphingosine. The wild-type hGBA is encoded by, for example, a gene having the nucleotide sequence represented by SEQ ID NO: 38.

The term "mouse glucocerebrosidase", "mouse β-glucosidase", or "mGBA" as used herein simply includes indistinguishably not only a normal wild-type mGBA having the amino acid sequence represented by SEQ ID NO: 43 but also mGBA mutants, which correspond to mGBAs having a substitution, deletion, and/or addition of one or more amino acid residues ("addition" of an amino acid residue herein means adding the residue to a terminal of a sequence or in the sequence) in the amino acid sequence represented by SEQ ID NO: 43, as long as the mutants have a function as the mGBA, such as an enzyme activity to decompose a glycolipid such as a galactocerebroside and/or a galactosylsphingosine. The wild-type mGBA is encoded by, for example, a gene having the nucleotide sequence represented by SEQ ID NO: 44.

Note that, the phrase that an hGBA has a function as the hGBA herein means that the hGBA has a specific activity of preferably 10% or more, more preferably 20% or more, further preferably 50% or more and further more preferably 80% or more when the specific activity of a normal wild-type hGBA is regarded as 100%. The specific activity herein refers to enzyme activity per mass of the protein. Note that, the specific activity of a fusion protein of an hGBA and a protein is obtained as an enzyme activity per mass of the moiety corresponding to the hGBA of the fusion protein. Note that, the specific activity of the hGBA in the fusion protein herein is computationally obtained by multiplying enzyme activity (µM/h/mg protein) of an hGBA per unit mass of the fusion protein by (the molecular weight of the fusion protein/the molecular weight of the moiety corresponding to the hGBA in the fusion protein). The same applies to GBAs of non-human animal species.

When an amino acid residue in the amino acid sequence of a wild-type hGBA is substituted with another amino acid residue, the number of amino acid residues to be substituted is 1 to 10, 1 to 5, or 1 to 3, for example, one or two. When an amino acid residue in the amino acid sequence of a wild-type hGBA is deleted, the number of amino acid residues to be deleted is 1 to 10, 1 to 5, or 1 to 3, for example, one or two. Mutants such as an hGBA mutant consisting of 496 amino acid residues obtained by deleing a single amino acid residue from the N terminal or C terminal of a wild-type hGBA and an hGBA mutant consisting of 495 amino acid residues obtained by deleing two amino acid residues from the N terminal or C terminal of a wild-type hGBA are also regarded as hGBAs. Also, a mutation having these substitution and deletion of amino acid residues in combination can be introduced to the amino acid sequence of a wild-type hGBA. The same applies to GBAs of non-human animal species.

When an amino acid residue is added to the amino acid sequence of a wild-type hGBA, one or more amino acid residues are added in the amino acid sequence of an hGBA or added to the N terminal or C terminal of the amino acid sequence thereof. The number of amino acid residues to be added herein is 1 to 10, 1 to 5, or 1 to 3, for example, one or two. A mutation having the addition of amino acid residues and the substitution mentioned above in combination can be introduced to the amino acid sequence of a wild-type hGBA, and a mutation having the addition of amino acid residues and the deletion mentioned above in combination can be introduced to the amino acid sequence of a wild-type hGBA. The same applies to the GBAs of non-human animal species.

Further, a combination of three types of mutations, i.e., substitution, deletion and addition of the amino acid residues, can be introduced to the amino acid sequence of a wild-type hGBA. For example, amino acid sequences obtained by deleting 1 to 10 amino acid residues from the amino acid sequence of a wild-type hGBA represented by SEQ ID NO: 37, substituting 1 to 10 amino acid residues thereof with different amino acid residues and adding 1 to 10 amino acid residues thereto are regarded as hGBAs; amino acid sequences obtained by deleting 1 to 5 amino acid residues from the amino acid sequence of a wild-type hGBA represented by SEQ ID NO: 37, substituting 1 to 5 amino acid residues thereof with different amino acid residues and adding 1 to 5 amino acid residues thereto are also regarded as hGBAs; amino acid sequences obtained by deleting 1 to 3 amino acid residues from the amino acid sequence of a wild-type hGBA represented by SEQ ID NO: 37, substituting 1 to 3 amino acid residues thereof with different amino acid residues and adding 1 to 3 amino acid residues thereto are also regarded as hGBAs; amino acid sequences obtained by deleting 1 or 2 amino acid residues from the amino acid sequence of a wild-type hGBA represented by SEQ ID NO: 37, substituting 1 or 2 amino acid residues thereof with different amino acid residues and adding 1 or 2 amino acid residues thereto are also regarded as hGBAs; and amino acid sequences obtained by deleting a single amino acid residue from the amino acid sequence of a wild-type hGBA represented by SEQ ID NO: 37, substituting a single amino acid residue thereof with a different amino acid residue and adding a single amino acid residue thereto are also regarded as hGBAs. The same applies to GBAs of non-human animal species.

The sites and types (deletion, substitution, and addition) of individual mutations in an hGBA mutant compared to a normal wild-type hGBA can be easily identified by alignment of the amino acid sequences of these hGBAs. The same applies to GBAs of non-human animal species.

The amino acid sequence of an hGBA mutant exhibits an identity of preferably 80% or more, 85% or more, 90% or more, or 95% or more, for example, an identity of 98% or more or 99% or more, to the amino acid sequence of a normal wild-type hGBA represented by SEQ ID NO: 37. The same applies to GBAs of non-human animal species.

In the present invention, the species from which serum albumin (SA) is originated is not particularly limited and human serum albumin (HSA) is preferable.

The term "human serum albumin" or "HSA" in the present invention include indistinguishably not only a wild-type human serum albumin consisting of 585 amino acids of the amino acid sequence represented by SEQ ID NO: 3, but also an HSA mutant, which corresponds to an HSA having a substitution, deletion, and/or addition of one or more amino acid residues ("addition" of an amino acid residue herein means adding the residue to a terminal of a sequence or in the sequence) in the amino acid sequence represented by SEQ ID NO: 3. The wild-type HSA is encoded by a gene having a nucleotide sequence represented by, for example, SEQ ID NO: 4.

Human serum albumin, when bound to each of wild-type human lysosome enzymes to prepare a fusion protein and expressed as a recombinant protein using a host cell such as CHO, particularly expressed as a recombinant protein so as to be secreted from the cell and accumulated in a culture solution, can provide an increased expression level as the human lysosome enzyme, with respect to at least one type of wild-type human lysosome enzyme, compared to that of the wild-type human lysosome enzyme expressed in the same manner as a recombinant protein. Particularly, human serum albumin, when bound to a wild-type hGALC or a wild-type hGBA to prepare a fusion protein and expressed as a recombinant protein using a host cell such as CHO, particularly expressed as a recombinant protein so as to be secreted from the cell and accumulated in a culture solution, can provide an increased expression level as the enzyme, compared to that of the wild-type enzyme expressed as recombinant proteins in the same manner. Note that, it is preferable that human serum albumin herein has a function as human serum albumin, such as a function to bind to an endogenous substance and an exogenous substance such as a drug in the blood and transport them, but is not limited to this.

The term "mouse serum albumin" or "MSA" in the present invention includes indistinguishably not only a wild-type mouse serum albumin having the amino acid sequence represented by SEQ ID NO: 16, but also an MSA mutant, which corresponds to an MSA having a substitution, deletion, and/or addition of one or more amino acid residues ("addition" of an amino acid residue herein means adding the residue to a terminal of a sequence or in the sequence) in the amino acid sequence represented by SEQ ID NO: 16. The wild-type MSA is encoded by a gene having a nucleotide sequence represented by, for example, SEQ ID NO: 17. Note that, it is preferable that MSA has a function as MSA, such as a function to bind to an endogenous substance and an exogenous substance such as a drug in the blood and transport them, but is not limited to this.

The term "serum albumin" or "SA" in the present invention expresses a comprehensive concept including serum albumin of mammals except humans, and mouse serum albumin (MSA) and bovine serum albumin (BSA) are included in this term. Serum albumin, when bound to each of wild-type human lysosome enzymes to prepare a fusion protein and expressed as a recombinant protein using a host cell such as CHO, particularly expressed as a recombinant protein so as to be secreted from the cell and accumulated in a culture solution, can provide an increased expression level as a human lysosome enzyme, with respect to at least one type of wild-type human lysosome enzymes, compared to that of the wild-type human lysosome enzyme expressed in the same manner as a recombinant protein. Particularly, serum albumin, when bound to a wild-type hGALC or a wild-type hGBA to prepare a fusion protein and expressed as a recombinant protein using a host cell such as CHO, particularly expressed as a recombinant protein so as to be secreted from the cell and accumulated in a culture solution, can provide an increased expression level as the enzyme, compared to that of the wild-type enzyme expressed as recombinant proteins in the same manner.

Human serum albumin (HSA) is known to have a plurality of wild-type variants. Human serum albumin Redhill is one of them. Human serum albumin Redhill differs from the amino acid sequence of the normal human serum albumin consisting of 585 amino acids mentioned above, in that the 320th amino acid residue from the N terminal is not alanine but threonine and a single arginine residue is added to the N terminal, and consists of 586 amino acids as represented by SEQ ID NO: 12. Since alanine changes to threonine as mentioned above, a sequence represented by Asn-Tyr-Thr appears in the amino acid sequence of human serum albumin Redhill and the Asn (asparagine) residue in this sequence is involved in N-linked glycosidation. Such human serum albumin Redhill is also a wild-type HSA.

Human serum albumin (HSA-A320T) consisting of 585 amino acids represented by SEQ ID NO: 13, which is obtained by substituting the 320th amino acid residue, alanine, from the N terminal of the amino acid sequence of a wild-type HSA represented by SEQ ID NO: 3, with threonine, is a preferable example of an HSA mutant. Human serum albumin obtained by substituting tyrosine at the 319th amino acid residues with an amino acid except proline while conserving asparagine at the 318th amino acid residues from the N terminal of the HSA mutant represented by SEQ ID NO: 13 is also a preferable example of an HSA mutant. Hereinafter, mutations to be introduced to a wild-type HSA amino acid sequence acceptable in an embodiment of the present invention will be more specifically described but the mutations can also apply to human serum albumin Redhill, HSA mutant represented by SEQ ID NO: 13, or to serum albumin of non-human animal species.

In a case where an amino acid residue in the amino acid sequence of a wild-type HSA or HSA-A320T is substituted with another amino acid residue, the number of amino acid residues to be substituted is 1 to 10, 1 to 5, or 1 to 3, for example, one or two. In a case where an amino acid residue in the HSA amino acid sequence of a wild-type or HSA-A320T is deleted, the number of amino acid residues to be deleted is 1 to 10, 1 to 5, or 1 to 3, for example, one or two. An HSA mutant obtained by deleting a single amino acid residue of the N terminal or C terminal of HSA of a wild-type or HSA-A320T and an HSA mutant obtained by deleting two amino acid residues of the N terminal or C terminal of HSA of a wild-type or HSA-A320T are regarded as HSA. A mutation having the substitution and deletion of amino acid residues in combination may be introduced to the HSA amino acid sequence of a wild-type or HSA-A320T. The same applies to SA of non-human animal species.

In a case where an amino acid residue is added to the amino acid sequence of a wild-type HSA or HSA-A320T, one or more amino acid residues are added in the amino acid sequence of the HSA or added to the N terminal or C terminal of the amino acid sequence thereof. The number of amino acid residues to be added herein is 1 to 10, 1 to 5, or 1 to 3, for example, one or two. An HSA mutant obtained by adding a single amino acid residue to the N terminal or C terminal of HSA of a wild-type or HSA-A320T and an HSA mutant obtained by adding two amino acid residues to the N terminal or C terminal of HSA of a wild-type or HSA-A320T are also regarded as HSA. A mutation having the addition of amino acid residues and the substitution mentioned above in combination can be introduced to the HSA amino acid sequence of a wild-type or HSA-A320T, and a mutation having the addition of amino acid residues and the deletion mentioned above in combination can be introduced to the HSA amino acid sequence of a wild-type or HSA-A320T. The same applies to SA of non-human animal species.

Further, a combination of three types of mutations, i.e., substitution, deletion and addition of the amino acid residues, can be introduced to the HSA amino acid sequence of a wild-type or HSA-A320T. For example, amino acid sequences obtained by deleting 1 to 10 amino acid residues from the amino acid sequence of a wild-type HSA represented by SEQ ID NO: 3, substituting 1 to 10 amino acid residues thereof with different amino acid residues and adding 1 to 10 amino acid residues thereto are regarded as HSAs; amino acid sequences obtained by deleting 1 to 5 amino acid residues from the amino acid sequence of a wild-type HSA represented by SEQ ID NO: 3, substituting 1 to 5 amino acid residues thereof with different amino acid residues and adding 1 to 5 amino acid residues thereto are also regarded as HSAs; amino acid sequences obtained by deleting 1 to 3 amino acid residues from the amino acid sequence of a wild-type HSA represented by SEQ ID NO: 3, substituting 1 to 3 amino acid residues thereof with different amino acid residues and adding 1 to 3 amino acid residues thereto are also regarded as HSAs; amino acid sequences obtained by deleting 1 or 2 amino acid residues from the amino acid sequence of a wild-type HSA represented by SEQ ID NO: 3, substituting 1 or 2 amino acid residues thereof with different amino acid residues and adding 1 or 2 amino acid residues thereto are also regarded as HSAs; and amino acid sequences obtained by deleting a single amino acid residue from the amino acid sequence of a wild-type HSA represented by SEQ ID NO: 3, substituting a single amino acid residue thereof with a different amino acid residue and adding a single amino acid residue thereto are also regarded as HSAs. The same applies to SA of non-human animal species.

The sites and types (deletion, substitution, and addition) of individual mutations in an HSA mutant compared to a normal wild-type HSA can be easily identified by alignment of the amino acid sequences of these HSAs. The same applies to SA of non-human animal species.

The amino acid sequence of an HSA mutant exhibits an identity of preferably 80% or more, 85% or more, 90% or more, or 95% or more, for example, an identity of 98% or more or 99% or more, to the amino acid sequence of a normal wild-type HSA represented by SEQ ID NO: 3. The same applies to SA of non-human animal species.

Note that, in an embodiment of the present invention, HSA, when bound to each of wild-type human lysosome enzymes to prepare a fusion protein and expressed as a recombinant protein using a host cell such as CHO, particularly expressed as a recombinant protein so as to be secreted from the cell and accumulated in a culture solution, can provide an expression level as a human lysosome enzyme in the culture supernatant in terms of concentration or enzyme activity, with respect to at least one of the wild-type human lysosome enzymes, that is at least 1.1 times or more, 1.2 times or more, 1.5 times or more, 2 times or more, 2.5 times or more, or 3.5 times or more, for example, 1.1 to 4 times, 1.5 to 3.6 times or 2 to 3.6 times that of the wild-type human lysosome enzyme expressed as a recombinant protein in the same manner.

Also, in an embodiment of the present invention, HSA, when bound to a wild-type hGALC to prepare a fusion protein and expressed as a recombinant protein using a host cell such as CHO, particularly expressed as a recombinant protein so as to be secreted from the cell and accumulated in a culture solution, can provide an expression level as hGALC in the culture supernatant in terms of concentration or enzyme activity, that is at least 1.1 times or more, 1.2 times or more, 1.5 times or more, 2 times or more, 2.5 times or more, or 3.5 times or more, for example, 1.1 to 4 times, 1.5 to 3.6 times, or 2 to 3.6 times that of the wild-type hGALC expressed as a recombinant protein in the same manner.

Also, in an embodiment of the present invention, HSA, when bound to a wild-type hGBA to prepare a fusion protein and expressed as a recombinant protein using a host cell such as CHO, particularly expressed as a recombinant protein so as to be secreted from the cell and accumulated in a culture solution, can provide an expression level as hGBA in the culture supernatant in terms of concentration or enzyme activity, that is at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, or 10 to 20 times that of the wild-type hGBA expressed as a recombinant protein in the same manner.

Also, in the present invention, the term "conservative amino acid substitution" means that an amino acid is substituted within a family of amino acids having a relevant side chain and chemical property. Such substitution within an amino acid family is presumed not to bring a significant change in the function of an original protein. Examples of the amino acid family include the following (1) to (12):
(1) acidic amino acids: aspartic acid and glutamic acid,
(2) basic amino acids: histidine, lysine, and arginine,
(3) aromatic amino acids: phenylalanine, tyrosine, and tryptophan,
(4) amino acids having a hydroxyl group (hydroxy amino acids): serine and threonine,
(5) hydrophobic amino acids: methionine, alanine, valine, leucine, and isoleucine,
(6) neutral hydrophilic amino acids: cysteine, serine, threonine, asparagine, and glutamine,
(7) amino acids affecting the orientation of a peptide chain: glycine and proline,
(8) amide-type amino acids (polar amino acids): asparagine and glutamine,
(9) aliphatic amino acids: alanine, leucine, isoleucine, and valine,
(10) amino acids having a small side chain: alanine, glycine, serine, and threonine,
(11) amino acids having a particularly small side chain: alanine and glycine,
(12) amino acids having a branched chain: valine, leucine, and isoleucine,
(13) amino acids having a side chain that may be hydroxylated: proline and serine.

The same applies to lysosome enzymes, GALC, GBA, and SA of non-human animal species.

The substitution of an amino acid in the amino acid sequence of each of a wild-type human lysosome enzyme and HSA with another amino acid is preferably a conservative amino acid substitution. The same applies to lysosome enzymes and SA of non-human animal species. Particularly, the conservative amino acid substitution is also applicable to a wild-type hGALC and hGBA.

The wild-type or mutant human lysosome enzyme, for example, hGALC or hGBA, having a constituent amino acid modified with a sugar chain is regarded as a human lysosome enzyme. Also, the wild-type or mutant human lysosome enzyme having a constituent amino acid modified with a phosphate group is regarded as a human lysosome enzyme. Also, the wild-type or mutant human lysosome enzyme having a constituent amino acid modified with a group except a sugar chain and a phosphate group is regarded as a human lysosome enzyme. Also, the wild-type or mutant human lysosome enzyme having a constituent amino acid whose side chain is converted by, e.g., a substitution reaction, is regarded as a human lysosome enzyme. Examples of such conversion include, but are not limited to, conversion of a cysteine residue to formylglycine. Also, the same applies to lysosome enzymes, GALC, and GBA of non-human animal species.

In other words, a human lysosome enzyme such as hGALC or hGBA, modified with a sugar chain is meant to be included in a human lysosome enzyme having an original amino acid sequence. Also, a human lysosome enzyme modified with a phosphate group is meant to be included in a human lysosome enzyme having an original amino acid sequence. Also, a human lysosome enzyme modified with a group except a sugar chain and a phosphate group is meant to be included in a human lysosome enzyme having an original amino acid sequence. Also, a human lysosome enzyme having a constituent amino acid whose side chain is converted by, e.g., a substitution reaction, is meant to be included in a human lysosome enzyme having an original amino acid sequence. Examples of such conversion include, but are not limited to, conversion of a cysteine residue to formylglycine. The same applies to lysosome enzymes, GALC, and GBA of non-human animal species.

The wild-type or mutant HSA having a constituent amino acid modified with a sugar chain is included in HSA. Also, the wild-type or mutant HSA having a constituent amino acid modified with a phosphate group is regarded as HSA. Also, HSA modified with a group except a sugar chain or a phosphate group is regarded as HSA. Also, the wild-type or mutant HSA having a constituent amino acid whose side chain is converted by, e.g., a substitution reaction, is regarded as HSA. Examples of such conversion include, but are not limited to, conversion of a cysteine residue into formylglycine. The same applies to SA of non-human animal species.

In short, HSA modified with a sugar chain is meant to be included in HSA having an original amino acid sequence. Also, HSA modified with a phosphate group is meant to be included in HSA having an original amino acid sequence. Also, HSA modified with a group except a sugar chain and a phosphate group is meant to be included in HSA having an original amino acid sequence. Also, HSA having a constituent amino acid whose side chain is converted by, e.g., a substitution reaction, is meant to be included in HSA having an original amino acid sequence. Examples of such conversion include, but are not limited to, conversion of a cysteine residue to formylglycine. The same applies to SA of non-human animal species.

In an embodiment, the present invention relates to a fusion protein obtained by binding a polypeptide containing an amino acid sequence of a wild-type or mutant human lysosome enzyme and a polypeptide containing an amino acid sequence of a wild-type or mutant SA. The phrase "binding polypeptides" refers to covalently binding different polypeptides directly or indirectly via a linker. SA herein is preferably HSA. Also, the human lysosome enzyme is, for example, hGALC or hGBA.

As an example method for binding two different polypeptides, it is general to employ the following method: to a downstream site of a gene encoding one of the polypeptides, a gene encoding the other polypeptide is bound in frame to prepare a DNA fragment, the DNA fragment is integrated into an expression vector, and a host cell is transformed with the expression vector and cultured to express a recombinant protein. The recombinant protein obtained is a single-chain polypeptide having the two polypeptides bound directly by a peptide bond or via another amino acid sequence.

In an embodiment of the present invention, the term "SA-human lysosome enzyme fusion protein" or "SA-human lysosome enzyme" refers to a fusion protein having an amino acid sequence in which the N terminal of the amino acid sequence of a human lysosome enzyme is bound to the C terminal of the SA amino acid sequence directly or via a linker, and having a function as a human lysosome enzyme. The animal species of SA is not particularly limited and SA is preferably SA of a mammal such as a primate, a mouse or a cow, more preferably primate SA, and further preferably HSA. The phrase that a human lysosome enzyme has a function as a human lysosome enzyme in the SA-human lysosome enzyme fusion protein means that the human lysosome enzyme has a specific activity of preferably 10% or more, more preferably 20% or more, further preferably 50% or more, and further more preferably 80% or more, when the specific activity of a normal wild-type human lysosome enzyme is regarded as 100%. Note that, the specific activity of the human lysosome enzyme in the SA-human lysosome enzyme fusion protein herein is computationally obtained by multiplying the enzyme activity (µM/h/mg protein) of a human lysosome enzyme of the fusion protein per unit mass by (the molecular weight of the fusion protein/the molecular weight of the moiety corresponding to the human lysosome enzyme in the fusion protein).

In an embodiment of the present invention, the term "HSA-human lysosome enzyme fusion protein" or "HSA-human lysosome enzyme" refers to a fusion protein having an amino acid sequence in which the N terminal of the amino acid sequence of a human lysosome enzyme is bound to the C terminal of the HSA amino acid sequence directly or via a linker, and having a function as a human lysosome enzyme. To the case where an HSA-human lysosome enzyme fusion protein has a function as a human lysosome enzyme, the definition in the case of the SA-human lysosome enzyme fusion protein mentioned above is applicable.

In the SA-human lysosome enzyme fusion protein, SA preferably has a function as SA, such as a function to bind to an endogenous substance and an exogenous substance such as a drug in the blood and transport them, but is not limited to this. The same applies to an HSA-human lysosome enzyme fusion protein.

In an embodiment of the present invention, a fusion protein having an amino acid sequence in which the N terminal of a lysosome enzyme of a non-human animal species is bound to the C terminal of the amino acid sequence of SA of a non-human animal species, directly or via a linker, and having a function as a human lysosome enzyme is expressed as a "(non-human animal species) SA - (non-human animal species) lysosome enzyme fusion protein". For example, a fusion protein of mouse SA and a mouse lysosome enzyme is expressed as an "MSA-mouse lysosome enzyme fusion protein" or "MSA-mouse lysosome enzyme". To a case where these fusion proteins having a function as a lysosome enzyme, the definition in the case of the SA-human lysosome enzyme fusion protein mentioned above is applicable.

In a case where a mutation is introduced to an HSA-human lysosome enzyme fusion protein, which is a fusion protein of a wild-type HSA and a wild-type human lysosome enzyme, the mutation can be introduced only to the HSA moiety and not introduced to the human lysosome enzyme moiety; the mutation can be introduced only to a human lysosome enzyme moiety without being introduced to the HSA moiety; or the mutation can be introduced to both of the HSA moiety and the human lysosome enzyme moiety. In a case where a mutation is introduced only to the HSA moiety, the amino acid sequence of the moiety is an amino acid sequence of HSA obtained by introducing a mutation to a wild-type HSA as mentioned above. In a case where a mutation is introduced only to a human lysosome enzyme moiety, the amino acid sequence of the moiety is an amino acid sequence of human lysosome enzyme obtained by introducing a mutation to a wild-type human lysosome enzyme as mentioned above. In a case where a mutation is introduced to both of the HSA moiety and the human lysosome enzyme moiety, the amino acid sequence of the HSA moiety is an amino acid sequence of HSA obtained by introducing a mutation to a wild-type HSA as mentioned above, and the amino acid sequence of the human lysosome enzyme moiety is an amino acid sequence of human lysosome enzyme obtained by introducing a mutation to a wild-type human lysosome enzyme as mentioned above. The same applies to a fusion protein of wild-type SA (including wild-type MSA) of a non-human animal species and a wild-type human lysosome enzyme.

An HSA-human lysosome enzyme fusion protein having a constituent amino acid modified with a sugar chain is also regarded as an HSA-human lysosome enzyme fusion protein. Also, an HSA-human lysosome enzyme fusion protein having a constituent amino acid modified with a phosphate group is regarded as an HSA-human lysosome enzyme fusion protein. Also, an HSA-human lysosome enzyme fusion protein modified with a group except a sugar chain and a phosphate group is regarded as an HSA-human lysosome enzyme fusion protein. Also, an HSA-human lysosome enzyme fusion protein having a constituent amino acid whose side chain is converted by, e.g., a substitution reaction, is regarded as an HSA-human lysosome enzyme fusion protein. Examples of such conversion include, but are not limited to, conversion of a cysteine residue to formylglycine. The same applies to a fusion protein (SA-human lysosome enzyme) of SA of a non-human animal species and a human lysosome enzyme and a fusion protein of SA of a non-human animal species and a lysosome enzyme of a non-human animal species, for example, MSA-mouse lysosome enzyme fusion protein.

More specifically, an HSA-human lysosome enzyme fusion protein modified with a sugar chain is meant to be included in an HSA-human lysosome enzyme fusion protein having an original amino acid sequence. Also, an HSA-human lysosome enzyme fusion protein modified with a phosphate group is meant to be included in an HSA-human lysosome enzyme fusion protein having an original amino acid sequence. Also, an HSA-human lysosome enzyme fusion protein modified with a group except a sugar chain and a phosphate group is meant to be included in an HSA-human lysosome enzyme fusion protein having an original amino acid sequence. Also, an HSA-human lysosome enzyme fusion protein having a constituent amino acid whose side chain is converted by, e.g., a substitution reaction, is meant to be included in an HSA-human lysosome enzyme fusion protein having an original amino acid sequence. Examples of such conversion include, but are not limited to, conversion of a cysteine residue to formylglycine. The same applies to a fusion protein (SA-human lysosome enzyme) of SA of a non-human animal species and a human lysosome enzyme and a fusion protein of SA of a non-human animal species and a lysosome enzyme of a non-human animal species, for example, an MSA-mouse lysosome enzyme fusion protein.

Also, an HSA-human lysosome enzyme fusion protein in which a constituent human lysosome enzyme is a precursor of a human lysosome enzyme is regarded as an HSA-human lysosome enzyme fusion protein. The precursor as used herein refers to a moiety which functions as a human lysosome enzyme of the HSA-human lysosome enzyme fusion protein biologically synthesized, is separated from the fusion protein during a production process or in a living body to which the fusion protein is administered and serves as a human lysosome enzyme by itself. In this case, sometimes, the HSA-human lysosome enzyme fusion protein biologically synthesized is cleaved at a predetermined site with, e.g., a hydrolytic enzyme to separate an HSA-containing moiety and a matured human lysosome enzyme-containing moiety. In this case, the resulting human lysosome enzyme is not a fusion protein with HSA but the HSA-human lysosome enzyme fusion protein is once synthesized during a process for producing the human lysosome enzyme. Accordingly, in a case where a human lysosome enzyme is produced by such a method, the production method is included in a method for producing HSA-human lysosome enzyme fusion protein. The same applies to a fusion protein (SA-human lysosome enzyme) of SA of a non-human animal species and a human lysosome enzyme, and a fusion protein of SA of a non-human animal species and a lysosome enzyme of a non-human animal species, for example, an MSA-mouse lysosome enzyme fusion protein.

In an embodiment of the present invention, the term "SA-hGALC fusion protein" or "SA-hGALC" refers to a fusion protein having an amino acid sequence in which the N terminal of the amino acid sequence of hGALC is bound to the C terminal of the amino acid sequence of SA directly or via a linker, and having a function as hGALC. The animal species of SA is not particularly limited and is preferably mammal SA, more preferably primate SA, and further preferably HSA. In an SA-hGALC fusion protein, the phrase that hGALC has a function as hGALC means that hGALC has a specific activity of preferably 10% or more, more preferably 20% or more, further preferably 50% or more, further more preferably 80% or more when the specific activity of a normal wild-type hGALC is regarded as 100%. Note that the specific activity of the hGALC in the SA-hGALC fusion protein herein is computationally obtained by multiplying the enzyme activity (µM/h/mg protein) of an hGALC per unit mass of the fusion protein by (the molecular weight of the fusion protein/the molecular weight of the moiety corresponding to the hGALC in the fusion protein).

In an embodiment of the present invention, the term "HSA-hGALC fusion protein" or "HSA-hGALC" refers to a fusion protein having an amino acid sequence in which the N terminal of the amino acid sequence of hGALC is bound to the C terminal of the amino acid sequence of HSA directly or via a linker, and having a function as hGALC. To a case where an HSA-hGALC fusion protein has a function as hGALC, the definition in the case of the SA-hGALC fusion protein mentioned above is applicable.

In an embodiment of the present invention, a fusion protein having an amino acid sequence in which the N terminal of the amino acid sequence of a non-human animal species is bound to the C terminal of the amino acid sequence of SA of a non-human animal species directly or via a linker, and having a function as GALC is expressed as a "(non-human animal species) SA - (non-human animal species) GALC fusion protein". For example, a fusion protein of mouse SA and a mouse GALC is expressed as an "MSA-mouse GALC fusion protein" or an "MSA-mouse GALC". To a case where these fusion proteins have a function as GALC, the definition in the case of the SA-hGALC fusion protein mentioned above is applicable. Also in these fusion proteins, the SA preferably has a function as SA, such as a function to bind an endogenous substance and an exogenous substance such as a drug in the blood and transport them, but is not limited to this.

In an embodiment of the present invention, a preferable HSA-hGALC fusion protein has the amino acid sequence represented by, for example, SEQ ID NO: 5. The HSA-hGALC fusion protein represented by SEQ ID NO: 5 is a fusion protein in which a wild-type hGALC is bound to the C terminal of a wild-type HSA via a linker sequence Gly-Ser. An HSA-hGALC fusion protein represented by SEQ ID NO: 5 is encoded by a gene having the nucleotide sequence represented by, for example, SEQ ID NO: 6. Also, a fusion protein having an amino acid sequence represented by SEQ ID NO: 5 and further having a mutation such as a substitution with different amino acid residues, a deletion, or an addition of one or more amino acid residues therein is included in the HSA-hGALC fusion protein as long as it has a function as hGALC. The HSA-hGALC fusion protein preferably has a function as human serum albumin, such as a function to bind an endogenous substance and an exogenous substance such as a drug in the blood and transport them, but is not limited to this. Note that, the GS linker mentioned in the specification refers to a linker constituted of Gly-Ser.

In an embodiment of the present invention, the term "MSA-mGALC fusion protein" or "MSA-mGALC" refers to a fusion protein having an amino acid sequence in which the N terminal of the amino acid sequence of mGALC is bound to the C terminal of the amino acid sequence of MSA directly or via a linker, and having a function as mGALC. A preferable MSA-mGALC fusion protein has the amino acid sequence represented by, for example, SEQ ID NO: 18. The MSA-mGALC fusion protein represented by SEQ ID NO: 18 is a fusion protein in which a wild-type mGALC is bound to the C terminal of wild-type MSA via a linker sequence Gly-Ser. The MSA-mGALC fusion protein represented by SEQ ID NO: 18 is encoded by a gene having a nucleotide sequence represented by, for example, SEQ ID NO: 19. Also, a fusion protein having an amino acid sequence represented by SEQ ID NO: 18 and further having a mutation such as a substitution with different amino acid residues, a deletion, or an addition of one or more amino acid residues therein is included in the MSA-mGALC fusion protein as long as it has a function as an mGALC. The MSA-mGALC fusion protein preferably has a function as mouse serum albumin, such as a function to bind an endogenous substance and an exogenous substance such as a drug in the blood and transport them, but is not limited to this.

In a case where a mutation is introduced to an HSA-hGALC fusion protein, which is a fusion protein of a wild-type HSA and a wild-type hGALC, the mutation can be introduced only to the HSA moiety and not introduced to the hGALC moiety; the mutation can be introduced only to the hGALC moiety without being introduced to the HSA moiety; or the mutation can be introduced to both of the HSA moiety and the hGALC moiety. In a case where a mutation is introduced only to the HSA moiety, the amino acid sequence of the moiety is an amino acid sequence of HSA obtained by introducing a mutation to a wild-type HSA as mentioned above. In a case where a mutation is introduced only to the hGALC moiety, the amino acid sequence of the moiety is an amino acid sequence of hGALC obtained by introducing a mutation to a wild-type hGALC as mentioned above. In a case where a mutation is introduced to both of the HSA moiety and the hGALC moiety, the amino acid sequence of the HSA moiety is an amino acid sequence of HSA obtained by introducing a mutation to a wild-type HSA as mentioned above, and the amino acid sequence of the hGALC moiety is an amino acid sequence of hGALC obtained by introducing a mutation to a wild-type hGALC as mentioned above. The same applies to the HSA-hGALC fusion protein including one having the amino acid sequence represented by SEQ ID NO: 5. Also, the same applies to the MSA-hGALC fusion protein having the amino acid sequence represented by SEQ ID NO: 18.

The MSA-mGALC fusion protein having the amino acid sequence represented by SEQ ID NO: 18 is encoded by a gene having a nucleotide sequence represented by, for example, SEQ ID NO: 19. The same applies to a fusion protein of wild-type SA (including wild-type MSA) of a non-human animal species and a wild-type hGALC.

The case where a mutation is introduced to an HSA-hGALC fusion protein having the amino acid sequence represented by SEQ ID NO: 5 will be described below. When an amino acid residue in the amino acid sequence represented by SEQ ID NO: 5 is substituted with another amino acid residue, the number of amino acid residues to be substituted is 1 to 10, 1 to 5, or 1 to 3, for example, one or two. When an amino acid residue is deleted, the number of amino acid residues to be deleted is 1 to 10, 1 to 5, or 1 to 3, for example, one or two. When an amino acid residue is added, one or more amino acid residues are added in the amino acid sequence represented by SEQ ID NO: 5 or added to the N terminal or C terminal of the amino acid sequence thereof. The HSA-hGALC fusion protein may have a combination of these substitution, deletion, and addition of amino acid residues. A mutation may be introduced only to the HSA moiety, only to the hGALC moiety, or to both of them. The same applies to a fusion protein (SA-hGALC) of SA of a non-human animal species and hGALC, and a fusion protein of SA of a non-human animal species and GALC of a non-human animal species, for example, an MSA-mouse GALC fusion protein.

When mutations are introduced to the amino acid sequence represented by SEQ ID NO: 5, the sites and types (deletion, substitution, and addition) of individual mutations can be easily identified by alignment of the amino acid sequences before and after introduction of the mutations. The amino acid sequence having a mutation introduced thereto exhibits an identity of preferably 80% or more, an identity of 85% or more, an identity of 90% or more, or an identity of 95% or more, for example, an identity of 98% or more or an identity of 99% or more, to the amino acid sequence represented by SEQ ID NO: 5.

An HSA-hGALC fusion protein having a constituent amino acid modified with a sugar chain is regarded as an HSA-hGALC fusion protein. Also, an HSA-hGALC fusion protein having a constituent amino acid modified with a phosphate group is regarded as an HSA-hGALC fusion protein. Also, an HSA-hGALC fusion protein modified with a group except a sugar chain and a phosphate group is regarded as an HSA-hGALC fusion protein. Also, an HSA-hGALC fusion protein having a constituent amino acid whose side chain is converted by, e.g., a substitution reaction, is regarded as an HSA-hGALC fusion protein. Examples of such conversion include, but are not limited to, conversion of a cysteine residue to formylglycine. The same applies to a fusion protein (SA-hGALC) of SA of a non-human animal species and hGALC, and a fusion protein of SA of a non-human animal species and GALC of a non-human animal species, for example, an MSA-mouse lysosome enzyme fusion protein.

More specifically, an HSA-hGALC fusion protein modified with a sugar chain is meant to be included in an HSA-hGALC fusion protein having an original amino acid sequence. Also, an HSA-hGALC fusion protein modified with a phosphate group is meant to be included in an HSA-hGALC fusion protein having an original amino acid sequence. Also, an HSA-hGALC fusion protein modified with a group except a sugar chain and a phosphate group is meant to be included in an HSA-hGALC fusion protein having an original amino acid sequence. Also, an HSA-hGALC fusion protein having a constituent amino acid whose side chain is converted by, e.g., a substitution reaction, is meant to be included in an HSA-hGALC fusion protein having an original amino acid sequence. Examples of such conversion include, but are not limited to, conversion of a cysteine residue to formylglycine. The same applies to a fusion protein (SA-hGALC) of SA of a non-human animal species and hGALC, and a fusion protein of SA of a non-human animal species and GALC of a non-human animal species, for example, an MSA-mouse GALC fusion protein.

Also, an HSA-hGALC fusion protein in which a constituent hGALC is a precursor of hGALC is regarded as an HSA-hGALC fusion protein. The precursor as used herein refers to a moiety which functions as hGALC of an HSA-hGALC fusion protein biologically synthesized, is separated from the fusion protein during a production process or in a living body administered with the fusion protein is administered and serves as an hGALC by itself. In this case, sometimes, the HSA-hGALC fusion protein biologically synthesized is cleaved at a predetermined site with, e.g., a hydrolytic enzyme to separate an HSA-containing moiety and a matured hGALC-containing moiety. In this case, the resulting hGALC is not a fusion protein with HSA but the HSA-hGALC fusion protein is once synthesized during a process for synthesizing the hGALC. Accordingly, in a case where hGALC is produced by such a method, the production method is included in a method for producing an HSA-hGALC fusion protein. The same applies to a fusion protein (SA-hGALC) of SA of a non-human animal species and hGALC, and a fusion protein of SA of a non-human animal species and GALC of a non-human animal species, for example, an MSA-mouse GALC fusion protein.

In an embodiment of the present invention, the term "SA-hGBA fusion protein" or "SA-hGBA" refers to a fusion protein having an amino acid sequence in which the N terminal of the amino acid sequence of hGBA is bound to the C terminal of the amino acid sequence of SA directly or via a linker, and having a function as hGBA. The animal species of SA is not particularly limited and SA is preferably mammal SA, more preferably primate SA, and further preferably HSA. The phrase that hGBA has a function as hGBA in an SA-hGBA fusion protein means that hGBA has a specific activity of preferably 10% or more, more preferably 20% or more, further preferably 50% or more, further more preferably 80% or more when the specific activity of a normal wild-type hGBA is regarded as 100%. Note that, the specific activity of the hGBA in the SA-hGBA fusion protein herein is computationally obtained by multiplying the enzyme activity (µM/h/mg protein) of an hGBA per unit mass of the fusion protein by (the molecular weight of the fusion protein/the molecular weight of the moiety corresponding to the hGBA in the fusion protein).

In an embodiment of the present invention, the term "HSA-hGBA fusion protein" or "HSA-hGBA" refers to a fusion protein having an amino acid sequence in which the N terminal of the amino acid sequence of hGBA is bound to the C terminal of the amino acid sequence of HSA directly or via a linker, and having a function as hGBA. To a case where HSA-hGBA fusion protein has a function as hGALC, the definition in the case of the SA-hGBA fusion protein mentioned above is applicable.

In an embodiment of the present invention, a fusion protein having an amino acid sequence in which the N terminal of GBA of a non-human animal species is bound to the C terminal of the amino acid sequence of SA of a non-human animal species directly or via a linker, and having a function as GBA is expressed as a "(non-human animal species) SA - (non-human animal species) GBA fusion protein". For example, a fusion protein of mouse SA and mouse GBA is expressed as an "MSA-mouse GBA fusion protein" or an "MSA-mouse GBA". To a case where these fusion proteins having a function as GBA, the definition in the case of the SA-hGBA fusion protein mentioned above is applicable. Also in these fusion proteins, SA preferably has a function as SA, such as a function to bind an endogenous substance and an exogenous substance such as a drug in the blood and transport them, but is not limited to this.

In an embodiment of the present invention, a preferable HSA-hGBA fusion protein has the amino acid sequence represented by, for example, SEQ ID NO: 39. The HSA-hGBA fusion protein represented by SEQ ID NO: 39 is a fusion protein in which a wild-type hGBA is bound to the C terminal of a wild-type HSA via a linker sequence having the amino acid sequence represented by SEQ ID NO: 9. The HSA-hGBA fusion protein represented by SEQ ID NO: 39 is encoded by a gene having the nucleotide sequence represented by, for example, SEQ ID NO: 40. Also, a fusion protein having an amino acid sequence represented by SEQ ID NO: 39 and further having a mutation such as a substitution with different amino acid residues, a deletion, or an addition of one or more amino acid residues therein is included in the HSA-hGBA fusion protein as long as it has a function as hGBA. The HSA-hGBA fusion protein preferably has a function as human serum albumin, such as a function to bind an endogenous substance and an exogenous substance such as a drug in the blood and transport them, but is not limited to this.

In an embodiment of the present invention, the term "MSA-mGBA fusion protein" or "MSA-mGBA" refers to a fusion protein having an amino acid sequence in which the N terminal of the amino acid sequence of mGBA is bound to the C terminal of the amino acid sequence of MSA directly or via a linker, and having a function as mGBA. A preferable MSA-mGBA fusion protein has the amino acid sequence represented by, for example, SEQ ID NO: 45. The MSA-mGBA fusion protein represented by SEQ ID NO: 45 is a fusion protein in which a wild-type mGBA is bound to the C terminal of wild-type MSA via a linker sequence having the amino acid sequence represented by SEQ ID NO: 9. The MSA-mGBA fusion protein represented by SEQ ID NO: 45 is encoded by a gene having a nucleotide sequence represented by, for example, SEQ ID NO: 46. Also, a fusion protein having an amino acid sequence represented by SEQ ID NO: 45 and further having a mutation such as a substitution with different amino acid residues, a deletion, or an addition of one or more amino acid residues therein is included in the MSA-mGBA fusion protein as long as it has a function as mGBA. The MSA-mGBA fusion protein preferably has a function as mouse serum albumin, such as a function to bind an endogenous substance and an exogenous substance such as a drug in the blood and transport them, but is not limited to this.

In a case where a mutation is introduced to an HSA-hGBA fusion protein, which is a fusion protein of a wild-type HSA and a wild-type hGBA, the mutation can be introduced only to the HSA moiety and not introduced to the hGBA moiety; the mutation can be introduced only to the hGBA moiety without being introduced to the HSA moiety; or the mutation can be introduced to both of the HSA moiety and the hGBA moiety. In a case where a mutation is introduced only to the HSA moiety, the amino acid sequence of the moiety is an amino acid sequence of HSA obtained by introducing a mutation to a wild-type HSA as mentioned above. In a case where a mutation is introduced only to the hGBA moiety, the amino acid sequence of the moiety is an amino acid sequence of hGBA obtained by introducing a mutation to a wild-type hGBA as mentioned above. In a case where a mutation is introduced to both of the HSA moiety and the hGBA moiety, the amino acid sequence of the HSA moiety is an amino acid sequence of HSA obtained by introducing a mutation to a wild-type HSA as mentioned above, and the amino acid sequence of the hGBA moiety is an amino acid sequence of hGBA obtained by introducing a mutation to a wild-type hGBA as mentioned above. The same applies to the HSA-hGBA fusion protein having the amino acid sequence represented by SEQ ID NO: 39. Also, the same applies to the MSA-mGBA fusion protein including one having the amino acid sequence represented by SEQ ID NO: 45. The MSA-mGBA fusion protein having the amino acid sequence represented by SEQ ID NO: 45 is encoded by a gene having a nucleotide sequence represented by, for example, SEQ ID NO: 46. The same applies to a fusion protein of wild-type SA (including wild-type MSA) of a non-human animal species and a wild-type hGBA.

The case where a mutation is introduced to an HSA-hGBA fusion protein having the amino acid sequence represented by SEQ ID NO: 39 will be described below. When an amino acid residue in the amino acid sequence represented by SEQ ID NO: 39 is substituted with another amino acid residue, the number of amino acid residues to be substituted is 1 to 10, 1 to 5, or 1 to 3, for example, one or two. When an amino acid residue is deleted, the number of amino acid residues to be deleted is 1 to 10, 1 to 5, or 1 to 3, for example, one or two. When an amino acid residue is added, one or more amino acid residues are added in the amino acid sequence represented by SEQ ID NO: 39 or added to the N terminal or C terminal of the amino acid sequence thereof. The HSA-hGBA fusion protein may have a combination of these substitution, deletion, and addition of amino acid residues. A mutation may be introduced only to the HSA moiety, only to the hGBA moiety, or to both of them. The same applies to a fusion protein (SA-hGBA) of SA of a non-human animal species and an hGBA, and a fusion protein of SA of a non-human animal species and GBA of a non-human animal species, for example, an MSA-mouse GBA fusion protein.

When mutations are introduced to the amino acid sequence represented by SEQ ID NO: 39, the sites and types (deletion, substitution, and addition) of individual mutations can be easily identified by alignment of the amino acid sequences before and after introduction of the mutations. The amino acid sequence having a mutation introduced thereto exhibits an identity of preferably 80% or more, an identity of 85% or more, an identity of 90% or more, or an identity of 95% or more, for example, an identity of 98% or more or an identity of 99% or more, to the amino acid sequence represented by SEQ ID NO: 39.

An HSA-hGBA fusion protein having a constituent amino acid modified with a sugar chain is regarded as an HSA-hGBA fusion protein. Also, an HSA-hGBA fusion protein having a constituent amino acid modified with a phosphate group is regarded as an HSA-hGBA fusion protein. Also, an HSA-hGBA fusion protein modified with a group except a sugar chain and a phosphate group is regarded as an HSA-hGBA fusion protein. Also, an HSA-hGBA fusion protein having a constituent amino acid whose side chain is converted by, e.g., a substitution reaction, is regarded as an HSA-hGBA fusion protein. Examples of such conversion include, but are not limited to, conversion of a cysteine residue to formylglycine. The same applies to a fusion protein (SA-hGBA) of SA of a non-human animal species and hGBA and a fusion protein of SA of a non-human animal species and GBA of a non-human animal species, for example, an MSA-mouse GBA fusion protein.

More specifically, an HSA-hGBA fusion protein modified with a sugar chain is meant to be included in an HSA-hGBA fusion protein having an original amino acid sequence. Also, an HSA-hGBA fusion protein modified with a phosphate group is meant to be included in an HSA-hGBA fusion protein having an original amino acid sequence. Also, an HSA-hGBA fusion protein modified with a group except a sugar chain and a phosphate group is meant to be included in an HSA-hGBA fusion protein having an original amino acid sequence. Also, an HSA-hGBA fusion protein having a constituent amino acid whose side chain is converted by, e.g., a substitution reaction, is meant to be included in an HSA-hGBA fusion protein having an original amino acid sequence. Examples of such conversion include, but are not limited to, conversion of a cysteine residue to formylglycine. The same applies to a fusion protein (SA-hGBA) of SA of a non-human animal species and hGBA, and a fusion protein of SA of a non-human animal species and GBA of a non-human animal species, for example, an MSA-mouse GBA fusion protein.

An HSA-hGBA fusion protein in which a constituent hGBA is a precursor of an hGBA is regarded as an HSA-hGBA fusion protein. The precursor as used herein refers to a moiety which functions as hGBA of the HSA-hGBA fusion protein biologically synthesized, is separated from the fusion protein during a production process or in a living body to which the fusion protein and serves as hGBA by itself. In this case, sometimes, the HSA-hGBA fusion protein biologically synthesized is cleaved at a predetermined site with, e.g., a hydrolytic enzyme, to separate an HSA-containing moiety and a matured hGBA-containing moiety. In this case, the resulting hGBA is not a fusion protein with HSA but the HSA-hGBA fusion protein is once synthesized during a process for synthesizing the hGBA. Accordingly, in a case where an hGBA is produced by such a method, the production method is included in a method for producing an HSA-hGBA fusion protein. The same applies to a fusion protein (SA-hGBA) of SA of a non-human animal species and hGBA, and a fusion protein of SA of a non-human animal species and GBA of a non-human animal species, for example, an MSA-mouse GBA fusion protein.

In an embodiment of the present invention, the term "human lysosome enzyme-SA fusion protein" or "human lysosome enzyme-SA" refers to a fusion protein having an amino acid sequence in which the N terminal of the amino acid sequence of SA is bound to the C terminal of the amino acid sequence of a human lysosome enzyme directly or via a linker, and having a function as a human lysosome enzyme. The phrase that a human lysosome enzyme has a function as a human lysosome enzyme in a human lysosome enzyme-SA fusion protein means that a human lysosome enzyme has a specific activity of preferably 10% or more, more preferably 20% or more, further preferably 50% or more, further more preferably 80% or more when the specific activity of a normal wild-type human lysosome enzyme is regarded as 100%. Note that, the specific activity of the human lysosome enzyme in the human lysosome enzyme-SA fusion protein herein is computationally obtained by multiplying the enzyme activity (µM/h/mg protein) of a human lysosome enzyme per unit mass of the fusion protein by (the molecular weight of the fusion protein/the molecular weight of the moiety corresponding to the human lysosome enzyme in the fusion protein).

In an embodiment of the present invention, the term "human lysosome enzyme-HSA fusion protein" or "human lysosome enzyme-HSA" refers to a fusion protein having an amino acid sequence in which the N terminal of the amino acid sequence of HSA is bound to the C terminal of the amino acid sequence of a human lysosome enzyme directly or via a linker, and having a function as a human lysosome enzyme. To a case where the human lysosome enzyme-HSA fusion protein has a function as a human lysosome enzyme, the definition in the case of the human lysosome enzyme-SA mentioned above is applicable.

In the human lysosome enzyme-SA fusion protein, SA preferably has a function as SA, such as a function to bind an endogenous substance and an exogenous substance such as a drug in the blood and transport them, but is not limited to this. The same applies to a human lysosome enzyme-HSA fusion protein.

In an embodiment of the present invention, a fusion protein having an amino acid sequence in which the N terminal of SA of a non-human animal species is bound to the C terminal of the amino acid sequence of a lysosome enzyme of a non-human animal species directly or via a linker, and having a function as human lysosome enzyme is expressed as a "(non-human animal species) lysosome enzyme - (non-human animal species) SA fusion protein". For example, a fusion protein of a mouse lysosome enzyme and mouse SA and is expressed as a "mouse lysosome enzyme-MSA fusion protein" or "mouse lysosome enzyme-MSA". To a case where these fusion proteins have a function as lysosome enzyme, the definition in the case of the human lysosome enzyme-SA fusion protein mentioned above is applicable.

In a case where a mutation is introduced to a human lysosome enzyme-HSA fusion protein, which is a fusion protein of a wild-type human lysosome enzyme and a wild-type HSA, the mutation can be introduced only to the human lysosome enzyme moiety and not introduced to the HSA moiety; the mutation can be introduced only to an HSA moiety without being introduced to the human lysosome enzyme moiety; or the mutation can be introduced to both of the human lysosome enzyme moiety and the HSA moiety. In a case where a mutation is introduced only to the human lysosome enzyme moiety, the amino acid sequence of the moiety is an amino acid sequence of the human lysosome enzyme obtained by introducing a mutation to a wild-type human lysosome enzyme as mentioned above. In a case where a mutation is introduced only to the HSA moiety, the amino acid sequence of the moiety is an amino acid sequence of HSA obtained by introducing a mutation to a wild-type HSA as mentioned above. In a case where a mutation is introduced to both of the human lysosome enzyme moiety and the HSA moiety, the amino acid sequence of the human lysosome enzyme moiety is an amino acid sequence of human lysosome enzyme obtained by introducing a mutation to a wild-type human lysosome enzyme as mentioned above, and the amino acid sequence of the HSA moiety is an amino acid sequence of HSA obtained by introducing a mutation to a wild-type HSA as mentioned above. The same applies to a fusion protein of a wild-type human lysosome enzyme and wild-type SA of a non-human animal species (including wild-type MSA).

A human lysosome enzyme-HSA fusion protein having a constituent amino acid modified with a sugar chain is regarded as a human lysosome enzyme-HSA fusion protein. Also, a human lysosome enzyme-HSA fusion protein having a constituent amino acid modified with a phosphate group is regarded as a human lysosome enzyme-HSA fusion protein. Also, a human lysosome enzyme-HSA fusion protein modified with a group except a sugar chain and a phosphate group is regarded as a human lysosome enzyme-HSA fusion protein. Also, a human lysosome enzyme-HSA fusion protein having a constituent amino acid whose side chain is converted by, e.g., a substitution reaction, is regarded as a human lysosome enzyme-HSA fusion protein. Examples of such conversion include, but are not limited to, conversion of a cysteine residue to formylglycine. The same applies to a fusion protein (human lysosome enzyme-SA) of a human lysosome enzyme and SA of a non-human animal species, and a fusion protein of a lysosome enzyme of a non-human animal species and SA of a non-human animal species, for example, a mouse lysosome enzyme-MSA.

More specifically, a human lysosome enzyme-HSA fusion protein modified with a sugar chain is meant to be included in a human lysosome enzyme-HSA fusion protein having an original amino acid sequence. Also, a human lysosome enzyme-HSA fusion protein modified with a phosphate group is meant to be included in a human lysosome enzyme-HSA fusion protein having an original amino acid sequence. Also, a human lysosome enzyme-HSA fusion protein modified with a group except a sugar chain and a phosphate group is meant to be included in a human lysosome enzyme-HSA fusion protein having an original amino acid sequence. Also, a human lysosome enzyme-HSA fusion protein having a constituent amino acid whose side chain is converted by, e.g., a substitution reaction, is meant to be included in a human lysosome enzyme-HSA fusion protein having an original amino acid sequence. Examples of such conversion include, but are not limited to, conversion of a cysteine residue to formylglycine. The same applies to a fusion protein (human lysosome enzyme-SA) of a human lysosome enzyme and SA of a non-human animal species and a fusion protein of a lysosome enzyme of a non-human animal species and SA of a non-human animal species.

Also, a human lysosome enzyme-HSA fusion protein in which a constituent human lysosome enzyme is a precursor of a human lysosome enzyme is regarded as a human lysosome enzyme-HSA fusion protein.

In an embodiment of the present invention, the term "hGALC-SA fusion protein" or "hGALC-SA" refers to a fusion protein having an amino acid sequence in which the N terminal of the amino acid sequence of SA is bound to the C terminal of the amino acid sequence of hGALC directly or via a linker, and having a function as hGALC. The phrase that hGALC has a function as hGALC in the hGALC-SA fusion protein means that hGALC has a specific activity of preferably 10% or more, more preferably 20% or more, further preferably 50% or more, further more preferably 80% or more when the specific activity of a normal wild-type hGALC is regarded as 100%. Note that, the specific activity of the hGALC in the hGALC-SA fusion protein herein is computationally obtained by multiplying the enzyme activity (µM/h/mg protein) of hGALC per unit mass of the fusion protein by (the molecular weight of the fusion protein/the molecular weight of the moiety corresponding to the hGALC in the fusion protein).

In an embodiment of the present invention, the term "hGALC-HSA fusion protein" or "hGALC-HSA" refers to a fusion protein having an amino acid sequence in which the N terminal of the amino acid sequence of HSA is bound to the C terminal of the amino acid sequence of hGALC directly or via a linker, and having a function as hGALC. To a case where the hGALC-HSA fusion protein has a function as hGALC, the definition in the case of the hGALC-SA fusion protein mentioned above is applicable.

In an embodiment of the present invention, a fusion protein having an amino acid sequence in which the N terminal of SA of a non-human animal species is bound to the C terminal of the amino acid sequence of GALC of a non-human animal species directly or via a linker, and having a function as GALC is expressed as a "(non-human animal species) GALC - (non-human animal species) SA fusion protein". For example, a fusion protein of mouse GALC and mouse SA is expressed as a "mouse GALC-MSA fusion protein" or "mouse GALC-MSA". To a case where these fusion proteins have a function as GALC, the definition in the case of the hGALC-HSA fusion protein mentioned above is applicable. Also in these fusion proteins, SA preferably has a function as SA, such as a function to bind an endogenous substance and an exogenous substance such as a drug in the blood and transport them, but is not limited to this.

In an embodiment of the present invention, a preferable hGALC-HSA fusion protein has the amino acid sequence represented by, for example, SEQ ID NO: 7. The hGALC-HSA fusion protein represented by SEQ ID NO: 7 is a fusion protein in which a wild-type HSA is bound to the C terminal of a wild-type hGALC via a linker sequence, Gly-Ser. The hGALC-HSA fusion protein represented by SEQ ID NO: 7 is encoded by a gene having the nucleotide sequence represented by, for example, SEQ ID NO: 8. Also, a fusion protein having an amino acid sequence represented by SEQ ID NO: 7 and further having a mutation such as a substitution with different amino acid residues, a deletion, or an addition of one or more amino acid residues therein is included in the hGALC-HSA fusion protein as long as it has a function as hGALC. The hGALC-HSA fusion protein preferably has a function as human serum albumin, such as a function to bind an endogenous substance and an exogenous substance such as a drug in the blood and transport them, but is not limited to this.

In an embodiment of the present invention, the term "mGALC-MSA fusion protein" or "mGALC-MSA" refers to a fusion protein having an amino acid sequence in which the N terminal of the amino acid sequence of MSA is bound to the C terminal of the amino acid sequence of mGALC directly or via a linker, and having a function as mGALC. A preferable mGALC-MSA fusion protein has the amino acid sequence represented by, for example, SEQ ID NO: 20. The mGALC-MSA fusion protein represented by SEQ ID NO: 20 is a fusion protein in which wild-type MSA is bound to the C terminal of a wild-type mGALC via a linker sequence, Gly-Ser. The mGALC-MSA fusion protein represented by SEQ ID NO: 20 is encoded by a gene having a nucleotide sequence represented by, for example, SEQ ID NO: 21. Also, a fusion protein having an amino acid sequence represented by SEQ ID NO: 20 and further having a mutation such as a substitution with different amino acid residues, a deletion, or an addition of one or more amino acid residues therein is included in the mGALC-MSA fusion protein as long as it has a function as an mGALC. The mGALC-MSA fusion protein preferably has a function as mouse serum albumin, such as a function to bind an endogenous substance and an exogenous substance such as a drug in the blood and transport them, but is not limited to this.

In a case where a mutation is introduced to hGALC-HSA fusion protein, which is a fusion protein of a wild-type hGALC and a wild-type HSA, the mutation can be introduced only to the hGALC moiety and not introduced to the HSA moiety; the mutation can be introduced only to the HSA moiety without being introduced to the hGALC moiety; or the mutation can be introduced to both of the hGALC moiety and the HSA moiety. In a case where a mutation is introduced only to the hGALC moiety, the amino acid sequence of the moiety is an amino acid sequence of hGALC obtained by introducing a mutation to a wild-type hGALC as mentioned above. In a case where a mutation is introduced only to the HSA moiety, the amino acid sequence of the moiety is an amino acid sequence of HSA obtained by introducing a mutation to a wild-type HSA as mentioned above. In a case where a mutation is introduced to both of the hGALC moiety and the HSA moiety, the amino acid sequence of the hGALC moiety is an amino acid sequence of hGALC obtained by introducing a mutation to a wild-type hGALC as mentioned above, and the amino acid sequence of the HSA moiety is an amino acid sequence of HSA obtained by introducing a mutation to a wild-type HSA as mentioned above. The same applies to an hGALC-HSA fusion protein having the amino acid sequence represented by SEQ ID NO: 7. The same applies to an mGALC-MSA fusion protein including one having the amino acid sequence represented by SEQ ID NO: 20. The mGALC-MSA fusion protein having the amino acid sequence represented by SEQ ID NO: 20 is encoded by a gene having the nucleotide sequence represented by, for example, SEQ ID NO: 21. The same applies to a fusion protein of a wild-type hGALC and wild-type SA of a non-human animal (including wild-type MSA).

In a case where a mutation is introduced to an hGALC-HSA fusion protein, which is a fusion protein of a wild-type hGALC and a wild-type HSA, the mutation can be introduced only to the HSA moiety and not introduced to the hGALC moiety; the mutation can be introduced only to the hGALC moiety without being introduced to the HSA moiety; or the mutation can be introduced to both of the HSA moiety and the hGALC moiety. In a case where a mutation is introduced only to the HSA moiety, the amino acid sequence of the HSA moiety is an amino acid sequence of HSA obtained by introducing a mutation to a wild-type HSA as mentioned above. In a case where a mutation is introduced only to the hGALC moiety, the amino acid sequence of the moiety is an amino acid sequence of hGALC obtained by introducing a mutation to a wild-type hGALC as mentioned above. In a case where a mutation is introduced to both of the HSA moiety and the hGALC moiety, the amino acid sequence of the HSA moiety is an amino acid sequence of HSA obtained by introducing a mutation to a wild-type HSA as mentioned above, and the amino acid sequence of the hGALC moiety is an amino acid sequence of hGALC obtained by introducing a mutation to a wild-type hGALC as mentioned above. The same applies to an hGALC-HSA fusion protein having the amino acid sequence represented by SEQ ID NO: 7. Also, the same applies to a fusion protein of an mGALC-MSA fusion protein including one having the amino acid sequence represented by SEQ ID NO: 20. The mGALC-MSA fusion protein having the amino acid sequence represented by SEQ ID NO: 20 is encoded by a gene having the nucleotide sequence represented by, for example, SEQ ID NO: 21. The same applies to a fusion protein of a wild-type hGALC and wild-type SA (hGALC-SA) of a non-human animal.

The case where a mutation is introduced to an hGALC-HSA fusion protein having the amino acid sequence represented by SEQ ID NO: 7 will be described below. When an amino acid residue in the amino acid sequence represented by SEQ ID NO: 7 is substituted with another amino acid residue, the number of amino acid residues to be substituted is 1 to 10, 1 to 5, or 1 to 3, for example, one or two. When an amino acid residue is deleted, the number of amino acid residues to be deleted is 1 to 10, 1 to 5, or 1 to 3, for example, one or two. When an amino acid residue is added, one or more amino acid residues are added in the amino acid sequence represented by SEQ ID NO: 7 or added to the N terminal or C terminal of the amino acid sequence thereof. The hGALC-HSA fusion protein may have a combination of these substitution, deletion, and addition of amino acid residues. A mutation may be introduced only to the HSA moiety, only to the hGALC moiety, or to both of them. The same applies to a fusion protein (SA-GALC) of SA of a non-human animal species and GALC, and a fusion protein of SA of a non-human animal species and GALC of a non-human animal species, for example, an MSA-mouse GALC fusion protein.

When mutations are introduced to the amino acid sequence represented by SEQ ID NO: 7, the sites and types (deletion, substitution, and addition) of individual mutations can be easily identified by alignment of the amino acid sequences before and after introduction of the mutations. The amino acid sequence having a mutation introduced thereto exhibits an identity of preferably 80% or more, an identity of 85% or more, an identity of 90% or more, or an identity of 95% or more, for example, an identity of 98% or more or an identity of 99% or more, to the amino acid sequence represented by SEQ ID NO: 7.

An hGALC-HSA fusion protein having a constituent amino acid modified with a sugar chain is regarded as an hGALC-HSA fusion protein. Also, an hGALC-HSA fusion protein having a constituent amino acid modified with a phosphate group is regarded as an hGALC-HSA fusion protein. Also, an hGALC-HSA fusion protein modified with a group except a sugar chain and a phosphate group is regarded as an hGALC-HSA fusion protein. Also, an hGALC-HSA fusion protein having a constituent amino acid whose side chain is converted by, e.g., a substitution reaction, is regarded as an hGALC-HSA fusion protein. Examples of such conversion include, but are not limited to, conversion of a cysteine residue to formylglycine. The same applies to a fusion protein (GALC-SA) of GALC of a non-human animal species and SA, and a fusion protein of GALC of a non-human animal species and SA of a non-human animal species, for example, a fusion protein of mouse GALC and MSA.

More specifically, an hGALC-HSA fusion protein modified with a sugar chain is meant to be included in an hGALC-HSA fusion protein having an original amino acid sequence. Also, an hGALC-HSA fusion protein modified with a phosphate group is meant to be included in an hGALC-HSA fusion protein having an original amino acid sequence. Also, an hGALC-HSA fusion protein modified with a group except a sugar chain and a phosphate group is meant to be included in an hGALC-HSA fusion protein having an original amino acid sequence. Also, an hGALC-HSA fusion protein having a constituent amino acid whose side chain is converted by, e.g., a substitution reaction, is meant to be included in an hGALC-HSA fusion protein having an original amino acid sequence. Examples of such conversion include, but are not limited to, conversion of a cysteine residue to formylglycine. The same applies to a fusion protein (GALC-SA) of GALC of a non-human animal species and SA and a fusion protein of GALC of a non-human animal species and SA of a non-human animal species.

Also, an hGALC-HSA fusion protein in which a constituent hGALC is a precursor of hGALC is regarded as an HSA-hGALC fusion protein.

In an embodiment of the present invention, the term "hGBA-SA fusion protein" or "hGBA-SA" refers to a fusion protein having an amino acid sequence in which the N terminal of the amino acid sequence of SA is bound to the C terminal of the amino acid sequence of hGBA directly or via a linker, and having a function as hGBA. The animal species of SA is not particularly limited and SA is preferably mammal SA, more preferably primate SA, and further preferably HSA. The phrase that an hGBA has a function as an hGBA in the hGBA-SA fusion protein means that the hGBA has a specific activity of preferably 10% or more, more preferably 20% or more, further preferably 50% or more, and further more preferably 80% or more, when the specific activity of a normal wild-type hGBA is regarded as 100%. Note that, the specific activity of the hGBA in the hGBA-SA fusion protein herein is computationally obtained by multiplying the enzyme activity (µM/h/mg protein) of the hGBA per unit mass of the fusion protein by (the molecular weight of the fusion protein/the molecular weight of the moiety corresponding to the hGBA in the fusion protein).

In an embodiment of the present invention, the term "hGBA-HSA fusion protein" or "hGBA-HSA" refers to a fusion protein having an amino acid sequence in which the N terminal of the amino acid sequence of HSA is bound to the C terminal of the amino acid sequence of hGBA directly or via a linker, and having a function as hGBA. To a case where the hGBA-HSA fusion protein has a function as hGALC, the definition in the case of the SA-hGBA fusion protein mentioned above is applicable.

In an embodiment of the present invention, a fusion protein having an amino acid sequence in which the N terminal of SA of a non-human animal species is bound to the C terminal of the amino acid sequence of GBA of a non-human animal species directly or via a linker, and having a function as GBA is expressed as "(non-human animal species) GBA - (non-human animal species) SA fusion protein". For example, a fusion protein of mouse GBA and mouse SA is expressed as a "mouse GBA-MSA fusion protein" or "mouse GBA-MSA". To a case where these fusion proteins have a function as GBA, the definition in the case of the hGBA-HSA fusion protein mentioned above is applicable. Also in these fusion proteins, SA preferably has a function as SA, such as a function to bind an endogenous substance and an exogenous substance such as a drug in the blood and transport them, but is not limited to this.

In an embodiment of the present invention, a preferable hGBA-HSA fusion protein has an amino acid sequence represented by, for example, SEQ ID NO: 41. The hGBA-HSA fusion protein represented by SEQ ID NO: 41 is a fusion protein in which a wild-type HSA is bound to the C terminal of a wild-type hGBA via a linker sequence having the amino acid sequence represented by SEQ ID NO: 9. The hGBA-HSA fusion protein represented by SEQ ID NO: 41 is encoded by a gene having a nucleotide sequence represented by, for example, SEQ ID NO: 42. Also, a fusion protein having an amino acid sequence represented by SEQ ID NO: 41 and further having a mutation such as a substitution with different amino acid residues, a deletion, or an addition of one or more amino acid residues therein is included in the hGBA-HSA fusion protein as long as it has a function as hGBA. The hGBA-HSA fusion protein preferably has a function as human serum albumin, such as a function to bind an endogenous substance and an exogenous substance such as a drug in the blood and transport them, but is not limited to this.

In an embodiment of the present invention, the term "mGBA-MSA fusion protein" or "mGBA-MSA" refers to a fusion protein having an amino acid sequence in which the N terminal of the amino acid sequence of MSA is bound to the C terminal of the amino acid sequence of mGBA directly or via a linker, and having a function as mGBA. A preferable mGBA-MSA fusion protein has the amino acid sequence represented by, for example, SEQ ID NO: 47. The mGBA-MSA fusion protein represented by SEQ ID NO: 47 is a fusion protein in which wild-type MSA is bound to the C terminal of a wild-type mGBA via a linker sequence having the amino acid sequence represented by SEQ ID NO: 9. The mGBA-MSA fusion protein represented by SEQ ID NO: 47 is encoded by a gene having a nucleotide sequence represented by, for example, SEQ ID NO: 48. Also, a fusion protein having an amino acid sequence represented by SEQ ID NO: 47 and further having a mutation such as a substitution with different amino acid residues, a deletion, or an addition of one or more amino acid residues therein is included in the mGBA-MSA fusion protein as long as it has a function as an mGBA. The mGBA-MSA fusion protein preferably has a function as mouse serum albumin, such as a function to bind an endogenous substance and an exogenous substance such as a drug in the blood and transport them, but is not limited to this.

In a case where a mutation is introduced to an hGBA-HSA fusion protein, which is a fusion protein of a wild-type hGBA and a wild-type HSA, the mutation can be introduced only to the hGBA moiety and not introduced to the HSA moiety; the mutation can be introduced only to the HSA moiety without being introduced to the hGBA moiety; or the mutation can be introduced to both of the hGBA moiety and the HSA moiety. In a case where a mutation is introduced only to the hGBA moiety, the amino acid sequence of the moiety is an amino acid sequence of hGBA obtained by introducing a mutation to a wild-type hGBA as mentioned above. In a case where a mutation is introduced only to the HSA moiety, the amino acid sequence of the moiety is an amino acid sequence of HSA obtained by introducing a mutation to a wild-type HSA as mentioned above. In a case where a mutation is introduced to both of the hGBA moiety and the HSA moiety, the amino acid sequence of the hGBA moiety is an amino acid sequence of hGBA obtained by introducing a mutation to a wild-type hGBA as mentioned above, and the amino acid sequence of the HSA moiety is an amino acid sequence of HSA obtained by introducing a mutation to a wild-type HSA as mentioned above. The same applies to the hGBA-HSA fusion protein having the amino acid sequence represented by SEQ ID NO: 41. Also, the same applies to the mGBA-MSA fusion protein including one having the amino acid sequence represented by SEQ ID NO: 47. The mGBA-MSA fusion protein having the amino acid sequence represented by SEQ ID NO: 47 is encoded by a gene having a nucleotide sequence represented by, for example, SEQ ID NO: 48. The same applies to a fusion protein of a wild-type hGBA and a wild-type SA (including wild-type MSA) of a non-human animal species.

The case where a mutation is introduced to an hGBA-HSA fusion protein having the amino acid sequence represented by SEQ ID NO: 41 will be described below. When an amino acid residue in the amino acid sequence represented by SEQ ID NO: 41 is substituted with another amino acid residue, the number of amino acid residues to be substituted is 1 to 10, 1 to 5, or 1 to 3, for example, one or two. When an amino acid residue is deleted, the number of amino acid residues to be deleted is 1 to 10, 1 to 5, or 1 to 3, for example, one or two. When an amino acid residue is added, one or more amino acid residues are added in the amino acid sequence represented by SEQ ID NO: 41 or added to the N terminal or C terminal of the amino acid sequence thereof. The hGBA-HSA fusion protein may have a combination of these substitution, deletion, and addition of amino acid residues. A mutation may be introduced only to the HSA moiety, only to the hGBA moiety, or to both of them. The same applies to a fusion protein (hGBA-SA) of hGBA and SA of a non-human animal species and a fusion protein of GBA of a non-human animal species and SA of a non-human animal species, for example, mouse GBA-MSA fusion protein.

When mutations are introduced to the amino acid sequence represented by SEQ ID NO: 41, the sites and types (deletion, substitution, and addition) of individual mutations can be easily identified by alignment of the amino acid sequences before and after introduction of the mutations. The amino acid sequence having a mutation introduced thereto exhibits an identity of preferably 80% or more, an identity of 85% or more, an identity of 90% or more, or an identity of 95% or more, for example, an identity of 98% or more or an identity of 99% or more, to the amino acid sequence represented by SEQ ID NO: 41.

An hGBA-HSA fusion protein having a constituent amino acid modified with a sugar chain is regarded as an hGBA-HSA fusion protein. Also, an hGBA-HSA fusion protein having a constituent amino acid modified with a phosphate group is regarded as an hGBA-HSA fusion protein. Also, an hGBA-HSA fusion protein modified with a group except a sugar chain and a phosphate group is regarded as an hGBA-HSA fusion protein. Also, hGBA-HSA fusion protein having a constituent amino acid whose side chain is converted by, e.g., a substitution reaction, is regarded as an hGBA-HSA fusion protein. Examples of such conversion include, but are not limited to, conversion of a cysteine residue to formylglycine. The same applies to a fusion protein (hGBA-SA) of hGBA and SA of a non-human animal species, and a fusion protein of GBA of a non-human animal species and SA of a non-human animal species, for example, a mouse GBA-MSA fusion protein.

More specifically, an hGBA-HSA fusion protein modified with a sugar chain is meant to be included in an hGBA-HSA fusion protein having an original amino acid sequence. Also, an hGBA-HSA fusion protein modified with a phosphate group is meant to be included in an hGBA-HSA fusion protein having an original amino acid sequence. Also, an hGBA-HSA fusion protein modified with a group except a sugar chain and a phosphate group is meant to be included in an hGBA-HSA fusion protein having an original amino acid sequence. Also, an hGBA-HSA fusion protein having a constituent amino acid whose side chain is converted by, e.g., a substitution reaction, is meant to be included in an hGBA-HSA fusion protein having an original amino acid sequence. Examples of such conversion include, but are not limited to, conversion of a cysteine residue to formylglycine. The same applies to a fusion protein (hGBA-SA) of hGBA and SA of a non-human animal species, and a fusion protein of GBA of a non-human animal species and SA of a non-human animal species, for example, a GBA-MSA fusion protein.

Also, an hGBA-HSA fusion protein in which a constituent hGBA is a precursor of an hGBA is regarded as an HSA-hGALC fusion protein.

In an embodiment of the present invention, the term "fusion protein of SA and lysosome enzyme", "fusion protein of lysosome enzyme and SA" or "fusion protein of serum albumin and lysosome enzyme", is meant to include both "SA-lysosome enzyme fusion protein" and "lysosome enzyme-SA fusion protein" as mentioned above. Also, the term "fusion protein of SA and GALC", "fusion protein of GALC and SA", or "fusion protein of serum albumin and galactosylceramidase", is meant to include both the "SA-GALC fusion protein" and the "GALC-SA fusion protein" as mentioned above. Also, the term "fusion protein of SA and GBA", "fusion protein of GBA and SA", or "fusion protein of serum albumin and glucocerebrosidase", is meant to include both the "SA-GBA fusion protein" and the "GBA-SA fusion protein" as mentioned above. The same applies to a case where SA is HSA; a case where a lysosome enzyme is a human lysosome enzyme; a case where GALC is hGALC; and a case where GBA is hGBA.

Now, a method for producing a fusion protein of SA and a lysosome enzyme will be more specifically described by way of a fusion protein of HSA and hGALC and a fusion protein of HSA and hGBA, below. The following description can apply to fusion proteins using SA derived from non-human species and using a lysosome enzyme other than GALC and GBA. For example, the following description can be applied to a fusion protein of SA of a non-human animal species and hGALC or hGBA, a fusion protein of SA of a non-human animal species and GALC or GBA of a non-human animal species, and a fusion protein of MSA and mGALC or mGBA.

In the fusion protein according to an embodiment of the present invention, SA and a lysosome enzyme are bound directly or via a linker. The "linker" used herein refers to a structure to be used for binding two types of proteins or to an independent moiety not belonging to any one of the proteins when two types or more of proteins are fused. Examples of the linker include peptide linkers and non-peptide linkers. A peptide linker constituting a part of a fusion protein can be called simply as a linker. The amino acid sequence of a peptide linker can be referred to as a linker sequence. In a fusion protein of SA and a lysosome enzyme, the linker refers to a moiety belonging to neither the amino acid sequence of SA nor the amino acid sequence of the lysosome enzyme. More specifically, the linker is a peptide chain present between SA and the lysosome enzyme. The linker has various functions. Examples of the function of the linker include a function to bind SA and a lysosome enzyme by being interposed between SA and a lysosome enzyme, a function to reduce mutual interference between SA and a lysosome enzyme by keeping an intramolecular distance within a fusion protein, and a function to serve as a hinge for connecting SA and a lysosome enzyme by being interposed between SA and a lysosome enzyme, thereby forming a flexible three dimensional structure of a fusion protein. The linker is present within a molecule of a fusion protein and exerts at least one of these functions. The same applies to a case where SA is HSA and a case where the lysosome enzyme is human lysosome enzyme such as hGALC or hGBA.

In the fusion protein of SA and a lysosome enzyme, the amino acid sequence of a linker is not particularly limited as long as it is present within the fusion protein molecule and produces a function as a linker. The length of a peptide linker is not particularly limited as long as it is present within a fusion protein molecule and produces a function as a linker. A peptide linker is constituted of one or more amino acids. In a case where a peptide linker is constituted of a plurality of amino acids, the number of the amino acids is preferably 2 to 50, more preferably 5 to 30, and further preferably 10 to 25. Suitable examples of a peptide linker include peptide linkers constituted of Gly-Ser, Gly-Gly-Ser, or the amino acid sequences represented by SEQ ID NOs: 9 to 11 (these are collectively referred to as a basic sequence), and peptide linkers containing these. For example, a peptide linker contains an amino acid sequence having 2 to 10, 2 to 6 or 3 to 5 repeats of the basic sequence. These amino acid sequences may have, e.g., a deletion, a substitution or addition of one or more amino acids. In a case where an amino acid is deleted, the number of amino acids to be deleted is preferably 1 or 2. In a case where an amino acid is substituted with a different one, the number of amino acids to be substituted is preferably 1 or 2. In a case where an amino acid is added, the number of amino acids to be added is preferably 1 or 2. The amino acid sequence of a desired linker moiety can be prepared by using a combination of these deletion, substitution, and addition of amino acids. A peptide linker may be constituted of a single amino acid, and the amino acid constituting the linker is, for example, glycine and serine. The same applies to a case where SA is HSA, and a case where the lysosome enzyme is a human lysosome enzyme such as hGALC or hGBA.

A fusion protein of HSA and hGALC can be prepared as a recombinant protein by preparing an expression vector integrating a DNA fragment in which a gene encoding hGALC is bound to a downstream or upstream site of a gene encoding HSA in frame, and culturing a host cell transformed by introduction of the expression vector. Also, a fusion protein of HSA and hGBA can be prepared as a recombinant protein by preparing an expression vector integrating a DNA fragment in which a gene encoding hGBA is bound to a downstream or upstream site of a gene encoding HSA in frame, and culturing a host cell transformed by introduction of the expression vector. The fusion protein prepared as a recombinant protein in this manner is constituted of a single-chain polypeptide. Also, in the present invention, the fusion protein prepared as a recombinant protein is referred to as a recombinant fusion protein.

In a case where an HSA-hGALC fusion protein is prepared as a recombinant fusion protein, a fusion protein having the amino acid sequence of hGALC at the C terminal of the amino acid sequence of HSA can be obtained by binding a gene encoding hGALC to a downstream site of a gene encoding HSA in frame. Conversely, an hGALC-HSA fusion protein having the amino acid sequence of hGALC at the N terminal of the amino acid sequence of HSA can be obtained by binding a gene encoding hGALC to an upstream site of a gene encoding HSA in frame. Also, an HSA-hGBA fusion protein having the amino acid sequence of hGBA at the C terminal of the amino acid sequence of HSA can be obtained by binding a gene encoding hGBA to a downstream site of a gene encoding HSA in frame. Conversely, an hGBA-HSA fusion protein having the amino acid sequence of hGBA at the N terminal of the amino acid sequence of HSA can be obtained by binding a gene encoding hGBA to an upstream site of a gene encoding HSA in frame. In either case, the fusion protein prepared as a recombinant fusion protein is a single-chain polypeptide.

Note that, in the present invention, the term "single-chain polypeptide" refers to a polypeptide having a single N terminal and a single C terminal and having no branched peptide chain. As long as a polypeptide satisfies the above condition, a polypeptide having an intramolecular disulfide bond, and a polypeptide modified with a sugar chain, a lipid or a phospholipid are single-chain polypeptides. Also, in a case where single-chain polypeptides are bound via a non-covalent bond to form a complex such as a dimer, the individual peptide chains forming the complex are understood as single-chain polypeptides and the complex itself is understood as an assemble of single-chain polypeptides.

In a single-chain polypeptide of a fusion protein, if the amino acid sequence of HSA is positioned on the N terminal side of the amino acid sequence of hGALC or hGBA, the C terminal of HSA and the N terminal of hGALC or hGBA are bound directly by a peptide bond, or via a linker. Figure 1 schematically shows an HSA-hGALC fusion protein of a single-chain polypeptide having HSA, a linker and hGALC in this order from the N terminal side. The HSA-hGALC fusion protein is a fusion protein in which the C terminal of HSA and the N terminal of the linker are bound by a peptide bond, and the C terminal of the linker and the N terminal of hGALC are bound by a peptide bond. Figure 2 schematically shows an HSA-hGBA fusion protein of a single-chain polypeptide having HSA, a linker and hGBA in this order from the N terminal side. The HSA-hGBA fusion protein is a fusion protein in which the C terminal of HSA and the N terminal of the linker are bound by a peptide bond, and the C terminal of the linker and the N terminal of hGBA are bound by a peptide bond.

Also, in a single-chain polypeptide of a fusion protein, if the amino acid sequence of hGALC or hGBA is positioned on the N terminal of the amino acid sequence of HSA, the C terminal of hGALC or hGBA and the N terminal of HSA are bound directly by a peptide bond, or via a linker. Figure 3 schematically shows an hGALC-HSA fusion protein of a single-chain polypeptide having hGALC, a linker and HSA in this order from the N terminal side. The hGALC-HSA fusion protein is a fusion protein in which the C terminal of hGALC and the N terminal of the linker are bound by a peptide bond, and the C terminal of the linker and the N terminal of HSA are bound by a peptide bond. Figure 4 schematically shows an hGBA-HSA fusion protein of a single-chain polypeptide having hGBA, a linker and HSA in this order from the N terminal side. The hGBA-HSA fusion protein is a fusion protein in which the C terminal of hGBA and the N terminal of the linker are bound by a peptide bond, and the C terminal of the linker and the N terminal of HSA are bound by a peptide bond.

In an embodiment of the present invention, a fusion protein of HSA and hGALC is meant to be characterized in that the fusion protein, when expressed as a recombinant protein in a host cell, particularly expressed as a recombinant protein so as to be secreted from the cell and accumulated in a culture solution, provides an expression level as hGALC in the culture supernatant in terms of concentration or enzyme activity, that is, for example, at least 1.1 times or more, 1.2 times or more, 1.5 times or more, 2 times or more, 2.5 times or more, or 3.5 times or more, for example, 1.1 to 4 times, 1.5 to 3.6 times, or 2 to 3.6 times that of a wild-type hGALC expressed as a recombinant protein in a host cell under the same conditions. Also, in an embodiment of the present invention, a fusion protein of HSA and hGBA is meant to be characterized in that the fusion protein, when expressed as a recombinant protein in a host cell, particularly expressed as a recombinant protein so as to be secreted from the cell and accumulated in a culture solution, provides an expression level as hGBA in the culture supernatant in terms of concentration or enzyme activity, that is, for example, at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, or 10 to 20 times that of a wild-type hGBA expressed as a recombinant protein in a host cell under the same conditions. The same conditions mean that the expression vector, host cell, culture conditions and others are the same. A preferable host cell to be used herein is a mammalian cell such as a CHO cell or an *NS*/*0* cell, in particular a CHO cell.

As preferable embodiments of such a fusion protein of HSA and hGALC, the following (1) and (2) are mentioned:
(1) a fusion protein having the amino acid sequence represented by SEQ ID NO: 5, in which the N terminal of the amino acid sequence of a wild-type hGALC represented by SEQ ID NO: 1 is bound to the C terminal of the amino acid sequence of a wild-type HSA represented by SEQ ID NO: 3 via a linker represented by Gly-Ser; and
(2) a fusion protein having the amino acid sequence represented by SEQ ID NO: 7, in which the N terminal of the amino acid sequence of a wild-type HSA represented by SEQ ID NO: 3 is bound to the C terminal of the amino acid sequence of a wild-type hGALC represented by SEQ ID NO: 1 via a linker represented by Gly-Ser.

Also, as preferable embodiments of such a fusion protein of HSA and hGBA, the following (1) and (2) are mentioned:
(1) a fusion protein having the amino acid sequence represented by SEQ ID NO: 39, in which the N terminal of the amino acid sequence of a wild-type hGBA represented by SEQ ID NO: 37 is bound to the C terminal of the amino acid sequence of a wild-type HSA represented by SEQ ID NO: 3 via a linker having an amino acid sequence represented by SEQ ID NO: 9; and
(2) a fusion protein having the amino acid sequence represented by SEQ ID NO: 41, in which the N terminal of the amino acid sequence of a wild-type HSA represented by SEQ ID NO: 3 is bound to the C terminal of the amino acid sequence of a wild-type hGBA represented by SEQ ID NO: 37 via a linker having an amino acid sequence represented by SEQ ID NO: 9.

In an embodiment of the present invention, the fusion protein of HSA and hGALC is characterized in that the fusion protein, when expressed as a recombinant protein in a host cell, particularly expressed as a recombinant protein so as to be secreted from the cell and accumulated in a culture solution, provides an increased expression level as hGALC in the culture supernatant in terms of concentration or enzyme activity, compared to that of a wild-type hGALC expressed as a recombinant protein in a host cell under the same conditions. Accordingly, when a fusion protein of HSA and hGALC is produced as a recombinant protein, a production efficiency thereof can be increased compared to a wild-type hGALC, and thus a production cost of the fusion protein can be reduced. Also, in an embodiment of the present invention, the fusion protein of HSA and hGBA is characterized in that the fusion protein, when expressed as a recombinant protein in a host cell, particularly expressed as a recombinant protein so as to be secreted from the cell and accumulated in a culture solution, provides an increased expression level as hGBA in the culture supernatant in terms of concentration or enzyme activity, compared to that of a wild-type hGBA expressed as a recombinant protein in a host cell under the same conditions. Accordingly, when a fusion protein of HSA and hGBA is produced as a recombinant protein, a production efficiency thereof can be increased compared to a wild-type hGBA, and thus a production cost of the fusion protein can be reduced.
Note that, it is known that medicaments containing a recombinant protein as an active ingredient are very expensive. Accordingly, if the amount of a recombinant protein produced under the same conditions is increased by several percent, for example, 3 to 9%, a large economic effect is produced. The same applies to a human lysosome enzyme such as hGALC or hGBA.

Note that, in the specification, when the expression level of a fusion protein of HSA and hGALC expressed in a host cell as a recombinant protein is compared to that of a wild-type hGALC expressed in a host cell as a recombinant protein under the same condition, comparison is preferably made not based on the mass of a protein expressed but based on the GALC enzyme activity of the protein expressed. The same applies to expression levels of a fusion protein of HSA and hGBA, a fusion protein of SA of a non-human animal species and hGALC or hGBA, and a fusion protein of SA of a non-human animal species and GALC or GBA of a non-human animal species, for example, a fusion protein of MSA and mGALC or mGBA.

A fusion protein of HSA and hGALC and a fusion protein of HSA and hGBA can be produced as a recombinant protein by culturing a host cell transformed with an expression vector integrating a gene encoding each of the fusion proteins.

The host cell used herein is not particularly limited as long as it can express a fusion protein of HSA and hGALC and a fusion protein of HSA and hGBA by introducing each of the expression vectors thereto. Any eukaryotic cell such as a mammalian cell, a yeast cell, a plant cell, and an insect cell can be used but a mammalian cell is particularly preferable.

When a mammalian cell is used as a host cell, the type of the mammalian cell is not particularly limited and a cell derived from a human, a mouse or a Chinese hamster is preferable, particularly a CHO cell derived from the ovary of a Chinese hamster, or a NS/0 cell derived from the mouse myeloma is preferable. Also, the expression vector to be used here for expression by integrating a DNA fragment containing a gene encoding a fusion protein of HSA and hGALC or a fusion protein of HSA and hGBA can be used without limitation as long as it can express the gene when it is introduced in a mammalian cell. The gene integrated in the expression vector is arranged downstream of a DNA sequence (gene expression regulatory site) capable of regulating the frequency of transcription of the gene in a mammalian cell. In the present invention, examples of the gene expression regulatory site that can be used include a cytomegalovirus-derived promoter, an SV40 early promoter, a human elongation factor-1α (EF-1α) promoter, and a human ubiquitin C promoter.

An expression vector prepared by arranging glutamine synthetase (GS) as a selection marker on the downstream side of a gene encoding a desired protein via an internal ribosome entry site (IRES) is known (International Patent Publications WO2012/063799, WO2013/161958). The expression vectors described in these literatures can be particularly suitably used for producing a fusion protein of HSA and hGALC or a fusion protein of HSA and hGBA.

Note that, in an embodiment of the present invention, the term "internal ribosome entry site" refers to a region (structure), which is present in a mRNA chain and to which a ribosome directly binds and can initiate translation in a cap-structure independent manner, or a region (structure) of a DNA chain, which is transcribed to provide the region of the mRNA chain. Also, in the present invention, the term "gene encoding an internal ribosome entry site" refers to a region (structure) of a DNA chain, which is transcribed to produce the region of the mRNA chain. The internal ribosome entry site is generally referred to as IRES and found in the 5' non-translated region of a virus such as *Picornaviridae* virus (e.g., poliovirus, rhinovirus, mouse encephalomyocarditis virus), a foot-and-mouth disease virus, hepatitis A virus, hepatitis C virus, coronavirus, bovine enteric virus, Theiler's murine encephalomyelitis virus, and Coxsackie B virus; and 5' non-translated region of genes such as human immunoglobulin heavy chain binding protein, *drosophila* antennapedia and *drosophila ultrabithorax.* In the case of picornavirus, the IRES is a region formed of about 450 bp and present in the 5' non-translated region of mRNA. The term "5' non-translated region of a virus" refers to a 5' non-translated region of mRNA of a virus or a region (structure) of a DNA chain, which is transcribed to form the region of the mRNA chain.

For example, an expression vector for expressing a desired protein, which contains a first gene expression regulatory site, a gene encoding the protein downstream thereof, an internal ribosome entry site further downstream and a gene encoding glutamine synthetase further downstream, and further contains a dihydrofolate reductase gene or a drug-resistant gene downstream the first gene expression regulatory site or another regulatory site, i.e., a second gene expression regulatory site, can be suitably used for production of a fusion protein of HSA and hGALC or a fusion protein of HSA and hGBA. In the expression vector, as the first gene expression regulatory site or second gene expression regulatory site, a cytomegalovirus-derived promoter, an SV40 early promoter, a human elongation factor-1α promoter (hEF-1α promoter) or a human ubiquitin C promoter can be suitably used, but an hEF-1α promoter is particularly preferable.

As the internal ribosome entry site, a site derived from the genome of a virus selected from the group consisting of *Picornaviridae* virus (including mouse encephalomyocarditis virus), foot-and-mouth disease virus, hepatitis A virus, hepatitis C virus, a coronavirus, a bovine enteric virus, Theiler's murine encephalomyelitis virus, and Coxsackie B virus, or a site derived from the 5' non-translated region of a gene selected from the group consisting of a gene of a human immunoglobulin heavy-chain binding protein, a *drosophila* antennapedia gene, and a *drosophila ultrabithorax* gene is suitably used but an internal ribosome entry site derived from the 5' non-translated region of the mouse encephalomyocarditis virus genome is particularly preferable. In a case where the internal ribosome entry site derived from the 5' non-translated region of the mouse encephalomyocarditis virus genome is used, not only a wild-type one but also a wild-type internal ribosome entry site having a plurality of initiation codons a part of which is destroyed can be suitably used. Also, in the expression vector, the drug-resistant gene suitably used is preferably a puromycin or neomycin resistant gene, and more preferably a puromycin resistant gene.

Also, for example, an expression vector for expressing a desired protein, which contains a human elongation factor-1α promoter, a gene encoding the protein downstream thereof, an internal ribosome entry site derived from the 5' non-translated region of a mouse encephalomyocarditis virus genome further downstream and a gene encoding glutamine synthetase further downstream, an expression regulatory site for another gene, and a dihydrofolate reductase gene further downstream, and in which the internal ribosome entry site is a wild-type internal ribosome entry site having a plurality of initiation codons a part of which is destroyed, can be suitably used for production of a fusion protein of HSA and hGALC or a fusion protein of HSA and hGBA. Examples of such an expression vector include an expression vector described in WO2013/161958.

For example, an expression vector for expressing a desired protein, which contains a human elongation factor-1α promoter, a gene encoding the protein downstream thereof, an internal ribosome entry site derived from the 5' non-translated region of a mouse encephalomyocarditis virus genome further downstream, a gene encoding glutamine synthetase further downstream, an expression regulatory site for another gene, and a drug-resistant gene further downstream, and in which the internal ribosome entry site is a wild-type internal ribosome entry site having a plurality of initiation codons a part of which is destroyed, can be suitably used for production of a fusion protein of HSA and hGALC or a fusion protein of HSA and hGBA. Examples of such an expression vector include pE-mIRES-GS-puro described in WO2012/063799 and pE-mIRES-GS-mNeo described in WO2013/161958.

In the 3' terminal of the internal ribosome entry site derived from the 5' non-translated region of a wild-type mouse encephalomyocarditis virus genome, three initiation codons (ATG) are present. The above pE-mIRES-GS-puro and pE-mIRES-GS-mNeo are expression vectors having IRES whose initiation codons are partly defective.

A fusion protein of HSA and hGALC or a fusion protein of HSA and hGBA can be expressed in a cell or a medium by culturing a host cell to which an expression vector integrating a gene encoding the fusion protein is introduced. In a case where a mammalian cell is a host cell, a method for expressing a fusion protein of HSA and hGALC or a fusion protein of HSA and hGBA will be more specifically described below.

As a medium for culturing a mammalian cell, any medium can be used without particular limitation as long as a mammalian cell can be proliferated by culturing, and preferably a serum-free medium is used. In the present invention, as the serum-free medium to be used as a culture medium for producing a recombinant protein, a medium containing, for example, amino acids (3 to 700 mg/L), vitamins (0.001 to 50 mg/L), monosaccharides (0.3 to 10 g/L), inorganic salts (0.1 to 10000 mg/L), trace elements (0.001 to 0.1 mg/L), nucleosides (0.1 to 50 mg/L), fatty acids (0.001 to 10 mg/L), biotin (0.01 to 1 mg/L), hydrocortisone (0.1 to 20 µg/L), insulin (0.1 to 20 mg/L), vitamin B12 (0.1 to 10 mg/L), putrescine (0.01 to 1 mg/L), sodium pyruvate (10 to 500 mg/L), and a water-soluble iron compound is suitably used. If desired, e.g., thymidine, hypoxanthine, and a pH indicator and antibiotic substance routinely used may be added to the medium.

As a serum-free medium to be used as a culture medium for producing a recombinant protein, DMEM/F12 medium (mixed medium of DMEM and F12) may be used as a basal medium, and these media are well known to those skilled in the art. Furthermore, as a serum-free medium, DMEM (HG) HAM improved (R5) medium containing sodium bicarbonate, L-glutamine, D-glucose, insulin, sodium selenite, diaminobutane, hydrocortisone, iron (II) sulfate, asparagine, aspartic acid, serine and polyvinyl alcohol may be used. Moreover, a commercially available serum-free medium, for example, CD OptiCHO^{™} medium, CHO-S-SFM II medium or CD CHO medium (Thermo Fisher Scientific), EX-CELL^{™}302 medium or EX-CELL^{™}325-PF medium (SAFC Biosciences), can be used as a basal medium.

The fusion protein of HSA and hGALC is characterized in that the fusion protein, when expressed as a recombinant protein by culturing a host cell to which an expression vector integrating a gene encoding the fusion protein is introduced, in the serum-free medium mentioned above, particularly expressed as a recombinant protein so as to be secreted from the cell and accumulated in a culture solution, provides an expression level as the hGALC in the culture supernatant in terms of concentration or enzyme activity, that is, for example, at least 1.1 times or more, 1.2 times or more, 1.5 times or more, 2 times or more, 2.5 times or more, or 3.5 times or more, for example, 1.1 to 4 times, 1.5 to 3.6 times, or 2 to 3.6 times that of a wild-type hGALC expressed as a recombinant protein in a host cell under the same conditions.

The fusion protein of HSA and hGBA is characterized in that the fusion protein, when expressed as a recombinant protein by culturing a host cell to which an expression vector integrating a gene encoding the fusion protein is introduced, in the serum-free medium mentioned above, particularly expressed as a recombinant protein so as to be secreted from the cell and accumulated in a culture solution, provides an expression level as the hGBA in the culture supernatant in terms of concentration or enzyme activity, that is, for example, at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times or 10 to 20 times that of a wild-type hGBA expressed as a recombinant protein in a host cell under the same conditions. The same conditions mean that the expression vector, host cell, culture conditions and others are the same. The host cell to be used herein is preferably a mammalian cell, particularly, a cell usually used for production of a recombinant protein, such as a CHO cell or an NS/0 cell.

When a wild-type hGALC is expressed as a recombinant by using a host cell transformed with an expression vector integrating a gene encoding it, it is difficult to effectively produce the recombinant since, for example, the survival rate of the host cell reduces. However, when hGALC is expressed as a fusion protein with HSA, the reduction in the survival rate of the host cell observed when a wild-type hGALC is expressed is suppressed. A fusion protein of HSA and hGALC, when expressed as a recombinant protein by culturing a mammalian cell to which an expression vector integrating a gene encoding it is introduced in a serum-free medium, particularly expressed as a recombinant protein so as to be secreted from the cell and accumulated in a culture solution, provides an increased expression level, compared to that of the wild-type hGALC expressed as a recombinant protein under the same conditions. It is considered that the suppression of a reduction in the survival rate contributes to the increase of an expression level. The same applies to a fusion protein of SA of a non-human animal species and hGALC, and a fusion protein of SA of a non-human animal species and GALC of a non-human animal species. Note that, the host cell to be used for expression of the fusion protein is preferably a mammalian cell, particularly, a cell usually used for production of a recombinant protein, such as a CHO cell or an NS/0 cell. More specifically, an embodiment of the present invention is a recombinant fusion protein of SA and GALC, particularly a recombinant fusion protein of HSA and hGALC.

A reduction in the survival rate of a host cell when hGALC and hGBA are expressed means an increase of dead cells. When cells are dead, the contents thereof flow out and become foreign substances. Thus, after the expression of a fusion protein, a purification step for removing foreign substances is required. More specifically, when hGALC and hGBA are expressed in the form of a fusion protein with HSA, the number of dead cells can be reduced, thereby reducing foreign substances produced during culturing, with the result that purification of the expressed fusion protein can be easily made. In this manner, the purification efficiency during a purification can be enhanced. Also, the amount of foreign substance contained in a fusion protein purified can be reduced.

A fusion protein of HSA and hGALC or a fusion protein of HSA and hGBA can be expressed within a cell or in a medium by culturing a host cell encoding the fusion protein. These fusion proteins can be purified by separating them from foreign substances by a method such as column chromatography. The fusion protein of HSA and hGALC or fusion protein of HSA and hGBA purified can be used as a pharmaceutical composition. Particularly, a fusion protein of HSA and hGALC can be used as a pharmaceutical composition targeted to Krabbe disease (galactosylceramide lipidosis, or globoid cell leukodystrophy). Particularly, a fusion protein of HSA and hGBA can be used as a pharmaceutical composition targeted to Gaucher's disease. Note that, the term of a pharmaceutical composition referred to in the specification means a composition containing a pharmaceutically acceptable excipient in addition of a fusion protein as an active ingredient.

A pharmaceutical composition containing a fusion protein of HSA and hGALC or a fusion protein of HSA and hGBA as an active ingredient can be administered intravenously, intramuscularly, intraperitoneally, subcutaneously or intracerebroventricularly as an injection. Such an injection can be supplied as a lyophilized preparation or an aqueous liquid. The aqueous liquid may be in a form it is filled in a vial or supplied as a prefilled preparation in a syringe. The lyophilized preparation is dissolved and restored in an aqueous medium before use and then used. In a fusion protein of HSA and hGALC or a fusion protein of HSA and hGBA contained in an aqueous liquid, the ratio (monomer (mass)/total protein (mass) × 100 (%)) of a monomer in the total protein is preferably 70% or more, more preferably 80% or more, further preferably 90% or more, for example, 95% or more, or 95% or more. The same applies to a solution prepared by dissolving and restoring a lyophilized preparation in an aqueous medium.

A fusion protein of HSA and hGALC or a fusion protein of HSA and hGBA can further form a conjugate with an antibody or a ligand. For example, a fusion protein of HSA and hGALC in an embodiment of the present invention can form a conjugate with an antibody or a ligand capable of specifically binding to a receptor on a cerebrovascular endothelial cell. Also, for example, a fusion protein of HSA and hGBA in an embodiment of the present invention can form a conjugate with an antibody or a ligand capable of specifically binding to a receptor on a cerebrovascular endothelial cell. A fusion protein of HSA and hGALC or a fusion protein of HSA and hGBA in the form of a conjugate with an antibody or a ligand capable of specifically binding to a receptor on a cerebrovascular endothelial cell can bind to a receptor on a cerebrovascular endothelial cell. The fusion protein bound to a receptor on a cerebrovascular endothelial cell can pass through the blood-brain barrier (BBB) to reach the tissue of the central nervous system (CNS). Thus, a fusion protein of HSA and hGALC or a fusion protein of HSA and hGBA in the form of a conjugate with such an antibody or a ligand, can pass through the blood-brain barrier (BBB) and exert a function thereof in the central nervous system (CNS).

The term "ligand" used in the present invention refers to a substance having an affinity for a predetermined substance, in particular, a protein having an affinity for a predetermined substance. In an example of the present invention, such a ligand is a substrate having affinity for a receptor present on a cerebrovascular endothelial cell, particularly a protein. Examples of the receptor present on a cerebrovascular endothelial cell include, but are not limited to, an insulin receptor, a transferrin receptor, a leptin receptor, a lipoprotein receptor, and an IGF receptor, particularly a transferrin receptor being mentioned, but are not limited to these. Also, the receptor is preferably a human-derived receptor. The ligands to an insulin receptor, a transferrin receptor, a leptin receptor, a lipoprotein receptor, and an IGF receptor are insulin, transferrin, leptin, lipoprotein, and IGF (IGF-1 and IGF-2), respectively. These ligands may be full length or a fragment thereof as long as they have an affinity for a receptor thereof and may be a wild-type or a mutant having a substitution, deletion, or/and addition introduction in a wild type.

In a conjugate of a fusion protein of HSA and hGALC with an antibody, the phrase that hGALC has a function as an hGALC means that hGALC has a specific activity of preferably 10% or more, more preferably 20% or more, further preferably 50% or more, further more preferably 80% or more, for example, 90% or more, or 95% or more when the specific activity of a normal wild-type hGALC is regarded as 100%. Note that, the specific activity of the hGALC in the conjugate herein is computationally obtained by multiplying the enzyme activity (µM/h/mg protein) of an hGALC per unit mass of the conjugate by (the molecular weight of the conjugate/the molecular weight of the moiety corresponding to the hGALC in the conjugate).

In a conjugate of a fusion protein of HSA and hGBA with an antibody, the phrase that hGBA has a function as hGBA means that hGBA has a specific activity of preferably 10% or more, more preferably 20% or more, further preferably 50% or more, further more preferably 80% or more, for example, 90% or more, or 95% or more when the specific activity of a normal wild-type hGBA is regarded as 100%. Note that, the specific activity of the hGBA in the conjugate herein is computationally obtained by multiplying the enzyme activity (µM/h/mg protein) of hGBA per unit mass of the conjugate by (the molecular weight of the conjugate/the molecular weight of the moiety corresponding to the hGBA in the conjugate).

In an embodiment of the present invention, a conjugate of a fusion protein of HSA and hGALC with an antibody is characterized in that the conjugate, when expressed as a recombinant protein in a host cell, particularly expressed as a recombinant protein so as to be secreted from the cell and accumulated in a culture solution, provides an expression level as the hGALC in the culture supernatant in terms of concentration or enzyme activity, that is, for example, at least 1.1 times or more, 1.2 times or more, 1.5 times or more, 2 times or more, 2.5 times or more, or 3.5 times or more, for example, 1.1 to 4 times, 1.5 to 3.6 times, or 2 to 3.6 times that of a wild-type hGALC expressed as a recombinant protein in a host cell under the same conditions. Also, in an embodiment of the present invention, a conjugate of a fusion protein of HSA and hGBA with an antibody is characterized in that the conjugate, when expressed as a recombinant protein in a host cell, particularly expressed as a recombinant protein so as to be secreted from the cell and accumulated in a culture solution, provides an expression level as hGBA in the culture supernatant in terms of concentration or enzyme activity, that is, for example, at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times or 10 to 20 times that of a wild-type hGBA expressed as a recombinant protein in a host cell under the same conditions.

Some of cytokines, in particular, interleukins, when being expressed as a recombinant protein by using a host cell to which an expression vector integrating a gene encoding wild-type one is introduced, are difficult to mass-produce because of limited expression levels. IL-10 is one of such interleukins. An embodiment of the present invention is a recombinant protein as a fusion protein of such a cytokine difficult to produce as a recombinant protein and SA. The recombinant protein is easier to mass-produce by using a host cell to which an expression vector integrating a gene encoding this is introduced, than the corresponding wild-type cytokine. The animal species of a cytokine to be bound to SA herein is not particularly limited and the cytokine is preferably a human cytokine. The animal species of SA to be bound to a cytokine is not particularly limited and the SA is preferably HSA.

The term "human cytokine" as used herein simply includes indistinguishably not only a normal wild-type human cytokine but also human cytokine mutants, which correspond to human cytokine mutants having a substitution, deletion, and/or addition of one or more amino acid residues ("addition" of an amino acid residue herein means adding the residue to a terminal of a sequence or in the sequence) in the amino acid sequence of a wild-type human cytokine, as long as the mutants have a function as the human cytokine, such as a physiological activity corresponding to the type of human cytokine. The same applies to cytokines of non-human animal species. Note that, the same applies to interleukins.

Note that, the phrase that a human cytokine has a function as the human cytokine herein means that the human cytokine has a specific activity of preferably 10% or more, more preferably 20% or more, further preferably 50% or more and further more preferably 80% or more when the specific activity of a normal wild-type human cytokine is regarded as 100%. The specific activity herein refers to physiological activity per mass of the protein. Note that, the specific activity of a fusion protein of a human cytokine and a protein is obtained as a physiological activity per mass of the moiety corresponding to the human cytokine of the fusion protein. Note that, the specific activity of a human cytokine in a fusion protein herein is computationally obtained by multiplying physiological activity of a human cytokine per unit mass of the fusion protein by (the molecular weight of the fusion protein/the molecular weight of the moiety corresponding to the human cytokine in the fusion protein). The same applies to cytokines of non-human animal species. Note that, the same applies to interleukins.

When an amino acid residue in the amino acid sequence of a wild-type human cytokine is substituted with another amino acid residue, the number of amino acid residues to be substituted is 1 to 10, 1 to 5, or 1 to 3, for example, one or two. When an amino acid residue in the amino acid sequence of a wild-type human cytokine is deleted, the number of amino acid residues to be deleted is 1 to 10, 1 to 5, or 1 to 3, for example, one or two. Mutants such as a human cytokine mutant consisting of an amino acid sequence obtained by deleting a single amino acid residue from the N terminal or C terminal of a wild-type human cytokine and a human cytokine mutant consisting of an amino acid sequence obtained by deleting two amino acid residues from the N terminal or C terminal of the wild-type human cytokine are also regarded as human cytokines. Also, a mutation having the substitution and deletion of amino acid residues in combination can be introduced to the amino acid sequence of the wild-type human cytokine. The same applies to cytokines of non-human animal species. Note that, the same applies to interleukins.

When an amino acid residue is added to the amino acid sequence of a wild-type human cytokine, one or more amino acid residues are added in the amino acid sequence of a human cytokine or added to the N terminal or C terminal of the amino acid sequence thereof. The number of amino acid residues to be added herein is 1 to 10, 1 to 5, or 1 to 3, for example, one or two. Also, a mutation having the addition of amino acid residues and the substitution mentioned above in combination can be introduced to the amino acid sequence of a wild-type human cytokine or a mutation having the addition of amino acid residues and the deletion mentioned above in combination can be introduced to the amino acid sequence of a wild-type human cytokine. The same applies to cytokines of non-human animal species. Note that, the same applies to interleukins.

Further, a combination of three types of mutations, i.e., substitution, deletion and addition of the amino acid residues, can be introduced to the amino acid sequence of a wild-type human cytokine. For example, amino acid sequences obtained by deleting 1 to 10 amino acid residues from the amino acid sequence of a wild-type human cytokine, substituting 1 to 10 amino acid residues thereof with different amino acid residues and adding 1 to 10 amino acid residues thereto are regarded as human cytokines; amino acid sequences obtained by deleting 1 to 5 amino acid residues from the amino acid sequence of a wild-type human cytokine, substituting 1 to 5 amino acid residues thereof with different amino acid residues and adding 1 to 5 amino acid residues thereto are also regarded as human cytokines; amino acid sequences obtained by deleting 1 to 3 amino acid residues from the amino acid sequence of a wild-type human cytokine, substituting 1 to 3 amino acid residues thereof with different amino acid residues and adding 1 to 3 amino acid residues thereto are also regarded as human cytokines; amino acid sequences obtained by deleting 1 or 2 amino acid residues from the amino acid sequence of a wild-type human cytokine, substituting 1 or 2 amino acid residues thereof with different amino acid residues and adding 1 or 2 amino acid residues thereto are also regarded as human cytokines; and amino acid sequences obtained by deleting a single amino acid residue from the amino acid sequence of a wild-type human cytokine, substituting a single amino acid residue thereof with another amino acid residue and adding a single amino acid residue thereto are also regarded as human cytokines. The same applies to cytokines of non-human animal species. Note that, the same applies to interleukins.

The sites and types (deletion, substitution, and addition) of individual mutations in a human cytokine mutant compared to a normal wild-type human cytokine can be easily identified by alignment of amino acid sequences of these human cytokines. The same applies to cytokines of non-human animal species. Note that, the same applies to interleukins.

The amino acid sequence of a human cytokine mutant exhibits an identity of preferably 80% or more, 85% or more, 90% or more, or 95% or more, for example, an identity of 98% or more or 99% or more, to the amino acid sequence of a normal wild-type human cytokine. The same applies to cytokines of non-human animal species. Note that, the same applies to interleukins.

The term "human interleukin 10", "human IL-10", or "hIL-10" as used herein simply includes indistinguishably not only a normal wild-type hIL-10 consisting of 160 amino acid residues represented by SEQ ID NO: 24 but also hIL-10 mutants, which correspond to hIL-10 mutants having a substitution, deletion, and/or addition of one or more amino acid residues ("addition" of an amino acid residue herein means adding the residue to a terminal of a sequence or in the sequence) in the amino acid sequence represented by SEQ ID NO: 24, as long as the mutants have a function as the hIL-10, such as a physiological activity as a suppressive cytokine to tranquilize immunoreaction. The wild-type hIL-10 is encoded by, for example, a gene having the nucleotide sequence represented by SEQ ID NO: 49. The same applies to IL-10s of non-human animal species.

Note that, the phrase that an hIL-10 has a function as the hIL-10 herein means that the hIL-10 has a specific activity of preferably 10% or more, more preferably 20% or more, further preferably 50% or more and further more preferably 80% or more when the specific activity of a normal wild-type hIL-10 is regarded as 100%. The specific activity herein refers to physiological activity per mass of the protein. Note that, the specific activity of a fusion protein of hIL-10 and a protein is obtained as a physiological activity per mass of the moiety corresponding to the hIL-10 in the fusion protein. Note that, the specific activity of an hIL-10 in the fusion protein herein is computationally obtained by multiplying physiological activity of the hIL-10 per unit mass of the fusion protein by (the molecular weight of the fusion protein/the molecular weight of the moiety corresponding to the hIL-10 in the fusion protein). The same applies to IL-10s of non-human animal species.

When an amino acid residue in the amino acid sequence of a wild-type hIL-10 is substituted with another amino acid residue, the number of amino acid residues to be substituted is 1 to 10, 1 to 5, or 1 to 3, for example, one or two. When an amino acid residue in the amino acid sequence of a wild-type hIL-10 is deleted, the number of amino acid residues to be deleted is 1 to 10, 1 to 5, or 1 to 3, for example, one or two. Mutants such as an hIL-10 mutant consisting of 159 amino acid residues obtained by deleing a single amino acid residue from the N terminal or C terminal of a wild-type hIL-10 and an hIL-10 mutant consisting of 158 amino acid residues obtained by deleting two amino acid residues from the N terminal or C terminal of a wild-type hIL-10 are also regarded as hIL-10s. Also, a mutation having the substitution and deletion of amino acid residues in combination can be introduced to the amino acid sequence of a wild-type hIL-10. The same applies to IL-10s of non-human animal species.

When an amino acid residue is added to the amino acid sequence of a wild-type hIL-10, one or more amino acid residues are added in the amino acid sequence of an hIL-10 or added to the N terminal or C terminal of the amino acid sequence thereof. The number of amino acid residues to be added herein is 1 to 10, 1 to 5, or 1 to 3, for example, one or two. Also, a mutation having the addition of amino acid residues and the substitution mentioned above in combination can be introduced to the amino acid sequence of a wild-type hIL-10 or a mutation having the addition of amino acid residues and the deletion mentioned above in combination can be introduced to the amino acid sequence of a wild-type hIL-10. The same applies to IL-10s of non-human animal species.

Further, a combination of three types of mutations, i.e., substitution, deletion and addition of the amino acid residues, can be introduced to the amino acid sequence of a wild-type hIL-10. For example, amino acid sequences obtained by deleting 1 to 10 amino acid residues from the amino acid sequence of a wild-type hIL-10 represented by SEQ ID NO: 24, substituting 1 to 10 amino acid residues thereof with different amino acid residues and adding 1 to 10 amino acid residues thereto are regarded as hIL-10s; amino acid sequences obtained by deleting 1 to 5 amino acid residues from the amino acid sequence of a wild-type hIL-10 represented by SEQ ID NO: 24, substituting 1 to 5 amino acid residues thereof with different amino acid residues and adding 1 to 5 amino acid residues thereto are also regarded as hIL-10s; amino acid sequences obtained by deleting 1 to 3 amino acid residues from the amino acid sequence of a wild-type hIL-10 represented by SEQ ID NO: 24, substituting 1 to 3 amino acid residues thereof with different amino acid residues and adding 1 to 3 amino acid residues thereto are also regarded as hIL-10s; amino acid sequences obtained by deleting 1 or 2 amino acid residues from the amino acid sequence of a wild-type hIL-10 represented by SEQ ID NO: 24, substituting 1 or 2 amino acid residues thereof with different amino acid residues and adding 1 or 2 amino acid residues thereto are also regarded as hIL-10s; and amino acid sequences obtained by deleting a single amino acid residue from the amino acid sequence of a wild-type hIL-10 represented by SEQ ID NO: 24, substituting a single amino acid residue thereof with another amino acid residue and adding a single amino acid residue thereto are also regarded as hIL-10s. The same applies to IL-10s of non-human animal species.

The sites and types (deletion, substitution, and addition) of individual mutations in an hIL-10 mutant compared to a normal wild-type hIL-10 can be easily identified by alignment of the amino acid sequences of these hIL-10s. The same applies to IL-10s of non-human animal species.

The amino acid sequence of an hIL-10 mutant exhibits an identity of preferably 80% or more, 85% or more, 90% or more, or 95% or more, for example, an identity of 98% or more or 99% or more, to the amino acid sequence of a normal wild-type hIL-10 represented by SEQ ID NO: 24. The same applies to IL-10s of non-human animal species.

The substitution of an amino acid in the amino acid sequence of a wild-type human cytokine with another amino acid, the substitution of an amino acid in the amino acid sequence of a wild-type human cytokine with another amino acid, and the substitution of an amino acid in the amino acid sequence of a wild-type hIL-10 with another amino acid are each preferably a conservative amino acid substitution. The same applies to a cytokine, interleukin, and IL-10 of non-human animal species.

The wild-type or mutant human cytokine having a constituent amino acid modified with a sugar chain is regarded as a human cytokine. Also, the wild-type or mutant human cytokine having a constituent amino acid modified with a phosphate group is regarded as a human cytokine. Also, the wild-type or mutant human cytokine having a constituent amino acid modified with a group except a sugar chain and a phosphate group is regarded as a human cytokine. Also, the wild-type or mutant human cytokine having a constituent amino acid whose side chain is converted by, e.g., a substitution reaction, is regarded as a human cytokine. Examples of such conversion include, but are not limited to, conversion of a cysteine residue to formylglycine. The same applies to cytokines of non-human animal species. Note that, the same applies to interleukins and IL-10s.

More specifically, a human cytokine modified with a sugar chain is meant to be included in a human cytokine having an original amino acid sequence. Also, a human cytokine modified with a phosphate group is meant to be included in a human cytokine having an original amino acid sequence. Also, a human cytokine modified with a group except a sugar chain and a phosphate group is meant to be included in a human cytokine having an original amino acid sequence. Also, a human cytokine having a constituent amino acid whose side chain is converted by, e.g., a substitution reaction, is meant to be included in a human cytokine having an original amino acid sequence. Examples of such conversion include, but are not limited to, conversion of a cysteine residue to formylglycine. The same applies to cytokines of non-human animal species. Note that, the same applies to interleukins and IL-10s.

In an embodiment of the present invention, the wild-type human cytokine is produced as a recombinant protein obtained by fusion with HSA. The fusion protein, when expressed as a recombinant protein using a host cell such as CHO, particularly expressed as a recombinant protein so as to be secreted from the cell and accumulated in a culture solution, can provide an expression level as a human cytokine in the culture supernatant in terms of concentration or physiological activity, that is at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, or 3 to 4 times that of the wild-type human cytokine expressed as a recombinant protein in the same manner.

Also, in an embodiment of the present invention, hIL-10 is produced as a recombinant protein obtained by fusion with HSA. The fusion protein, when expressed as a recombinant protein using a host cell such as CHO, particularly expressed as a recombinant protein so as to be secreted from the cell and accumulated in a culture solution, can provide an expression level as hIL-10 in the culture supernatant in terms of concentration or physiological activity, that is at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, or 3 to 4 times that of the wild-type hIL-10 expressed as a recombinant protein in the same manner.

More specifically, an embodiment of the present invention is a fusion protein obtained by binding a polypeptide containing an amino acid sequence of a cytokine and a polypeptide containing an amino acid sequence of SA. The phrase "binding polypeptides" refers to covalently binding different polypeptides directly or indirectly via a linker. The cytokine and SA herein are preferably derived from a human.

Also, an embodiment of the present invention is a fusion protein obtained by binding a polypeptide containing an amino acid sequence of IL-10 and a polypeptide containing an amino acid sequence of SA. The phrase "binding polypeptides" refers to covalently binding different polypeptides directly or indirectly via a linker. The IL-10 and SA herein are preferably derived from a human.

As an example method for binding two different polypeptides, it is general to employ the following method: to a downstream site of a gene encoding one of the polypeptides, a gene encoding the other polypeptide is bound in frame to prepare a DNA fragment. The DNA fragment is integrated into an expression vector. A host cell is transformed with the expression vector and cultured to express a recombinant protein. The recombinant protein obtained is a single-chain polypeptide having the two polypeptides bound directly by a peptide bond, or via another amino acid sequence.

In an embodiment of the present invention, the term "SA-human cytokine fusion protein" or "SA-human cytokine" refers to a fusion protein having an amino acid sequence in which the N terminal of the amino acid sequence of a human cytokine is bound to the C terminal of the SA amino acid sequence directly or via a linker, and having a function as a human cytokine. The animal species of SA is not particularly limited and SA is preferably mammalian SA, more preferably primate SA, and further preferably HSA. The phrase that a human cytokine has a function as a human cytokine in the SA-human cytokine fusion protein means that the human cytokine has a specific activity of preferably 10% or more, more preferably 20% or more, further preferably 50% or more, and further more preferably 80% or more, when the specific activity of a normal wild-type human cytokine is regarded as 100%. Note that, the specific activity of the human cytokine in the SA-human cytokine fusion protein herein is computationally obtained by multiplying the physiological activity of a human cytokine per unit mass of the fusion protein by (the molecular weight of the fusion protein/the molecular weight of the moiety corresponding to the human cytokine in the fusion protein).

In an embodiment of the present invention, the term "HSA-human cytokine fusion protein" or "HSA-human cytokine" refers to a fusion protein having an amino acid sequence in which the N terminal of the amino acid sequence of a human cytokine is bound to the C terminal of the HSA amino acid sequence directly or via a linker, and having a function as a human cytokine. To the case where an HSA-human cytokine fusion protein has a function as a human cytokine, the definition in the case of the SA-human cytokine fusion protein mentioned above is applicable.

In the SA-human cytokine fusion protein, SA preferably has a function as SA, such as a function to bind to an endogenous substance and an exogenous substance such as a drug in the blood and transport them, but is not limited to this. The same applies to an HSA-human lysosomal enzyme fusion protein.

In a case where a mutation is introduced to an HSA-human cytokine fusion protein, which is a fusion protein of a wild-type HSA and a wild-type human cytokine, the mutation can be introduced only to the HSA moiety and not introduced to the human cytokine moiety; the mutation can be introduced only to a human cytokine moiety without being introduced to the HSA moiety; or the mutation can be introduced to both of the HSA moiety and the human cytokine moiety. In a case where a mutation is introduced only to the HSA moiety, the amino acid sequence of the moiety is an amino acid sequence of HSA obtained by introducing a mutation to a wild-type HSA as mentioned above. In a case where a mutation is introduced only to a human cytokine moiety, the amino acid sequence of the moiety is an amino acid sequence of a human cytokine obtained by introducing a mutation to a wild-type human cytokine as mentioned above. In a case where a mutation is introduced to both of the HSA moiety and the human cytokine moiety, the amino acid sequence of the HSA moiety is an amino acid sequence of HSA obtained by introducing a mutation to a wild-type HSA as mentioned above, and the amino acid sequence of the human cytokine moiety is an amino acid sequence of a human cytokine obtained by introducing a mutation to of a wild-type human cytokine as mentioned above. The same applies to a fusion protein of wild-type SA of a non-human animal species and a human cytokine.

An HSA-human cytokine fusion protein having a constituent amino acid modified with a sugar chain is also regarded as an HSA-human cytokine fusion protein. Also, an HSA-human cytokine fusion protein having a constituent amino acid modified with a phosphate group is regarded as an HSA-human cytokine fusion protein. Also, an HSA-human cytokine fusion protein modified with a group except a sugar chain and a phosphate group is regarded as an HSA-human cytokine fusion protein. Also, an HSA-human cytokine fusion protein having a constituent amino acid whose side chain is converted by, e.g., a substitution reaction, is regarded as an HSA-human cytokine fusion protein. Examples of such conversion include, but are not limited to, conversion of a cysteine residue to formylglycine. The same applies to a fusion protein (SA-human cytokine) of SA of a non-human animal species and a human cytokine.

More specifically, an HSA-human cytokine fusion protein modified with a sugar chain is meant to be included in an HSA-human cytokine fusion protein having an original amino acid sequence. Also, an HSA-human cytokine fusion protein modified with a phosphate group is meant to be included in an HSA-human cytokine fusion protein having an original amino acid sequence. Also, an HSA-human cytokine fusion protein modified with a group except a sugar chain and a phosphate group is meant to be included in an HSA-human cytokine fusion protein having an original amino acid sequence. Also, an HSA-human cytokine fusion protein having a constituent amino acid whose side chain is converted by, e.g., a substitution reaction, is meant to be included in an HSA-human cytokine fusion protein having an original amino acid sequence. Examples of such conversion include, but are not limited to, conversion of a cysteine residue to formylglycine. The same applies to a fusion protein (SA-human cytokine) of SA of another animal and a human cytokine. The same applies to a fusion protein (SA-human cytokine) of SA of a non-human animal species and a human cytokine.

Also, an HSA-human cytokine fusion protein in which a constituent human cytokine is a precursor of a human cytokine is regarded as an HSA-human cytokine fusion protein. The precursor as used herein refers to a moiety which functions as a human cytokine of an HSA-human cytokine fusion protein biologically synthesized, is separated from the fusion protein during a production process or in a living body to which the fusion protein is administered and serves as a human cytokine by itself. In this case, sometimes, the HSA-human cytokine fusion protein biologically synthesized is cleaved at a predetermined site with, e.g., a hydrolytic enzyme to separate an HSA-containing moiety and a matured human cytokine-containing moiety. In this case, the resulting human cytokine is not a fusion protein with HSA but the HSA-human cytokine fusion protein is once synthesized during a process for producing the cytokine.
Accordingly, in a case where a human cytokine is produced by such a method, the production method is included in a method for producing HSA-human cytokine. The same applies to a fusion protein (SA-human cytokine) of SA of a non-human animal species and a human cytokine, and a fusion protein of SA of a non-human animal species and a cytokine of a non-human animal species, for example, an MSA-mouse cytokine fusion protein.

In an embodiment of the present invention, the term "SA-hIL-10 fusion protein" or "SA-hIL-10" refers to a fusion protein having an amino acid sequence in which the N terminal of the amino acid sequence of hIL-10 is bound to the C terminal of the amino acid sequence of SA directly or via a linker, and having a function as hIL-10. The animal species of SA is not particularly limited and is preferably mammal SA, more preferably primate SA, and further preferably HSA. In an SA-hIL-10 fusion protein, the phrase that hIL-10 has a function as hIL-10 means that hIL-10 has a specific activity of preferably 10% or more, more preferably 20% or more, further preferably 50% or more, further more preferably 80% or more when the specific activity of a normal wild-type hIL-10 is regarded as 100%. Note that the specific activity of the hIL-10 in the SA-hIL-10 fusion protein herein is computationally obtained by multiplying the physiological activity of an hIL-10 per unit mass of the fusion protein by (the molecular weight of the fusion protein/the molecular weight of the moiety corresponding to the hIL-10 in the fusion protein).

In an embodiment of the present invention, the term "HSA-hIL-10 fusion protein" or "HSA-hIL-10" refers to a fusion protein having an amino acid sequence in which the N terminal of the amino acid sequence of hIL-10 is bound to the C terminal of the amino acid sequence of HSA directly or via a linker, and having a function as hIL-10. To a case where an HSA-hIL-10 fusion protein has a function as hIL-10, the definition in the case of the SA-hIL-10 fusion protein mentioned above is applicable. Also in these fusion proteins, SA preferably has a function as SA, such as a function to bind an endogenous substance and an exogenous substance such as a drug in the blood and transport them, but is not limited to this.

In an embodiment of the present invention, a preferable HSA-hIL-10 fusion protein has the amino acid sequence represented by, for example, SEQ ID NO: 50. The HSA-hIL-10 fusion protein represented by SEQ ID NO: 50 is a fusion protein in which a wild-type hIL-10 is bound directly to the C terminal of a wild-type HSA. An HSA-hIL-10 fusion protein represented by SEQ ID NO: 50 is encoded by a gene having the nucleotide sequence represented by, for example, SEQ ID NO: 51. Also, a fusion protein having an amino acid sequence represented by SEQ ID NO: 50 and further having a mutation such as a substitution with different amino acid residues, a deletion, or an addition of one or more amino acid residues therein is included in the HSA-hIL-10 fusion protein as long as it has a function as hIL-10. The HSA-hIL-10 fusion protein preferably has a function as human serum albumin, such as a function to bind an endogenous substance and an exogenous substance such as a drug in the blood and transport them, but is not limited to this.

When mutations are introduced to the amino acid sequence represented by SEQ ID NO: 50, the sites and types (deletion, substitution, and addition) of individual mutations can be easily identified by alignment of the amino acid sequences before and after introduction of the mutations. The amino acid sequence having a mutation introduced thereto exhibits an identity of preferably 80% or more, 85% or more, 90% or more, or 95% or more, for example, an identity of 98% or more or 99% or more, to the amino acid sequence represented by SEQ ID NO: 50.

In a case where a mutation is introduced to an HSA-hIL-10 fusion protein, which is a fusion protein of a wild-type HSA and a wild-type hIL-10, the mutation can be introduced only to the HSA moiety and not introduced to the hIL-10 moiety; the mutation can be introduced only to an hIL-10 moiety without being introduced to the HSA moiety; or the mutation can be added to both of the HSA moiety and the hIL-10 moiety. In a case where a mutation is introduced only to the HSA moiety, the amino acid sequence of the moiety is an amino acid sequence of HSA obtained by introducing a mutation to a wild-type HSA as mentioned above. In a case where a mutation is introduced only to an hIL-10 moiety, the amino acid sequence of the moiety is an amino acid sequence of an hIL-10 of obtained by introducing a mutation to a wild-type hIL-10 as mentioned above. In a case where a mutation is introduced to both of the HSA moiety and the hIL-10 moiety, the amino acid sequence of the HSA moiety is an amino acid sequence of HSA of obtained by introducing a mutation to a wild-type HSA as mentioned above, whereas the amino acid sequence of the hIL-10 moiety is an amino acid sequence of an hIL-10 of obtained by introducing a mutation to a wild-type hIL-10 as mentioned above. The same applies to an HSA-hIL-10 fusion protein having the amino acid sequence represented by SEQ ID NO: 50. The same applies to a fusion protein of wild-type SA of a non-human animal species and a wild-type hIL-10.

The case where a mutation is introduced to an HSA-hIL-10 fusion protein having the amino acid sequence represented by SEQ ID NO: 50 will be described below. When an amino acid residue in the amino acid sequence represented by SEQ ID NO: 50 is substituted with another amino acid residue, the number of amino acid residues to be substituted is 1 to 10, 1 to 5, or 1 to 3, for example, one or two. When an amino acid residue is deleted, the number of amino acid residues to be deleted is 1 to 10, 1 to 5, or 1 to 3, for example, one or two. When an amino acid residue is added, one or more amino acid residues are added in the amino acid sequence represented by SEQ ID NO: 50 or added to the N terminal or C terminal of the amino acid sequence. The HSA-hIL-10 fusion protein may have a combination of the substitution, deletion, and addition of amino acid residues. A mutation may be introduced only to the HSA moiety, only to the hIL-10 moiety, or to both of them.

When mutations are introduced to the amino acid sequence represented by SEQ ID NO: 50, the sites and types (deletion, substitution, and addition) of individual mutations can be easily identified by alignment of the amino acid sequences before and after introduction of the mutations. The amino acid sequence having a mutation introduced thereto exhibits an identity of preferably 80% or more, 85% or more, 90% or more, or 95% or more, for example, an identity of 98% or more or 99% or more, to the amino acid sequence represented by SEQ ID NO: 50.

An HSA-hIL-10 fusion protein having a constituent amino acid modified with a sugar chain is regarded as an HSA-hIL-10 fusion protein. Also, an HSA-hIL-10 fusion protein having a constituent amino acid modified with a phosphate group is regarded as an HSA-hIL-10 fusion protein. Also, an HSA-hIL-10 fusion protein modified with a group except a sugar chain and a phosphate group is regarded as an HSA-hIL-10 fusion protein. Also, an HSA-hIL-10 fusion protein having a constituent amino acid whose side chain is converted by, e.g., a substitution reaction, is regarded as an HSA-hIL-10 fusion protein. Examples of such conversion include, but are not limited to, conversion of a cysteine residue to formylglycine. The same applies to a fusion protein (SA-hIL-10) of SA of another animal and hIL-10. The same applies to a fusion protein (SA-hIL-10) of SA of a non-human animal species and hIL-10.

More specifically, an HSA-hIL-10 fusion protein modified with a sugar chain is meant to be included in an HSA-hIL-10 fusion protein having an original amino acid sequence. Also, an HSA-hIL-10 fusion protein modified with a phosphate group is meant to be included in an HSA-hIL-10 fusion protein having an original amino acid sequence. Also, an HSA-hIL-10 fusion protein modified with a group except a sugar chain and a phosphate group is meant to be included in an HSA-hIL-10 fusion protein having an original amino acid sequence. Also, an HSA-hIL-10 fusion protein having a constituent amino acid whose side chain is converted by, e.g., a substitution reaction, is meant to be included in an HSA-hIL-10 fusion protein having an original amino acid sequence. Examples of such conversion include, but are not limited to, conversion of a cysteine residue to formylglycine. The same applies to a fusion protein (SA-hIL-10) of SA of a non-human animal species and hIL-10.

An HSA-hIL-10 fusion protein in which a constituent hIL-10 is a precursor of hIL-10 is regarded as an HSA-hIL-10 fusion protein. The precursor as used herein refers to a moiety which functions as an hIL-10 of an HSA-hIL-10 fusion protein biologically synthesized, is separated from the fusion protein during a production process or in a living body to which the fusion protein is administered and serves as an hIL-10 by itself. In this case, sometimes, the HSA-hIL-10 fusion protein biologically synthesized is cleaved at a predetermined site with, e.g., a hydrolytic enzyme to separate an HSA-containing moiety and a matured hIL-10-containing moiety. In this case, the resulting hIL-10 is not a fusion protein with HSA but the HSA-hIL-10 fusion protein is once synthesized during a process for synthesizing the hIL-10. Accordingly, in a case where hIL-10 is produced by such a method, the production method is included in a method for producing an HSA-hIL-10 fusion protein. The same applies to a fusion protein (SA-hIL-10) of SA of a non-human animal species and hIL-10.

In an embodiment of the present invention, the term "human cytokine-SA fusion protein" or "human cytokine-SA" refers to a fusion protein having an amino acid sequence in which the N terminal of the amino acid sequence of SA is bound to the C terminal of the amino acid sequence of a human cytokine directly or via a linker, and having a function as a human cytokine. The phrase that a human cytokine has a function as a human cytokine in a human cytokine-SA fusion protein means that a human cytokine has a specific activity of preferably 10% or more, more preferably 20% or more, further preferably 50% or more, further more preferably 80% or more when the specific activity of a normal wild-type human cytokine is regarded as 100%. Note that, the specific activity of the human cytokine in the human cytokine-SA fusion protein herein is computationally obtained by multiplying the physiological activity of a human cytokine per unit mass of the fusion protein by (the molecular weight of the fusion protein/the molecular weight of the moiety corresponding to the human cytokine in the fusion protein).

In an embodiment of the present invention, the term "human cytokine-HSA fusion protein" or "human cytokine-HSA" refers to a fusion protein having an amino acid sequence in which the N terminal of the amino acid sequence of HSA is bound to the C terminal of the amino acid sequence of a human cytokine directly or via a linker, and having a function as a human cytokine. To a case where the human cytokine-HSA fusion protein has a function as a human cytokine, the definition in the case of the human cytokine-SA mentioned above is applicable.

In the human cytokine-SA fusion protein, SA preferably has a function as SA, such as a function to bind an endogenous substance and an exogenous substance such as a drug in the blood and transport them, but is not limited to this. The same applies to a human cytokine-HSA fusion protein.

In a case where a mutation is introduced to a human cytokine-HSA fusion protein, which is a fusion protein of a wild-type human cytokine and a wild-type HSA, the mutation can be introduced only to the human cytokine moiety and not introduced to the HSA moiety; the mutation can be introduced only to an HSA moiety without being introduced to the human cytokine moiety; or the mutation can be introduced to both of the human cytokine moiety and the HSA moiety. In a case where a mutation is introduced only to the human cytokine moiety, the amino acid sequence of the moiety is an amino acid sequence of the human cytokine obtained by introducing a mutation to a wild-type human cytokine as mentioned above. In a case where a mutation is introduced only to the HSA moiety, the amino acid sequence of the moiety is an amino acid sequence of HSA obtained by introducing a mutation to a wild-type HSA as mentioned above. In a case where a mutation is introduced to both of the human cytokine moiety and the HSA moiety, the amino acid sequence of the human cytokine moiety is an amino acid sequence of human cytokine obtained by introducing a mutation to a wild-type human cytokine as mentioned above, and the amino acid sequence of the HSA moiety is an amino acid sequence of HSA obtained by introducing a mutation to a wild-type HSA as mentioned above. The same applies to a fusion protein of a wild-type human cytokine and a wild-type SA of a non-human animal species.

A human cytokine-HSA fusion protein having a constituent amino acid modified with a sugar chain is regarded as a human cytokine-HSA fusion protein. Also, a human cytokine-HSA fusion protein having a constituent amino acid modified with a phosphate group is regarded as a human cytokine-HSA fusion protein. Also, a human cytokine-HSA fusion protein modified with a group except a sugar chain and a phosphate group is regarded as a human cytokine-HSA fusion protein. Also, a human cytokine-HSA fusion protein having a constituent amino acid whose side chain is converted by, e.g., a substitution reaction, is regarded as a human cytokine-HSA fusion protein. Examples of such conversion include, but are not limited to, conversion of a cysteine residue to formylglycine. The same applies to a fusion protein (human cytokine-SA) of a human cytokine and SA of a non-human animal species.

More specifically, a human cytokine-HSA fusion protein modified with a sugar chain is meant to be included in a human cytokine-HSA fusion protein having an original amino acid sequence. Also, a human cytokine-HSA fusion protein modified with a phosphate group is meant to be included in a human cytokine-HSA fusion protein having an original amino acid sequence. Also, a human cytokine-HSA fusion protein modified with a group except a sugar chain and a phosphate group is meant to be included in a human cytokine-HSA fusion protein having an original amino acid sequence. Also, a human cytokine-HSA fusion protein having a constituent amino acid whose side chain is converted by, e.g., a substitution reaction, is meant to be included in a human cytokine-HSA fusion protein having an original amino acid sequence. Examples of such conversion include, but are not limited to, conversion of a cysteine residue to formylglycine. The same applies to a fusion protein (human cytokine-SA) of a human cytokine and SA of a non-human animal species.

A human cytokine-HSA fusion protein in which a constituent human cytokine is a precursor of a human cytokine is regarded as a human cytokine-HSA fusion protein. The same applies to a fusion protein (human cytokine-SA) of a human cytokine and SA of a non-human animal species.

In an embodiment of the present invention, the term "hIL-10-SA fusion protein" or "hIL-10-SA" refers to a fusion protein having an amino acid sequence in which the N terminal of the amino acid sequence of SA is bound to the C terminal of the amino acid sequence of hIL-10 directly or via a linker, and having a function as hIL-10. The phrase that hIL-10 has a function as hIL-10 in the hIL-10-SA fusion protein means that hIL-10 has a specific activity of preferably 10% or more, more preferably 20% or more, further preferably 50% or more, further more preferably 80% or more when the specific activity of a normal wild-type hIL-10 is regarded as 100%. Note that, the specific activity of the hIL-10 in the hIL-10-SA fusion protein herein is computationally obtained by multiplying the physiological activity of hIL-10 per unit mass of the fusion protein by (the molecular weight of the fusion protein/the molecular weight of the moiety corresponding to the hIL-10 in the fusion protein).

In an embodiment of the present invention, the term "hIL-10-HSA fusion protein" or "hIL-10-HSA" refers to a fusion protein having an amino acid sequence in which the N terminal of the amino acid sequence of HSA is bound to the C terminal of the amino acid sequence of hIL-10 directly or via a linker, and having a function as hIL-10. To a case where the hIL-10-HSA fusion protein has a function as hIL-10, the definition in the case of the hIL-10-SA mentioned above is applicable. Also in these fusion proteins, SA preferably has a function as SA, such as a function to bind an endogenous substance and an exogenous substance such as a drug in the blood and transport them, but is not limited to this.

In an embodiment of the present invention, a preferable hIL-10-HSA fusion protein has the amino acid sequence represented by, for example, SEQ ID NO: 52. The hIL-10-HSA fusion protein represented by SEQ ID NO: 52 is a fusion protein in which a wild-type HSA is bound directly to the C terminal of a wild-type hIL-10. The hIL-10-HSA fusion protein represented by SEQ ID NO: 52 is encoded by a gene having the nucleotide sequence represented by, for example, SEQ ID NO: 53. Also, a fusion protein having an amino acid sequence represented by SEQ ID NO: 52 and further having a mutation such as a substitution with different amino acid residues, a deletion, or an addition of one or more amino acid residues therein is included in the hIL-10-HSA fusion protein as long as it has a function as hIL-10. The hIL-10-HSA fusion protein preferably has a function as human serum albumin, such as a function to bind an endogenous substance and an exogenous substance such as a drug in the blood and transport them, but is not limited to this.

When mutations are introduced to the amino acid sequence represented by SEQ ID NO: 52, the sites and types (deletion, substitution, and addition) of individual mutations can be easily identified by alignment of the amino acid sequences before and after introduction of the mutations. The amino acid sequence having a mutation introduced thereto exhibits an identity of preferably 80% or more, 85% or more, 90% or more, or 95% or more, for example, an identity of 98% or more or 99% or more, to the amino acid sequence represented by SEQ ID NO: 52.

In a case where a mutation is introduced to an hIL-10-HSA fusion protein, which is a fusion protein of a wild-type hIL-10 and a wild-type HSA, the mutation can be introduced only to the HSA moiety and not introduced to the hIL-10 moiety; the mutation can be introduced only to the hIL-10 moiety without being introduced to the HSA moiety; or the mutation can be introduced to both of the HSA moiety and the hIL-10 moiety. In a case where a mutation is introduced only to the HSA moiety, the amino acid sequence of the moiety is an amino acid sequence of HSA obtained by introducing a mutation to a wild-type HSA as mentioned above. In a case where a mutation is introduced only to the hIL-10 moiety, the amino acid sequence of the moiety is an amino acid sequence of an hIL-10 obtained by introducing a mutation to a wild-type hIL-10 as mentioned above. In a case where a mutation is introduced to both of the HSA moiety and the hIL-10 moiety, the amino acid sequence of the HSA moiety is an amino acid sequence of HSA obtained by introducing a mutation to a wild-type HSA as mentioned above, and the amino acid sequence of the hIL-10 moiety is an amino acid sequence of hIL-10 obtained by introducing a mutation to a wild-type hIL-10 as mentioned above. The same applies to an hIL-10-HSA fusion protein having the amino acid sequence represented by SEQ ID NO: 52. The same applies to a fusion protein (hIL-10-SA) of a wild-type hIL-10 and wild-type SA of a non-human animal species.

The case where a mutation is introduced to an hIL-10-HSA fusion protein having the amino acid sequence represented by SEQ ID NO: 52 will be described below. When an amino acid residue in the amino acid sequence represented by SEQ ID NO: 52 is substituted with another amino acid residue, the number of amino acid residues to be substituted is 1 to 10, 1 to 5, or 1 to 3, for example, one or two. When an amino acid residue is deleted, the number of amino acid residues to be deleted is 1 to 10, 1 to 5, or 1 to 3, for example, one or two. When an amino acid residue is added, one or more amino acid residues are added in the amino acid sequence represented by SEQ ID NO: 52 or added to the N terminal or C terminal of the amino acid sequence. The hIL-10-HSA fusion protein may have a combination of the substitution, deletion, and addition of amino acid residues. A mutation may be introduced only to the HSA moiety, only to the hIL-10 moiety, or to both of them.

When mutations are introduced to the amino acid sequence represented by SEQ ID NO: 52, the sites and types (deletion, substitution, and addition) of individual mutations can be easily identified by alignment of the amino acid sequences before and after introduction of the mutations. The amino acid sequence having a mutation introduced thereto exhibits an identity of preferably 80% or more, 85% or more, 90% or more, or 95% or more, for example, an identity of 98% or more or 99% or more, to the amino acid sequence represented by SEQ ID NO: 52.

An hIL-10-HSA fusion protein having a constituent amino acid modified with a sugar chain is regarded as an hIL-10-HSA fusion protein. Also, an hIL-10-HSA fusion protein having a constituent amino acid modified with a phosphate group is regarded as an hIL-10-HSA fusion protein. Also, an hIL-10-HSA fusion protein modified with a group except a sugar chain and a phosphate group is regarded as an hIL-10-HSA fusion protein. Also, an hIL-10-HSA fusion protein having a constituent amino acid whose side chain is converted by, e.g., a substitution reaction, is regarded as an hIL-10-HSA fusion protein. Examples of such conversion include, but are not limited to, conversion of a cysteine residue to formylglycine. The same applies to a fusion protein (SA-hIL-10) of SA of a non-human animal species and hIL-10.

More specifically, an hIL-10-HSA fusion protein modified with a sugar chain is meant to be included in an hIL-10-HSA fusion protein having an original amino acid sequence. Also, an hIL-10-HSA fusion protein modified with a phosphate group is meant to be included in an hIL-10-HSA fusion protein having an original amino acid sequence. Also, an hIL-10-HSA fusion protein modified with a group except a sugar chain and a phosphate group is meant to be included in an hIL-10-HSA fusion protein having an original amino acid sequence. Also, an hIL-10-HSA fusion protein having a constituent amino acid whose side chain is converted by, e.g., a substitution reaction, is meant to be included in an hIL-10-HSA fusion protein having an original amino acid sequence. Examples of such conversion include, but are not limited to, conversion of a cysteine residue to formylglycine. The same applies to a fusion protein (SA-hIL-10) of SA of a non-human animal species and hIL-10.

An hIL-10-HSA fusion protein in which a constituent hIL-10 is a precursor of hIL-10 is regarded as an hIL-10-HSA fusion protein. The same applies to a fusion protein (SA-hIL-10) of SA of a non-human animal species and hIL-10. The same applies to a fusion protein (SA-hIL-10) of SA of a non-human animal species and hIL-10 and a fusion protein of SA of a non-human animal species and IL-10 of a non-human animal species, for example, an MSA-mouse IL-10 fusion protein.

In an embodiment of the present invention, the term "fusion protein of HSA and human cytokine", "fusion protein of human cytokine and HSA" or "fusion protein of human serum albumin and human cytokine", is meant to include both the "HSA-human cytokine fusion protein" and "human cytokine-HSA fusion protein" as mentioned above. Also, the term "fusion protein of HSA and hIL-10", "fusion protein of hIL-10 and HSA" , or "fusion protein of human serum albumin and human cytokine 10", is meant to include both the "HSA-hIL-10 fusion protein" and "hIL-10-HSA fusion protein" as mentioned above.

Now, a method for producing a fusion protein of HSA and hIL-10 will be more specifically described, below. The following description can apply to fusion proteins using a cytokine other than IL-10, using SA derived from non-human species, and using a cytokine derived from non-human species. For example, the following description can be applied to a fusion protein of SA of a non-human animal species and hIL-10 and a fusion protein of SA of a non-human animal species and IL-10 of a non-human animal species.

In the fusion protein according to an embodiment of the present invention, SA and a cytokine are bound directly or via a linker. The "linker" used herein refers to a moiety not belonging to any one of the amino acid sequence of SA and the amino acid sequence of the cytokine. More specifically, the linker is a peptide chain present between SA and the cytokine. The linker has various functions. Examples of the function of the linker include a function to bind SA and a cytokine by being interposed between SA and a cytokine, a function to reduce mutual interference between SA and a cytokine by keeping an intramolecular distance within a fusion protein, and a function to serve as a hinge for connecting SA and a cytokine by being interposed between SA and a cytokine, thereby forming a flexible three dimensional structure of a fusion protein. The linker is present within a molecule of a fusion protein and exerts at least one of these functions. The same applies to a case where SA is HSA and a case where the cytokine is human cytokine such as hIL-10.

In the fusion protein of SA and a cytokine, the amino acid sequence of a peptide linker is not particularly limited as long as it is present within the fusion protein molecule and produces a function as a linker. The length of a peptide linker is not particularly limited as long as it is present within a fusion protein molecule and produces a function as a linker. A peptide linker is constituted of one or more amino acids. In a case where a peptide linker is constituted of a plurality of amino acids, the number of amino acids is preferably 2 to 50, more preferably 5 to 30, and further preferably 10 to 25. Suitable examples of a peptide linker include peptide linkers constituted of Gly-Ser, Gly-Gly-Ser, or the amino acid sequences represented by SEQ ID NOs: 9 to 11 (these are collectively referred to as a basic sequence), and peptide linkers containing these. For example, a peptide linker contains an amino acid sequence having 2 to 10, 2 to 6 or 3 to 5 repeats of the basic sequence. These amino acid sequences may have, e.g., a deletion, a substitution or addition of one or more amino acids. In a case where an amino acid is deleted, the number of amino acids to be deleted is preferably 1 or 2. In a case where an amino acid is substituted with a different one, the number of amino acids to be substituted is preferably 1 or 2. In a case where an amino acid is added, the number of amino acids to be added is preferably 1 or 2. The amino acid sequence of a desired linker moiety can be prepared by using a combination of the deletion, substitution, and addition of amino acids. A peptide linker may be constituted of a single amino acid, and the amino acid constituting the linker is, for example, glycine and serine.

A fusion protein of HSA and hIL-10 can be prepared as a recombinant protein by preparing an expression vector integrating a DNA fragment in which a gene encoding hIL-10 is bound to a downstream or upstream site of a gene encoding HSA in frame and culturing a host cell transformed by introduction the expression vector. The fusion protein prepared as a recombinant protein in this manner is constituted of a single-chain polypeptide.

In a case where the fusion protein is prepared as a recombinant fusion protein, an HSA-hIL-10 fusion protein having the amino acid sequence of hIL-10 at the C terminal of the amino acid sequence of HSA can be obtained by binding a gene encoding hIL-10 to a downstream site of a gene encoding HSA in frame. Conversely, an hIL-10-HSA fusion protein having the amino acid sequence of hIL-10 at the N terminal of the amino acid sequence of HSA can be obtained by binding a gene encoding hIL-10 to an upstream site of a gene encoding HSA in frame. In either case, the fusion protein prepared as a recombinant fusion protein is a single-chain polypeptide.

In a single-chain polypeptide of a fusion protein, if the amino acid sequence of HSA is positioned on the N terminal side of the amino acid sequence of hIL-10, the C terminal of HSA and the N terminal of hIL-10 are bound directly by a peptide bond, or via a linker. Figure 5 schematically shows an HSA-hIL-10 fusion protein of a single-chain polypeptide having HSA, a linker and hIL-10 in this order from the N terminal side. The HSA-hIL-10 fusion protein is a fusion protein in which the C terminal of HSA and the N terminal of the linker are bound by a peptide bond, and the C terminal of the linker and the N terminal of hIL-10 are bound by a peptide bond.

In a single-chain polypeptide of a fusion protein, if the amino acid sequence of hIL-10 is positioned on the N terminal of the amino acid sequence of HSA, the C terminal of hIL-10 and the N terminal of HSA are bound directly by a peptide bond, or via a linker. Figure 6 schematically shows an hIL-10-HSA fusion protein of a single-chain polypeptide having hIL-10, a linker and HSA in this order from the N terminal side. The hIL-10-HSA fusion protein is a fusion protein in which the C terminal of hIL-10 and the N terminal of the linker are bound by a peptide bond, and the C terminal of the linker and the N terminal of HSA are bound by a peptide bond.

In an embodiment of the present invention, a fusion protein of HSA and hIL-10 is meant to be characterized in that the fusion protein, when expressed as a recombinant protein in a host cell, particularly expressed as a recombinant protein so as to be secreted from the cell and accumulated in a culture solution, provides an expression level as hIL-10 in the culture supernatant in terms of concentration or physiological activity, that is, for example, at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, or 3 to 4 times that of the wild-type hIL-10 expressed as a recombinant protein in a host cell under the same conditions. The same conditions mean that the expression vector, host cell, culture conditions and others are the same. A preferable host cell to be used herein is a mammalian cell such as a CHO cell or an NS/0 cell, in particular a CHO cell.

As preferable embodiments of such a fusion protein of HSA and hIL-10, the following (1) to (4) are mentioned:
(1) a fusion protein having the amino acid sequence represented by SEQ ID NO: 50, in which the N terminal of the amino acid sequence of a wild-type hIL-10 represented by SEQ ID NO: 24 is bound directly to the C terminal of the amino acid sequence of a wild-type HSA represented by SEQ ID NO: 3;
(2) a fusion protein having the amino acid sequence represented by SEQ ID NO: 52, in which the N terminal of the amino acid sequence of a wild-type HSA represented by SEQ ID NO: 3 is bound directly to the C terminal of the amino acid sequence of a wild-type hIL-10 represented by SEQ ID NO: 24;
(3) a fusion protein having the amino acid sequence represented by SEQ ID NO: 54, in which the N terminal of the amino acid sequence of a wild-type hIL-10 represented by SEQ ID NO: 24 is bound to the C terminal of the amino acid sequence of a wild-type HSA represented by SEQ ID NO: 3 via a linker represented by SEQ ID NO: 9; and
(4) a fusion protein having the amino acid sequence represented by SEQ ID NO: 55, in which the N terminal of the amino acid sequence of a wild-type HSA represented by SEQ ID NO: 3 is bound to the C terminal of the amino acid sequence of a wild-type hIL-10 represented by SEQ ID NO: 24 via a linker represented by SEQ ID NO: 9.

Also, as preferable embodiments of such a fusion protein of HSA and hIL-10, the following (5) to (8) using human serum albumin (HSA-A320T) consisting of 585 amino acids represented by SEQ ID NO: 13, which is obtained by substituting the 320th amino acid residue, alanine, from the N terminal of the amino acid sequence of a wild-type HSA represented by SEQ ID NO: 3, with threonine, are further mentioned:
(5) a fusion protein having the amino acid sequence represented by SEQ ID NO: 56, in which the N terminal of the amino acid sequence of a wild-type hIL-10 represented by SEQ ID NO: 24 is bound directly to the C terminal of the amino acid sequence of human serum albumin (HSA-A320T) represented by SEQ ID NO: 13;
(6) a fusion protein having the amino acid sequence represented by SEQ ID NO: 57 as a whole, in which the N terminal of the amino acid sequence of human serum albumin (HSA-A320T) represented by SEQ ID NO: 13 is bound directly to the C terminal of the amino acid sequence of a wild-type hIL-10 represented by SEQ ID NO: 24;
(7) a fusion protein having the amino acid sequence represented by SEQ ID NO: 58, in which the N terminal of the amino acid sequence of a wild-type hIL-10 represented by SEQ ID NO: 24 is bound to the C terminal of the amino acid sequence of human serum albumin (HSA-A320T) represented by SEQ ID NO: 13 via a linker represented by SEQ ID NO: 9; and
(8) a fusion protein having the amino acid sequence represented by SEQ ID NO: 59, in which the N terminal of the amino acid sequence of human serum albumin (HSA-A320T) represented by SEQ ID NO: 13 is bound to the C terminal of the amino acid sequence of a wild-type hIL-10 represented by SEQ ID NO: 24 via a linker represented by SEQ ID NO: 9.

In an embodiment of the present invention, the fusion protein of HSA and hIL-10 is characterized in that the fusion protein, when expressed as a recombinant protein in a host cell, particularly expressed as a recombinant protein so as to be secreted from the cell and accumulated in a culture solution, provides an increased expression level as hIL-10 in the culture supernatant in terms of concentration or physiological activity, compared to that of a wild-type hIL-10 expressed as a recombinant protein in a host cell under the same conditions. Accordingly, when a fusion protein of HSA and hIL-10 is produced as a recombinant protein, a production efficiency thereof can be increased compared to a wild-type hIL-10, and thus a production cost of the fusion protein can be reduced. Note that, it is known that medicaments containing a recombinant protein as an active ingredient are very expensive. Accordingly, if the amount of a recombinant protein produced under the same conditions is increased by several percent, for example, 3 to 9%, a large economic effect is produced. The same applies to a cytokine such as hIL-10.

Note that, in the specification, when the expression level of a fusion protein of HSA and hIL-10 expressed in a host cell as a recombinant protein is compared to that of a wild-type hIL-10 expressed in a host cell as a recombinant protein under the same condition, comparison is preferably made not based on the mass of a protein expressed but based on the number of molecules or hIL-10 physiological activity of the protein expressed. The same applies to a fusion protein of SA of a non-human animal species and hIL-10 and a fusion protein of SA and another cytokine.

A fusion protein of HSA and hIL-10 can be produced by using the expression vector, host cell, medium and others that can be used for producing the fusion protein of HSA and hGALC or fusion protein of HSA and hGBA mentioned above. Note that, not only to this, the same universally applies to fusion proteins of SA and a cytokine.

The fusion protein of HSA and hIL-10 is characterized in that the fusion protein, when expressed as a recombinant protein by culturing a host cell to which an expression vector integrating a gene encoding the fusion protein is introduced, in the serum-free medium mentioned above, particularly expressed as a recombinant protein so as to be secreted from the cell and accumulated in a culture solution, provides an expression level as the hIL-10 in the culture supernatant in terms of concentration or physiological activity, that is, for example, at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, or 3 to 4 times that of a wild-type hIL-10 expressed as a recombinant protein in a host cell under the same conditions. The same conditions mean that the expression vector, host cell, culture conditions and others are the same. The host cell used herein is preferably a mammalian cell, particularly, a cell usually used for production of a recombinant protein, such as a CHO cell or an NS/0 cell.

A wild-type hIL-10, when expressed as a recombinant by using a host cell transformed with an expression vector integrating a gene encoding it, tends to exhibit low expression levels, and it is difficult to efficiently mass-produce it as a recombinant. However, hIL-10 can be expressed as a recombinant at relatively high expression levels by expressing hIL-10 as a fusion protein with HSA. More specifically, a fusion protein of HSA and hIL-10, when expressed as a recombinant protein by culturing a host to which an expression vector integrating a gene encoding it is introduced, in a serum-free medium, particularly expressed as a recombinant protein so as to be secreted from the cell and accumulated in a culture solution, can be efficiently expressed compared to a case where a wild-type hIL-10 is expressed as a recombinant protein under the same conditions, thus being suitable for mass production. The same applies to a fusion protein of SA of a non-human animal species and hIL-10. Note that, the host cell to be used for expression of the fusion protein is preferably a mammalian cell, particularly, a cell usually used for production of a recombinant protein, such as a CHO cell or an NS/0 cell. More specifically, an embodiment of the present invention is a recombinant fusion protein of SA and IL-10, particularly a recombinant fusion protein of HSA and hIL-10.

A fusion protein of HSA and hIL-10 can be expressed within a cell or in a medium by culturing a host cell encoding the fusion protein. The fusion protein of HSA and hIL-10 can be purified by separating it from foreign substances by a method such as column chromatography. The fusion protein of HSA and hIL-10 purified can be used as a pharmaceutical composition. Particularly, a fusion protein of HSA and hIL-10 can be used as a pharmaceutical composition targeted to inflammatory diseases or cancer.

A pharmaceutical composition containing a fusion protein of HSA and hIL-10 as an active ingredient can be administered intravenously, intramuscularly, intraperitoneally, subcutaneously or intracerebroventricularly as an injection. Such an injection can be supplied as a lyophilized preparation or an aqueous liquid. The aqueous liquid may be in a form it is filled in a vial or supplied as a prefilled preparation in a syringe. The lyophilized preparation is dissolved and restored in an aqueous medium before use and then used.

A fusion protein of HSA and hIL-10 can further form a conjugate with an antibody or a ligand. For example, a fusion protein of HSA and hIL-10 in an embodiment of the present invention can form a conjugate with an antibody or a ligand capable of specifically binding to a receptor on a cerebrovascular endothelial cell. A fusion protein of HSA and hIL-10 or a fusion protein of HSA and hGBA in the form of a conjugate with an antibody or a ligand capable of specifically binding to a receptor on a cerebrovascular endothelial cell can bind to a receptor on a cerebrovascular endothelial cell. The fusion protein bound to a receptor on a cerebrovascular endothelial cell can pass through the blood-brain barrier (BBB) to reach the tissue of the central nervous system (CNS). Thus, a fusion protein of HSA and hIL-10 in the form of a conjugate with such an antibody or a ligand, can pass through the blood-brain barrier (BBB) and exert a function thereof in the central nervous system (CNS).

In a conjugate of a fusion protein of HSA and hIL-10 with an antibody, the phrase that hIL-10 has a function as an hIL-10 means that hIL-10 has a specific activity of preferably 10% or more, more preferably 20% or more, further preferably 50% or more, further more preferably 80% or more, for example, 90% or more, or 95% or more when the specific activity of a normal wild-type hIL-10 is regarded as 100%. Note that, the specific activity of the hIL-10 in the conjugate herein is computationally obtained by multiplying the physiological activity of an hIL-10 per unit mass of the conjugate by (the molecular weight of the conjugate/the molecular weight of the moiety corresponding to the hIL-10 in the conjugate).

In an embodiment of the present invention, a conjugate of a fusion protein of HSA and hIL-10 with an antibody is characterized in that the conjugate, when expressed as a recombinant protein in a host cell, particularly expressed as a recombinant protein so as to be secreted from the cell and accumulated in a culture solution, provides an expression level as the hIL-10 in the culture supernatant in terms of concentration or physiological activity, that is, for example, at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, or 3 to 4 times that of a wild-type hIL-10 expressed as a recombinant protein in a host cell under the same conditions.

Some of neurotrophic factors, when expressed as a recombinant protein by using a host cell to which an expression vector integrating a gene encoding wild-type one is introduced, are relatively difficult to mass-produce because of limited expression levels. Such neurotrophic factors include BDNF (brain-derived neurotrophic factor), NGF (nerve growth factor), NT-3 (neurotrophin-3), NT-4 (neurotrophin-4), and NT-5 (neurotrophin-5). The structural difference between NT-4 and NT-5 is caused by interspecific mutations, which are believed to be the same factor and hence NT-4 and NT-5 are occasionally expressed as NT-4/5 or the like, but expressed as NT-4 in the specification. GDNF family ligands (GFLs) are a family including four proteins: glial cell line-derived neurotrophic factor (GDNF), neurturin (NRTN), artemin (ARTN), and persephin (PSPN). The four proteins included in the GDNF family ligands (GFLs) are also included in neurotrophic factors. Also, cerebral dopamine neurotrophic factor (CDNF) and mesencephalic astrocyte-derived neurotrophic factor (MANF) are included in neurotrophic factors. An embodiment of the present invention is a recombinant protein as a fusion protein of such a neurotrophic factor that is relatively difficult to produce as a recombinant protein and SA. The recombinant protein is easier to mass-produce by using a host cell to which an expression vector integrating a gene encoding this is introduced, than the corresponding neurotrophic factor. The animal species of a neurotrophic factor to be bound to SA herein is not particularly limited and the neurotrophic factor is preferably a human neurotrophic factor. The animal species of SA to be bound to a neurotrophic factor is not particularly limited and the SA is preferably HSA.

The term "human neurotrophic factor" as used herein simply includes indistinguishably not only a normal wild-type human neurotrophic factor but also human neurotrophic factor mutants, which correspond to human neurotrophic factor mutants having a substitution, deletion, and/or addition of one or more amino acid residues ("addition" of an amino acid residue herein means adding the residue to a terminal of a sequence or in the sequence) in the amino acid sequence of a wild-type human neurotrophic factor, as long as the mutants have a function as the human neurotrophic factor, such as a physiological activity corresponding to the type of human neurotrophic factor. The same applies to neurotrophic factors of non-human animal species.

Note that, the phrase that a human neurotrophic factor has a function as the human neurotrophic factor herein means that the human neurotrophic factor has a specific activity of preferably 10% or more, more preferably 20% or more, further preferably 50% or more and further more preferably 80% or more when the specific activity of a normal wild-type human neurotrophic factor is regarded as 100%. The specific activity herein refers to physiological activity per mass of the protein. Note that, the specific activity of a fusion protein of a human neurotrophic factor and a protein is obtained as a physiological activity per mass of the moiety corresponding to the human neurotrophic factor of the fusion protein. Note that, the specific activity of a human neurotrophic factor in a fusion protein herein is computationally obtained by multiplying physiological activity of a human neurotrophic factor per unit mass of the fusion protein by (the molecular weight of the fusion protein/the molecular weight of the moiety corresponding to the human neurotrophic factor in the fusion protein). The same applies to neurotrophic factors of non-human animal species.

When an amino acid residue in the amino acid sequence of a wild-type human neurotrophic factor is substituted with another amino acid residue, the number of amino acid residues to be substituted is 1 to 10, 1 to 5, or 1 to 3, for example, one or two. When an amino acid residue in the amino acid sequence of a wild-type human neurotrophic factor is deleted, the number of amino acid residues to be deleted is 1 to 10, 1 to 5, or 1 to 3, for example, one or two. Mutants such as a human neurotrophic factor mutant consisting of an amino acid sequence obtained by deleting a single amino acid residue from the N terminal or C terminal of a wild-type human neurotrophic factor and a human neurotrophic factor mutant consisting of an amino acid sequence obtained by deleting two amino acid residues from the N terminal or C terminal of the wild-type human neurotrophic factor are also regarded as human neurotrophic factors. Also, a mutation having the substitution and deletion of amino acid residues in combination can be introduced to the amino acid sequence of the wild-type human neurotrophic factor. The same applies to neurotrophic factors of non-human animal species.

When an amino acid residue is added to the amino acid sequence of a wild-type human neurotrophic factor, one or more amino acid residues are added in the amino acid sequence of a human neurotrophic factor or added to the N terminal or C terminal of the amino acid sequence thereof. The number of amino acid residues to be added herein is 1 to 10, 1 to 5, or 1 to 3, for example, one or two. Also, a mutation having the addition of amino acid residues and the substitution mentioned above in combination can be introduced to the amino acid sequence of a wild-type human neurotrophic factor or a mutation having the addition of amino acid residues and the deletion mentioned above in combination can be introduced to the amino acid sequence of a wild-type human neurotrophic factor. The same applies to neurotrophic factors of non-human animal species.

Further, a combination of three types of mutations, i.e., substitution, deletion and addition of the amino acid residues, can be introduced to the amino acid sequence of a wild-type human neurotrophic factor. For example, amino acid sequences obtained by deleting 1 to 10 amino acid residues from the amino acid sequence of a wild-type human neurotrophic factor, substituting 1 to 10 amino acid residues thereof with different amino acid residues and adding 1 to 10 amino acid residues thereto are regarded as human neurotrophic factors; amino acid sequences obtained by deleting 1 to 5 amino acid residues from the amino acid sequence of a wild-type human neurotrophic factor, substituting 1 to 5 amino acid residues thereof with different amino acid residues and adding 1 to 5 amino acid residues thereto are also regarded as human neurotrophic factors; amino acid sequences obtained by deleting 1 to 3 amino acid residues from the amino acid sequence of a wild-type human neurotrophic factor, substituting 1 to 3 amino acid residues thereof with different amino acid residues and adding 1 to 3 amino acid residues thereto are also regarded as human neurotrophic factors; amino acid sequences obtained by deleting 1 or 2 amino acid residues from the amino acid sequence of a wild-type human neurotrophic factor, substituting 1 or 2 amino acid residues thereof with different amino acid residues and adding 1 or 2 amino acid residues thereto are also regarded as human neurotrophic factors; and amino acid sequences obtained by deleting a single amino acid residue from the amino acid sequence of a wild-type human neurotrophic factor, substituting a single amino acid residue thereof with another amino acid residue and adding a single amino acid residue thereto are also regarded as human neurotrophic factors. The same applies to neurotrophic factors of non-human animal species.

The sites and types (deletion, substitution, and addition) of individual mutations in a human neurotrophic factor mutant compared to a normal wild-type human neurotrophic factor can be easily identified by alignment of amino acid sequences of these human neurotrophic factors. The same applies to neurotrophic factors of non-human animal species.

The amino acid sequence of a human neurotrophic factor mutant exhibits an identity of preferably 80% or more, 85% or more, 90% or more, or 95% or more, for example, an identity of 98% or more or 99% or more, to the amino acid sequence of a normal wild-type human neurotrophic factor. The same applies to neurotrophic factors of non-human animal species.

The substitution of an amino acid in the amino acid sequence of a wild-type human neurotrophic factor with another amino acid is preferably a conservative amino acid substitution. The same applies to neurotrophic factors of non-human animal species.

The wild-type or mutant human neurotrophic factor having a constituent amino acid modified with a sugar chain is regarded as a human neurotrophic factor. Also, the wild-type or mutant human neurotrophic factor having a constituent amino acid modified with a phosphate group is regarded as a human neurotrophic factor. Also, the wild-type or mutant human neurotrophic factor having a constituent amino acid modified with a group except a sugar chain and a phosphate group is regarded as a human neurotrophic factor. Also, the wild-type or mutant human neurotrophic factor having a constituent amino acid whose side chain is converted by, e.g., a substitution reaction, is regarded as a human neurotrophic factor. Examples of such conversion include, but are not limited to, conversion of a cysteine residue to formylglycine. The same applies to neurotrophic factors of non-human animal species.

More specifically, a human neurotrophic factor modified with a sugar chain is meant to be included in a human neurotrophic factor having an original amino acid sequence. Also, a human neurotrophic factor modified with a phosphate group is meant to be included in a human neurotrophic factor having an original amino acid sequence. Also, a human neurotrophic factor modified with a group except a sugar chain and a phosphate group is meant to be included in a human neurotrophic factor having an original amino acid sequence. Also, a human neurotrophic factor having a constituent amino acid whose side chain is converted by, e.g., a substitution reaction, is meant to be included in a human neurotrophic factor having an original amino acid sequence. Examples of such conversion include, but are not limited to, conversion of a cysteine residue to formylglycine. The same applies to neurotrophic factors of non-human animal species.

The term "human brain-derived neurotrophic factor", "human BDNF", or "hBDNF" as used herein simply includes indistinguishably not only a normal wild-type hBDNF consisting of 119 amino acid residues represented by SEQ ID NO: 60 but also hBDNF mutants, which correspond to hBDNF mutants having a substitution, deletion, and/or addition of one or more amino acid residues ("addition" of an amino acid residue herein means adding the residue to a terminal of a sequence or in the sequence) in the amino acid sequence represented by SEQ ID NO: 60, as long as the mutants have a function as the hBDNF, such as a physiological activity as a neurotrophic factor to bind to the specific receptor TrkB on target cell surfaces and promote the generation, growth, maintenance, and regeneration of nerve cells. The wild-type hBDNF is encoded by, for example, a gene having the nucleotide sequence represented by SEQ ID NO: 61. The same applies to BDNFs of non-human animal species.

Note that, the phrase that an hBDNF has a function as the hBDNF herein means that the hBDNF has a specific activity of preferably 10% or more, more preferably 20% or more, further preferably 50% or more and further more preferably 80% or more when the specific activity of a normal wild-type hBDNF is regarded as 100%. The specific activity herein refers to physiological activity per mass of the protein. Note that, the specific activity of a fusion protein of hBDNF and a protein is obtained as a physiological activity per mass of the moiety corresponding to the hBDNF in the fusion protein. Note that, the specific activity of an hBDNF in the fusion protein herein is computationally obtained by multiplying physiological activity of the hBDNF per unit mass of the fusion protein by (the molecular weight of the fusion protein/the molecular weight of the moiety corresponding to the hBDNF in the fusion protein). The same applies to BDNFs of non-human animal species.

When an amino acid residue in the amino acid sequence of a wild-type hBDNF is substituted with another amino acid residue, the number of amino acid residues to be substituted is 1 to 10, 1 to 5, or 1 to 3, for example, one or two. When an amino acid residue in the amino acid sequence of a wild-type hBDNF is deleted, the number of amino acid residues to be deleted is 1 to 10, 1 to 5, or 1 to 3, for example, one or two. Mutants such as an hBDNF mutant consisting of 118 amino acid residues obtained by deleing a single amino acid residue from the N terminal or C terminal of a wild-type hBDNF and an hBDNF mutant consisting of 117 amino acid residues obtained by deleting two amino acid residues from the N terminal or C terminal of a wild-type hBDNF are also regarded as hBDNFs. Also, a mutation having the substitution and deletion of amino acid residues in combination can be introduced to the amino acid sequence of a wild-type hBDNF. The same applies to BDNFs of non-human animal species.

When an amino acid residue is added to the amino acid sequence of a wild-type hBDNF, one or more amino acid residues are added in the amino acid sequence of an hBDNF or added to the N terminal or C terminal of the amino acid sequence thereof. The number of amino acid residues to be added herein is 1 to 10, 1 to 5, or 1 to 3, for example, one or two. Also, a mutation having the addition of amino acid residues and the substitution mentioned above in combination can be introduced to the amino acid sequence of a wild-type hBDNF or a mutation having the addition of amino acid residues and the deletion mentioned above in combination can be introduced to the amino acid sequence of a wild-type hBDNF. The same applies to BDNFs of non-human animal species.

Further, a combination of three types of mutations, i.e., substitution, deletion and addition of the amino acid residues, can be introduced to the amino acid sequence of a wild-type hBDNF. For example, amino acid sequences obtained by deleting 1 to 10 amino acid residues from the amino acid sequence of a wild-type hBDNF represented by SEQ ID NO: 60, substituting 1 to 10 amino acid residues thereof with different amino acid residues and adding 1 to 10 amino acid residues thereto are regarded as hBDNFs; amino acid sequences obtained by deleting 1 to 5 amino acid residues from the amino acid sequence of a wild-type hBDNF represented by SEQ ID NO: 60, substituting 1 to 5 amino acid residues thereof with different amino acid residues and adding 1 to 5 amino acid residues thereto are also regarded as hBDNFs; amino acid sequences obtained by deleting 1 to 3 amino acid residues from the amino acid sequence of a wild-type hBDNF represented by SEQ ID NO: 60, substituting 1 to 3 amino acid residues thereof with different amino acid residues and adding 1 to 3 amino acid residues thereto are also regarded as hBDNFs; amino acid sequences obtained by deleting 1 or 2 amino acid residues from the amino acid sequence of a wild-type hBDNF represented by SEQ ID NO: 60, substituting 1 or 2 amino acid residues thereof with different amino acid residues and adding 1 or 2 amino acid residues thereto are also regarded as hBDNFs; and amino acid sequences obtained by deleting a single amino acid residue from the amino acid sequence of a wild-type hBDNF represented by SEQ ID NO: 60, substituting a single amino acid residue thereof with another amino acid residue and adding a single amino acid residue thereto are also regarded as hBDNFs. The same applies to BDNFs of non-human animal species.

The sites and types (deletion, substitution, and addition) of individual mutations in an hBDNF mutant compared to a normal wild-type hBDNF can be easily identified by alignment of the amino acid sequences of these hBDNFs. The same applies to BDNFs of non-human animal species.

The amino acid sequence of an hBDNF mutant exhibits an identity of preferably 80% or more, 85% or more, 90% or more, or 95% or more, for example, an identity of 98% or more or 99% or more, to the amino acid sequence of a normal wild-type hBDNF represented by SEQ ID NO: 60.

The term "human nerve growth factor", "human NGF", or "hNGF" as used herein simply includes indistinguishably not only a normal wild-type hNGF consisting of 120 amino acid residues represented by SEQ ID NO: 62, but also hNGF mutants, which correspond to hNGF mutants having a substitution, deletion, and/or addition of one or more amino acid residues ("addition" of an amino acid residue herein means adding the residue to a terminal of a sequence or in the sequence) in the amino acid sequence represented by SEQ ID NO: 62, as long as the mutants have a function as the hNGF, such as a physiological activity as a neurotrophic factor such as an action to promote elongation of neuroaxes and synthesis of neurotransmitters, an action to maintain nerve cells, a repairing action for cell injury, and an action to promote functional recovery of cerebral nerves to prevent aging. The wild-type hNGF is encoded by, for example, a gene having the nucleotide sequence represented by SEQ ID NO: 63. The same applies to NGFs of non-human animal species.

Note that, the phrase that hNGF has a function as the hNGF herein means that hNGF has a specific activity of preferably 10% or more, more preferably 20% or more, further preferably 50% or more and further more preferably 80% or more when the specific activity of a normal wild-type hNGF is regarded as 100%. The specific activity herein refers to physiological activity per mass of the protein. Note that, the specific activity of a fusion protein of hNGF and a protein is obtained as a physiological activity per mass of the moiety corresponding to the hNGF in the fusion protein. Note that, the specific activity of hNGF in a fusion protein herein is computationally obtained by multiplying physiological activity of the hNGF per unit mass of the fusion protein by (the molecular weight of the fusion protein/the molecular weight of the moiety corresponding to the hNGF in the fusion protein). The same applies to NGFs of non-human animal species.

When an amino acid residue in the amino acid sequence of a wild-type hNGF is substituted with another amino acid residue, the number of amino acid residues to be substituted is 1 to 10, 1 to 5, or 1 to 3, for example, one or two. When an amino acid residue in the amino acid sequence of a wild-type hNGF is deleted, the number of amino acid residues to be deleted is 1 to 10, 1 to 5, or 1 to 3, for example, one or two. Mutants such as an hNGF mutant consisting of 119 amino acid residues

obtained by deleing a single amino acid residue from the N terminal or C terminal of a wild-type hNGF and an hNGF mutant consisting of 118 amino acid residues obtained by deleting two amino acid residues from the N terminal or C terminal of a wild-type hNGF are also regarded as hNGFs. Also, a mutation having the substitution and deletion of amino acid residues in combination can be introduced to the amino acid sequence of a wild-type hNGF. The same applies to NGFs of non-human animal species.

When an amino acid residue is added to the amino acid sequence of a wild-type hNGF, one or more amino acid residues are added in the amino acid sequence of an hNGF or added to the N terminal or C terminal of the amino acid sequence thereof. The number of amino acid residues to be added herein is 1 to 10, 1 to 5, or 1 to 3, for example, one or two. Also, a mutation having the addition of amino acid residues and the substitution mentioned above in combination can be introduced to the amino acid sequence of a wild-type hNGF or a mutation having the addition of amino acid residues and the deletion mentioned above in combination can be introduced to the amino acid sequence of a wild-type hNGF. The same applies to NGFs of non-human animal species.

Further, a combination of three types of mutations, i.e., substitution, deletion and addition of the amino acid residues, can be introduced to the amino acid sequence of a wild-type hNGF. For example, amino acid sequences obtained by deleting 1 to 10 amino acid residues from the amino acid sequence of a wild-type hNGF represented by SEQ ID NO: 62, substituting 1 to 10 amino acid residues thereof with different amino acid residues and adding 1 to 10 amino acid residues thereto are regarded as hNGFs; amino acid sequences obtained by deleting 1 to 5 amino acid residues from the amino acid sequence of a wild-type hNGF represented by SEQ ID NO: 62, substituting 1 to 5 amino acid residues thereof with different amino acid residues and adding 1 to 5 amino acid residues thereto are also regarded as hNGFs; amino acid sequences obtained by deleting 1 to 3 amino acid residues from the amino acid sequence of a wild-type hNGF represented by SEQ ID NO: 62, substituting 1 to 3 amino acid residues thereof with different amino acid residues and adding 1 to 3 amino acid residues thereto are also regarded as hNGFs; amino acid sequences obtained by deleting 1 or 2 amino acid residues from the amino acid sequence of a wild-type hNGF represented by SEQ ID NO: 62, substituting 1 or 2 amino acid residues thereof with different amino acid residues and adding 1 or 2 amino acid residues thereto are also regarded as hNGFs; and amino acid sequences obtained by deleting a single amino acid residue from the amino acid sequence of a wild-type hNGF represented by SEQ ID NO: 62, substituting a single amino acid residue thereof with another amino acid residue and adding a single amino acid residue thereto are also regarded as hNGFs. The same applies to NGFs of non-human animal species.

The sites and types (deletion, substitution, and addition) of individual mutations in an hNGF mutant compared to a normal wild-type hNGF can be easily identified by alignment of the amino acid sequences of these hNGFs. The same applies to NGFs of non-human animal species.

The amino acid sequence of an hNGF mutant exhibits an identity of preferably 80% or more, 85% or more, 90% or more, or 95% or more, for example, an identity of 98% or more or 99% or more, to the amino acid sequence of a normal wild-type hNGF represented by SEQ ID NO: 62.

The term "human neurotrophin-3", "human NT-3", or "hNT-3" as used herein simply includes indistinguishably not only a normal wild-type hNT-3 consisting of 119 amino acid residues represented by SEQ ID NO: 64 but also hNT-3 mutants, which correspond to hNT-3 mutants having a substitution, deletion, and/or addition of one or more amino acid residues ("addition" of an amino acid residue herein means adding the residue to a terminal of a sequence or in the sequence) in the amino acid sequence represented by SEQ ID NO: 64, as long as the mutants have a function as hNT-3, such as a physiological activity as a nerve growth factor to act via TrkC, the physiological activity being necessary for maintaining the survival of and promoting the differentiation of nerve cells. The wild-type hNT-3 is encoded by, for example, a gene having the nucleotide sequence represented by SEQ ID NO: 65. The same applies to hNT-3s of non-human animal species.

Note that, the phrase that hNT-3 has a function as the hNT-3 herein means that hNT-3 has a specific activity of preferably 10% or more, more preferably 20% or more, further preferably 50% or more and further more preferably 80% or more when the specific activity of a normal wild-type hNT-3 is regarded as 100%. The specific activity herein refers to physiological activity per mass of the protein. Note that, the specific activity of a fusion protein of hNT-3 and a protein is obtained as a physiological activity per mass of the moiety corresponding to the hNT-3 in the fusion protein. Note that, the specific activity of hNT-3 in a fusion protein herein is computationally obtained by multiplying physiological activity of the hNT-3 per unit mass of the fusion protein by (the molecular weight of the fusion protein/the molecular weight of the moiety corresponding to the hNT-3 in the fusion protein). The same applies to hNT-3s of non-human animal species.

When an amino acid residue in the amino acid sequence of a wild-type hNT-3 is substituted with another amino acid residue, the number of amino acid residues to be substituted is 1 to 10, 1 to 5, or 1 to 3, for example, one or two. When an amino acid residue in the amino acid sequence of a wild-type hNT-3 is deleted, the number of amino acid residues to be deleted is 1 to 10, 1 to 5, or 1 to 3, for example, one or two. Mutants such as an hNT-3 mutant consisting of 118 amino acid residues obtained by deleing a single amino acid residue from the N terminal or C terminal of a wild-type hNT-3 and an hNT-3 mutant consisting of 117 amino acid residues obtained by deleting two amino acid residues from the N terminal or C terminal of a wild-type hNT-3 are also regarded as hNT-3s. Also, a mutation having the substitution and deletion of amino acid residues in combination can be introduced to the amino acid sequence of a wild-type hNT-3. The same applies to hNT-3s of non-human animal species.

When an amino acid residue is added to the amino acid sequence of a wild-type hNT-3, one or more amino acid residues are added in the amino acid sequence of an hNT-3 or added to the N terminal or C terminal of the amino acid sequence thereof. The number of amino acid residues to be added herein is 1 to 10, 1 to 5, or 1 to 3, for example, one or two. Also, a mutation having the addition of amino acid residues and the substitution mentioned above in combination can be introduced to the amino acid sequence of a wild-type hNT-3 or a mutation having the addition of amino acid residues and the deletion mentioned above in combination can be introduced to the amino acid sequence of a wild-type hNT-3. The same applies to hNT-3s of non-human animal species.

Further, a combination of three types of mutations, i.e., substitution, deletion and addition of the amino acid residues, can be introduced to the amino acid sequence of a wild-type hNT-3. For example, amino acid sequences obtained by deleting 1 to 10 amino acid residues from the amino acid sequence of a wild-type hNT-3 represented by SEQ ID NO: 64, substituting 1 to 10 amino acid residues thereof with different amino acid residues and adding 1 to 10 amino acid residues thereto are regarded as hNT-3s; amino acid sequences obtained by deleting 1 to 5 amino acid residues from the amino acid sequence of a wild-type hNT-3 represented by SEQ ID NO: 64, substituting 1 to 5 amino acid residues thereof with different amino acid residues and adding 1 to 5 amino acid residues thereto are also regarded as hNT-3s; amino acid sequences obtained by deleting 1 to 3 amino acid residues from the amino acid sequence of a wild-type hNT-3 represented by SEQ ID NO: 64, substituting 1 to 3 amino acid residues thereof with different amino acid residues and adding 1 to 3 amino acid residues thereto are also regarded as hNT-3s; amino acid sequences obtained by deleting 1 or 2 amino acid residues from the amino acid sequence of a wild-type hNT-3 represented by SEQ ID NO: 64, substituting 1 or 2 amino acid residues thereof with different amino acid residues and adding 1 or 2 amino acid residues thereto are also regarded as hNT-3s; and amino acid sequences obtained by deleting a single amino acid residue from the amino acid sequence of a wild-type hNT-3 represented by SEQ ID NO: 64, substituting a single amino acid residue thereof with another amino acid residue and adding a single amino acid residue thereto are also regarded as hNT-3s. The same applies to hNT-3s of non-human animal species.

The sites and types (deletion, substitution, and addition) of individual mutations in an hNT-3 mutant compared to a normal wild-type hNT-3 can be easily identified by alignment of the amino acid sequences of these hNT-3s. The same applies to hNT-3s of non-human animal species.

The amino acid sequence of an hNT-3 mutant exhibits an identity of preferably 80% or more, 85% or more, 90% or more, or 95% or more, for example, an identity of 98% or more or 99% or more, to the amino acid sequence of a normal wild-type hNT-3 represented by SEQ ID NO: 64.

The term "human neurotrophin-4", "human NT-4", or "hNT-4" as used herein simply includes indistinguishably not only a normal wild-type hNT-4 consisting of 130 amino acid residues represented by SEQ ID NO: 66 but also hNT-4 mutants, which correspond to hNT-4 mutants having a substitution, deletion, and/or addition of one or more amino acid residues ("addition" of an amino acid residue herein means adding the residue to a terminal of a sequence or in the sequence) in the amino acid sequence represented by SEQ ID NO: 66, as long as the mutants have a function as hNT-4, such as a physiological activity as a nerve growth factor to act via TrkB, the physiological activity being necessary for maintaining the survival of and promoting the differentiation of nerve cells. The wild-type hNT-4 is encoded by, for example, a gene having the nucleotide sequence represented by SEQ ID NO: 67. The same applies to hNT-4s of non-human animal species.

Note that, the phrase that hNT-4 has a function as the hNT-4 herein means that hNT-4 has a specific activity of preferably 10% or more, more preferably 20% or more, further preferably 50% or more and further more preferably 80% or more when the specific activity of a normal wild-type hNT-4 is regarded as 100%. The specific activity herein refers to physiological activity per mass of the protein. Note that, the specific activity of a fusion protein of hNT-4 and a protein is obtained as a physiological activity per mass of the moiety corresponding to the hNT-4 in the fusion protein. Note that, the specific activity of hNT-4 in a fusion protein herein is computationally obtained by multiplying physiological activity of the hNT-4 per unit mass of the fusion protein by (the molecular weight of the fusion protein/the molecular weight of the moiety corresponding to the hNT-4 in the fusion protein). The same applies to hNT-4s of non-human animal species.

When an amino acid residue in the amino acid sequence of a wild-type hNT-4 is substituted with another amino acid residue, the number of amino acid residues to be substituted is 1 to 10, 1 to 5, or 1 to 3, for example, one or two. When an amino acid residue in the amino acid sequence of a wild-type hNT-4 is deleted, the number of amino acid residues to be deleted is 1 to 10, 1 to 5, or 1 to 3, for example, one or two. Mutants such as an hNT-4 mutant consisting of 129 amino acid residues obtained by deleing a single amino acid residue from the N terminal or C terminal of a wild-type hNT-4 and an hNT-4 mutant consisting of 128 amino acid residues obtained by deleting two amino acid residues from the N terminal or C terminal of a wild-type hNT-4 are also regarded as hNT-4s. Also, a mutation having the substitution and deletion of amino acid residues in combination can be introduced to the amino acid sequence of a wild-type hNT-4. The same applies to hNT-4s of non-human animal species.

When an amino acid residue is added to the amino acid sequence of a wild-type hNT-4, one or more amino acid residues are added in the amino acid sequence of an hNT-4 or added to the N terminal or C terminal of the amino acid sequence thereof. The number of amino acid residues to be added herein is 1 to 10, 1 to 5, or 1 to 3, for example, one or two. Also, a mutation having the addition of amino acid residues and the substitution mentioned above in combination can be introduced to the amino acid sequence of a wild-type hNT-4 or a mutation having the addition of amino acid residues and the deletion mentioned above in combination can be introduced to the amino acid sequence of a wild-type hNT-4. The same applies to hNT-4s of non-human animal species.

Further, a combination of three types of mutations, i.e., substitution, deletion and addition of the amino acid residues, can be introduced to the amino acid sequence of a wild-type hNT-4. For example, amino acid sequences obtained by deleting 1 to 10 amino acid residues from the amino acid sequence of a wild-type hNT-4 represented by SEQ ID NO: 66, substituting 1 to 10 amino acid residues thereof with different amino acid residues and adding 1 to 10 amino acid residues thereto are regarded as hNT-4s; amino acid sequences obtained by deleting 1 to 5 amino acid residues from the amino acid sequence of a wild-type hNT-4 represented by SEQ ID NO: 66, substituting 1 to 5 amino acid residues thereof with different amino acid residues and adding 1 to 5 amino acid residues thereto are also regarded as hNT-4s; amino acid sequences obtained by deleting 1 to 3 amino acid residues from the amino acid sequence of a wild-type hNT-4 represented by SEQ ID NO: 66, substituting 1 to 3 amino acid residues thereof with different amino acid residues and adding 1 to 3 amino acid residues thereto are also regarded as hNT-4s; amino acid sequences obtained by deleting 1 or 2 amino acid residues from the amino acid sequence of a wild-type hNT-4 represented by SEQ ID NO: 66, substituting 1 or 2 amino acid residues thereof with different amino acid residues and adding 1 or 2 amino acid residues thereto are also regarded as hNT-4s; and amino acid sequences obtained by deleting a single amino acid residue from the amino acid sequence of a wild-type hNT-4 represented by SEQ ID NO: 66, substituting a single amino acid residue thereof with another amino acid residue and adding a single amino acid residue thereto are also regarded as hNT-4s. The same applies to hNT-4s of non-human animal species.

The sites and types (deletion, substitution, and addition) of individual mutations in an hNT-4 mutant compared to a normal wild-type hNT-4 can be easily identified by alignment of the amino acid sequences of these hNT-4s. The same applies to hNT-4s of non-human animal species.

The amino acid sequence of an hNT-4 mutant exhibits an identity of preferably 80% or more, 85% or more, 90% or more, or 95% or more, for example, an identity of 98% or more or 99% or more, to the amino acid sequence of a normal wild-type hNT-4 represented by SEQ ID NO: 66.

The term "human cerebral dopamine neurotrophic factor", "human CDNF", or "hCDNF" as used herein simply includes indistinguishably not only a normal wild-type hCDNF having an amino acid sequence obtained by removing from the amino acid sequence represented by SEQ ID NO: 156 the amino acid sequence of His6 at the C terminal but also hCDNF mutants, which correspond to hCDNF mutants having a substitution, deletion, and/or addition of one or more amino acid residues ("addition" of an amino acid residue herein means adding the residue to a terminal of a sequence or in the sequence) in the wild-type amino acid sequence, as long as the mutants have a function as the hCDNF, such as promotion of the survival and differentiation of dopaminergic neurons. The same applies to human mesencephalic astrocyte-derived neurotrophic factor (hMANF), which belongs to the same family as hCDNF. The above matters on hBDNF, hNGF, hNT-3, and hNT-4 can also be applied to hCDNF and hMANF.

The term "human glial cell line-derived neurotrophic factor", "human GDNF", or "hGDNF" as used herein simply includes indistinguishably not only a normal wild-type hGDNF having an amino acid sequence obtained by removing from the amino acid sequence represented by SEQ ID NO: 162 the amino acid sequence of His6 at the C terminal but also hGDNF mutants, which correspond to hGDNF mutants having a substitution, deletion, and/or addition of one or more amino acid residues ("addition" of an amino acid residue herein means adding the residue to a terminal of a sequence or in the sequence) in the wild-type amino acid sequence, as long as the mutants have a function as the GCDNF, such as promotion of the survival and differentiation of dopaminergic neurons. The above matters on hBDNF, hNGF, hNT-3, and hNT-4 can also be applied to hGDNF.

The term "human neurturin", "human NRTN", or "hNRTN" as used herein simply includes indistinguishably not only a normal wild-type hNRTN having an amino acid sequence obtained by removing from the amino acid sequence represented by SEQ ID NO: 168 the amino acid sequence of His6 at the C terminal but also hNRTN mutants, which correspond to hNRTN mutants having a substitution, deletion, and/or addition of one or more amino acid residues ("addition" of an amino acid residue herein means adding the residue to a terminal of a sequence or in the sequence) in the wild-type amino acid sequence, as long as the mutants have a function as the hNRTN, such as maintenance of the survival of many types of neurons such as dopaminergic neurons. The above matters on hBDNF, hNGF, hNT-3, and hNT-4 can also be applied to hNRTN.

The term "human artemin", "human ARTN" , or "hARTN" as used herein simply includes indistinguishably not only a normal wild-type hARTN having an amino acid sequence obtained by removing from the amino acid sequence represented by SEQ ID NO: 174 the amino acid sequence of His6 at the C terminal but also hARTN mutants, which correspond to hARTN mutants having a substitution, deletion, and/or addition of one or more amino acid residues ("addition" of an amino acid residue herein means adding the residue to a terminal of a sequence or in the sequence) in the wild-type amino acid sequence, as long as the mutants have a function as the hARTN, such as promotion of the survival and differentiation of dopaminergic neurons. The above matters on hBDNF, hNGF, hNT-3, and hNT-4 can also be applied to hARTN.

The term "human persephin", "human PSPN", or "hPSPN" as used herein simply includes indistinguishably not only a normal wild-type hPSPN having an amino acid sequence obtained by removing from the amino acid sequence represented by SEQ ID NO: 180 the amino acid sequence of His6 at the C terminal but also hPSPN mutants, which correspond to hPSPN mutants having a substitution, deletion, and/or addition of one or more amino acid residues ("addition" of an amino acid residue herein means adding the residue to a terminal of a sequence or in the sequence) in the wild-type amino acid sequence, as long as the mutants have a function as the hPSPN, such as promotion of the survival of cholinergic neurons in the basal forebrain and motor neurons in the spinal cord. The above matters on hBDNF, hNGF, hNT-3, and hNT-4 can also be applied to hPSPN.

The substitution of an amino acid in the amino acid sequence of a wild-type hBDNF with another amino acid, the substitution of an amino acid in the amino acid sequence of a wild-type hNGF with another amino acid, the substitution of an amino acid in the amino acid sequence of a wild-type hNT-3 with another amino acid, and the substitution of an amino acid in the amino acid sequence of a wild-type hNT-4 with another amino acid are each a conservative amino acid substitution, for example. The same applies to any of those neurotrophic factors of non-human animal species. The same applies to other neurotrophic factors.

The wild-type or mutant hBDNF, hNGF, hNT-3, or hNT-4 having a constituent amino acid modified with a sugar chain is regarded as hBDNF, hNGF, hNT-3, or hNT-4, respectively. Also, the same applies to any of those wild-type or mutant human neurotrophic factors having a constituent amino acid modified with a phosphate group. Also, the same applies to any of those wild-type or mutant human neurotrophic factors having a constituent amino acid modified with a group except a sugar chain and a phosphate group. Also, the same applies to any of those wild-type or mutant human neurotrophic factors having a constituent amino acid whose side chain is converted by, e.g., a substitution reaction. Examples of such conversion include, but are not limited to, conversion of a cysteine residue to formylglycine. The same applies to hBDNF, hNGF, hNT-3, and hNT-4. The same applies to any of those neurotrophic factors of non-human animal species. The same applies to other neurotrophic factors.

More specifically, hBDNF, hNGF, hNT-3, or hNT-4 modified with a sugar chain is meant to be included respectively in hBDNF, hNGF, hNT-3, or hNT-4 having an original amino acid sequence. Also, the same applies to any of those human neurotrophic factors modified with a phosphate group. Also, the same applies to any of those human neurotrophic factors modified with a group except a sugar chain and a phosphate group. Also, the same applies to any of those human neurotrophic factors having a constituent amino acid whose side chain is converted by, e.g., a substitution reaction. Examples of such conversion include conversion of a cysteine residue to formylglycine. The same applies to neurotrophic factors of non-human animal species. The same applies to other neurotrophic factors.

An embodiment of the present invention is a recombinant protein obtained by fusing a wild-type or mutant neurotrophic factor with HSA. The fusion protein, when expressed as a recombinant protein using a host cell such as CHO, particularly expressed as a recombinant protein so as to be secreted from the cell and accumulated in a culture solution, can provide an expression level as a human neurotrophic factor in the culture supernatant in terms of concentration or physiological activity, that is at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, 10 to 20 times, or 20 to 30 times that of the wild-type human neurotrophic factors expressed as a recombinant protein in the same manner.

Also, an embodiment of the present invention is a recombinant protein obtained by fusing a wild-type or mutant hBDNF with HSA. The fusion protein, when expressed as a recombinant protein using a host cell such as CHO, particularly expressed as a recombinant protein so as to be secreted from the cell and accumulated in a culture solution, can provide an expression level as hBDNF in the culture supernatant in terms of concentration or physiological activity, that is at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, 10 to 20 times, or 20 to 30 times that of the wild-type hBDNF expressed as a recombinant protein in the same manner.

Also, an embodiment of the present invention is a recombinant protein obtained by fusing a wild-type or mutant hNGF with HSA. The fusion protein, when expressed as a recombinant protein using a host cell such as CHO, particularly expressed as a recombinant protein so as to be secreted from the cell and accumulated in a culture solution, can provide an expression level as hNGF in the culture supernatant in terms of concentration or physiological activity, that is at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, 10 to 20 times, or 20 to 30 times that of the wild-type hNGF expressed as a recombinant protein in the same manner.

Also, an embodiment of the present invention is a recombinant protein obtained by fusing a wild-type or mutant hNT-3 with HSA. The fusion protein, when expressed as a recombinant protein using a host cell such as CHO, particularly expressed as a recombinant protein so as to be secreted from the cell and accumulated in a culture solution, can provide an expression level as hNT-3 in the culture supernatant in terms of concentration or physiological activity, that is at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, 10 to 20 times, or 20 to 30 times that of the wild-type hNT-3 expressed as a recombinant protein in the same manner.

Also, an embodiment of the present invention is a recombinant protein obtained by fusing a wild-type or mutant hNT-4 with HSA. The fusion protein, when expressed as a recombinant protein using a host cell such as CHO, particularly expressed as a recombinant protein so as to be secreted from the cell and accumulated in a culture solution, can provide an expression level as hNT-4 in the culture supernatant in terms of concentration or physiological activity, that is at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, 10 to 20 times, or 20 to 30 times that of the wild-type hNT-4 expressed as a recombinant protein in the same manner.

Also, an embodiment of the present invention is a recombinant protein obtained by fusing a wild-type hCDNF with HSA. The fusion protein, when expressed as a recombinant protein using a host cell such as CHO, particularly expressed as a recombinant protein so as to be secreted from the cell and accumulated in a culture solution, can provide an expression level as hCDNF in the culture supernatant in terms of concentration or physiological activity, that is at least 1.5 times or more, 2 times or more, 2.5 times or more, or 5 times or more, for example, 1.5 to 4 times or 2.0 to 5.0 times that of the wild-type hCDNF expressed as a recombinant protein in the same manner. The same applies to hMANF.

Also, an embodiment of the present invention is a recombinant protein obtained by fusing a wild-type hGDNF with HSA. The fusion protein, when expressed as a recombinant protein using a host cell such as CHO, particularly expressed as a recombinant protein so as to be secreted from the cell and accumulated in a culture solution, can provide an expression level as hGDNF in the culture supernatant in terms of concentration or physiological activity, that is at least 1.5 times or more, 2 times or more, 2.5 times or more, or 5 times or more, for example, 1.5 to 4 times or 2.0 to 5.0 times that of the wild-type hGDNF expressed as a recombinant protein in the same manner.

Also, an embodiment of the present invention is a recombinant protein obtained by fusing a wild-type hNRTN with HSA, a recombinant protein obtained by fusing a wild-type hARTN with HSA, and a recombinant protein obtained by fusing a wild-type PSPN with HSA. The fusion proteins can be each obtained as a recombinant protein in a culture solution when it is expressed as a recombinant protein using a host cell such as CHO, particularly expressed as a recombinant protein so as to be secreted from the cell and accumulated in a culture solution. The wild-type hNRTN, wild-type hARTN, and wild-type PSPN are hardly expressed with the same method.

An embodiment of the present invention relates to a fusion protein obtained by binding a polypeptide containing an amino acid sequence of a human neurotrophic factor and a polypeptide containing an amino acid sequence of SA. The phrase "binding polypeptides" refers to covalently binding different polypeptides directly or indirectly via a linker. The neurotrophic factor herein is preferably a human neurotrophic factor. Also, the SA is preferably HSA. Also, the human neurotrophic factor is, for example, hBDNF, hNGF, hNT-3, or hNT-4. The same applies to neurotrophic factors of non-human animal species. The same applies to other neurotrophic factors.

As an example method for binding two different polypeptides, it is general to employ the following method: to a downstream site of a gene encoding one of the polypeptides, a gene encoding the other polypeptide is bound in frame to prepare a DNA fragment, the DNA fragment is integrated into an expression vector, and a host cell is transformed with the expression vector and cultured to express a recombinant protein. The recombinant protein obtained is a single-chain polypeptide having the two polypeptides bound directly by a peptide bond, or via another amino acid sequence.

In an embodiment of the present invention, the term "SA-human neurotrophic factor fusion protein" or "SA-human neurotrophic factor" refers to a fusion protein having an amino acid sequence in which the N terminal of the amino acid sequence of a human neurotrophic factor is bound to the C terminal of the SA amino acid sequence directly or via a linker, and having a function as a human neurotrophic factor. The animal species of SA is not particularly limited and SA is preferably mammal SA, more preferably primate SA, and further preferably HSA. The phrase that a human neurotrophic factor has a function as a human neurotrophic factor in the SA-human neurotrophic factor fusion protein means that the human neurotrophic factor has a specific activity of preferably 10% or more, more preferably 20% or more, further preferably 50% or more and further more preferably 80% or more, when the specific activity of a normal wild-type human neurotrophic factor is regarded as 100%. Note that, the specific activity of the human neurotrophic factor in the SA-human neurotrophic factor fusion protein herein is computationally obtained by multiplying the physiological activity of a human neurotrophic factor of the fusion protein per unit mass by (the molecular weight of the fusion protein/the molecular weight of the moiety corresponding to the human neurotrophic factor in the fusion protein).

In an embodiment of the present invention, the term "HSA-human neurotrophic factor fusion protein" or "HSA-human neurotrophic factor" refers to a fusion protein having an amino acid sequence in which the N terminal of the amino acid sequence of a human neurotrophic factor is bound to the C terminal of the HSA amino acid sequence directly or via a linker, and having a function as a human neurotrophic factor. To the case where an HSA-human neurotrophic factor fusion protein has a function as a human neurotrophic factor, the definition in the case of the SA-neurotrophic factor fusion protein mentioned above is applicable.

In the SA-human neurotrophic factor fusion protein, SA preferably has a function as SA, such as a function to bind an endogenous substance and an exogenous substance such as a drug in the blood and transport them, but is not limited to this. The same applies to an HSA-human neurotrophic factor fusion protein. The same applies to an HSA-hBDNF fusion protein, an HSA-hNGF fusion protein, an HSA-hNT-3 fusion protein, and an HSA-hNT-4 fusion protein. The same applies to other nutritional factors.

In a case where a mutation is introduced to an HSA-human neurotrophic factor fusion protein, which is a fusion protein of a wild-type HSA and a wild-type human neurotrophic factor, the mutation can be introduced only to the HSA moiety and not introduced to the human neurotrophic factor moiety; the mutation can be introduced only to the human neurotrophic factor moiety without being introduced to the HSA moiety; or the mutation can be introduced to both of the HSA moiety and the human neurotrophic factor moiety. In a case where a mutation is introduced only to the HSA moiety, the amino acid sequence of the moiety is an amino acid sequence of HSA obtained by introducing a mutation to a wild-type HSA as mentioned above. In a case where a mutation is introduced only to a human neurotrophic factor moiety, the amino acid sequence of the moiety is an amino acid sequence of a human neurotrophic factor having a mutation to a wild-type human neurotrophic factor as mentioned above. In a case where a mutation is introduced to both of the HSA moiety and the human neurotrophic factor moiety, the amino acid sequence of the HSA moiety is an amino acid sequence of HSA obtained by introducing a mutation to a wild-type HSA as mentioned above, and the amino acid sequence of the human neurotrophic factor moiety is an amino acid sequence of a human neurotrophic factor obtained by introducing a mutation to a wild-type human neurotrophic factor as mentioned above. The same applies to a fusion protein of wild-type SA of a non-human animal species and a wild-type human neurotrophic factor.

An HSA-human neurotrophic factor fusion protein having a constituent amino acid modified with a sugar chain is also regarded as an HSA-human neurotrophic factor fusion protein. Also, an HSA-human neurotrophic factor fusion protein having a constituent amino acid modified with a phosphate group is regarded as an HSA-human neurotrophic factor fusion protein. Also, an HSA-human neurotrophic factor fusion protein modified with a group except a sugar chain and a phosphate group is regarded as an HSA-human neurotrophic factor fusion protein. Also, an HSA-human neurotrophic factor fusion protein having a constituent amino acid whose side chain is converted by, e.g., a substitution reaction, is regarded as an HSA-human neurotrophic factor fusion protein. Examples of such conversion include, but are not limited to, conversion of a cysteine residue to formylglycine. The same applies to a fusion protein (SA-human neurotrophic factor) of SA of a non-human animal species and a human neurotrophic factor.

More specifically, an HSA-human neurotrophic factor fusion protein modified with a sugar chain is meant to be included in an HSA-human neurotrophic factor fusion protein having an original amino acid sequence. Also, an HSA-human neurotrophic factor fusion protein modified with a phosphate group is meant to be included in an HSA-human neurotrophic factor fusion protein having an original amino acid sequence. Also, an HSA-human neurotrophic factor fusion protein modified with a group except a sugar chain and a phosphate group is meant to be included in an HSA-human neurotrophic factor fusion protein having an original amino acid sequence. Also, an HSA-human neurotrophic factor fusion protein having a constituent amino acid whose side chain is converted by, e.g., a substitution reaction, is meant to be included in an HSA-human neurotrophic factor fusion protein having an original amino acid sequence. Examples of such conversion include, but are not limited to, conversion of a cysteine residue to formylglycine. The same applies to a fusion protein (SA-human neurotrophic factor) of SA of a non-human animal species and a human neurotrophic factor.

Also, an HSA-human neurotrophic factor fusion protein in which a constituent human neurotrophic factor is a precursor of a human neurotrophic factor is regarded as an HSA-human neurotrophic factor fusion protein. The precursor as used herein refers to a moiety which functions as a human neurotrophic factor of an HSA-human neurotrophic factor fusion protein biologically synthesized, is separated from the fusion protein during a production process or in a living body to which the fusion protein is administered and serves as a human neurotrophic factor by itself. In this case, sometimes, the HSA-human neurotrophic factor fusion protein biologically synthesized is cleaved at a predetermined site with, e.g., a hydrolytic enzyme to separate an HSA-containing moiety and a matured human neurotrophic factor-containing moiety. In this case, the human resulting neurotrophic factor is not a fusion protein with HSA but the HSA-human neurotrophic factor fusion protein is once synthesized during a process for producing the human neurotrophic factor. Accordingly, in a case where a human neurotrophic factor is produced by such a method, the production method is included in a method for producing HSA-human neurotrophic factor fusion protein. The same applies to a fusion protein (SA-human neurotrophic factor) of SA of a non-human animal species and a human neurotrophic factor.

In an embodiment of the present invention, the term "SA-hBDNF fusion protein" or "SA-hBDNF" refers to a fusion protein having an amino acid sequence in which the N terminal of the amino acid sequence of hBDNF is bound to the C terminal of the amino acid sequence of SA directly or via a linker, and having a function as hBDNF. The animal species of SA is not particularly limited and SA is preferably mammal SA, more preferably primate SA, and further preferably HSA. In an SA-hBDNF fusion protein, the phrase that hBDNF has a function as hBDNF means that hBDNF has a specific activity of preferably 10% or more, more preferably 20% or more, further preferably 50% or more, further more preferably 80% or more when the specific activity of a normal wild-type hBDNF is regarded as 100%. Note that the specific activity of the hBDNF in the SA-hBDNF fusion protein herein is computationally obtained by multiplying the physiological activity of an hBDNF per unit mass of the fusion protein by (the molecular weight of the fusion protein/the molecular weight of the moiety corresponding to the hBDNF in the fusion protein).

In an embodiment of the present invention, the term "HSA-hBDNF fusion protein" or "HSA-hBDNF" refers to a fusion protein having an amino acid sequence in which the N terminal of the amino acid sequence of hBDNF is bound to the C terminal of the amino acid sequence of HSA directly or via a linker, and having a function as hBDNF. To a case where an HSA-hBDNF fusion protein has a function as hBDNF, the definition in the case of the SA-hBDNF fusion protein mentioned above is applicable.

In an embodiment of the present invention, a preferable HSA-hBDNF fusion protein has the amino acid sequence represented by, for example, SEQ ID NO: 68. The HSA-hBDNF fusion protein represented by SEQ ID NO: 68 is a fusion protein in which a wild-type hBDNF is bound to the C terminal of a wild-type HSA via a linker sequence Gly-Ser. An HSA-hBDNF fusion protein represented by SEQ ID NO: 68 is encoded by a gene having the nucleotide sequence represented by, for example, SEQ ID NO: 69. Also, a fusion protein having an amino acid sequence represented by SEQ ID NO: 68 and further having a mutation such as a substitution with different amino acid residues, a deletion, or an addition of one or more amino acid residues therein is included in the HSA-hBDNF fusion protein as long as it has a function as hBDNF. The HSA-hBDNF fusion protein preferably has a function as human serum albumin, such as a function to bind an endogenous substance and an exogenous substance such as a drug in the blood and transport them, but is not limited to this.

In a case where a mutation is introduced to an HSA-hBDNF fusion protein, which is a fusion protein of a wild-type HSA and a wild-type hBDNF, the mutation can be introduced only to the HSA moiety and not introduced to the hBDNF moiety; the mutation can be introduced only to the hBDNF moiety without being introduced to the HSA moiety; or the mutation can be introduced to both of the HSA moiety and the hBDNF moiety. In a case where a mutation is introduced only to the HSA moiety, the amino acid sequence of the moiety is an amino acid sequence of HSA obtained by introducing a mutation to a wild-type HSA as mentioned above. In a case where a mutation is introduced only to the hBDNF moiety, the amino acid sequence of the moiety is an amino acid sequence of hBDNF obtained by introducing a mutation to the wild-type hBDNF as mentioned above. In a case where a mutation is introduced to both of the HSA moiety and the hBDNF moiety, the amino acid sequence of the HSA moiety is an amino acid sequence of HSA obtained by introducing a mutation to a wild-type HSA as mentioned above, and the amino acid sequence of the hBDNF moiety is and amino acid sequence of hBDNF obtained by introducing a mutation to a wild-type hBDNF as mentioned above. The same applies to the HSA-hBDNF fusion protein having the amino acid sequence represented by SEQ ID NO: 68. The same applies to a fusion protein of a wild-type SA of a non-human animal species and a wild-type hBDNF.

The case where a mutation is introduced to an HSA-hBDNF fusion protein having the amino acid sequence represented by SEQ ID NO: 68 will be described below. When an amino acid residue in the amino acid sequence represented by SEQ ID NO: 68 is substituted with another amino acid residue, the number of amino acid residues to be substituted is 1 to 10, 1 to 5, or 1 to 3, for example, one or two. When an amino acid residue is deleted, the number of amino acid residues to be deleted is 1 to 10, 1 to 5, or 1 to 3, for example, one or two. When an amino acid residue is added, one or more amino acid residues are added in the amino acid sequence represented by SEQ ID NO: 68 or added to the N terminal or C terminal of the amino acid sequence. The HSA-hBDNF fusion protein may have a combination of the substitution, deletion, and addition of amino acid residues. A mutation may be introduced only to the HSA moiety, or only to the hIL-10 moiety, or to both of them.

When mutations are introduced to the amino acid sequence represented by SEQ ID NO: 68, the sites and types (deletion, substitution, and addition) of individual mutations can be easily identified by alignment of the amino acid sequences before and after introduction of the mutations. The amino acid sequence having a mutation introduced thereto exhibits an identity of preferably 80% or more, 85% or more, 90% or more, or 95% or more, for example, an identity of 98% or more or 99% or more, to the amino acid sequence represented by SEQ ID NO: 68.

An HSA-hBDNF fusion protein having a constituent amino acid modified with a sugar chain is regarded as an HSA-hBDNF fusion protein. Also, an HSA-hBDNF fusion protein having a constituent amino acid modified with a phosphate group is regarded as an HSA-hBDNF fusion protein. Also, an HSA-hBDNF fusion protein modified with a group except a sugar chain and a phosphate group is regarded as an HSA-hBDNF fusion protein. Also, an HSA-hBDNF fusion protein having a constituent amino acid whose side chain is converted by, e.g., a substitution reaction, is regarded as an HSA-hBDNF fusion protein. Examples of such conversion include, but are not limited to, conversion of a cysteine residue to formylglycine. The same applies to a fusion protein (SA-hBDNF) of SA of a non-human animal species and hBDNF.

More specifically, an HSA-hBDNF fusion protein modified with a sugar chain is meant to be included in an HSA-hBDNF fusion protein having an original amino acid sequence. Also, an HSA-hBDNF fusion protein modified with a phosphate group is meant to be included in an HSA-hBDNF fusion protein having an original amino acid sequence. Also, an HSA-hBDNF fusion protein modified with a group except a sugar chain and a phosphate group is meant to be included in an HSA-hBDNF fusion protein having an original amino acid sequence. Also, an HSA-hBDNF fusion protein having a constituent amino acid whose side chain is converted by, e.g., a substitution reaction, is meant to be included in an HSA-hBDNF fusion protein having an original amino acid sequence. Examples of such conversion include, but are not limited to, conversion of a cysteine residue to formylglycine. The same applies to a fusion protein (SA-hBDNF) of SA of a non-human animal species and hBDNF.

An HSA-hBDNF fusion protein in which a constituent hBDNF is a precursor of hBDNF is regarded as an HSA-hBDNF fusion protein. The precursor as used herein refers to a moiety which functions as an hBDNF of an HSA-hBDNF fusion protein biologically synthesized, is separated from the fusion protein during a production process or in a living body to which the fusion protein is administered and serves as an hBDNF by itself. In this case, sometimes the HSA-hBDNF fusion protein biologically synthesized is cleaved at a predetermined site with, e.g., a hydrolytic enzyme to separate an HSA-containing moiety and a matured hBDNF-containing moiety. The resulting hBDNF is not a fusion protein with HSA but the HSA-hBDNF fusion protein is once synthesized during a process for producing hBDNF. Accordingly, in a case where hBDNF is produced by such a method, the production method is included in a method for producing an HSA-hBDNF fusion protein. The same applies to a fusion protein (SA-hBDNF) of SA of a non-human animal species and hBDNF.

In an embodiment of the present invention, the term "SA-hNGF fusion protein" or "SA-hNGF" refers to a fusion protein having an amino acid sequence in which the N terminal of the amino acid sequence of hNGF is bound to the C terminal of the amino acid sequence of SA, directly or via a linker, and having functions as hNGF. The animal species of SA is not particularly limited and is preferably mammal SA, more preferably primate SA, and further preferably HSA. In an SA-hNGF fusion protein, the phrase that hNGF has a function as hNGF means that hNGF has a specific activity of preferably 10% or more, more preferably 20% or more, further preferably 50% or more, further more preferably 80% or more when the specific activity of a normal wild-type hNGF is regarded as 100%. Note that the specific activity of the hNGF in the SA-hNGF fusion protein herein is computationally obtained by multiplying the physiological activity of an hNGF per unit mass of the fusion protein by (the molecular weight of the fusion protein/the molecular weight of the moiety corresponding to the hNGF in the fusion protein).

In an embodiment of the present invention, the term "HSA-hNGF fusion protein" or "HSA-hNGF" refers to a fusion protein having an amino acid sequence in which the N terminal of the amino acid sequence of hNGF is bound to the C terminal of the amino acid sequence of HSA directly or via a linker, and having a function as hNGF. To a case where an HSA-hNGF fusion protein has a function as hNGF, the definition in the case of the SA-hNGF fusion protein mentioned above is applicable.

In an embodiment of the present invention, a preferable HSA-hNGF fusion protein has the amino acid sequence represented by, for example, SEQ ID NO: 70. The HSA-hNGF fusion protein represented by SEQ ID NO: 70 is a fusion protein in which a wild-type hNGF is bound to the C terminal of a wild-type HSA via a linker sequence Gly-Ser. An HSA-hNGF fusion protein represented by SEQ ID NO: 70 is encoded by a gene having the nucleotide sequence represented by, for example, SEQ ID NO: 71. Also, a fusion protein having an amino acid sequence represented by SEQ ID NO: 70 and further having a mutation such as a substitution with different amino acid residues, a deletion, or an addition of one or more amino acid residues therein is included in the HSA-hNGF fusion protein as long as it has a function as hNGF. The HSA-hNGF fusion protein preferably has a function as human serum albumin, such as a function to bind an endogenous substance and an exogenous substance such as a drug in the blood and transport them, but is not limited to this.

In a case where a mutation is introduced to an HSA-hNGF fusion protein, which is a fusion protein of a wild-type HSA and a wild-type hNGF, the mutation can be introduced only to the HSA moiety and not introduced to the hNGF moiety; the mutation can be introduced only to the hNGF moiety without being introduced to the HSA moiety; or the mutation can be introduced to both of the HSA moiety and the hNGF moiety. In a case where a mutation is introduced only to the HSA moiety, the amino acid sequence of the moiety is an amino acid sequence of HSA obtained by introducing a mutation to a wild-type HSA as mentioned above. In a case where a mutation is introduced only to the hNGF moiety, the amino acid sequence of the moiety is an amino acid sequence of hNGF obtained by introducing a mutation to the wild-type hNGF as mentioned above. In a case where a mutation is introduced to both of the HSA moiety and the hNGF moiety, the amino acid sequence of the HSA moiety is an amino acid sequence of HSA having a mutation to a wild-type HSA as mentioned above, and the amino acid sequence of the hNGF moiety is an amino acid sequence of hNGF obtained by introducing a mutation to a wild-type hNGF as mentioned above. The same applies to the HSA-hNGF fusion protein having the amino acid sequence represented by SEQ ID NO: 70. The same applies to a fusion protein of a wild-type SA of a non-human animal species and a wild-type hNGF.

The case where a mutation is introduced to an HSA-hNGF fusion protein having the amino acid sequence represented by SEQ ID NO: 70 will be described below. When an amino acid residue in the amino acid sequence represented by SEQ ID NO: 70 is substituted with another amino acid residue, the number of amino acid residues to be substituted is 1 to 10, 1 to 5, or 1 to 3, for example, one or two. When an amino acid residue is deleted, the number of amino acid residues to be deleted is 1 to 10, 1 to 5, or 1 to 3, for example, one or two. When an amino acid residue is added, one or more amino acid residues are added in the amino acid sequence represented by SEQ ID NO: 70 or added to the N terminal or C terminal of the amino acid sequence. The HSA-hNGF fusion protein may have a combination of the substitution, deletion, and addition of amino acid residues. A mutation may be introduced only to the HSA moiety, or only to the hNGF moiety, or to both of them. The same applies to a fusion protein (SA-hNGF) of SA of a non-human animal species and hNGF.

When mutations are introduced to the amino acid sequence represented by SEQ ID NO: 70, the sites and types (deletion, substitution, and addition) of individual mutations can be easily identified by alignment of the amino acid sequences before and after introduction of the mutations. The amino acid sequence having a mutation introduced thereto exhibits an identity of preferably 80% or more, 85% or more, 90% or more, or 95% or more, for example, an identity of 98% or more or 99% or more, to the amino acid sequence represented by SEQ ID NO: 70.

An HSA-hNGF fusion protein having a constituent amino acid modified with a sugar chain is regarded as an HSA-hNGF fusion protein. Also, an HSA-hNGF fusion protein having a constituent amino acid modified with a phosphate group is regarded as an HSA-hNGF fusion protein. Also, an HSA-hNGF fusion protein modified with a group except a sugar chain and a phosphate group is regarded as an HSA-hNGF fusion protein. Also, an HSA-hNGF fusion protein having a constituent amino acid whose side chain is converted by, e.g., a substitution reaction, is regarded as an HSA-hNGF fusion protein. Examples of such conversion include, but are not limited to, conversion of a cysteine residue to formylglycine. A mutation may be introduced only to the HSA moiety, or only to the hNGF moiety, or to both of them. The same applies to a fusion protein (SA-hNGF) of SA of a non-human animal species and hNGF.

More specifically, an HSA-hNGF fusion protein modified with a sugar chain is meant to be included in an HSA-hNGF fusion protein having an original amino acid sequence. Also, an HSA-hNGF fusion protein modified with a phosphate group is meant to be included in an HSA-hNGF fusion protein having an original amino acid sequence. Also, an HSA-hNGF fusion protein modified with a group except a sugar chain and a phosphate group is meant to be included in an HSA-hNGF fusion protein having an original amino acid sequence. Also, an HSA-hNGF fusion protein having a constituent amino acid whose side chain is converted by, e.g., a substitution reaction, is meant to be included in an HSA-hNGF fusion protein having an original amino acid sequence. Examples of such conversion include, but are not limited to, conversion of a cysteine residue to formylglycine. A mutation may be introduced only to the HSA moiety, or only to the hNGF moiety, or to both of them. The same applies to a fusion protein (SA-hNGF) of SA of a non-human animal species and hNGF.

An HSA-hNGF fusion protein in which a constituent hNGF is a precursor of hNGF is regarded as an HSA-hNGF fusion protein. The precursor as used herein refers to a moiety which functions as an hNGF of an HSA-hNGF fusion protein biologically synthesized, is separated from the fusion protein during a production process or in a living body to which the fusion protein is administered and serves as an hNGF by itself. In this case, sometimes the HSA-hNGF fusion protein biologically synthesized is cleaved at a predetermined site with, e.g., a hydrolytic enzyme to separate an HSA-containing moiety and a matured hNGF-containing moiety. The resulting hNGF is not a fusion protein with HSA but the HSA-hNGF fusion protein is once synthesized during a process for producing hNGF. Accordingly, in a case where hNGF is produced by such a method, the production method is included in a method for producing an HSA-hNGF fusion protein. A mutation may be introduced only to the HSA moiety, or only to the hNGF moiety, or to both of them. The same applies to a fusion protein (SA-hNGF) of SA of a non-human animal species and hNGF.

In an embodiment of the present invention, the term "SA-hNT-3 fusion protein" or "SA-hNT-3" refers to a fusion protein having an amino acid sequence in which the N terminal of the amino acid sequence of hNT-3 is bound to the C terminal of the amino acid sequence of SA, directly or via a linker, and having functions as hNT-3. The animal species of SA is not particularly limited and is preferably mammal SA, more preferably primate SA, and further preferably HSA. In an SA-hNT-3 fusion protein, the phrase that hNT-3 has a function as hNT-3 means that hNT-3 has a specific activity of preferably 10% or more, more preferably 20% or more, further preferably 50% or more, further more preferably 80% or more when the specific activity of a normal wild-type hNT-3 is regarded as 100%. Note that the specific activity of the hNT-3 in the SA-hNT-3 fusion protein herein is computationally obtained by multiplying the physiological activity of an hNT-3 per unit mass of the fusion protein by (the molecular weight of the fusion protein/the molecular weight of the moiety corresponding to the hNT-3 in the fusion protein).

In an embodiment of the present invention, the term "HSA-hNT-3 fusion protein" or "HSA-hNT-3" refers to a fusion protein having an amino acid sequence in which the N terminal of the amino acid sequence of hNT-3 is bound to the C terminal of the amino acid sequence of HSA directly or via a linker, and having a function as hNT-3. To a case where an HSA-hNT-3 fusion protein has a function as hNT-3, the definition in the case of the SA-hNT-3 fusion protein mentioned above is applicable.

In an embodiment of the present invention, a preferable HSA-hNT-3 fusion protein has the amino acid sequence represented by, for example, SEQ ID NO: 72. The HSA-hNT-3 fusion protein represented by SEQ ID NO: 72 is a fusion protein in which a wild-type hNT-3 is bound to the C terminal of a wild-type HSA via a linker sequence Gly-Ser. An HSA-hNT-3 fusion protein represented by SEQ ID NO: 72 is encoded by a gene having the nucleotide sequence represented by, for example, SEQ ID NO: 73. Also, a fusion protein having an amino acid sequence represented by SEQ ID NO: 72 and further having a mutation such as a substitution with different amino acid residues, a deletion, or an addition of one or more amino acid residues therein is included in the HSA-hNT-3 fusion protein as long as it has a function as hNT-3. The HSA-hNT-3 fusion protein preferably has a function as human serum albumin, such as a function to bind an endogenous substance and an exogenous substance such as a drug in the blood and transport them, but is not limited to this.

In a case where a mutation is introduced to an HSA-hNT-3 fusion protein, which is a fusion protein of a wild-type HSA and a wild-type hNT-3, the mutation can be introduced only to the HSA moiety and not introduced to the hNT-3 moiety; the mutation can be introduced only to the hNT-3 moiety without being introduced to the HSA moiety; or the mutation can be introduced to both of the HSA moiety and the hNT-3 moiety. In a case where a mutation is introduced only to the HSA moiety, the amino acid sequence of the moiety is an amino acid sequence of HSA obtained by introducing a mutation to a wild-type HSA as mentioned above. In a case where a mutation is introduced only to the hNT-3 moiety, the amino acid sequence of the moiety is an amino acid sequence of hNT-3 obtained by introducing a mutation to the wild-type hNT-3 as mentioned above. In a case where a mutation is introduced to both of the HSA moiety and the hNT-3 moiety, the amino acid sequence of the HSA moiety is an amino acid sequence of HSA obtained by introducing a mutation to a wild-type HSA as mentioned above, and the amino acid sequence of the hNT-3 moiety is an amino acid sequence of hNT-3 obtained by introducing a mutation to a wild-type hNT-3 as mentioned above. The same applies to the HSA-hNT-3 fusion protein having the amino acid sequence represented by SEQ ID NO: 72. The same applies to a fusion protein of a wild-type SA of a non-human animal species and a wild-type hNT-3.

The case where a mutation is introduced to an HSA-hNT-3 fusion protein having the amino acid sequence represented by SEQ ID NO: 72 will be described below. When an amino acid residue in the amino acid sequence represented by SEQ ID NO: 72 is substituted with another amino acid residue, the number of amino acid residues to be substituted is 1 to 10, 1 to 5, or 1 to 3, for example, one or two. When an amino acid residue is deleted, the number of amino acid residues to be deleted is 1 to 10, 1 to 5, or 1 to 3, for example, one or two. When an amino acid residue is added, one or more amino acid residues are added in the amino acid sequence represented by SEQ ID NO: 72 or added to the N terminal or C terminal of the amino acid sequence. The HSA-hNT-3 fusion protein may have a combination of the substitution, deletion, and addition of amino acid residues. A mutation may be introduced only to the HSA moiety, or only to the hGALC moiety, or to both of them.

When mutations are introduced to the amino acid sequence represented by SEQ ID NO: 72, the sites and types (deletion, substitution, and addition) of individual mutations can be easily identified by alignment of the amino acid sequences before and after introduction of the mutations. The amino acid sequence having a mutation introduced thereto exhibits an identity of preferably 80% or more, 85% or more, 90% or more, or 95% or more, for example, an identity of 98% or more or 99% or more, to the amino acid sequence represented by SEQ ID NO: 72.

An HSA-hNT-3 fusion protein having a constituent amino acid modified with a sugar chain is regarded as an HSA-hNT-3 fusion protein. Also, an HSA-hNT-3 fusion protein having a constituent amino acid modified with a phosphate group is regarded as an HSA-hNT-3 fusion protein. Also, an HSA-hNT-3 fusion protein modified with a group except a sugar chain and a phosphate group is regarded as an HSA-hNT-3 fusion protein. Also, an HSA-hNT-3 fusion protein having a constituent amino acid whose side chain is converted by, e.g., a substitution reaction, is regarded as an HSA-hNT-3 fusion protein. Examples of such conversion include, but are not limited to, conversion of a cysteine residue to formylglycine. The same applies to a fusion protein (SA-hNT-3) of SA of a non-human animal species and hNT-3.

More specifically, an HSA-hNT-3 fusion protein modified with a sugar chain is meant to be included in an HSA-hNT-3 fusion protein having an original amino acid sequence. Also, an HSA-hNT-3 fusion protein modified with a phosphate group is meant to be included in an HSA-hNT-3 fusion protein having an original amino acid sequence. Also, an HSA-hNT-3 fusion protein modified with a group except a sugar chain and a phosphate group is meant to be included in an HSA-hNT-3 fusion protein having an original amino acid sequence. Also, an HSA-hNT-3 fusion protein having a constituent amino acid whose side chain is converted by, e.g., a substitution reaction, is meant to be included in an HSA-hNT-3 fusion protein having an original amino acid sequence. Examples of such conversion include, but are not limited to, conversion of a cysteine residue to formylglycine. The same applies to a fusion protein (SA-hNT-3) of SA of a non-human animal species and hNT-3.

An HSA-hNT-3 fusion protein in which a constituent hNT-3 is a precursor of hNT-3 is regarded as an HSA-hNT-3 fusion protein. The precursor as used herein refers to a moiety which functions as an hNT-3 of an HSA-hNT-3 fusion protein biologically synthesized, is separated from the fusion protein during a production process or in a living body to which the fusion protein is administered and serves as an hNT-3 by itself. In this case, sometimes the HSA-hNT-3 fusion protein biologically synthesized is cleaved at a predetermined site with, e.g., a hydrolytic enzyme to separate an HSA-containing moiety and a matured hNT-3-containing moiety. The resulting hNT-3 is not a fusion protein with HSA but the HSA-hNT-3 fusion protein is once synthesized during a process for synthesizing the hNT-3. Accordingly, in a case where hNT-3 is produced by such a method, the production method is included in a method for producing an HSA-hNT-3 fusion protein. The same applies to a fusion protein (SA-hNT-3) of SA of a non-human animal species and hNT-3.

In an embodiment of the present invention, the term "SA-hNT-4 fusion protein" or "SA-hNT-4" refers to a fusion protein having an amino acid sequence in which the N terminal of the amino acid sequence of hNT-4 is bound to the C terminal of the amino acid sequence of SA, directly or via a linker, and having functions as hNT-4. The animal species of SA is not particularly limited and is preferably mammal SA, more preferably primate SA, and further preferably HSA. In an SA-hNT-4 fusion protein, the phrase that hNT-4 has a function as hNT-4 means that hNT-4 has a specific activity of preferably 10% or more, more preferably 20% or more, further preferably 50% or more, further more preferably 80% or more when the specific activity of a normal wild-type hNT-4 is regarded as 100%. Note that the specific activity of the hNT-4 in the SA-hNT-4 fusion protein herein is computationally obtained by multiplying the physiological activity of an hNT-4 per unit mass of the fusion protein by (the molecular weight of the fusion protein/the molecular weight of the moiety corresponding to the hNT-4 in the fusion protein).

In an embodiment of the present invention, the term "HSA-hNT-4 fusion protein" or "HSA-hNT-4" refers to a fusion protein having an amino acid sequence in which the N terminal of the amino acid sequence of hNT-4 is bound to the C terminal of the amino acid sequence of HSA directly or via a linker, and having a function as hNT-4. To a case where an HSA-hNT-4 fusion protein has a function as hNT-4, the definition in the case of the SA-hNT-4 fusion protein mentioned above is applicable.

In an embodiment of the present invention, a preferable HSA-hNT-4 fusion protein has the amino acid sequence represented by, for example, SEQ ID NO: 74. The HSA-hNT-4 fusion protein represented by SEQ ID NO: 74 is a fusion protein in which a wild-type hNT-4 is bound to the C terminal of a wild-type HSA via a linker sequence Gly-Ser. An HSA-hNT-4 fusion protein represented by SEQ ID NO: 74 is encoded by a gene having the nucleotide sequence represented by, for example, SEQ ID NO: 75. Also, a fusion protein having an amino acid sequence represented by SEQ ID NO: 74 and further having a mutation such as a substitution with different amino acid residues, a deletion, or an addition of one or more amino acid residues therein is included in the HSA-hNT-4 fusion protein as long as it has a function as hNT-4. The HSA-hNT-4 fusion protein preferably has a function as human serum albumin, such as a function to bind an endogenous substance and an exogenous substance such as a drug in the blood and transport them, but is not limited to this.

In a case where a mutation is introduced to an HSA-hNT-4 fusion protein, which is a fusion protein of a wild-type HSA and a wild-type hNT-4, the mutation can be introduced only to the HSA moiety and not introduced to the hNT-4 moiety; the mutation can be introduced only to the hNT-4 moiety without being introduced to the HSA moiety; or the mutation can be introduced to both of the HSA moiety and the hNT-4 moiety. In a case where a mutation is introduced only to the HSA moiety, the amino acid sequence of the moiety is an amino acid sequence of HSA obtained by introducing an mutation to a wild-type HSA as mentioned above. In a case where a mutation is introduced only to the hNT-4 moiety, the amino acid sequence of the moiety is an amino acid sequence of hNT-4 obtained by introducing a mutation to the wild-type hNT-4 as mentioned above. In a case where a mutation is introduced to both of the HSA moiety and the hNT-4 moiety, the amino acid sequence of the HSA moiety is an amino acid sequence of HSA obtained by introducing a mutation to a wild-type HSA as mentioned above, and the amino acid sequence of the hNT-4 moiety is an amino acid sequence of hNT-4 obtained by introducing a mutation to a wild-type hNT-4 as mentioned above. The same applies to the HSA-hNT-4 fusion protein having the amino acid sequence represented by SEQ ID NO: 74. The same applies to a fusion protein of a wild-type SA of a non-human animal species and a wild-type hNT-4.

The case where a mutation is introduced to an HSA-hNT-4 fusion protein having the amino acid sequence represented by SEQ ID NO: 74 will be described below. When an amino acid residue in the amino acid sequence represented by SEQ ID NO: 74 is substituted with another amino acid residue, the number of amino acid residues to be substituted is 1 to 10, 1 to 5, or 1 to 3, for example, one or two. When an amino acid residue is deleted, the number of amino acid residues to be deleted is 1 to 10, 1 to 5, or 1 to 3, for example, one or two. When an amino acid residue is added, one or more amino acid residues are added in the amino acid sequence represented by SEQ ID NO: 74 or added to the N terminal or C terminal of the amino acid sequence. The HSA-hNT-4 fusion protein may have a combination of the substitution, deletion, and addition of amino acid residues. A mutation may be introduced only to the HSA moiety, or only to the hNT-4 moiety, or to both of them. The same applies to a fusion protein (SA-hNT-4) of SA of a non-human animal species and hNT-3.

When mutations are introduced to the amino acid sequence represented by SEQ ID NO: 74, the sites and types (deletion, substitution, and addition) of individual mutations can be easily identified by alignment of the amino acid sequences before and after introduction of the mutations. The amino acid sequence having a mutation introduced thereto exhibits an identity of preferably 80% or more, 85% or more, 90% or more, or 95% or more, for example, an identity of 98% or more or 99% or more, to the amino acid sequence represented by SEQ ID NO: 74.

An HSA-hNT-4 fusion protein having a constituent amino acid modified with a sugar chain is regarded as an HSA-hNT-4 fusion protein. Also, an HSA-hNT-4 fusion protein having a constituent amino acid modified with a phosphate group is regarded as an HSA-hNT-4 fusion protein. Also, an HSA-hNT-4 fusion protein modified with a group except a sugar chain and a phosphate group is regarded as an HSA-hNT-4 fusion protein. Also, an HSA-hNT-4 fusion protein having a constituent amino acid whose side chain is converted by, e.g., a substitution reaction, is regarded as an HSA-hNT-4 fusion protein. Examples of such conversion include, but are not limited to, conversion of a cysteine residue to formylglycine. The same applies to a fusion protein (SA-hNT-4) of SA of a non-human animal species and hNT-3.

More specifically, an HSA-hNT-4 fusion protein modified with a sugar chain is meant to be included in an HSA-hNT-4 fusion protein having an original amino acid sequence. Also, an HSA-hNT-4 fusion protein modified with a phosphate group is meant to be included in an HSA-hNT-4 fusion protein having an original amino acid sequence. Also, an HSA-hNT-4 fusion protein modified with a group except a sugar chain and a phosphate group is meant to be included in an HSA-hNT-4 fusion protein having an original amino acid sequence. Also, an HSA-hNT-4 fusion protein having a constituent amino acid whose side chain is converted by, e.g., a substitution reaction, is meant to be included in an HSA-hNT-4 fusion protein having an original amino acid sequence. Examples of such conversion include, but are not limited to, conversion of a cysteine residue to formylglycine. The same applies to a fusion protein (SA-hNT-4) of SA of a non-human animal species and hNT-3.

An HSA-hNT-4 fusion protein in which a constituent hNT-4 is a precursor of hNT-4 is regarded as an HSA-hNT-4 fusion protein. The precursor as used herein refers to a moiety which functions as an hNT-4 of an HSA-hNT-4 fusion protein biologically synthesized, is separated from the fusion protein during a production process or in a living body to which the fusion protein is administered and serves as an hNT-4 by itself. In this case, sometimes the HSA-hNT-4 fusion protein biologically synthesized is cleaved at a predetermined site with, e.g., a hydrolytic enzyme to separate an HSA-containing moiety and an hNT-4-containing moiety. The resulting hNT-4 is not a fusion protein with HSA but the HSA-hNT-4 fusion protein is once synthesized during a process for synthesizing the hNT-4. Accordingly, in a case where hNT-4 is produced by such a method, the production method is included in a method for producing an HSA-hNT-4 fusion protein. The same applies to a fusion protein (SA-hNT-4) of SA of a non-human animal species and hNT-4.

In an embodiment of the present invention, the term "HSA-hCDNF fusion protein" or "HSA-hCDNF" refers to a fusion protein having an amino acid sequence in which the N terminal of the amino acid sequence of hCDNF is bound to the C terminal of the amino acid sequence of HSA directly or via a linker, and having a function as hCDNF. In an embodiment of the present invention, the term "HSA-hGDNF fusion protein" or "HSA-hGDNF" refers to a fusion protein having an amino acid sequence in which the N terminal of the amino acid sequence of hGDNF is bound to the C terminal of the amino acid sequence of HSA directly or via a linker, and having a function as hGDNF. In an embodiment of the present invention, the term "HSA-hNRTN fusion protein" or "HSA-hNRTN" refers to a fusion protein having an amino acid sequence in which the N terminal of the amino acid sequence of hNRTN is bound to the C terminal of the amino acid sequence of HSA directly or via a linker, and having a function as hNRTN. In an embodiment of the present invention, the term "HSA-hARTN fusion protein" or "HSA-hARTN" refers to a fusion protein having an amino acid sequence in which the N terminal of the amino acid sequence of hARTN is bound to the C terminal of the amino acid sequence of HSA directly or via a linker, and having a function as hARTN. In an embodiment of the present invention, the term "HSA-hPSPN fusion protein" or "HSA-hPSPN" refers to a fusion protein having an amino acid sequence in which the N terminal of the amino acid sequence of hPSPN is bound to the C terminal of the amino acid sequence of HSA directly or via a linker, and having a function as hPSPN. The above matters on HSA-hBDNF are also applied to these fusion proteins.

In an embodiment of the present invention, the term "human neurotrophic factor-SA fusion protein" or "human neurotrophic factor-SA" refers to a fusion protein having an amino acid sequence in which the N terminal of the amino acid sequence of SA is bound to the C terminal of the amino acid sequence of a human neurotrophic factor directly or via a linker, and having a function as a human neurotrophic factor. In a human neurotrophic factor-SA fusion protein, the phrase that a human neurotrophic factor has a function as the human neurotrophic factor means that the human neurotrophic factor has a specific activity of preferably 10% or more, more preferably 20% or more, further preferably 50% or more, further more preferably 80% or more when the specific activity of a normal wild-type human neurotrophic factor is regarded as 100%. Note that, the specific activity of the human neurotrophic factor in the human neurotrophic factor-SA fusion protein herein is computationally obtained by multiplying the physiological activity of a human neurotrophic factor per unit mass of the fusion protein by (the molecular weight of the fusion protein/the molecular weight of the moiety corresponding to the human neurotrophic factor in the fusion protein).

In an embodiment of the present invention, the term "human neurotrophic factor-HSA fusion protein" or "human neurotrophic factor-HSA" refers to a fusion protein having an amino acid sequence in which the N terminal of the amino acid sequence of HSA is bound to the C terminal of the amino acid sequence of a human neurotrophic factor directly or via a linker, and having a function as a human neurotrophic factor. To the case where a human neurotrophic factor-HSA fusion protein has a function as a human neurotrophic factor, the definition in the case of the neurotrophic factor-HSA fusion protein mentioned above is applicable.

In the human neurotrophic factor-SA fusion protein, SA preferably has a function as SA, such as a function to bind an endogenous substance and an exogenous substance such as a drug in the blood and transport them, but is not limited to this. The same applies to a human neurotrophic factor-HSA fusion protein. The same applies to an hBDNF-HSA fusion protein, an hNGF-HSA fusion protein, an hNT-3-HSA fusion protein, and an hNT-4-HSA fusion protein.

In an embodiment of the present invention, a fusion protein having an amino acid sequence in which the N terminal of SA of a non-human animal species is bound to the C terminal of the amino acid sequence of a neurotrophic factor of the non-human animal species, directly or via a linker, and having a function as a neurotrophic factor is expressed as a "(non-human animal species) neurotrophic factor - (non-human animal species) SA fusion protein". For example, a fusion protein of a mouse neurotrophic factor and mouse SA is expressed as a "mouse neurotrophic factor-MSA fusion protein" or "mouse neurotrophic factor-MSA". To a case where these fusion proteins have a function as a neurotrophic factor, the definition in the case of the human neurotrophic factor-SA fusion protein mentioned above is applicable. Also in these fusion proteins, SA preferably has a function as SA, such as a function to bind an endogenous substance and an exogenous substance such as a drug in the blood and transport them, but is not limited to this.

In a case where a mutation is introduced to a human neurotrophic factor-HSA fusion protein, which is a fusion protein of a wild-type human neurotrophic factor and a wild-type HSA, the mutation can be introduced only to the human neurotrophic factor moiety and not introduced to the HSA moiety; the mutation can be introduced only to an HSA moiety without being introduced to the human neurotrophic factor moiety; or the mutation can be introduced to both of the human neurotrophic factor moiety and the HSA moiety. In a case where a mutation is introduced only to the human neurotrophic factor moiety, the amino acid sequence of the moiety is an amino acid sequence of a human neurotrophic factor obtained by introducing a mutation to a wild-type human neurotrophic factor as mentioned above. In a case where a mutation is introduced only to the HSA moiety, the amino acid sequence of the moiety is an amino acid sequence of HSA obtained by introducing the mutation to a wild-type HSA as mentioned above. In a case where a mutation is introduced to both of the human neurotrophic factor moiety and the HSA moiety, the amino acid sequence of the human neurotrophic factor moiety is an amino acid sequence of human neurotrophic factor obtained by introducing a mutation to a wild-type human neurotrophic factor as mentioned above, and the amino acid sequence of the HSA moiety is an amino acid sequence of HSA obtained by introducing a mutation to a wild-type HSA as mentioned above. The same applies to a fusion protein of a wild-type human neurotrophic factor and a wild-type SA of a non-human animal species.

A human neurotrophic factor-HSA fusion protein having a constituent amino acid modified with a sugar chain is regarded as a human neurotrophic factor-HSA fusion protein. Also, a human neurotrophic factor-HSA fusion protein having a constituent amino acid modified with a phosphate group is regarded as a human neurotrophic factor-HSA fusion protein. Also, a human neurotrophic factor-HSA fusion protein modified with a group except a sugar chain and a phosphate group is regarded as a human neurotrophic factor-HSA fusion protein. Also, a human neurotrophic factor-HSA fusion protein having a constituent amino acid whose side chain is converted by, e.g., a substitution reaction, is regarded as a human neurotrophic factor-HSA fusion protein. Examples of such conversion include, but are not limited to, conversion of a cysteine residue to formylglycine. The same applies to a fusion protein (human neurotrophic factor-SA) of a human neurotrophic factor and SA of a non-human animal species.

More specifically, a human neurotrophic factor-HSA fusion protein modified with a sugar chain is meant to be included in a human neurotrophic factor-HSA fusion protein having an original amino acid sequence. Also, a human neurotrophic factor-HSA fusion protein modified with a phosphate group is meant to be included in a human neurotrophic factor-HSA fusion protein having an original amino acid sequence. Also, a human neurotrophic factor-HSA fusion protein modified with a group except a sugar chain and a phosphate group is meant to be included in a human neurotrophic factor-HSA fusion protein having an original amino acid sequence. Also, a human neurotrophic factor-HSA fusion protein having a constituent amino acid whose side chain is converted by, e.g., a substitution reaction, is meant to be included in a human neurotrophic factor-HSA fusion protein having an original amino acid sequence. Examples of such conversion include, but are not limited to, conversion of a cysteine residue to formylglycine. The same applies to a fusion protein (human neurotrophic factor-SA) of a human neurotrophic factor and SA of a non-human animal species.

A human neurotrophic factor-HSA fusion protein in which a constituent human neurotrophic factor is a precursor of a human neurotrophic factor is regarded as a human neurotrophic factor-HSA fusion protein. In a case where the precursor of a human neurotrophic factor does not exhibit any function as the human neurotrophic factor, however, the precursor is needed to be converted to exhibit a function as the human neurotrophic factor through in vivo or in vitro processing.

In an embodiment of the present invention, the term "hBDNF-SA fusion protein" or "hBDNF-SA" refers to a fusion protein having an amino acid sequence in which the N terminal of the amino acid sequence of SA is bound to the C terminal of the amino acid sequence of hBDNF directly or via a linker, and having a function as hBDNF. The phrase that hBDNF has a function as hBDNF in the hBDNF-SA fusion protein means that hBDNF has a specific activity of preferably 10% or more, more preferably 20% or more, further preferably 50% or more, further more preferably 80% or more when the specific activity of a normal wild-type hBDNF is regarded as 100%. Note that, the specific activity of the hBDNF in the hBDNF-SA fusion protein herein is computationally obtained by multiplying the physiological activity of hBDNF per unit mass of the fusion protein by (the molecular weight of the fusion protein/the molecular weight of the moiety corresponding to the hBDNF in the fusion protein).

In an embodiment of the present invention, the term "hBDNF-HSA fusion protein" or "hBDNF-HSA" refers to a fusion protein having an amino acid sequence in which the N terminal of the amino acid sequence of HSA is bound to the C terminal of the amino acid sequence of hBDNF directly or via a linker, and having a function as hBDNF. To a case where the hBDNF-HSA fusion protein has a function as hBDNF, the definition in the case of the hBDNF-SA fusion protein mentioned above is applicable.

In the present invention, a fusion protein having an amino acid sequence in which the N terminal of SA of a non-human animal species is bound to the C terminal of the amino acid sequence of BDNF of a non-human animal species, directly or via a linker, and having a function as BDNF is expressed as a "(non-human animal species) BDNF - (non-human animal species) SA fusion protein". For example, a fusion protein of mouse BDNF and mouse SA is expressed as a "mouse BDNF-mouse SA fusion protein" or "mouse BDNF-MSA". To a case where these fusion proteins have a function as BDNF, the definition in the case of the hBDNF-SA fusion protein mentioned above is applicable.

In an embodiment of the present invention, a preferable hBDNF-HSA fusion protein has the amino acid sequence represented by, for example, SEQ ID NO: 76. The hBDNF-HSA fusion protein represented by SEQ ID NO: 76 is a fusion protein in which a wild-type HSA is bound to the C terminal of a wild-type hBDNF via a linker sequence, Gly-Ser. The hBDNF-HSA fusion protein represented by SEQ ID NO: 76 is encoded by a gene having the nucleotide sequence represented by, for example, SEQ ID NO: 77. Also, a fusion protein having an amino acid sequence represented by SEQ ID NO: 76 and further having a mutation such as a substitution with different amino acid residues, a deletion, or an addition of one or more amino acid residues therein is included in the hBDNF-HSA fusion protein as long as it has a function as hBDNF. The hBDNF-HSA fusion protein preferably has a function as human serum albumin, such as a function to bind an endogenous substance and an exogenous substance such as a drug in the blood and transport them, but is not limited to this.

When mutations are introduced to the amino acid sequence represented by SEQ ID NO: 76, the sites and types (deletion, substitution, and addition) of individual mutations can be easily identified by alignment of the amino acid sequences before and after introduction of the mutations. The amino acid sequence having a mutation introduced thereto exhibits an identity of preferably 80% or more, 85% or more, 90% or more, or 95% or more, for example, an identity of 98% or more or 99% or more, to the amino acid sequence represented by SEQ ID NO: 76.

In a case where a mutation is introduced to an hBDNF-HSA fusion protein, which is a fusion protein of a wild-type hBDNF and a wild-type HSA, the mutation can be introduced only to the HSA moiety and not introduced to the hBDNF moiety; the mutation can be introduced only to the hBDNF moiety without being introduced to the HSA moiety; or the mutation can be introduced to both of the HSA moiety and the hBDNF moiety. In a case where a mutation is introduced only to the HSA moiety, the amino acid sequence of the moiety is an amino acid sequence of HSA of a wild-type HSA obtained by introducing an mutation to as mentioned above. In a case where a mutation is introduced only to the hBDNF moiety, the amino acid sequence of the moiety is an amino acid sequence of an hBDNF obtained by introducing a mutation to a wild-type hBDNF as mentioned above. In a case where a mutation is introduced to both of the HSA moiety and the hBDNF moiety, the amino acid sequence of the HSA moiety is an amino acid sequence of HSA obtained by introducing a mutation to a wild-type HSA as mentioned above, and the amino acid sequence of the hBDNF moiety is an amino acid sequence of hBDNF obtained by introducing a mutation to a wild-type hBDNF as mentioned above. The same applies to an hBDNF-HSA fusion protein having the amino acid sequence represented by SEQ ID NO: 76. The same applies to a fusion protein (hBDNF-SA) of a wild-type hBDNF and a wild-type SA of a non-human animal species.

The case where a mutation is introduced to an hBDNF-HSA fusion protein having the amino acid sequence represented by SEQ ID NO: 76 will be described below. When an amino acid residue in the amino acid sequence represented by SEQ ID NO: 76 is substituted with another amino acid residue, the number of amino acid residues to be substituted is 1 to 10, 1 to 5, or 1 to 3, for example, one or two. When an amino acid residue is deleted, the number of amino acid residues to be deleted is 1 to 10, 1 to 5, or 1 to 3, for example, one or two. When an amino acid residue is added, one or more amino acid residues are added in the amino acid sequence represented by SEQ ID NO: 76 or added to the N terminal or C terminal of the amino acid sequence. The hBDNF-HSA fusion protein may have a combination of the substitution, deletion, and addition of amino acid residues. A mutation may be introduced only to the HSA moiety, or only to the hBDNF moiety, or to both of them. The same applies to a fusion protein (hBDNF-SA) of hBDNF and SA of a non-human animal species.

An hBDNF-HSA fusion protein having a constituent amino acid modified with a sugar chain is regarded as an hBDNF-HSA fusion protein. Also, an hBDNF-HSA fusion protein having a constituent amino acid modified with a phosphate group is regarded as an hBDNF-HSA fusion protein. Also, an hBDNF-HSA fusion protein modified with a group except a sugar chain and a phosphate group is regarded as an hBDNF-HSA fusion protein. Also, an hBDNF-HSA fusion protein having a constituent amino acid whose side chain is converted by, e.g., a substitution reaction, is regarded as an hBDNF-HSA fusion protein. Examples of such conversion include, but are not limited to, conversion of a cysteine residue to formylglycine. The same applies to a fusion protein (hBDNF-SA) of hBDNF and SA of a non-human animal species.

More specifically, an hBDNF-HSA fusion protein modified with a sugar chain is meant to be included in an hBDNF-HSA fusion protein having an original amino acid sequence. Also, an hBDNF-HSA fusion protein modified with a phosphate group is meant to be included in an hBDNF-HSA fusion protein having an original amino acid sequence. Also, an hBDNF-HSA fusion protein modified with a group except a sugar chain and a phosphate group is meant to be included in an hBDNF-HSA fusion protein having an original amino acid sequence. Also, an hBDNF-HSA fusion protein having a constituent amino acid whose side chain is converted by, e.g., a substitution reaction, is meant to be included in an hBDNF-HSA fusion protein having an original amino acid sequence. Examples of such conversion include, but are not limited to, conversion of a cysteine residue to formylglycine. The same applies to a fusion protein (hBDNF-SA) of hBDNF and SA of a non-human animal species.

An hBDNF-HSA fusion protein in which a constituent hBDNF is a precursor of hBDNF is regarded as an hBDNF-HSA fusion protein. In a case where the hBDNF-HSA fusion protein in which the hBDNF is in the form of a precursor does not exhibit any function as hBDNF, however, the precursor is needed to be converted to exhibit a function as hBDNF through in vivo or in vitro processing.

In an embodiment of the present invention, the term "hNGF-SA fusion protein" or "hNGF-SA" refers to a fusion protein having an amino acid sequence in which the N terminal of the amino acid sequence of SA is bound to the C terminal of the hNGF amino acid sequence directly or via a linker, and having a function as hNGF. The phrase that an hNGF has a function as an hNGF in the hNGF-SA fusion protein means that the hNGF has a specific activity of preferably 10% or more, more preferably 20% or more, further preferably 50% or more, and further more preferably 80% or more, when the specific activity of a normal wild-type hNGF is regarded as 100%. Note that, the specific activity of the hNGF in the hNGF-SA fusion protein herein is computationally obtained by multiplying the physiological activity of the hNGF per unit mass of the fusion protein by (the molecular weight of the fusion protein/the molecular weight of the moiety corresponding to the hNGF in the fusion protein).

In an embodiment of the present invention, the term "hNGF-HSA fusion protein" or "hNGF-HSA" refers to a fusion protein having an amino acid sequence in which the N terminal of the amino acid sequence of HSA is bound to the C terminal of the amino acid sequence of hNGF directly or via a linker, and having a function as hNGF. To a case where an hNGF-HSA fusion protein has a function as an hNGF, the definition in the case of the hNGF-SA fusion protein mentioned above is applicable.

In an embodiment of the present invention, a fusion protein having an amino acid sequence in which the N terminal of SA of a non-human animal species is bound to the C terminal of the amino acid sequence of NGF of a non-human animal species directly or via a linker, and having a function as NGF is expressed as "(non-human animal species) NGF - (non-human animal species) SA fusion protein". For example, a fusion protein of mouse NGF and mouse SA is expressed as a "mouse NGF-mouse SA fusion protein" or "mouse NGF-MSA". To a case where these fusion proteins have a function as NGF, the definition in the case of the hNGF-SA fusion protein mentioned above is applicable.

In an embodiment of the present invention, a preferable hNGF-HSA fusion protein has the amino acid sequence represented by, for example, SEQ ID NO: 78. The hNGF-HSA fusion protein represented by SEQ ID NO: 78 is a fusion protein in which a wild-type HSA is bound to the C terminal of a wild-type hNGF via a linker sequence, Gly-Ser. The hNGF-HSA fusion protein represented by SEQ ID NO: 78 is encoded by a gene having the nucleotide sequence represented by, for example, SEQ ID NO: 79. Also, a fusion protein having an amino acid sequence represented by SEQ ID NO: 78 and further having a mutation such as a substitution with different amino acid residues, a deletion, or an addition of one or more amino acid residues therein is included in the hNGF-HSA fusion protein as long as it has a function as hNGF. The hNGF-HSA fusion protein preferably has a function as human serum albumin, such as a function to bind an endogenous substance and an exogenous substance such as a drug in the blood and transport them, but is not limited to this.

When mutations are introduced to the amino acid sequence represented by SEQ ID NO: 78, the sites and types (deletion, substitution, and addition) of individual mutations can be easily identified by alignment of the amino acid sequences before and after introduction of the mutations. The amino acid sequence having a mutation introduced thereto exhibits an identity of preferably 80% or more, 85% or more, 90% or more, or 95% or more, for example, an identity of 98% or more or 99% or more, to the amino acid sequence represented by SEQ ID NO: 78.

In a case where a mutation is introduced to an hNGF-HSA fusion protein, which is a fusion protein of a wild-type hNGF and a wild-type HSA, the mutation can be introduced only to the HSA moiety and not introduced to the hNGF moiety; the mutation can be introduced only to the hNGF moiety without being introduced to the HSA moiety; or the mutation can be introduced to both of the HSA moiety and the hNGF moiety. In a case where a mutation is introduced only to the HSA moiety, the amino acid sequence of the moiety is an amino acid sequence of HSA obtained by introducing a mutation to a wild-type HSA as mentioned above. In a case where a mutation is introduced only to the hNGF moiety, the amino acid sequence of the moiety is an amino acid sequence of an hNGF obtained by introducing a mutation to a wild-type hNGF as mentioned above. In a case where a mutation is introduced to both of the HSA moiety and the hNGF moiety, the amino acid sequence of the HSA moiety is an amino acid sequence of HSA obtained by introducing a mutation to a wild-type HSA as mentioned above, and the amino acid sequence of the hNGF moiety is an amino acid sequence of hNGF obtained by introducing a mutation to a wild-type hNGF as mentioned above. The same applies to an hNGF-HSA fusion protein having the amino acid sequence represented by SEQ ID NO: 78. The same applies to a fusion protein (hNGF-SA) of a wild-type hNGF and a wild-type SA of a non-human animal species.

The case where a mutation is introduced to an hNGF-HSA fusion protein having the amino acid sequence represented by SEQ ID NO: 78 will be described below. When an amino acid residue in the amino acid sequence represented by SEQ ID NO: 78 is substituted with another amino acid residue, the number of amino acid residues to be substituted is 1 to 10, 1 to 5, or 1 to 3, for example, one or two. When an amino acid residue is deleted, the number of amino acid residues to be deleted is 1 to 10, 1 to 5, or 1 to 3, for example, one or two. When an amino acid residue is added, one or more amino acid residues are added in the amino acid sequence represented by SEQ ID NO: 78 or added to the N terminal or C terminal of the amino acid sequence. The hNGF-HSA fusion protein may have a combination of the substitution, deletion, and addition of amino acid residues. A mutation may be introduced only to the HSA moiety, or only to the hNGF moiety, or to both of them. The same applies to a fusion protein (NGF-SA) of NGF of a non-human animal species and HSA and a fusion protein of NGF of a non-human animal species and SA of a non-human animal species.

An hNGF-HSA fusion protein having a constituent amino acid modified with a sugar chain is regarded as an hNGF-HSA fusion protein. Also, an hNGF-HSA fusion protein having a constituent amino acid modified with a phosphate group is regarded as an hNGF-HSA fusion protein. Also, an hNGF-HSA fusion protein modified with a group except a sugar chain and a phosphate group is regarded as an hNGF-HSA fusion protein. Also, an hNGF-HSA fusion protein having a constituent amino acid whose side chain is converted by, e.g., a substitution reaction, is regarded as an hNGF-HSA fusion protein. Examples of such conversion include, but are not limited to, conversion of a cysteine residue to formylglycine. The same applies to a fusion protein (hNGF-SA) of hNGF and SA of a non-human animal species.

More specifically, an hNGF-HSA fusion protein modified with a sugar chain is meant to be included in an hNGF-HSA fusion protein having an original amino acid sequence. Also, an hNGF-HSA fusion protein modified with a phosphate group is meant to be included in an hNGF-HSA fusion protein having an original amino acid sequence. Also, an hNGF-HSA fusion protein modified with a group except a sugar chain and a phosphate group is meant to be included in an hNGF-HSA fusion protein having an original amino acid sequence. Also, an hNGF-HSA fusion protein having a constituent amino acid whose side chain is converted by, e.g., a substitution reaction, is meant to be included in an hNGF-HSA fusion protein having an original amino acid sequence. Examples of such conversion include, but are not limited to, conversion of a cysteine residue to formylglycine. The same applies to a fusion protein (hNGF-SA) of hNGF and SA of a non-human animal species.

An hNGF-HSA fusion protein in which a constituent hNGF is a precursor of hNGF is regarded as an hNGF-HSA fusion protein. In a case where the hNGF-HSA fusion protein in which the hNGF is in the form of a precursor does not exhibit any function as hNGF, however, the precursor is needed to be converted to exhibit a function as hNGF through in vivo or in vitro processing. The same applies to a fusion protein (hNGF-SA) of hNGF and SA of a non-human animal species.

In an embodiment of the present invention, the term "hNT-3-SA fusion protein" or "hNT-3-SA" refers to a fusion protein having an amino acid sequence in which the N terminal of the amino acid sequence of SA is bound to the C terminal of the hNT-3 amino acid sequence directly or via a linker, and having a function as hNT-3. The phrase that an hNT-3 has a function as an hNT-3 in the hNT-3-SA fusion protein means that the hNT-3 has a specific activity of preferably 10% or more, more preferably 20% or more, further preferably 50% or more, and further more preferably 80% or more, when the specific activity of a normal wild-type hNT-3 is regarded as 100%. Note that, the specific activity of the hNT--3 in the hNT-3-SA fusion protein herein is computationally obtained by multiplying the physiological activity of the hNT-3 per unit mass of the fusion protein by (the molecular weight of the fusion protein/the molecular weight of the moiety corresponding to the hNT-3 in the fusion protein).

In an embodiment of the present invention, the term "hNT-3-HSA fusion protein" or "hNT-3-HSA" refers to a fusion protein having an amino acid sequence in which the N terminal of the amino acid sequence of HSA is bound to the C terminal of the amino acid sequence of hNT-3 directly or via a linker, and having a function as hNT-3. To a case where an hNT-3-HSA fusion protein has a function as an hNT-3, the definition in the case of the hNT-3-SA fusion protein mentioned above is applicable.

In an embodiment of the present invention, a fusion protein having an amino acid sequence in which the N terminal of SA of a non-human animal species is bound to the C terminal of the amino acid sequence of NT-3 of a non-human animal species directly or via a linker, and having a function as NT-3 is expressed as "(non-human animal species) NT-3 - (non-human animal species) SA fusion protein". For example, a fusion protein of mouse NT-3 and mouse SA is expressed as a "mouse NT-3-mouse SA fusion protein" or "mouse NT-3-MSA". To a case where these fusion proteins have a function as NT-3, the definition in the case of the hNT-3-SA fusion protein mentioned above is applicable.

In an embodiment of the present invention, a preferable hNT-3-HSA fusion protein has the amino acid sequence represented by, for example, SEQ ID NO: 80. The hNT-3-HSA fusion protein represented by SEQ ID NO: 80 is a fusion protein in which a wild-type HSA is bound to the C terminal of a wild-type hNT-3 via a linker sequence, Gly-Ser. The hNT-3-HSA fusion protein represented by SEQ ID NO: 80 is encoded by a gene having the nucleotide sequence represented by, for example, SEQ ID NO: 81. Also, a fusion protein having an amino acid sequence represented by SEQ ID NO: 80 and further having a mutation such as a substitution with different amino acid residues, a deletion, or an addition of one or more amino acid residues therein is included in the hNT-3-HSA fusion protein as long as it has a function as hNT-3. The hNT-3-HSA fusion protein preferably has a function as human serum albumin, such as a function to bind an endogenous substance and an exogenous substance such as a drug in the blood and transport them, but is not limited to this.

When mutations are introduced to the amino acid sequence represented by SEQ ID NO: 80, the sites and types (deletion, substitution, and addition) of individual mutations can be easily identified by alignment of the amino acid sequences before and after introduction of the mutations. The amino acid sequence having a mutation introduced thereto exhibits an identity of preferably 80% or more, 85% or more, 90% or more, or 95% or more, for example, an identity of 98% or more or 99% or more, to the amino acid sequence represented by SEQ ID NO: 80.

In a case where a mutation is introduced to an hNT-3-HSA fusion protein, which is a fusion protein of a wild-type hNT-3 and a wild-type HSA, the mutation can be introduced only to the HSA moiety and not introduced to the hNT-3 moiety; the mutation can be introduced only to the hNT-3 moiety without being introduced to the HSA moiety; or the mutation can be introduced to both of the HSA moiety and the hNT-3 moiety. In a case where a mutation is introduced only to the HSA moiety, the amino acid sequence of the moiety is an amino acid sequence of HSA obtained by introducing an mutation to a wild-type HSA as mentioned above. In a case where a mutation is introduced only to the hNT-3 moiety, the amino acid sequence of the moiety is an amino acid sequence of an hNT-3 obtained by introducing a mutation to a wild-type hNT-3 as mentioned above. In a case where a mutation is introduced to both of the HSA moiety and the hNT-3 moiety, the amino acid sequence of the HSA moiety is an amino acid sequence of HSA obtained by introducing a mutation to a wild-type HSA as mentioned above, and the amino acid sequence of the hNT-3 moiety is an amino acid sequence of hNT-3 obtained by introducing a mutation to a wild-type hNT-3 as mentioned above. The same applies to an hNT-3-HSA fusion protein having the amino acid sequence represented by SEQ ID NO: 80. The same applies to a fusion protein (hNT-3-SA) of a wild-type hNT-3 and a wild-type SA of a non-human animal species.

The case where a mutation is introduced to an hNT-3-HSA fusion protein having the amino acid sequence represented by SEQ ID NO: 80 will be described below. When an amino acid residue in the amino acid sequence represented by SEQ ID NO: 80 is substituted with another amino acid residue, the number of amino acid residues to be substituted is 1 to 10, 1 to 5, or 1 to 3, for example, one or two. When an amino acid residue is deleted, the number of amino acid residues to be deleted is 1 to 10, 1 to 5, or 1 to 3, for example, one or two. When an amino acid residue is added, one or more amino acid residues are added in the amino acid sequence represented by SEQ ID NO: 80 or added to the N terminal or C terminal of the amino acid sequence. The hNT-3-HSA fusion protein may have a combination of the substitution, deletion, and addition of amino acid residues. A mutation may be introduced only to the HSA moiety, or only to the hNT-3 moiety, or to both of them. The same applies to a fusion protein (hNT-3-SA) of hNT-3 and SA of a non-human animal species.

An hNT-3-HSA fusion protein having a constituent amino acid modified with a sugar chain is regarded as an hNT-3-HSA fusion protein. Also, an hNT-3-HSA fusion protein having a constituent amino acid modified with a phosphate group is regarded as an hNT-3-HSA fusion protein. Also, an hNT-3-HSA fusion protein modified with a group except a sugar chain and a phosphate group is regarded as an hNT-3-HSA fusion protein. Also, an hNT-3-HSA fusion protein having a constituent amino acid whose side chain is converted by, e.g., a substitution reaction, is regarded as an hNT-3-HSA fusion protein. Examples of such conversion include, but are not limited to, conversion of a cysteine residue to formylglycine. The same applies to a fusion protein (hNT-3-SA) of hNT-3 and SA of a non-human animal species.

More specifically, an hNT-3-HSA fusion protein modified with a sugar chain is meant to be included in an hNT-3-HSA fusion protein having an original amino acid sequence. Also, an hNT-3-HSA fusion protein modified with a phosphate group is meant to be included in an hNT-3-HSA fusion protein having an original amino acid sequence. Also, an hNT-3-HSA fusion protein modified with a group except a sugar chain and a phosphate group is meant to be included in an hNT-3-HSA fusion protein having an original amino acid sequence. Also, an hNT-3-HSA fusion protein having a constituent amino acid whose side chain is converted by, e.g., a substitution reaction, is meant to be included in an hNT-3-HSA fusion protein having an original amino acid sequence. Examples of such conversion include, but are not limited to, conversion of a cysteine residue to formylglycine. The same applies to a fusion protein (hNT-3-SA) of hNT-3 and SA of a non-human animal species.

An hNT-3-HSA fusion protein in which a constituent hNT-3 is a precursor of hNT-3 is regarded as an hNT-3-HSA fusion protein. In a case where the hNT-3-HSA fusion protein in which the hNT-3 is in the form of a precursor does not exhibit any function as hNT-3, however, the precursor is needed to be converted to exhibit a function as hNT-3 through in vivo or in vitro processing. The same applies to a fusion protein (hNT-3-SA) of hNT-3 and SA of a non-human animal species.

In an embodiment of the present invention, the term "hNT-4-SA fusion protein" or "hNT-4-SA" refers to a fusion protein having an amino acid sequence in which the N terminal of the amino acid sequence of SA is bound to the C terminal of the hNT-4 amino acid sequence, directly or via a linker, and having a function as hNT-4. The phrase that an hNT-4 has a function as an hNT-4 in the hNT-4-SA fusion protein means that the hNT-4 has a specific activity of preferably 10% or more, more preferably 20% or more, further preferably 50% or more, and further more preferably 80% or more, when the specific activity of a normal wild-type hNT-4 is regarded as 100%. Note that, the specific activity of the hNT-4 in the hNT-4-SA fusion protein herein is computationally obtained by multiplying the physiological activity of the hNT-4 per unit mass of the fusion protein by (the molecular weight of the fusion protein/the molecular weight of the moiety corresponding to the hNT-4 in the fusion protein).

In an embodiment of the present invention, the term "hNT-4-HSA fusion protein" or "hNT-4-HSA" refers to a fusion protein having an amino acid sequence in which the N terminal of the amino acid sequence of HSA is bound to the C terminal of the amino acid sequence of hNT-4 directly or via a linker, and having a function as hNT-4. To a case where an hNT-4-HSA fusion protein has a function as an hNT-4, the definition in the case of the hNT-4-SA fusion protein mentioned above is applicable.

In an embodiment of the present invention, a fusion protein having an amino acid sequence in which the N terminal of SA of a non-human animal species is bound to the C terminal of the amino acid sequence of NT-4 of a non-human animal species directly or via a linker, and having a function as NT-4 is expressed as "(non-human animal species) NT-4 - (non-human animal species) SA fusion protein". For example, a fusion protein of mouse NT-4 and mouse SA is expressed as a "mouse NT-4-mouse SA fusion protein" or "mouse NT-4-MSA". To a case where these fusion proteins have a function as NT-4, the definition in the case of the hNT-4-SA fusion protein mentioned above is applicable.

In an embodiment of the present invention, a preferable hNT-4-HSA fusion protein has the amino acid sequence represented by, for example, SEQ ID NO: 82. The hNT-4-HSA fusion protein represented by SEQ ID NO: 82 is a fusion protein in which a wild-type HSA is bound to the C terminal of a wild-type hNT-4 via a linker sequence, Gly-Ser. The hNT-4-HSA fusion protein represented by SEQ ID NO: 82 is encoded by a gene having the nucleotide sequence represented by, for example, SEQ ID NO: 83. Also, a fusion protein having an amino acid sequence represented by SEQ ID NO: 82 and further having a mutation such as a substitution with different amino acid residues, a deletion, or an addition of one or more amino acid residues therein is included in the hNT-4-HSA fusion protein as long as it has a function as hNT-4. The hNT-4-HSA fusion protein preferably has a function as human serum albumin, such as a function to bind an endogenous substance and an exogenous substance such as a drug in the blood and transport them, but is not limited to this.

When mutations are introduced to the amino acid sequence represented by SEQ ID NO: 82, the sites and types (deletion, substitution, and addition) of individual mutations can be easily identified by alignment of the amino acid sequences before and after introduction of the mutations. The amino acid sequence having a mutation introduced thereto exhibits an identity of preferably 80% or more, 85% or more, 90% or more, or 95% or more, for example, an identity of 98% or more or 99% or more, to the amino acid sequence represented by SEQ ID NO: 82.

In a case where a mutation is introduced to an hNT-4-HSA fusion protein, which is a fusion protein of a wild-type hNT-4 and a wild-type HSA, the mutation can be introduced only to the HSA moiety and not introduced to the hNT-4 moiety; the mutation can be introduced only to the hNT-4 moiety without being introduced to the HSA moiety; or the mutation can be introduced to both of the HSA moiety and the hNT-4 moiety. In a case where a mutation is introduced only to the HSA moiety, the amino acid sequence of the moiety is an amino acid sequence of HSA obtained by introducing an mutation to a wild-type HSA as mentioned above. In a case where a mutation is introduced only to the hNT-4 moiety, the amino acid sequence of the moiety is an amino acid sequence of an hNT-4 obtained by introducing a mutation to a wild-type hNT-4 as mentioned above. In a case where a mutation is introduced to both of the HSA moiety and the hNT-4 moiety, the amino acid sequence of the HSA moiety is an amino acid sequence of HSA obtained by introducing a mutation to a wild-type HSA as mentioned above, and the amino acid sequence of the hNT-4 moiety is an amino acid sequence of hNT-4 obtained by introducing a mutation to a wild-type hNT-4 as mentioned above. The same applies to an hNT-4-HSA fusion protein having the amino acid sequence represented by SEQ ID NO: 82. The same applies to a fusion protein (hNT-4-SA) of a wild-type hNT-4 and a wild-type SA of a non-human animal species.

The case where a mutation is introduced to an hNT-4-HSA fusion protein having the amino acid sequence represented by SEQ ID NO: 82 will be described below. When an amino acid residue in the amino acid sequence represented by SEQ ID NO: 82 is substituted with another amino acid residue, the number of amino acid residues to be substituted is 1 to 10, 1 to 5, or 1 to 3, for example, one or two. When an amino acid residue is deleted, the number of amino acid residues to be deleted is 1 to 10, 1 to 5, or 1 to 3, for example, one or two. When an amino acid residue is added, one or more amino acid residues are added in the amino acid sequence represented by SEQ ID NO: 82 or added to the N terminal or C terminal of the amino acid sequence. The hNT-4-HSA fusion protein may have a combination of the substitution, deletion, and addition of amino acid residues. A mutation may be introduced only to the HSA moiety, or only to the hNT-4 moiety, or to both of them. The same applies to a fusion protein (hNT-4-SA) of hNT-4 and SA of a non-human animal species.

An hNT-4-HSA fusion protein having a constituent amino acid modified with a sugar chain is regarded as an hNT-4-HSA fusion protein. Also, an hNT-4-HSA fusion protein having a constituent amino acid modified with a phosphate group is regarded as an hNT-4-HSA fusion protein. Also, an hNT-4-HSA fusion protein modified with a group except a sugar chain and a phosphate group is regarded as an hNT-4-HSA fusion protein. Also, hNT-4-HSA fusion protein having a constituent amino acid whose side chain is converted by, e.g., a substitution reaction, is regarded as an hNT-4-HSA fusion protein. Examples of such conversion include, but are not limited to, conversion of a cysteine residue to formylglycine. The same applies to a fusion protein (NT-4-SA) of NT-4 of a non-human animal species and HSA and a fusion protein of NT-4 of a non-human animal species and SA of a non-human animal species.

More specifically, an hNT-4-HSA fusion protein modified with a sugar chain is meant to be included in an hNT-4-HSA fusion protein having an original amino acid sequence. Also, an hNT-4-HSA fusion protein modified with a phosphate group is meant to be included in an hNT-4-HSA fusion protein having an original amino acid sequence. Also, an hNT-4-HSA fusion protein modified with a group except a sugar chain and a phosphate group is meant to be included in an hNT-4-HSA fusion protein having an original amino acid sequence. Also, an hNT-4-HSA fusion protein having a constituent amino acid whose side chain is converted by, e.g., a substitution reaction, is meant to be included in an hNT-4-HSA fusion protein having an original amino acid sequence. Examples of such conversion include, but are not limited to, conversion of a cysteine residue to formylglycine. The same applies to a fusion protein (NT-4-SA) of NT-4 of a non-human animal species and HSA and a fusion protein of NT-4 of a non-human animal species and SA of a non-human animal species.

An hNT-4-HSA fusion protein in which a constituent hNT-4 is a precursor of an hNT-4 is regarded as an hNT-4-HSA fusion protein. In a case where the hNT-4-HSA fusion protein in which the hNT-4 is in the form of a precursor does not exhibit any function as hNT-4, however, the precursor is needed to be converted to exhibit a function as hNT-4 through in vivo or in vitro processing.

In an embodiment of the present invention, the term "hCDNF-HSA fusion protein" or "hCDNF-HSA" refers to a fusion protein having an amino acid sequence in which the N terminal of the amino acid sequence of HSA is bound to the C terminal of the hCDNF amino acid sequence directly or via a linker, and having a function as hCDNF. In an embodiment of the present invention, the term "hGDNF-HSA fusion protein" or "hGDNF-HSA" refers to a fusion protein having an amino acid sequence in which the N terminal of the amino acid sequence of HSA is bound to the C terminal of the hGDNF amino acid sequence directly or via a linker, and having a function as hGDNF. In an embodiment of the present invention, the term "HNRTN-HSA fusion protein" or "hNRTN-HSA" refers to a fusion protein having an amino acid sequence in which the N terminal of the amino acid sequence of HSA is bound to the C terminal of the hNRTN amino acid sequence directly or via a linker, and having a function as hNRTN. In an embodiment of the present invention, the term "hARTN-HSA fusion protein" or "hARTN-HSA" refers to a fusion protein having an amino acid sequence in which the N terminal of the amino acid sequence of HSA is bound to the C terminal of the hARTN amino acid sequence directly or via a linker, and having a function as hARTN. In an embodiment of the present invention, the term "hPSPN-HSA fusion protein" or "hPSPN-HSA" refers to a fusion protein having an amino acid sequence in which the N terminal of the amino acid sequence of HSA is bound to the C terminal of the hPSPN amino acid sequence directly or via a linker, and having a function as hPSPN. The above matters on HSA-hBDNF, HSA-hNGF, HSA-hNT-3, and HSA-hNT-4 are also applied to these fusion proteins.

Preferable examples of hCDNF-HSA include an hCDNF-HSA having an amino acid sequence obtained by removing the amino acid sequence of His6 from the C terminal of the amino acid sequence of SEQ ID NO: 158. This fusion protein is a fusion protein in which the N terminal of HSA is bound to the C terminal of hCDNF via a GS linker. Preferable examples of HSA-hCDNF include an HSA-hCDNF having an amino acid sequence obtained by removing the amino acid sequence of His6 from the C terminal of the amino acid sequence of SEQ ID NO: 160. This fusion protein is a fusion protein in which the N terminal of the pro-form of hCDNF is bound to the C terminal of HSA via a GS linker. Preferable examples of hGDNF-HSA include an hGDNF-HSA having an amino acid sequence obtained by removing the amino acid sequence of His6 from the C terminal of the amino acid sequence of SEQ ID NO: 164. This fusion protein is a fusion protein in which the N terminal of HSA is bound to the C terminal of hGDNF via a GS linker. Preferable examples of HSA-hGDNF include an HSA-hGDNF having an amino acid sequence obtained by removing the amino acid sequence of His6 from the C terminal of the amino acid sequence of SEQ ID NO: 166. This fusion protein is a fusion protein in which the N terminal of the pro-form of hGDNF is bound to the C terminal of HSA via a GS linker. Preferable examples of hNRTN-HSA include an hNRTN-HSA having an amino acid sequence obtained by removing the amino acid sequence of His6 from the C terminal of the amino acid sequence of SEQ ID NO: 170. This fusion protein is a fusion protein in which the N terminal of HSA is bound to the C terminal of hNRTN via a GS linker. Preferable examples of HSA-hNRTN include an HSA-hNRTN having amino acid sequence obtained by removing the amino acid sequence of His6 from the C terminal of the amino acid sequence of SEQ ID NO: 172. This fusion protein is a fusion protein in which the N terminal of the pro-form of hNRTN is bound to the C terminal of HSA via a GS linker. Preferable examples of hARTN-HSA include an hARTN-HSA having an amino acid sequence obtained by removing the amino acid sequence of His6 from the C terminal of the amino acid sequence of SEQ ID NO: 176. This fusion protein is a fusion protein in which the N terminal of HSA is bound to the C terminal of hARTN via a GS linker. Preferable examples of HSA-hARTN include an HSA-hARTN having an amino acid sequence obtained by removing the amino acid sequence of His6 from the C terminal of the amino acid sequence of SEQ ID NO: 178. This fusion protein is a fusion protein in which the N terminal of the pro-form of hARTN is bound to the C terminal of HSA via a GS linker. Preferable examples of hPSPN-HSA include an hPSPN-HSA having an amino acid sequence obtained by removing the amino acid sequence of His6 from the C terminal of the amino acid sequence of SEQ ID NO: 182. This fusion protein is a fusion protein in which the N terminal of HSA is bound to the C terminal of hPSPN via a GS linker. Preferable examples of HSA-hPSPN include an HSA-hPSPN having an amino acid sequence obtained by removing the amino acid sequence of His6 from the C terminal of the amino acid sequence of SEQ ID NO: 184. This fusion protein is a fusion protein in which the N terminal of the pro-form of hPSPN is bound to the C terminal of HSA via a GS linker. The above matters on HSA-hBDNF, HSA-hNGF, HSA-hNT-3, and HSA-hNT-4 are also applied to these fusion proteins.

In an embodiment of the present invention, the term "fusion protein of HSA and human neurotrophic factor", "fusion protein of human neurotrophic factor and HSA", or "fusion protein of human serum albumin and human neurotrophic factor" is meant to include both "HSA-human neurotrophic factor fusion protein" and "human neurotrophic factor-HSA fusion protein" as mentioned above. These matters are applied to both a case where SA is derived from a non-human animal species and a case where the nerve growth factor is derived from a non-human animal species.

Also, the term "fusion protein of HSA and hBDNF", "fusion protein of hBDNF and HSA", or "fusion protein of human serum albumin and human cerebral nerve-derived trophic factor" is meant to include both "HSA-hBDNF fusion protein" and "hBDNF-HSA fusion protein" mentioned above. These matters are applied to both a case where SA is derived from a non-human animal species and a case where the BDNF is derived from a non-human animal species.

Also, the term "fusion protein of HSA and hNGF", "fusion protein of hNGF and HSA", or "fusion protein of human serum albumin and human nerve growth factor" is meant to include both "HSA-hNGF fusion protein" and "hNGF-HSA fusion protein" mentioned above. These matters are applied to both a case where SA is derived from a non-human animal species and a case where the NGF is derived from a non-human animal species.

Also, the term "fusion protein of HSA and hNT-3", "fusion protein of hNT-3 and HSA", or "fusion protein of human serum albumin and human neurotrophin-3" is meant to include both "HSA-hNT-3 fusion protein" and "hNT-3-HSA fusion protein" mentioned above. These matters are applied to both a case where SA is derived from a non-human animal species and a case where the NT-3 is derived from a non-human animal species.

Also, the term "fusion protein of HSA and hNT-4", "fusion protein of hNT-4 and HSA", or "fusion protein of human serum albumin and human neurotrophin-4" is meant to include both "HSA-hNT-4 fusion protein" and "hNT-4-HSA fusion protein" mentioned above. These matters are applied to both a case where SA is derived from a non-human animal species and a case where the NT-4 is derived from a non-human animal species. The same applies to hCDNF, hGDNF, hNRTN, hARTN, and hPSPN.

In the fusion protein of SA and a neurotrophic factor according to an embodiment of the present invention, SA and a neurotrophic factor are bound directly or via a linker. The "linker" used herein refers to a moiety not belonging to any one of the amino acid sequence of SA and the amino acid sequence of the neurotrophic factor. More specifically, the linker is a peptide chain present between SA and the neurotrophic factor. The linker has various functions. Examples of the function of the linker include a function to bind SA and a neurotrophic factor by being interposed between SA and a neurotrophic factor, a function to reduce mutual interference between SA and a neurotrophic factor by keeping an intramolecular distance within a fusion protein, and a function to serve as a hinge for connecting SA and a neurotrophic factor by being interposed between SA and a neurotrophic factor, thereby forming a flexible three dimensional structure of a fusion protein. The linker is present within a molecule of a fusion protein and exerts at least one of these functions. These matters are applied irrespective of the animal species from which SA is derived and the animal species from which a nerve growth factor is derived. The matters are applied not only to a case of a neurotrophic factor being BDNF, NGF, NT-3, or NT-4 but also a case of another neurotrophic factor.

Now, a method for producing a fusion protein of SA and a neurotrophic factor will be more specifically described by way of a fusion protein of HSA and hBDNF, a fusion protein of HSA and hNGF, a fusion protein of HSA and hNT-3, and a fusion protein of HSA and hNT-4, below. The matters specifically described below can be applied to a case where SA is derived from a non-human animal species, and a case where BDNF, NGF, NT-3, or NT-4 is derived from a non-human animal species. Also, the matters can be applied to neurotrophic factors other than BDNF, NGF, NT-3, and NT-4.

A fusion protein of HSA and hBDNF can be prepared as a recombinant protein by preparing an expression vector integrating a DNA fragment in which a gene encoding hBDNF is bound to a downstream or upstream site of a gene encoding HSA in frame, and culturing a host cell transformed by introduction of the expression vector. Also, a fusion protein of HSA and hNGF can be prepared as a recombinant protein by preparing an expression vector integrating a DNA fragment in which a gene encoding hNGF is bound to a downstream or upstream site of a gene encoding HSA in frame, and culturing a host cell transformed by introduction of the expression vector. Also, a fusion protein of HSA and hNT-3 can be prepared as a recombinant protein by preparing an expression vector integrating a DNA fragment in which a gene encoding hNT-3 is bound to a downstream or upstream site of a gene encoding HSA in frame, and culturing a host cell transformed by introduction of the expression vector. Also, a fusion protein of HSA and hNT-4 can be prepared as a recombinant protein by preparing an expression vector integrating a DNA fragment in which a gene encoding hNT-4 is bound to a downstream or upstream site of a gene encoding HSA in frame, and culturing a host cell transformed by introduction of the expression vector. The fusion protein prepared as a recombinant protein in this manner is constituted of a single-chain polypeptide.

In a case where the fusion protein is prepared as a recombinant fusion protein, a fusion protein having the amino acid sequence of hBDNF at the C terminal of the amino acid sequence of HSA can be obtained by binding a gene encoding hBDNF to a downstream site of a gene encoding HSA in frame. Conversely, a fusion protein having the amino acid sequence of hBDNF at the N terminal of the amino acid sequence of HSA can be obtained by binding a gene encoding hBDNF to an upstream site of a gene encoding HSA in frame. Also, in a case where the fusion protein is prepared as a recombinant fusion protein, a fusion protein having the amino acid sequence of hNGF at the C terminal of the amino acid sequence of HSA can be obtained by binding a gene encoding hNGF to a downstream site of a gene encoding HSA in frame. Conversely, a fusion protein having the amino acid sequence of hNGF at the N terminal of the amino acid sequence of HSA can be obtained by binding a gene encoding hNGF to an upstream site of a gene encoding HSA in frame. Also, in a case where the fusion protein is prepared as a recombinant fusion protein, a fusion protein having the amino acid sequence of hNT-3 at the C terminal of the amino acid sequence of HSA can be obtained by binding a gene encoding hNT-3 to a downstream site of a gene encoding HSA in frame. Conversely, a fusion protein having the amino acid sequence of hNT-3 at the N terminal of the amino acid sequence of HSA can be obtained by binding a gene encoding hNT-3 to an upstream site of a gene encoding HSA in frame. Also, in a case where the fusion protein is prepared as a recombinant fusion protein, a fusion protein having the amino acid sequence of hNT-4 at the C terminal of the amino acid sequence of HSA can be obtained by binding a gene encoding hNT-4 to a downstream site of a gene encoding HSA in frame. Conversely, a fusion protein having the amino acid sequence of hNT-4 at the N terminal of the amino acid sequence of HSA can be obtained by binding a gene encoding hNT-4 to an upstream site of a gene encoding HSA in frame. In either case, the fusion protein prepared as a recombinant fusion protein is a single-chain polypeptide.

In a single-chain polypeptide of a fusion protein, if the amino acid sequence of HSA is positioned on the N terminal side of the amino acid sequence of hBDNF, hNGF, hNT-3, or hNT-4, the C terminal of HSA and the N terminal of hBDNF, hNGF, hNT-3, or hNT-4 are bound directly by a peptide bond, or via a linker. The "linker" herein refers to a moiety having an amino acid sequence that is present between the C terminal of HSA and the N terminal of hBDNF, hNGF, hNT-3, or hNT-4 and does not belong to any one of HSA and hBDNF, hNGF, hNT-3, or hNT-4 in the single-chain polypeptide. Figure 7 schematically shows an HSA-hBDNF fusion protein of a single-chain polypeptide having HSA, a linker and hBDNF in this order from the N terminal side. The HSA-hBDNF fusion protein is a fusion protein in which the C terminal of HSA and the N terminal of the linker are bound by a peptide bond, and the C terminal of the linker and the N terminal of hBDNF are bound by a peptide bond. Also, Figure 8 schematically shows an HSA-hNGF fusion protein of a single-chain polypeptide having HSA, a linker and hNGF in this order from the N terminal side. The HSA-hNGF fusion protein is a fusion protein in which the C terminal of HSA and the N terminal of the linker are bound by a peptide bond, and the C terminal of the linker and the N terminal of hNGF are bound by a peptide bond. Also, Figure 9 schematically shows an HSA-hNT-3 fusion protein of a single-chain polypeptide having HSA, a linker and hNT-3 in this order from the N terminal side. The HSA-hNT-3 fusion protein is a fusion protein in which the C terminal of HSA and the N terminal of the linker are bound by a peptide bond, and the C terminal of the linker and the N terminal of hNT-3 are bound by a peptide bond. Also, Figure 10 schematically shows an HSA-hNT-4 fusion protein of a single-chain polypeptide having HSA, a linker and hNT-4 in this order from the N terminal side. The HSA-hNT-4 fusion protein is a fusion protein in which the C terminal of HSA and the N terminal of the linker are bound by a peptide bond, and the C terminal of the linker and the N terminal of hNT-4 are bound by a peptide bond.

In a single-chain polypeptide of a fusion protein, if the amino acid sequence of hBDNF, hNGF, hNT-3, or hNT-4 is positioned on the N terminal of the amino acid sequence of HSA, the C terminal of hBDNF, hNGF, hNT-3, or hNT-4 and the N terminal of HSA are bound directly by a peptide bond, or via a linker. The "linker" herein refers to a moiety having an amino acid sequence that is present between the C terminal of hBDNF, hNGF, hNT-3, or hNT-4 and the N terminal of HSA and does not belong to any one of HSA and hBDNF, hNGF, hNT-3, or hNT-4 in the single-chain polypeptide. Figure 11 schematically shows an hBDNF-HSA fusion protein of a single-chain polypeptide having hBDNF, a linker and HSA in this order from the N terminal side. The hBDNF-HSA fusion protein is a fusion protein in which the C terminal of hBDNF and the N terminal of the linker are bound by a peptide bond, and the C terminal of the linker and the N terminal of HSA are bound by a peptide bond. Also, Figure 12 schematically shows an hNGF-HSA fusion protein of a single-chain polypeptide having hNGF, a linker and HSA in this order from the N terminal side. The hNGF-HSA fusion protein is a fusion protein in which the C terminal of hNGF and the N terminal of the linker are bound by a peptide bond, and the C terminal of the linker and the N terminal of HSA are bound by a peptide bond. Also, Figure 13 schematically shows an hNT-3-HSA fusion protein of a single-chain polypeptide having hNT-3, a linker and HSA in this order from the N terminal side. The hNT-3-HSA fusion protein is a fusion protein in which the C terminal of hNT-3 and the N terminal of the linker are bound by a peptide bond, and the C terminal of the linker and the N terminal of HSA are bound by a peptide bond. Also, Figure 14 schematically shows an hNT-4-HSA fusion protein of a single-chain polypeptide having hNT-4, a linker and HSA in this order from the N terminal side. The hNT-4-HSA fusion protein is a fusion protein in which the C terminal of hNT-4 and the N terminal of the linker are bound by a peptide bond, and the C terminal of the linker and the N terminal of HSA are bound by a peptide bond.

The amino acid sequence of a peptide linker is not particularly limited as long as it is present within the fusion protein molecule and produces a function as a linker. The length of a peptide linker is not particularly limited as long as it is present within a fusion protein molecule and produces a function as a linker. A peptide linker is constituted of one or more amino acids. In a case where a peptide linker is constituted of a plurality of amino acids, the number of amino acids is preferably 2 to 50, more preferably 5 to 30, and further preferably 10 to 25. Suitable examples of a peptide linker include peptide linkers constituted of Gly-Ser, Gly-Gly-Ser, or the amino acid sequences represented by SEQ ID NOs: 9 to 11 (these are collectively referred to as a basic sequence), and peptide linkers containing these. For example, a peptide linker contains an amino acid sequence having 2 to 10, 2 to 6 or 3 to 5 repeats of the basic sequence. These amino acid sequences may have, e.g., a deletion, a substitution or addition of one or more amino acids. In a case where an amino acid is deleted, the number of amino acids to be deleted is preferably 1 or 2. In a case where an amino acid is substituted with a different one, the number of amino acids to be substituted is preferably 1 or 2. In a case where an amino acid is added, the number of amino acids to be added is preferably 1 or 2. The amino acid sequence of a desired linker moiety can be prepared by using a combination of the deletion, substitution, and addition of amino acids. A peptide linker may be constituted of a single amino acid, and the amino acid constituting the linker is, for example, glycine and serine.

In an embodiment of the present invention,
(1) a fusion protein of HSA and hBDNF refers to a fusion protein is characterized in that the fusion protein, when expressed as a recombinant protein in a host cell, particularly expressed as a recombinant protein so as to be secreted from the cell and accumulated in a culture solution, provides an expression level as hBDNF in the culture supernatant in terms of concentration or physiological activity, that is at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, 10 to 20 times, or 20 to 30 times that of a wild-type hBDNF expressed as a recombinant protein in a host cell under the same conditions.
(2) In an embodiment of the present invention, a fusion protein of HSA and hNGF is meant to be characterized in that the fusion protein, when expressed as a recombinant protein in a host cell, particularly expressed as a recombinant protein so as to be secreted from the cell and accumulated in a culture solution, provides an expression level as hNGF in the culture supernatant in terms of concentration or physiological activity, that is at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, 10 to 20 times, or 20 to 30 times that of a wild-type hNGF expressed as a recombinant protein in a host cell under the same conditions.
(3) In an embodiment of the present invention, a fusion protein of HSA and hNT-3 is meant to be characterized in that the fusion protein, when expressed as a recombinant protein in a host cell, particularly expressed as a recombinant protein so as to be secreted from the cell and accumulated in a culture solution, provides an expression level as hNT-3 in the culture supernatant in terms of concentration or physiological activity, that is at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, 10 to 20 times, or 20 to 30 times that of a wild-type hNT-3 expressed as a recombinant protein in a host cell under the same conditions.
(4) In an embodiment of the present invention, a fusion protein of HSA and hNT-4 is meant to be characterized in that the fusion protein, when expressed as a recombinant protein in a host cell, particularly expressed as a recombinant protein so as to be secreted from the cell and accumulated in a culture solution, provides an expression level as hNT-4 in the culture supernatant in terms of concentration or physiological activity, that is at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, 10 to 20 times, or 20 to 30 times that of a wild-type hNT-4 expressed as a recombinant protein in a host cell under the same conditions.

In the embodiments of (1) to (4) in the above, the same conditions mean that the expression vector, host cell, culture conditions and others are the same. A preferable host cell to be used herein is a mammalian cell such as a CHO cell or an NS/0 cell, in particular a CHO cell.

As preferable embodiments of such a fusion protein of HSA and hBDNF, the following (1) to (4) are mentioned:
(1) a fusion protein having the amino acid sequence represented by SEQ ID NO: 68, in which the N terminal of the amino acid sequence of a wild-type hBDNF represented by SEQ ID NO: 60 is bound to the C terminal of the amino acid sequence of a wild-type HSA represented by SEQ ID NO: 3 via a linker sequence Gly-Ser;
(2) a fusion protein having the amino acid sequence represented by SEQ ID NO: 76, in which the N terminal of the amino acid sequence of a wild-type HSA represented by SEQ ID NO: 3 is bound to the C terminal of the amino acid sequence of a wild-type hBDNF represented by SEQ ID NO: 60 via a linker sequence Gly-Ser;
(3) a fusion protein having the amino acid sequence represented by SEQ ID NO: 85, in which the N terminal of the amino acid sequence of a wild-type hBDNF (pro-form) represented by SEQ ID NO: 84 is bound to the C terminal of the amino acid sequence of a wild-type HSA represented by SEQ ID NO: 3 via a linker sequence Gly-Ser; and
(4) a fusion protein having the amino acid sequence represented by SEQ ID NO: 86, in which the N terminal of the amino acid sequence of a wild-type HSA represented by SEQ ID NO: 3 is bound to the C terminal of the amino acid sequence of a wild-type hBDNF (pro-form) represented by SEQ ID NO: 84 via a linker sequence Gly-Ser.

Also, as preferable embodiments of such a fusion protein of HSA and hNGF, the following (1) to (4) are mentioned:
(1) a fusion protein having the amino acid sequence represented by SEQ ID NO: 70, in which the N terminal of the amino acid sequence of a wild-type hNGF represented by SEQ ID NO: 62 is bound to the C terminal of the amino acid sequence of a wild-type HSA represented by SEQ ID NO: 3 via a linker sequence Gly-Ser;
(2) a fusion protein having the amino acid sequence represented by SEQ ID NO: 78, in which the N terminal of the amino acid sequence of a wild-type HSA represented by SEQ ID NO: 3 is bound to the C terminal of the amino acid sequence of a wild-type hNGF represented by SEQ ID NO: 62 via a linker sequence Gly-Ser;
(3) a fusion protein having the amino acid sequence represented by SEQ ID NO: 88, in which the N terminal of the amino acid sequence of a wild-type hNGF (pro-form) represented by SEQ ID NO: 87 is bound to the C terminal of the amino acid sequence of a wild-type HSA represented by SEQ ID NO: 3 via a linker sequence Gly-Ser; and
(4) a fusion protein having the amino acid sequence represented by SEQ ID NO: 89, in which the N terminal of the amino acid sequence of a wild-type HSA represented by SEQ ID NO: 3 is bound to the C terminal of the amino acid sequence of a wild-type hNGF (pro-form) represented by SEQ ID NO: 87 via a linker sequence Gly-Ser.

Also, as preferable embodiments of such a fusion protein of HSA and hNT-3, the following (1) to (4) are mentioned:
(1) a fusion protein having the amino acid sequence represented by SEQ ID NO: 72, in which the N terminal of the amino acid sequence of a wild-type hNT-3 represented by SEQ ID NO: 64 is bound to the C terminal of the amino acid sequence of a wild-type HSA represented by SEQ ID NO: 3 via a linker sequence Gly-Ser;
(2) a fusion protein having the amino acid sequence represented by SEQ ID NO: 80, in which the N terminal of the amino acid sequence of a wild-type HSA represented by SEQ ID NO: 3 is bound to the C terminal of the amino acid sequence of a wild-type hNT-3 represented by SEQ ID NO: 64 via a linker sequence Gly-Ser;
(3) a fusion protein having the amino acid sequence represented by SEQ ID NO: 91, in which the N terminal of the amino acid sequence of a wild-type hNT-3 (pro-form) represented by SEQ ID NO: 90 is bound to the C terminal of the amino acid sequence of a wild-type HSA represented by SEQ ID NO: 3 via a linker sequence Gly-Ser; and
(4) a fusion protein having the amino acid sequence represented by SEQ ID NO: 92, in which the N terminal of the amino acid sequence of a wild-type HSA represented by SEQ ID NO: 3 is bound to the C terminal of the amino acid sequence of a wild-type hNT-3 (pro-form) represented by SEQ ID NO: 90 via a linker sequence Gly-Ser.

Also, as preferable embodiments of such a fusion protein of HSA and hNT-4, the following (1) to (4) are mentioned:
(1) a fusion protein having the amino acid sequence represented by SEQ ID NO: 74, in which the N terminal of the amino acid sequence of a wild-type hNT-4 represented by SEQ ID NO: 66 is bound to the C terminal of the amino acid sequence of a wild-type HSA represented by SEQ ID NO: 3 via a linker sequence Gly-Ser;
(2) a fusion protein having the amino acid sequence represented by SEQ ID NO: 82, in which the N terminal of the amino acid sequence of a wild-type HSA represented by SEQ ID NO: 3 is bound to the C terminal of the amino acid sequence of a wild-type hNT-4 represented by SEQ ID NO: 66 via a linker sequence Gly-Ser;
(3) a fusion protein having the amino acid sequence represented by SEQ ID NO: 94, in which the N terminal of the amino acid sequence of a wild-type hNT-4 (pro-form) represented by SEQ ID NO: 93 is bound to the C terminal of the amino acid sequence of a wild-type HSA represented by SEQ ID NO: 3 via a linker sequence Gly-Ser; and
(4) a fusion protein having the amino acid sequence represented by SEQ ID NO: 95, in which the N terminal of the amino acid sequence of a wild-type HSA represented by SEQ ID NO: 3 is bound to the C terminal of the amino acid sequence of a wild-type hNT-4 (pro-form) represented by SEQ ID NO: 93 via a linker sequence Gly-Ser.

In an embodiment of the present invention, the fusion protein of HSA and hBDNF is characterized in that the fusion protein, when expressed as a recombinant protein in a host cell, particularly expressed as a recombinant protein so as to be secreted from the cell and accumulated in a culture solution, provides an increased expression level as hBDNF in the culture supernatant in terms of concentration or physiological activity, compared to that of a wild-type hBDNF expressed as a recombinant protein in a host cell under the same conditions. Accordingly, when a fusion protein of HSA and hBDNF is produced as a recombinant protein, a production efficiency thereof can be increased compared to a wild-type hBDNF, and thus a production cost of the fusion protein can be reduced. Note that, it is known that medicaments containing a recombinant protein as an active ingredient are very expensive. Accordingly, if the amount of a recombinant protein produced under the same conditions is increased by several percent, for example, 3 to 9%, a large economic effect is produced. The same applies to neurotrophic factors such as hBDNF, hNGF, hNT-3, and hNT-4.

Note that, in the specification, when the expression level of a fusion protein of HSA and hBDNF expressed in a host cell as a recombinant protein is compared that of a wild-type hBDNF expressed in a host cell as a recombinant protein under the same condition, comparison is preferably made not based on the mass of a protein expressed but based on the number of molecules or hBDNF physiological activity of the protein expressed. This rule is also applied to a fusion protein of HSA and hNGF, a fusion protein of HSA and hNT-3, and a fusion protein of HSA and hNT-4. In addition, this rule is applied irrespective of the animal species from which constituent SA in a fusion protein is derived and the animal species from which a constituent nerve growth factor therein is derived.

A fusion protein of HSA and hBDNF, a fusion protein of HSA and hNGF, a fusion protein of HSA and hNT-3, and a fusion protein of HSA and hNT-4 can be produced as a recombinant protein by culturing a host cell transformed with an expression vector integrating a gene encoding each of the fusion proteins. In this case, the fusion proteins can be produced by using the expression vector, host cell, medium and others that can be used for producing the fusion protein of HSA and hGALC or fusion protein of HSA and hGBA mentioned above.

A fusion protein of HSA and hBDNF, a fusion protein of HSA and hNGF, a fusion protein of HSA and hNT-3, or a fusion protein of HSA and hNT-4 can be expressed within a cell or in a medium by culturing a host cell encoding the fusion protein. These fusion proteins can be purified by separating them from foreign substances by a method such as column chromatography.

The fusion protein of HSA and hBDNF, fusion protein of HSA and hNGF, fusion protein of HSA and hNT-3, or fusion protein of HSA and hNT-4 purified can be used as a pharmaceutical composition. Particularly, a fusion protein of HSA and hBDNF can be used as a pharmaceutical composition targeted to neurodegenerative diseases such as Alzheimer's disease, Parkinson's disease, and Huntington's disease, spinal degenerative diseases such as amyotrophic lateral sclerosis, developmental impairments such as diabetic neuropathy, ischemic cerebral disease and Rett syndrome, schizophrenia, depression and Rett syndrome. Particularly, a fusion protein of HSA and hNGF can be used as a pharmaceutical composition targeted to neurodegenerative diseases and the like such as Alzheimer's disease, Parkinson's disease, and Huntington's disease. Particularly, a fusion protein of HSA and hNT-3 and a fusion protein of HSA and hNT-4 can be used as a pharmaceutical composition targeted to neurodegenerative diseases and the like.

A pharmaceutical composition containing a fusion protein of HSA and hBDNF, a fusion protein of HSA and hNGF, a fusion protein of HSA and hNT-3, or a fusion protein of HSA and hNT-4 as an active ingredient can be administered intravenously, intramuscularly, intraperitoneally, subcutaneously or intracerebroventricularly as an injection. Such an injection can be supplied as a lyophilized preparation or an aqueous liquid. The aqueous liquid may be in a form it is filled in a vial or supplied as a prefilled preparation in a syringe. The lyophilized preparation is dissolved and restored in an aqueous medium before use and then used. The same applies to HSA and other neurotrophic factors.

A fusion protein of HSA and hBDNF, a fusion protein of HSA and hNGF, a fusion protein of HSA and hNT-3, or a fusion protein of HSA and hNT-4 can further form a conjugate with an antibody or a ligand. For example, a fusion protein of HSA and hBDNF or a ligand, a fusion protein of HSA and hNGF, a fusion protein of HSA and hNT-3, and a fusion protein of HSA and hNT-4 can each form a conjugate with an antibody or a ligand capable of specifically binding to a receptor on a cerebrovascular endothelial cell. A fusion protein of any of HSA and hBDNF, HSA and hNGF, HSA and hNT-3, and HSA and hNT-4 in the form of a conjugate with an antibody or a ligand capable of specifically binding to a receptor on a cerebrovascular endothelial cell can bind to a receptor on a cerebrovascular endothelial cell. The fusion protein bound to a receptor on a cerebrovascular endothelial cell can pass through the blood-brain barrier (BBB) to reach the tissue of the central nervous system (CNS). Thus, each of those fusion proteins in the form of a conjugate with such an antibody or a ligand, can pass through the blood-brain barrier (BBB) and exert a function thereof in the central nervous system (CNS). In a case where SA is SA of a non-human animal species, the same applies to cases where BDNF, NGF, hNT-3, and hNT-4 are derived from non-human animal species. Note that, the same applies to a case where the neurotrophic factor is a neurotrophic factor other than BDNF, NGF, hNT-3, and hNT-4.

In a conjugate of a fusion protein of HSA and hBDNF with an antibody or a ligand, the phrase that hBDNF has a function as an hBDNF means that hBDNF has a specific activity of preferably 10% or more, more preferably 20% or more, further preferably 50% or more, further more preferably 80% or more, for example, 90% or more, or 95% or more when the specific activity of a normal wild-type hBDNF is regarded as 100%. Note that, the specific activity of the hBDNF in the conjugate herein is computationally obtained by multiplying the physiological activity of an hBDNF per unit mass of the conjugate by (the molecular weight of the conjugate/the molecular weight of the moiety corresponding to the hBDNF in the conjugate). This is applied to both a case where SA is derived from a non-human animal species and a case where BDNF is derived from a non-human animal species.

In a conjugate of a fusion protein of HSA and hNGF with an antibody or a ligand, the phrase that hNGF has a function as an hNGF means that hNGF has a specific activity of preferably 10% or more, more preferably 20% or more, further preferably 50% or more, further more preferably 80% or more, for example, 90% or more, or 95% or more when the specific activity of a normal wild-type hNGF is regarded as 100%. Note that, the specific activity of the hNGF in the conjugate herein is computationally obtained by multiplying the physiological activity of an hNGF per unit mass of the conjugate by (the molecular weight of the conjugate/the molecular weight of the moiety corresponding to the hNGF in the conjugate). This is applied to both a case where SA is derived from a non-human animal species and a case where NGF is derived from a non-human animal species.

In a conjugate of a fusion protein of HSA and hNT-3 with an antibody or a ligand, the phrase that hNT-3 has a function as an hNT-3 means that hNT-3 has a specific activity of preferably 10% or more, more preferably 20% or more, further preferably 50% or more, further more preferably 80% or more, for example, 90% or more, or 95% or more when the specific activity of a normal wild-type hNT-3 is regarded as 100%. Note that, the specific activity of the hNT-3 in the conjugate herein is computationally obtained by multiplying the physiological activity of an hNT-3 per unit mass of the conjugate by (the molecular weight of the conjugate/the molecular weight of the moiety corresponding to the hNT-3 in the conjugate). This is applied to both a case where SA is derived from a non-human animal species and a case where NT-3 is derived from a non-human animal species.

In a conjugate of a fusion protein of HSA and hNT-4 with an antibody or a ligand, the phrase that hNT-4 has a function as an hNT-4 means that hNT-4 has a specific activity of preferably 10% or more, more preferably 20% or more, further preferably 50% or more, further more preferably 80% or more, for example, 90% or more, or 95% or more when the specific activity of a normal wild-type hNT-4 is regarded as 100%. Note that, the specific activity of the hNT-4 in the conjugate herein is computationally obtained by multiplying the physiological activity of an hNT-4 per unit mass of the conjugate by (the molecular weight of the conjugate/the molecular weight of the moiety corresponding to the hNT-4 in the conjugate). This is applied to both a case where SA is derived from a non-human animal species and a case where NT-4 is derived from a non-human animal species.

In an embodiment of the present invention, a conjugate of a fusion protein of HSA and hBDNF with an antibody or a ligand is characterized in that the conjugate, when expressed as a recombinant protein in a host cell, particularly expressed as a recombinant protein so as to be secreted from the cell and accumulated in a culture solution, provides an expression level as the hBDNF in the culture supernatant in terms of concentration or physiological activity, that is at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, 10 to 20 times, or 20 to 30 times that of a wild-type hBDNF expressed as a recombinant protein in a host cell under the same conditions.

Also, in an embodiment of the present invention, a conjugate of a fusion protein of HSA and hNGF with an antibody or a ligand is characterized in that the conjugate, when expressed as a recombinant protein in a host cell, particularly expressed as a recombinant protein so as to be secreted from the cell and accumulated in a culture solution, provides an expression level as the hNGF in the culture supernatant in terms of concentration or physiological activity, that is at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, 10 to 20 times, or 20 to 30 times that of a wild-type hNGF expressed as a recombinant protein in a host cell under the same conditions.

Also, in an embodiment of the present invention, a conjugate of a fusion protein of HSA and hNT-3 with an antibody or a ligand is characterized in that the conjugate, when expressed as a recombinant protein in a host cell, particularly expressed as a recombinant protein so as to be secreted from the cell and accumulated in a culture solution, provides an expression level as the hNT-3 in the culture supernatant in terms of concentration or physiological activity, that is at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, 10 to 20 times, or 20 to 30 times that of a wild-type hNT-3 expressed as a recombinant protein in a host cell under the same conditions.

Also, in an embodiment of the present invention, a conjugate of a fusion protein of HSA and hNT-4 with an antibody or a ligand is characterized in that the conjugate, when is expressed as a recombinant protein in a host cell, particularly expressed as a recombinant protein so as to be secreted from the cell and accumulated in a culture solution, provides an expression level as the hNT-4 in the culture supernatant in terms of concentration or physiological activity, that is at least 1.1 times or more, 1.2 times or more, 1.5 times or more, 2 times or more, 2.5 times or more, or 3.5 times or more, for example, 1.1 to 4 times, 1.5 to 3.6 times, 2 to 3.6 times, 10 to 20 times, or 20 to 30 times that of a wild-type hNT-4 expressed as a recombinant protein in a host cell under the same conditions.

Some of neurotrophic factors, when being expressed as a recombinant protein by using a host cell to which an expression vector integrating a gene encoding wild-type one is introduced, are difficult to mass-produce because of limited expression levels. Such neurotrophic factors include BDNF, NGF, NT-3, and NT-4. An embodiment of the present invention is a recombinant protein as a fusion protein of such a neurotrophic factor difficult to produce as a recombinant protein and SA. The recombinant protein is easier to mass-produce by using a host cell to which an expression vector integrating a gene encoding this is introduced, than the corresponding wild-type neurotrophic factor. The animal species of a neurotrophic factor to be bound to SA herein is not particularly limited and the neurotrophic factor is preferably a human neurotrophic factor. Also, the animal species of SA to be bound to a neurotrophic factor is not particularly limited and the SA is preferably HSA.

GDNF family ligands (GFLs) are a family consisting of four neurotrophic factors: glial cell line-derived neurotrophic factor (GDNF), neurturin (NRTN), artemin (ARTN), and persephin (PSPN). Also, a fusion protein of any of them and HSA and a conjugate obtained by binding an antibody or a ligand to a fusion protein of any of them and HSA are preferable embodiments of the present invention. Also, a fusion protein of cerebral dopamine neurotrophic factor (CDNF) and HSA and a conjugate obtained by binding an antibody or a ligand to a fusion protein of CDNF and HSA are preferable embodiments of the present invention.

GDNF has functions, for example, to promote the survival and differentiation of dopaminergic neurons and to prevent the apoptosis of motor neurons due to axonal break. A fusion protein of GDNF and HSA and a conjugate obtained by binding an antibody or a ligand to a fusion protein of GDNF and HSA can be used as a pharmaceutical composition for synucleinopathy (including Parkinson's disease (PD), dementia with Lewy bodies (DLB), and multiple system atrophy (MSA)), polyglutamine disease (including Huntington's disease (HD), spinocerebellar degeneration, bulbospinal muscular atrophy, and frontotemporal lobar degeneration), amyotrophic lateral sclerosis (ALS), and alcoholism.

NRTN has functions, for example, to maintain the survival of many types of neurons such as cholinergic neurons and dopaminergic neurons. A fusion protein of NRTN and HSA and a conjugate obtained by binding an antibody or a ligand to a fusion protein of NRTN and HSA can be used as a pharmaceutical composition for synucleinopathy (including Parkinson's disease (PD), dementia with Lewy bodies (DLB), and multiple system atrophy (MSA)), and chronic pain.

ARTN has functions, for example, to promote the survival and differentiation of dopaminergic neurons. A fusion protein of ARTN and HSA and a conjugate obtained by binding an antibody or a ligand to a fusion protein of NRTN and HSA can be used as a pharmaceutical composition for synucleinopathy (including Parkinson's disease (PD), dementia with Lewy bodies (DLB), and multiple system atrophy (MSA)) and chronic pain.

PSPN has functions, for example, to promote the survival of cholinergic neurons in the basal forebrain and motor neurons in the spinal cord. A fusion protein of NRTN and HSA and a conjugate obtained by binding an antibody or a ligand to a fusion protein of NRTN and HSA can be used as a pharmaceutical composition for synucleinopathy (including Parkinson's disease (PD), dementia with Lewy bodies (DLB), and multiple system atrophy (MSA)), polyglutamine disease (including Huntington's disease (HD), spinocerebellar degeneration, bulbospinal muscular atrophy, and frontotemporal lobar degeneration), Alzheimer's disease, and stroke.

CDNF has activities, for example, to promote the survival and differentiation of dopaminergic neurons. A fusion protein of CDNF and HSA and a conjugate obtained by binding an antibody or a ligand to a fusion protein of CDNF and HSA can be used as a pharmaceutical composition for synucleinopathy (including Parkinson's disease (PD), dementia with Lewy bodies (DLB), and multiple system atrophy (MSA)) and polyglutamine disease (including Huntington's disease (HD), spinocerebellar degeneration, bulbospinal muscular atrophy, and frontotemporal lobar degeneration). The same applies to a fusion protein of MANF and HSA and a conjugate obtained by binding an antibody to a fusion protein of MANF and HSA.

In the present invention, the term "antibody" mainly refers to any of a human antibody, a mouse antibody, a humanized antibody, an antibody derived from any of Camelidae animals (including alpaca), a chimeric antibody of a human antibody and another mammalian antibody, and a chimeric antibody of a mouse antibody and another mammalian antibody, but the meaning is not limited thereto as long as the antibody has a characteristic to specifically bind to a specific antigen, and the animal species of an antibody is not particularly limited, either.

In the present invention, the term "human antibody" refers to an antibody the whole protein of which is encoded by a gene derived from a human. However, an antibody encoded by a gene obtained by introducing a mutation to the original human gene without causing any change to the original amino acid sequence, for example, for the purpose of increasing the expression efficiency of the gene is also included in "human antibodies". Also, an antibody prepared by replacing part of a human antibody with part of another human antibody with a combination of two or more genes each encoding a human antibody is regarded as a "human antibody". A human antibody has complementarity-determining regions (CDRs) at three positions in each light chain and complementarity-determining regions (CDRs) at three positions in each heavy chain. The CDRs at three positions in each light chain are referred to as CDR1, CDR2, and CDR3 in order from the N terminal side. Likewise, the CDRs at three positions in each heavy chain are referred to as CDR1, CDR2, and CDR3 in order from the N terminal side. An antibody obtained by modifying the antigen specificity, affinity, or the like of a human antibody through replacing a CDR of the human antibody with a CDR of another human antibody is also included in human antibodies.

In the present invention, an antibody obtained by introducing a mutation such as a substitution, a deletion, and an addition to the amino acid sequence of an original human antibody through modification of a gene of the original antibody is also included in "human antibodies". When an amino acid in the amino acid sequence of an original antibody is substituted with another amino acid, the number of amino acids to be substituted is preferably 1 to 20, more preferably 1 to 5, and further preferably 1 to 3. When an amino acid in the amino acid sequence of an original antibody is deleted, the number of amino acids to be deleted is preferably 1 to 20, more preferably 1 to 5, and further preferably 1 to 3. Also, an antibody obtained by introducing a mutation having the substitution and deletion of amino acids in combination is regarded as a human antibody. When an amino acid is added, preferably 1 to 20 amino acids, more preferably 1 to 5 amino acids, or further preferably 1 to 3 amino acids are added in the amino acid sequence of an original antibody or added to the N terminal or C terminal thereof. Also, an antibody obtained by introducing a mutation having the addition, substitution, and deletion of amino acids in combination is regarded as a human antibody. The amino acid sequence of an antibody having a mutation introduced thereto exhibits an identity of preferably 80% or more, more preferably 90% or more, further preferably 95% or more, or further more preferably 98% or more to the amino acid sequence of the original antibody. In a case where the above mutation is introduced to a human antibody, the mutation can be introduced to a variable region of the antibody. In a case where the above mutation is introduced to a variable region of an antibody, the mutation may be introduced to any of the CDRs and framework regions of the variable region, and is particularly introduced to a framework region. That is, the phrase "a gene derived from a human" includes not only the original gene derived from a human but also a gene obtained by introducing modification thereto.

In the present invention, the term "humanized antibody" refers to an antibody in which part of the amino acid sequence of the variable region (for example, all or some of the CDRs, in particular) is derived from a non-human mammal and the other region is derived from a human. Examples of the humanized antibody include an antibody prepared by replacing complementarity-determining regions (CDRs) at three positions in each light chain constituting a human antibody and complementarity-determining regions (CDRs) at three positions in each heavy chain with CDRs of another mammal. The biological species of another mammal from which CDRs to be transplanted at proper positions of a human antibody are derived is not particularly limited as long as the mammal is a non-human mammal, and is preferably a mouse, a rat, a rabbit, a horse, or a non-human primate, more preferably any of a mouse and a rat, and further preferably a mouse. An antibody obtained by introducing a mutation the same as the above mutation that can be introduced to a human antibody to the amino acid sequence of an original humanized antibody is also included in "humanized antibodies".

In the present invention, the term "chimeric antibody" refers to an antibody in which fragments of two or more different antibodies derived from two or more different species are linked together.

A chimeric antibody of a human antibody and an antibody of another mammal is an antibody obtained by replacing part of a human antibody with part of an antibody of a non-human mammal. An antibody consists of an Fc region, Fab region, and hinge part described later. Specific examples of the chimeric antibody include a chimeric antibody in which the Fc region is derived from a human antibody and the Fab region is derived from an antibody of another mammal. The hinge part is derived from either a human antibody or an antibody of another mammal. Another example is a chimeric antibody in which conversely the Fc region is derived from another mammal and the Fab region is derived form a human antibody. The hinge part is derived from either a human antibody or an antibody of another mammal.

Alternatively, it can be said that an antibody consists of a variable region and a constant region. Other specific examples of the chimeric antibody include a chimeric antibody in which the heavy chain constant region (C_{H}) and the light chain constant region (C_{L}) are derived from a human antibody and the heavy chain variable region (V_{H}) and the light chain variable region (V_{L}) are derived from an antibody of another mammal, and a chimeric antibody in which conversely the heavy chain constant region (C_{H}) and the light chain constant region (C_{L}) are derived from an antibody of another mammal and the heavy chain variable region (V_{H}) and the light chain variable region (V_{L}) are derived from a human antibody. Here, the biological species of another mammal is not particularly limited as long as the mammal is a non-human mammal, and is preferably a mouse, a rat, a rabbit, a horse, or a non-human primate, for example, a mouse.

An antibody in an embodiment of the present invention has a basic structure consisting of two immunoglobulin light chains (or simply "light chains") and two immunoglobulin heavy chains (or simply "heavy chains"), four polypeptide chains in total. It should be noted that the term "antibody" includes not only those having such basic structure but also:
(1) an antibody consisting of one light chain and one heavy chain, two polypeptide chains in total;
(2) an antibody consisting of a Fab region, which results from deletion of the Fc region from the basic structure of an antibody in the original meaning, and an antibody consisting of the whole or part of the Fab region and hinge part (including Fab, F(ab'), and F(ab')₂);
(3) a single chain antibody obtained by binding a linker to the C terminal side of a light chain and further binding a heavy chain to the C terminal side thereof;
(4) a single chain antibody obtained by binding a linker to the C terminal side of a heavy chain and further binding a light chain to the C terminal side thereof;
(5) an antibody consisting of an Fc region, which results from deletion of the Fab region from the basic structure of an antibody in the original meaning, with the amino acid sequence of the Fc region modified to have a characteristic to specifically bind to a specific antigen (Fc antibody); and
(6) a single domain antibody described later.

An antibody in an embodiment of the present invention is an antibody derived from a Camelidae animal (including alpaca). Antibodies of Camelidae animals include an antibody consisting of two heavy chains linked together via a disulfide bond. This antibody consisting of two heavy chains is referred to as a heavy chain antibody. VHH is an antibody consisting of one heavy chain containing the variable region of a heavy chain constituting a heavy chain antibody, or an antibody consisting of one heavy chain lacking the constant region (CH) constituting a heavy chain antibody. VHH is also one of antibodies in embodiments of the present invention. Also, an antibody consisting of two light chains linked together via a disulfide bond is one of antibodies in embodiments of the present invention. This antibody consisting of two light chains is referred to as a light chain antibody. Also, an antibody obtained by introducing a mutation to the amino acid sequence of an antibody of a Camelidae animal in order to reduce antigenicity when an antibody (including VHH) derived from a Camelidae animal is administered to a human is regarded as the antibody in an embodiment of the present invention. In a case where a mutation is introduced to the amino acids of an antibody of a Camelidae animal, mutations the same as the mutations that can be introduced to the antibodies described herein can be introduced.

An antibody in an embodiment of the present invention is an antibody derived from a shark. The antibody of a shark consists of two heavy chains linked via a disulfide bond. This antibody consisting of two heavy chains is referred to as a heavy chain antibody. VNAR is an antibody consisting of one heavy chain containing the variable region of a heavy chain constituting a heavy chain antibody, or an antibody consisting of one heavy chain lacking the constant region (CH) constituting a heavy chain antibody. VNAR is also one of the antibodies in embodiments of the present invention. An antibody obtained by introducing a mutation to the amino acid sequence of an antibody of a shark in order to reduce antigenicity when an antibody (including VNAR) derived from a shark is administered to a human is also the antibody in an embodiment of the present invention. When a mutation is introduced to the amino acids of an antibody of a shark, mutations the same as the mutations that can be introduced to the antibodies described herein can be introduced. An antibody obtained by humanizing an antibody of a shark is also one of the antibodies in embodiments of the present invention.

An antibody having a basic structure consisting of two light chains and two heavy chains, four polypeptide chains in total, has complementarity-determining regions (CDRs) at three positions in the variable region (V_{L}) of each light chain and complementarity-determining regions (CDRs) at three positions in the variable region (V_{H}) of each heavy chain. The CDRs at three positions in each light chain are referred to as CDR1, CDR2, and CDR3 in order from the N terminal side. Likewise, the CDRs at three positions in each heavy chain are referred to as CDR1, CDR2, and CDR3 in order from the N terminal side. However, even a molecule in which some or all of the CDRs are incomplete or absent is included in antibodies as long as the molecule has a characteristic to specifically bind to a specific antigen. Regions other than the CDRs in each of the variable regions (V_{L} and V_{H}) of the light chains and heavy chains are referred to as framework regions (FRs). The FRs are referred to as FR1, FR2, FR3, and FR4 in order from the N terminal side. Normally, the CDRs and FRs are present in the order of FR1, CDR1, FR2, CDR2, FR3, CDR3, and FR4 from the N terminal side.

In an embodiment of the present invention, an antibody obtained by introducing a mutation such as a substitution, a deletion, and an addition to the amino acid sequence of an original antibody is also included in antibodies. When an amino acid in the amino acid sequence of an original antibody is substituted with another amino acid, the number of amino acids to be substituted is preferably 1 to 20, more preferably 1 to 5, and further preferably 1 to 3. When an amino acid in the amino acid sequence of an original antibody is deleted, the number of amino acids to be deleted is preferably 1 to 20, more preferably 1 to 5, and further preferably 1 to 3. Also, a mutant obtained by introducing a mutation having the substitution and deletion of amino acids in combination is regarded as an antibody. When an amino acid is added, preferably 1 to 20, more preferably 1 to 5, or further preferably 1 to 3 amino acids are added in the amino acid sequence of an original antibody or added to the N terminal or C terminal thereof. A mutant obtained by introducing a mutation having the addition, substitution, and deletion of amino acids in combination is also regarded as an antibody. The amino acid sequence of an antibody having a mutation introduced thereto exhibits an identity of preferably 80% or more, more preferably 85% or more, or further preferably 90% or more, 95% or more, or 98% or more, to the amino acid sequence of the original antibody.

In an embodiment of the present invention, an antibody obtained by introducing a mutation such as a substitution, a deletion, and an addition to the amino acid sequence of the variable region of the original antibody is also included in antibodies. When an amino acid in the amino acid sequence of an original antibody is substituted with another amino acid, the number of amino acids to be substituted is preferably 1 to 10, more preferably 1 to 5, and further preferably 1 to 3. When an amino acid in the amino acid sequence of an original antibody is deleted, the number of amino acids to be deleted is preferably 1 to 10, more preferably 1 to 5, and further preferably 1 to 3. Also, a mutant obtained by introducing a mutation having the substitution and deletion of amino acids in combination is regarded as an antibody. When an amino acid is added, preferably 1 to 10, more preferably 1 to 5, or further preferably 1 to 3 amino acids are added in the amino acid sequence of an original antibody or added to the N terminal or C terminal thereof. A mutant obtained by introducing a mutation having the addition, substitution, and deletion of amino acids in combination is also regarded as an antibody. The amino acid sequence of an antibody having a mutation introduced thereto exhibits an identity of preferably 80% or more, more preferably 85% or more, or further preferably 90% or more, 95% or more, or 98% or more, to the amino acid sequence of the original antibody. When a mutation is introduced to the variable region of an antibody, the mutation may be introduced to any of the CDRs and framework regions of the variable region, but is particularly introduced to a framework region.

In an embodiment of the present invention, an antibody obtained by introducing a mutation such as a substitution, a deletion, and an addition to the amino acid sequence of a framework region of the variable region of an original antibody is also included in antibodies. When an amino acid in the amino acid sequence of an original antibody is substituted with another amino acid, the number of amino acids to be substituted is preferably 1 to 10, more preferably 1 to 5, and further preferably 1 to 3. When an amino acid in the amino acid sequence of an original antibody is deleted, the number of amino acids to be deleted is preferably 1 to 10, more preferably 1 to 5, and further preferably 1 to 3. Also, a mutant obtained by introducing a mutation having the substitution and deletion of amino acids in combination is regarded as an antibody. When an amino acid is added, preferably 1 to 10, more preferably 1 to 5, or further preferably 1 to 3 amino acids are added in the amino acid sequence of an original antibody or added to the N terminal or C terminal thereof. A mutant obtained by introducing a mutation having the addition, substitution, and deletion of amino acids in combination is also regarded as an antibody. The amino acid sequence of an antibody having a mutation introduced thereto exhibits an identity of preferably 80% or more, more preferably 85% or more, or further preferably 90% or more, 95% or more, or 98% or more, to the amino acid sequence of the original antibody.

In an embodiment of the present invention, an antibody obtained by introducing a mutation such as a substitution, a deletion, and an addition to the amino acid sequence of a CDR region of the variable region of the original antibody is also included in antibodies. When an amino acid in the amino acid sequence of an original antibody is substituted with another amino acid, the number of amino acids to be substituted is preferably 1 to 5, more preferably 1 to 3, and further preferably 1 or 2. When an amino acid in the amino acid sequence of an original antibody is deleted, the number of amino acids to be deleted is preferably 1 to 5, more preferably 1 to 3, and further preferably 1 or 2. Also, a mutant obtained by introducing a mutation having the substitution and deletion of amino acids in combination is regarded as an antibody. When an amino acid is added, preferably 1 to 5, more preferably 1 to 3, or further preferably 1 or 2 amino acids are added in the amino acid sequence of an original antibody or added to the N terminal or C terminal thereof. A mutant obtained by introducing a mutation having the addition, substitution, and deletion of amino acids in combination is also regarded as an antibody. The amino acid sequence of an antibody having a mutation introduced thereto exhibits an identity of preferably 80% or more, more preferably 85% or more, or further preferably 90% or more, 95% or more, or 98% or more, to the amino acid sequence of the original antibody.

In an embodiment of the present invention, the term Fab refers to a molecule in which one light chain containing its variable region and C_{L} region (constant region of light chain) and one heavy chain containing its variable region and C_{H}1 region (part 1 of constant region of heavy chain) bind together via a disulfide bond between a cysteine residue present in one and that present in the other. The heavy chain in Fab may further contain part of a hinge part in addition to the variable region and C_{H}1 region (part 1 of constant region of heavy chain), and the hinge part in this case lacks cysteine residues that are present in the hinge part and bind the two heavy chains of an antibody together. The light chain and the heavy chain in Fab bind together via a disulfide bond formed between a cysteine residue present in the constant region (C_{L} region) of the light chain and a cysteine residue present in the constant region (C_{H}1 region) of the heavy chain or in a hinge part. The heavy chain forming Fab is referred to as a Fab heavy chain. Lacking cysteine residues that are present in the hinge part and bind the two heavy chains of an antibody together, Fab consists of one light chain and one heavy chain. The light chain constituting Fab contains the variable region and C_{L} region. The heavy chain constituting Fab may consist of the variable region and C_{H}1 region, and may contain part of a hinge part in addition to the variable region and C_{H}1 region. It should be noted that in this case the hinge part is selected in such a manner that any cysteine residue that binds two heavy chains is not contained in order not to allow the formation of a disulfide bond between two heavy chains at the hinge part. In F(ab'), the heavy chain contains the whole or part of a hinge part containing a cysteine residue for binding two heavy chains in addition to the variable region and C_{H}1 region. The term F(ab')₂ refers to a molecule in which two molecules of F(ab') bind together via a disulfide bond between a cysteine residue present in the hinge part of one and a cysteine residue present in the hinge part of the other. The heavy chain forming F(ab') or F(ab')₂ is referred to as a Fab' heavy chain. Also, a polymer in which a plurality of antibody molecules are bound directly or via a linker, such as a dimer and a trimer, are regarded as an antibody. Further, not only these but also molecules containing part of an antibody molecule and having a characteristic to specifically bind to an antigen are all included in "antibodies" defined in the present invention. Specifically, the term light chain includes a molecule derived from a light chain and having the amino acid sequence of the whole or part of the variable region. The term heavy chain includes a molecule derived from a heavy chain and having the amino acid sequence of the whole or part of the variable region. Accordingly, a product, for example, obtained by deleting the Fc region of a heavy chain is also regarded as a heavy chain as long as the product has the whole or part of the amino acid sequence of the variable region.

The term Fc or Fc region herein refers to a region containing a fragment consisting of the C_{H}2 region (part 2 of constant region of heavy chain) and C_{H}3 region (part 3 of constant region of heavy chain) of an antibody molecule.

Further included in antibodies in an embodiment of the present invention are:
(7) scFab, scF(ab'), and scF(ab')₂, each of which is a single chain antibody obtained by binding the light chain and heavy chain constituting Fab, F(ab'), or F(ab')₂ shown in (2) in the above via a linker. Herein, scFab, scF(ab'), and scF(ab')₂ may be ones obtained by binding a linker to the C terminal side of a light chain and further binding a heavy chain to the C terminal side thereof, or ones obtained by binding a linker to the C terminal side of a heavy chain and further binding a light chain to the C terminal side thereof. Further, scFv, which is a single chain antibody obtained by binding the variable region of a light chain and the variable region of a heavy chain together via a linker, is also included in antibodies. scFv may be one obtained by binding a linker to the C terminal side of the variable region of a light chain and further binding the variable region of a heavy chain to the C terminal side thereof, or one obtained by binding a linker to the C terminal side of the variable region of a heavy chain and further binding the variable region of a light chain to the C terminal side thereof.

In addition to full-length antibodies and those show in (1) to (7) in the above, antigen-binding fragments (antibody fragments) of any form, which are obtained by deleting part of a full-length antibody, the term showing a wider concept including (1) to (7), are included in "antibodies" defined in the specification. Antigen-binding fragments also include heavy chain antibodies, light chain antibodies, VHH, VNAR, and products obtained by deleting part of any of them.

The term "antigen-binding fragment" refers to a fragment of an antibody, the fragment retaining at least part of the specific binding activity to an antigen. Examples of the binding fragment include Fab, Fab', F(ab')₂, a variable region (Fv), a single chain antibody obtained by linking a heavy chain variable region (V_{H}) and a light chain variable region (V_{L}) via an appropriate linker (scFv), a diabody, which is a dimer of polypeptide containing a heavy chain variable region (V_{H}) and a light chain variable region (V_{L}), a minibody, which is a dimer of a molecule in which a part (C_{H}3) of the constant region is bound to the heavy chain (H chain) of scFv, and other fragmented antibodies. However, the antigen-binding fragment is not limited to these molecules as long as the antigen-binding fragment has binding ability to an antigen.

In an embodiment of the present invention, the term "single chain antibody" refers to a protein in which a linker is bound to the C terminal side of an amino acid sequence containing the whole or part of the variable region of a light chain, and further an amino acid sequence containing the whole or part of the variable region of a heavy chain is bound to the C terminal side thereof, and being capable of specifically binding to a specific antigen. Also, a protein in which a linker is bound to the C terminal side of an amino acid sequence containing the whole or part of the variable region of a heavy chain, and further an amino acid sequence containing the whole or part of the variable region of a light chain is bound to the C terminal side thereof, and being capable of specifically binding to a specific antigen is included in a "single chain antibody". In a single chain antibody in which a light chain is bound to the C terminal side of a heavy chain via a linker, the Fc region of the heavy chain has been normally deleted. The variable region of a light chain has three complementarity-determining regions (CDRs) involved in the antigen specificity of an antibody. Likewise, the variable region of a heavy chain has three CDRs. These CDRs are main regions that determine the antigen specificity of an antibody. Therefore, all of the three CDRs of a heavy chain and all of the three CDRs of a light chain are preferably contained in a single chain antibody. However, a single chain antibody with one or more CDRs deleted can be used as long as the antigen-specific affinity of the antibody is maintained.

In a single chain antibody, the linker disposed between the light chain and heavy chain of the antibody is a peptide chain constituted of preferably 2 to 50, more preferably 8 to 50, further preferably 10 to 30, further more preferably 12 to 18 or 15 to 25, for example, 15 or 25 amino acid residues. The amino acid sequence of the linker is not limited as long as an anti-hTfR antibody obtained by linking the two chains via the linker retains the affinity for hTfR, and preferably an amino acid sequence constituted of glycine alone or glycine and serine, for example, an amino acid sequence containing amino acid sequence Gly-Ser, amino acid sequence Gly-Gly-Ser, amino acid sequence Gly-Gly-Gly, amino acid sequence Gly-Gly-Gly-Gly-Ser (SEQ ID NO: 9), amino acid sequence Gly-Gly-Gly-Gly-Gly-Ser (SEQ ID NO: 10), amino acid sequence Ser-Gly-Gly-Gly-Gly (SEQ ID NO: 11), or an amino acid sequence of 2 to 10 or 2 to 5 repeats of any of these amino acid sequences. When the variable region of a light chain is bound to the C terminal side of the amino acid sequence consisting of the whole region of the variable region of a heavy chain via a linker, for example, a linker containing 15 amino acids in total corresponding to three consecutive repeats of amino acid sequence Gly-Gly-Gly-Gly-Ser (SEQ ID NO: 9) is preferable.

In an embodiment of the present invention, the term single domain antibody refers to an antibody having a characteristic to specifically bind to an antigen with a single variable region. Single domain antibodies include antibodies in which the variable region consists only of the variable region of a heavy chain (heavy chain single domain antibodies) and antibodies in which the variable region consists only of the variable region of a light chain (light chain single domain antibodies). VHH and VNAR are each one of single domain antibodies.

In an embodiment of the present invention, the term "human transferrin receptor" refers to a membrane protein having the amino acid sequence represented by SEQ ID NO: 22. The antibody of the present invention in an embodiment thereof is an antibody that specifically binds to a part ranging from the 89th cysteine residue as counted from the N terminal side to phenylalanine at the C terminal in the amino acid sequence represented by SEQ ID NO: 22 (extracellular region of transferrin receptor), but is not limited thereto.

Common as a method for preparing an antibody for a protein of interest is a method involving using a cell to which an expression vector integrating a gene encoding the protein is introduced to prepare a recombinant protein, and immunizing an animal such as a mouse with use of the recombinant protein to obtain an antibody. A hybridoma cell having an ability to produce an antibody for the recombinant protein can be prepared by taking out a cell producing an antibody for the recombinant protein from the immunized animal and fusing the cell with a myeloma cell.

Alternatively, a cell producing an antibody for a protein of interest can be obtained by immunizing an immune cell obtained from an animal such as a mouse by *in vitro* immunization with the protein. When *in vitro* immunization is used, the animal species from which the immune cell is derived is not particularly limited, preferably any of mice, rats, rabbits, guinea pigs, dogs, cats, horses, and primates including humans, more preferably any of mice, rats, and humans, and further preferably any of mice and humans. Examples of applicable mouse immune cells include splenocytes prepared from the spleen of a mouse. Examples of applicable human immune cells include cells prepared from the peripheral blood, bone marrow, or spleen of a human. Immunization of a human immune cell by *in vitro* immunization gives a human antibody for a recombinant protein.

After immunization of an immune cell by *in vitro* immunization, a hybridoma cell having an ability to produce an antibody can be prepared by fusing the cell with a myeloma cell. Alternatively, mRNA is extracted from an immunized cell to synthesize cDNA, a DNA fragment containing genes encoding the light chain and heavy chain of immunoglobulin is amplified through PCR reaction with the cDNA as a template, and the resultant can be used for artificially reconstructing an antibody gene.

The hybridoma cells as obtained by the above method also include cells producing an antibody that recognizes a non-target protein as an antigen. In addition, not all of the hybridoma cells producing an antibody for a protein of interest produce an antibody that exhibits desired properties such as high affinity for the protein.

Similarly, the artificially reconstructed antibody genes also include genes encoding an antibody that recognizes a non-target protein as an antigen. In addition, not all of the genes encoding an antibody for a protein of interest encode an antibody that exhibits desired properties such as high affinity for the protein.

Therefore, a step of selecting hybridoma cells producing an antibody having desired properties from the hybridoma cells as obtained in the above is needed. For the artificially reconstructed antibody genes, a step of selecting a gene encoding an antibody having desired properties from those antibody genes is needed. For example, a method described in detail below is effective as a method of selecting hybridoma cells producing an antibody that exhibits high affinity for a protein of interest (high-affinity antibody) or a gene encoding a high-affinity antibody.

When hybridoma cells producing an antibody having high affinity for a protein of interest are selected, for example, a method is used in which the protein is added to a plate and allowed to be held thereon, a culture supernatant for hybridoma cells is then added, subsequently antibodies not bound to the protein are removed from the plate, and the amount of an antibody held on the plate is measured. In this method, the higher the affinity of an antibody contained in a culture supernatant for hybridoma cells that has been added to a plate for the protein, the larger the amount of the antibody held on the plate. Accordingly, the amounts of antibodies held on plates are measured, and hybridoma cells corresponding to a plate on which a larger amount of an antibody is held can be selected as a cell strain producing an antibody having relatively high affinity for the protein. Furthermore, mRNA is extracted from the cell strain selected in this manner to synthesize cDNA, a DNA fragment containing a gene encoding an antibody for the protein is amplified by using a PCR method with the cDNA as a template, and as a result a gene encoding a high-affinity antibody can be isolated.

When a gene encoding an antibody having high affinity for a protein of interest is selected from the above artificially reconstructed antibody genes, the artificially reconstructed antibody genes are each temporarily integrated into an expression vector, and the expression vector is introduced into a host cell. Although a prokaryotic cell and an eukaryotic are both applicable as a cell to be used as a host cell in this case without any limitation, as long as the cell can be allowed to express an artificially reconstructed antibody gene by introducing an expression vector integrating the antibody gene, the cell is preferably a cell derived from a mammal such as a human, a mouse, and a Chinese hamster, and particularly preferably a CHO cell derived from the ovary of a Chinese hamster, or an NS/0 cell derived from mouse myeloma. Also, the expression vector to be used for expression by integrating a gene encoding an antibody gene is not particularly limited as long as it can express the gene when it is introduced in a mammalian cell. The gene integrated in an expression vector is arranged downstream of a DNA sequence (gene expression regulatory site) capable of regulating the frequency of transcription of the gene in a mammalian cell. In the present invention, examples of the gene expression regulatory site that can be used include a cytomegalovirus-derived promoter, an SV40 early promoter, a human elongation factor-1α (EF-1α) promoter, and a human ubiquitin C promoter.

A mammalian cell having such an expression vector introduced therein comes to express the above-described artificially reconstructed antibody integrated in the expression vector. When cells producing an antibody having high affinity for an antibody for a protein of interest are selected from the thus-obtained cells expressing artificially reconstructed antibodies, a method is used in which the protein is added to a plate and held thereon, a culture supernatant of cells is then brought into contact with the protein, subsequently antibodies not bound to the protein are removed from the plate, and the amount of an antibody held on the plate is measured. In this method, the higher the affinity of an antibody contained in a culture supernatant for cells for the protein, the larger the amount of the antibody held on a plate. Accordingly, the amounts of antibodies held on plates are measured, and cells corresponding to a plate on which a larger amount of an antibody is held can be selected as a cell strain producing an antibody having relatively high affinity for the protein, and eventually a gene encoding an antibody having high affinity for the protein can be selected. Furthermore, a DNA fragment containing a gene encoding an antibody for the protein is obtained from the thus-selected cell strain and amplified by using a PCR method, and as a result a gene encoding a high-affinity antibody can be isolated.

Examples of methods for producing a conjugate in which a fusion protein of SA and a lysosome enzyme, a fusion protein of SA and a cytokine, or a fusion protein of SA and a neurotrophic factor is bound to an antibody in an embodiment of the present invention include a method of binding a fusion protein to an antibody via a non-peptide linker or a peptide linker. For the non-peptide linker, any of polyethylene glycol, polypropylene glycol, copolymer of ethylene glycol and propylene glycol, polyoxyethylated polyol, polyvinyl alcohol, polysaccharides, dextran, polyvinyl ether, biodegradable polymers, lipid polymers, chitins, and hyaluronic acid, or a derivative of any of them, or a combination of any of them can be used. The peptide linker is a peptide chain constituted of 1 to 50 amino acids bound by peptide bonds or a derivative thereof, and the N terminal and the C terminal of the linker each form a covalent bond with either a fusion protein or an antibody to bind the fusion protein and the antibody. The SA, lysosome enzyme, cytokine, and neurotrophic factor in this case may be each derived from a human, or derived from a non-human animal species.

The conjugate of a fusion protein with an antibody is produced in such a manner that a fusion protein and an antibody are separately produced, and they are then reacted with a non-peptide linker or a peptide linker to allow the fusion protein to bind to one end of the linker and the antibody to bind to the other end. For each of the fusion protein and the antibody, a fusion protein or antibody produced as a recombinant protein can be used. With this production method, for example, a conjugate in which a fusion protein of HSA and a human lysosome enzyme and an antibody are bound together via a non-peptide linker or a peptide linker, a conjugate in which a fusion protein of HSA and a human cytokine and an antibody are bound together via a non-peptide linker or a peptide linker, and a conjugate in which a fusion protein of HSA and a human neurotrophic factor and an antibody are bound together via a non-peptide linker or a peptide linker are produced.

With the production method, for example, a conjugate in which a fusion protein of HSA and hGALC, a fusion protein of HSA and hGBA, a fusion protein of HSA and hIL-10, a fusion protein of HSA and hBDNF, a fusion protein of HSA and hNGF, a fusion protein of HSA and hNT-3, or a fusion protein of HSA and hNT-4 is bound to an antibody or a ligand via a non-peptide linker or a peptide linker can be obtained.

Examples of methods for producing a conjugate in which a fusion protein of SA and a lysosome enzyme, a fusion protein of SA and a cytokine, or a fusion protein of SA and a neurotrophic factor is bound to an antibody or a ligand in an embodiment of the present invention include a method of producing a conjugate by binding the C terminal or N terminal of such a fusion protein to the N terminal or C terminal, respectively of an antibody or a ligand, via a linker or directly, by a peptide bond. The conjugate can be produced as a recombinant protein by a production method shown as an example in the following.

For example, an antibody and a fusion protein of HSA and hGALC can be produced as a conjugate by binding the N terminal or C terminal of the fusion protein to the C terminal side or N terminal side, respectively of a heavy chain or light chain of the antibody, via a linker or directly, by a peptide bond. The conjugate in which an antibody and a fusion protein of HSA and hGALC are bound as described can be obtained as a conjugate protein in such a manner that a DNA fragment in which cDNA encoding a fusion protein of HSA and hGALC is disposed in flame on the 3' terminal side or 5' terminal side of cDNA encoding a heavy chain or light chain of an antibody, directly or with a DNA fragment encoding a linker intervening therebetween, is integrated in an expression vector for mammalian cells, and a mammalian cell having the expression vector introduced therein is cultured. For the mammalian cell, in a case where a DNA fragment encoding a fusion protein of HSA and hGALC is bound to a heavy chain, an expression vector for mammalian cells having a cDNA fragment encoding a light chain of the antibody integrated therein can also be introduced in the same host cell; in a case where a DNA fragment encoding a fusion protein of HSA and hGALC is bound to a light chain, an expression vector for mammalian cells having a cDNA fragment encoding a heavy chain of the antibody integrated therein can also be introduced in the same host cell. In a case where the antibody is a single chain antibody or VHH, a conjugate in which the antibody and a fusion protein of HSA and hGALC are bound can be obtained as a recombinant protein in such a manner that a DNA fragment in which cDNA encoding a single chain antibody or VHH is linked to the 5' terminal side or 3' terminal side of cDNA encoding a fusion protein of HSA and hGALC, directly or with a DNA fragment encoding a linker intervening therebetween, is integrated in an expression vector (for mammalian cells, eukaryotes such as yeasts, or prokaryotic cells such as *Escherichia coli*), and an appropriate cell having the expression vector introduced therein is allowed to express therein. Also, a conjugate of a fusion protein of HSA and hGBA with an antibody, a conjugate of a fusion protein of HSA and hIL-10 with an antibody, a conjugate of a fusion protein of HSA and hBDNF with an antibody, a conjugate of a fusion protein of HSA and hNGF with an antibody, a conjugate of a fusion protein of HSA and hNT-3 with an antibody, and a conjugate of a fusion protein of HSA and hNT-4 with an antibody can be each obtained as a recombinant protein in the same manner as for a conjugate of a fusion protein of HSA and hGALC with an antibody.

An antibody, HSA, and hGALC can be formed even as a conjugate having the antibody intervening between HSA and hGALC. This conjugate can be produced by binding the N terminal of a heavy chain or light chain of an antibody to the C terminal of HSA, via a linker or directly, and further binding hGALC to the C terminal thereof, via a linker or directly, each by a peptide bond. Alternatively, the conjugate can be produced by binding the N terminal of a heavy chain or light chain of an antibody to the C terminal of hGALC, via a linker or directly, and further binding HSA to the C terminal thereof, via a linker or directly, each by a peptide bond. Also in a case where the antibody is a single chain antibody or VHH, the antibody, HSA, and hGALC can be formed as a fusion protein having the antibody intervening between HSA and hGALC. This conjugate can be produced by binding the N terminal of a single chain antibody or VHH to the C terminal of HSA, via a linker or directly, and further binding hGALC to the C terminal thereof, via a linker or directly, each by a peptide bond. Alternatively, the conjugate can be produced by binding the N terminal of a single chain antibody or VHH to the C terminal of hGALC, via a linker or directly, and further binding HSA to the C terminal thereof, via a linker or directly, each by a peptide bond. Such a conjugate can be produced for a lysosome enzyme other than hGALC such as hGBA, also for a cytokine such as hIL-10, and also for a neurotrophic factor such as hBDNF, hNGF, hNT-3, and hNT-4. For convenience, such conjugates having an intervening antibody are also referred to as a fusion protein of an antibody, another human lysosome enzyme, and HSA, a fusion protein of an antibody, another human cytokine, and HSA, and a fusion protein of an antibody, another human neurotrophic factor, and HSA.

An antibody, HSA, and hGALC can be formed even as a conjugate having the antibody intervening between HSA and hGALC. This conjugate can be produced by binding the N terminal of a heavy chain or light chain of an antibody to the C terminal of HSA, via a linker or directly, and further binding hGALC to the C terminal thereof, via a linker or directly, each by a peptide bond. Alternatively, the conjugate can be produced by binding the N terminal of a heavy chain or light chain of an antibody to the C terminal of hGALC, via a linker or directly, and further binding HSA to the C terminal thereof, via a linker or directly, each by a peptide bond. Also in a case where the antibody is a single chain antibody or VHH, the antibody, HSA, and hGALC can be formed as a fusion protein having the antibody intervening between HSA and hGALC. This conjugate can be produced by binding the N terminal of a single chain antibody or VHH to the C terminal of HSA, via a linker or directly, and further binding hGALC to the C terminal thereof, via a linker or directly, each by a peptide bond. Alternatively, the conjugate can be produced by binding the N terminal of a single chain antibody or VHH to the C terminal of hGALC, via a linker or directly, and further binding HSA to the C terminal thereof, via a linker or directly, each by a peptide bond. Such a conjugate can be produced for a lysosome enzyme other than hGALC such as hGBA, also for a cytokine such as hIL-10, and also for a neurotrophic factor such as hBDNF, hNGF, hNT-3, and hNT-4. For convenience, such conjugates having an intervening antibody are also referred to as a fusion protein of an antibody, another human lysosome enzyme, and HSA, a fusion protein of an antibody, another human cytokine, and HSA, and a fusion protein of an antibody, another human neurotrophic factor, and HSA.

The above method can be applied to production of a conjugate of a fusion protein of another human lysosome enzyme and HSA, a fusion protein of another human cytokine and HSA, or a fusion protein of another human neurotrophic factor and HSA, with an antibody. Also, the above method can be applied to production of a conjugate in which SA is derived from a non-human animal species and a conjugate in which a lysosome enzyme, cytokine, or neurotrophic factor is derived from a non-human animal species.

The expression vector, host cell, medium and others that can be used for producing the fusion protein of HSA and hGALC or fusion protein of HSA and hGBA mentioned above can be used also in production of any of the conjugates of a fusion protein with an antibody mentioned above.

Examples of preferable embodiments of the conjugate of an antibody, HSA, and hGALC include the following (1) to (10):
(1) a molecule containing: a conjugate in which a fusion protein of HSA and hGALC is bound to the C terminal of a heavy chain of an antibody directly or via a linker; and a light chain of an antibody;
(2) a molecule containing: a conjugate in which a fusion protein of HSA and hGALC is bound to the N terminal of a heavy chain of an antibody directly or via a linker; and a light chain of an antibody;
(3) a molecule containing: a conjugate in which a fusion protein of HSA and hGALC is bound to the C terminal of a light chain of an antibody directly or via a linker; and a heavy chain of an antibody;
(4) a molecule containing: a conjugate in which a fusion protein of HSA and hGALC is bound to the N terminal of a light chain of an antibody directly or via a linker; and a heavy chain of an antibody;
(5) a conjugate in which a single chain antibody or VHH is bound to the C terminal of HSA directly or via a linker, and hGALC is further bound to the C terminal thereof;
(6) a conjugate in which a single chain antibody or VHH is bound to the C terminal of hGALC, directly or via a linker, and HSA is further bound to the C terminal thereof;
(7) a conjugate in which HSA is bound to the C terminal of hGALC directly or via a linker, and a single chain antibody or VHH is further bound to the C terminal thereof;
(8) a conjugate in which hGALC is bound to the C terminal of HSA directly or via a linker, and a single chain antibody or VHH is further bound to the C terminal thereof;
(9) a conjugate in which HSA is bound to the C terminal of a single chain antibody or VHH directly or via a linker, and hGALC is further bound to the C terminal thereof; and
(10) a conjugate in which hGALC is bound to the C terminal of a single chain antibody or VHH directly or via a linker, and HSA is further bound to the C terminal thereof.

A preferable example of (1) is a molecule containing: conjugate A in which a linker having an amino acid sequence of three repeats of SEQ ID NO: 9 is bound to the C terminal of a Fab heavy chain having the amino acid sequence of SEQ ID NO: 25, HSA having the amino acid sequence of SEQ ID NO: 3 is further bound to the C terminal thereof, a linker having the amino acid sequence of SEQ ID NO: 9 is further bound to the C terminal thereof, and hGALC having the amino acid sequence of SEQ ID NO: 1 is further bound to the C terminal thereof; and a light chain of SEQ ID NO: 23. Here, conjugate A has the amino acid sequence of SEQ ID NO: 28.

A preferable example of (2) is a molecule containing: conjugate B in which a linker having the amino acid sequence of SEQ ID NO: 9 is bound to the C terminal of HSA having the amino acid sequence of SEQ ID NO: 3, hGALC having the amino acid sequence of SEQ ID NO: 1 is further bound to the C terminal thereof, a linker having an amino acid sequence of three repeats of SEQ ID NO: 9 is further bound to the C terminal thereof, and a Fab heavy chain having the amino acid sequence of SEQ ID NO: 25 is further bound to the C terminal thereof; and a light chain of SEQ ID NO: 23. Here, conjugate B has the amino acid sequence of SEQ ID NO: 30.

A preferable example of (3) is a molecule containing: conjugate C in which a linker having an amino acid sequence of three repeats of SEQ ID NO: 9 is bound to the C terminal of a Fab heavy chain having the amino acid sequence of SEQ ID NO: 25, hGALC having the amino acid sequence of SEQ ID NO: 1 is further bound to the C terminal thereof, a linker having the amino acid sequence of SEQ ID NO: 9 is further bound to the C terminal thereof, and HSA having the amino acid sequence of SEQ ID NO: 3 is further bound to the C terminal thereof; and a light chain of SEQ ID NO: 23. Here, conjugate C has the amino acid sequence of SEQ ID NO: 32.

A preferable example of (4) is a molecule containing: conjugate D in which a linker having the amino acid sequence of SEQ ID NO: 9 is bound to the C terminal of hGALC having the amino acid sequence of SEQ ID NO: 1, HSA having the amino acid sequence of SEQ ID NO: 3 is further bound to the C terminal thereof, a linker having an amino acid sequence of three repeats of SEQ ID NO: 9 is further bound to the C terminal thereof, and a Fab heavy chain having the amino acid sequence of SEQ ID NO: 25 is further bound to the C terminal thereof; and a light chain of SEQ ID NO: 23. Here, conjugate C has the amino acid sequence of SEQ ID NO: 34.

Examples of preferable embodiments of the conjugate of an antibody, HSA, and hGBA include the following (1) to (10):
(1) a molecule containing: a conjugate in which a fusion protein of HSA and hGBA is bound to the C terminal of a heavy chain of an antibody directly or via a linker; and a light chain of an antibody;
(2) a molecule containing: a conjugate in which a fusion protein of HSA and hGBA is bound to the N terminal of a heavy chain of an antibody directly or via a linker; and a light chain of an antibody;
(3) a molecule containing: a conjugate in which a fusion protein of HSA and hGBA is bound to the C terminal of a light chain of an antibody directly or via a linker; and a heavy chain of an antibody;
(4) a molecule containing: a conjugate in which a fusion protein of HSA and hGBA is bound to the N terminal of a light chain of an antibody directly or via a linker; and a heavy chain of an antibody;
(5) a conjugate in which a single chain antibody or VHH is bound to the C terminal of HSA directly or via a linker, and hGBA is further bound to the C terminal thereof;
(6) a conjugate in which a single chain antibody or VHH is bound to the C terminal of hGBA directly or via a linker, and HSA is further bound to the C terminal thereof;
(7) a conjugate in which HSA is bound to the C terminal of hGBA directly or via a linker, and a single chain antibody or VHH is further bound to the C terminal thereof;
(8) a conjugate in which hGBA is bound to the C terminal of HSA directly or via a linker, and a single chain antibody or VHH is further bound to the C terminal thereof;
(9) a conjugate in which HSA is bound to the C terminal of a single chain antibody or VHH directly or via a linker, and hGBA is further bound to the C terminal thereof; and
(10) a conjugate in which hGBA is bound to the C terminal of a single chain antibody or VHH directly or via a linker, and HSA is further bound to the C terminal thereof.

Examples of preferable embodiments of the conjugate of an antibody, HSA, and hIL-10 include the following (1) to (10):
(1) a molecule containing: a conjugate in which a fusion protein of HSA and hIL-10 is bound to the C terminal of a heavy chain of an antibody directly or via a linker; and a light chain of an antibody;
(2) a molecule containing: a conjugate in which a fusion protein of HSA and hIL-10 is bound to the N terminal of a heavy chain of an antibody directly or via a linker; and a light chain of an antibody;
(3) a molecule containing: a conjugate in which a fusion protein of HSA and hIL-10 is bound to the C terminal of a light chain of an antibody directly or via a linker; and a heavy chain of an antibody;
(4) a molecule containing: a conjugate in which a fusion protein of HSA and hIL-10 is bound to the N terminal of a light chain of an antibody directly or via a linker; and a heavy chain of an antibody;
(5) a conjugate in which a single chain antibody or VHH is bound to the C terminal of HSA directly or via a linker, and hIL-10 is further bound to the C terminal thereof;
(6) a conjugate in which a single chain antibody or VHH is bound to the C terminal of hIL-10 directly or via a linker, and HSA is further bound to the C terminal thereof;
(7) a conjugate in which HSA is bound to the C terminal of hIL-10 directly or via a linker, and a single chain antibody or VHH is further bound to the C terminal thereof;
(8) a conjugate in which hIL-10 is bound to the C terminal of HSA directly or via a linker, and a single chain antibody or VHH is further bound to the C terminal thereof;
(9) a conjugate in which HSA is bound to the C terminal of a single chain antibody or VHH directly or via a linker, and hIL-10 is further bound to the C terminal thereof; and
(10) a conjugate in which hIL-10 is bound to the C terminal of a single chain antibody or VHH directly or via a linker, and HSA is further bound to the C terminal thereof.

Examples of preferable embodiments of the conjugate of an antibody, HSA, and hBDNF include the following (1) to (10):
(1) a molecule containing: a conjugate in which a fusion protein of HSA and hBDNF is bound to the C terminal of a heavy chain of an antibody directly or via a linker; and a light chain of an antibody;
(2) a molecule containing: a conjugate in which a fusion protein of HSA and hBDNF is bound to the N terminal of a heavy chain of an antibody directly or via a linker; and a light chain of an antibody;
(3) a molecule containing: a conjugate in which a fusion protein of HSA and hBDNF is bound to the C terminal of a light chain of an antibody directly or via a linker; and a heavy chain of an antibody;
(4) a molecule containing: a conjugate in which a fusion protein of HSA and hBDNF is bound to the N terminal of a light chain of an antibody directly or via a linker; and a heavy chain of an antibody;
(5) a conjugate in which a single chain antibody or VHH is bound to the C terminal of HSA directly or via a linker, and hBDNF is further bound to the C terminal thereof;
(6) a conjugate in which a single chain antibody or VHH is bound to the C terminal of hBDNF directly or via a linker, and HSA is further bound to the C terminal thereof;
(7) a conjugate in which HSA is bound to the C terminal of hBDNF directly or via a linker, and a single chain antibody or VHH is further bound to the C terminal thereof;
(8) a conjugate in which hBDNF is bound to the C terminal of HSA directly or via a linker, and a single chain antibody or VHH is further bound to the C terminal thereof;
(9) a conjugate in which HSA is bound to the C terminal of a single chain antibody or VHH, directly or via a linker, and hBDNF is further bound to the C terminal thereof; and
(10) a conjugate in which hBDNF is bound to the C terminal of a single chain antibody or VHH directly or via a linker, and HSA is further bound to the C terminal thereof.

A preferable example of (6) is a conjugate having the amino acid sequence of SEQ ID NO: 192 in which a GS linker is bound to the C terminal of a wild-type hBDNF, HSA having the amino acid sequence of SEQ ID NO: 3 is further bound to the C terminal thereof, a linker having the amino acid sequence of SEQ ID NO: 196 is further bound to the C terminal thereof, and VHH having the amino acid sequence of SEQ ID NO: 186 is further bound to the C terminal thereof. A preferable example of (7) is a conjugate having the amino acid sequence of SEQ ID NO: 193 in which a linker having the amino acid sequence of SEQ ID NO: 196 is bound to the C terminal of a wild-type hBDNF, VHH having the amino acid sequence of SEQ ID NO: 186 is further bound to the C terminal thereof, a GS linker is further bound to the C terminal thereof, and HSA having the amino acid sequence of SEQ ID NO: 3 is further bound to the C terminal thereof.

Examples of preferable embodiments of the conjugate of an antibody, HSA, and hNGF include the following (1) to (10):
(1) a molecule containing: a conjugate in which a fusion protein of HSA and hNGF is bound to the C terminal of a heavy chain of an antibody directly or via a linker; and a light chain of an antibody;
(2) a molecule containing: a conjugate in which a fusion protein of HSA and hNGF is bound to the N terminal of a heavy chain of an antibody directly or via a linker; and a light chain of an antibody;
(3) a molecule containing: a conjugate in which a fusion protein of HSA and hNGF is bound to the C terminal of a light chain of an antibody directly or via a linker; and a heavy chain of an antibody;
(4) a molecule containing: a conjugate in which a fusion protein of HSA and hNGF is bound to the N terminal of a light chain of an antibody directly or via a linker; and a heavy chain of an antibody;
(5) a conjugate in which a single chain antibody or VHH is bound to the C terminal of HSA directly or via a linker, and hNGF is further bound to the C terminal thereof;
(6) a conjugate in which a single chain antibody or VHH is bound to the C terminal of hNGF directly or via a linker, and HSA is further bound to the C terminal thereof;
(7) a conjugate in which HSA is bound to the C terminal of hNGF directly or via a linker, and a single chain antibody or VHH is further bound to the C terminal thereof;
(8) a conjugate in which hNGF is bound to the C terminal of HSA directly or via a linker, and a single chain antibody or VHH is further bound to the C terminal thereof;
(9) a conjugate in which HSA is bound to the C terminal of a single chain antibody or VHH directly or via a linker, and hNGF is further bound to the C terminal thereof; and
(10) a conjugate in which hNGF is bound to the C terminal of a single chain antibody or VHH directly or via a linker, and HSA is further bound to the C terminal thereof.

Examples of preferable embodiments of the conjugate of an antibody, HSA, and hNT-3 include the following (1) to (10):
(1) a molecule containing: a conjugate in which a fusion protein of HSA and hNT-3 is bound to the C terminal of a heavy chain of an antibody directly or via a linker; and a light chain of an antibody;
(2) a molecule containing: a conjugate in which a fusion protein of HSA and hNT-3 is bound to the N terminal of a heavy chain of an antibody directly or via a linker; and a light chain of an antibody;
(3) a molecule containing: a conjugate in which a fusion protein of HSA and hNT-3 is bound to the C terminal of a light chain of an antibody directly or via a linker; and a heavy chain of an antibody;
(4) a molecule containing: a conjugate in which a fusion protein of HSA and hNT-3 is bound to the N terminal of a light chain of an antibody directly or via a linker; and a heavy chain of an antibody;
(5) a conjugate in which a single chain antibody or VHH is bound to the C terminal of HSA directly or via a linker, and hNT-3 is further bound to the C terminal thereof;
(6) a conjugate in which a single chain antibody or VHH is bound to the C terminal of hNT-3 directly or via a linker, and HSA is further bound to the C terminal thereof;
(7) a conjugate in which HSA is bound to the C terminal of hNT-3 directly or via a linker, and a single chain antibody or VHH is further bound to the C terminal thereof;
(8) a conjugate in which hNT-3 is bound to the C terminal of HSA directly or via a linker, and a single chain antibody or VHH is further bound to the C terminal thereof;
(9) a conjugate in which HSA is bound to the C terminal of a single chain antibody or VHH directly or via a linker, and hNT-3 is further bound to the C terminal thereof; and
(10) a conjugate in which hNT-3 is bound to the C terminal of a single chain antibody or VHH directly or via a linker, and HSA is further bound to the C terminal thereof.

Examples of preferable embodiments of the conjugate of an antibody, HSA, and hNT-4 include the following (1) to (10):
(1) a molecule containing: a conjugate in which a fusion protein of HSA and hNT-4 is bound to the C terminal of a heavy chain of an antibody directly or via a linker; and a light chain of an antibody;
(2) a molecule containing: a conjugate in which a fusion protein of HSA and hNT-4 is bound to the N terminal of a heavy chain of an antibody directly or via a linker; and a light chain of an antibody;
(3) a molecule containing: a conjugate in which a fusion protein of HSA and hNT-4 is bound to the C terminal of a light chain of an antibody directly or via a linker; and a heavy chain of an antibody;
(4) a molecule containing: a conjugate in which a fusion protein of HSA and hNT-4 is bound to the N terminal of a light chain of an antibody directly or via a linker; and a heavy chain of an antibody;
(5) a conjugate in which a single chain antibody or VHH is bound to the C terminal of HSA directly or via a linker, and hNT-4 is further bound to the C terminal thereof;
(6) a conjugate in which a single chain antibody or VHH is bound to the C terminal of hNT-4 directly or via a linker, and HSA is further bound to the C terminal thereof;
(7) a conjugate in which HSA is bound to the C terminal of hNT-4 directly or via a linker, and a single chain antibody or VHH is further bound to the C terminal thereof;
(8) a conjugate in which hNT-4 is bound to the C terminal of HSA directly or via a linker, and a single chain antibody or VHH is further bound to the C terminal thereof;
(9) a conjugate in which HSA is bound to the C terminal of a single chain antibody or VHH directly or via a linker, and hNT-4 is further bound to the C terminal thereof; and
(10) a conjugate in which hNT-4 is bound to the C terminal of a single chain antibody or VHH directly or via a linker, and HSA is further bound to the C terminal thereof.

Examples of preferable embodiments of the conjugate of an antibody, HSA, and hCDNF include the following (1) to (10):
(1) a molecule containing: a conjugate in which a fusion protein of HSA and hCDNF is bound to the C terminal of a heavy chain of an antibody directly or via a linker; and a light chain of an antibody;
(2) a molecule containing: a conjugate in which a fusion protein of HSA and hCDNF is bound to the N terminal of a heavy chain of an antibody directly or via a linker; and a light chain of an antibody;
(3) a molecule containing: a conjugate in which a fusion protein of HSA and hCDNF is bound to the C terminal of a light chain of an antibody directly or via a linker; and a heavy chain of an antibody;
(4) a molecule containing: a conjugate in which a fusion protein of HSA and hCDNF is bound to the N terminal of a light chain of an antibody directly or via a linker; and a heavy chain of an antibody;
(5) a conjugate in which a single chain antibody or VHH is bound to the C terminal of HSA directly or via a linker, and hCDNF is further bound to the C terminal thereof;
(6) a conjugate in which a single chain antibody or VHH is bound to the C terminal of hCDNF directly or via a linker, and HSA is further bound to the C terminal thereof;
(7) a conjugate in which HSA is bound to the C terminal of hCDNF directly or via a linker, and a single chain antibody or VHH is further bound to the C terminal thereof;
(8) a conjugate in which hCDNF is bound to the C terminal of HSA directly or via a linker, and a single chain antibody or VHH is further bound to the C terminal thereof;
(9) a conjugate in which HSA is bound to the C terminal of a single chain antibody or VHH directly or via a linker, and hCDNF is further bound to the C terminal thereof; and
(10) a conjugate in which hCDNF is bound to the C terminal of a single chain antibody or VHH directly or via a linker, and HSA is further bound to the C terminal thereof.

A preferable example of (6) is conjugate in which a GS linker is bound to the C terminal of a wild-type hCDNF, HSA having the amino acid sequence of SEQ ID NO: 3 is further bound to the C terminal thereof, a linker having the amino acid sequence of SEQ ID NO: 196 is further bound to the C terminal thereof, and VHH having the amino acid sequence of SEQ ID NO: 186 is further bound to the C terminal thereof. A preferable example of (7) is a conjugate in which a linker having the amino acid sequence of SEQ ID NO: 196 is bound to the C terminal of a wild-type hCDNF, VHH having the amino acid sequence of SEQ ID NO: 186 is further bound to the C terminal thereof, a GS linker is further bound to the C terminal thereof, and HSA having the amino acid sequence of SEQ ID NO: 3 is further bound to the C terminal thereof.

Examples of preferable embodiments of the conjugate of an antibody, HSA, and hGDNF include the following (1) to (10):
(1) a molecule containing: a conjugate in which a fusion protein of HSA and hGDNF is bound to the C terminal of a heavy chain of an antibody directly or via a linker; and a light chain of an antibody;
(2) a molecule containing: a conjugate in which a fusion protein of HSA and hGDNF is bound to the N terminal of a heavy chain of an antibody directly or via a linker; and a light chain of an antibody;
(3) a molecule containing: a conjugate in which a fusion protein of HSA and hGDNF is bound to the C terminal of a light chain of an antibody directly or via a linker; and a heavy chain of an antibody;
(4) a molecule containing: a conjugate in which a fusion protein of HSA and hGDNF is bound to the N terminal of a light chain of an antibody directly or via a linker; and a heavy chain of an antibody;
(5) a conjugate in which a single chain antibody or VHH is bound to the C terminal of HSA directly or via a linker, and hGDNF is further bound to the C terminal thereof;
(6) a conjugate in which a single chain antibody or VHH is bound to the C terminal of hGDNF directly or via a linker, and HSA is further bound to the C terminal thereof;
(7) a conjugate in which HSA is bound to the C terminal of hGDNF directly or via a linker, and a single chain antibody or VHH is further bound to the C terminal thereof;
(8) a conjugate in which hGDNF is bound to the C terminal of HSA directly or via a linker, and a single chain antibody or VHH is further bound to the C terminal thereof;
(9) a conjugate in which HSA is bound to the C terminal of a single chain antibody or VHH directly or via a linker, and hGDNF is further bound to the C terminal thereof; and
(10) a conjugate in which hGDNF is bound to the C terminal of a single chain antibody or VHH directly or via a linker, and HSA is further bound to the C terminal thereof.

A preferable example of (6) is a conjugate in which a GS linker is bound to the C terminal of wild-type hGDNF, HSA having the amino acid sequence of SEQ ID NO: 3 is further bound to the C terminal thereof, a linker having the amino acid sequence of SEQ ID NO: 196 is further bound to the C terminal thereof, and VHH having the amino acid sequence of SEQ ID NO: 186 is further bound to the C terminal thereof. A preferable example of (7) is a conjugate in which a linker having the amino acid sequence of SEQ ID NO: 196 is bound to the C terminal of wild-type hGDNF, VHH having the amino acid sequence of SEQ ID NO: 186 is further bound to the C terminal thereof, a GS linker is further bound to the C terminal thereof, and HSA having the amino acid sequence of SEQ ID NO: 3 is further bound to the C terminal thereof.

Examples of preferable embodiments of the conjugate of an antibody, HSA, and hNRTN include the following (1) to (10):
(1) a molecule containing: a conjugate in which a fusion protein of HSA and hNRTN is bound to the C terminal of a heavy chain of an antibody directly or via a linker; and a light chain of an antibody;
(2) a molecule containing: a conjugate in which a fusion protein of HSA and hNRTN is bound to the N terminal of a heavy chain of an antibody directly or via a linker; and a light chain of an antibody;
(3) a molecule containing: a conjugate in which a fusion protein of HSA and hNRTN is bound to the C terminal of a light chain of an antibody directly or via a linker; and a heavy chain of an antibody;
(4) a molecule containing: a conjugate in which a fusion protein of HSA and hNRTN is bound to the N terminal of a light chain of an antibody directly or via a linker; and a heavy chain of an antibody;
(5) a conjugate in which a single chain antibody or VHH is bound to the C terminal of HSA directly or via a linker, and hNRTN is further bound to the C terminal thereof;
(6) a conjugate in which a single chain antibody or VHH is bound to the C terminal of hNRTN directly or via a linker, and HSA is further bound to the C terminal thereof;
(7) a conjugate in which HSA is bound to the C terminal of hNRTN directly or via a linker, and a single chain antibody or VHH is further bound to the C terminal thereof;
(8) a conjugate in which hNRTN is bound to the C terminal of HSA directly or via a linker, and a single chain antibody or VHH is further bound to the C terminal thereof;
(9) a conjugate in which HSA is bound to the C terminal of a single chain antibody or VHH directly or via a linker, and hNRTN is further bound to the C terminal thereof; and
(10) a conjugate in which hNRTN is bound to the C terminal of a single chain antibody or VHH directly or via a linker, and HSA is further bound to the C terminal thereof.

A preferable example of (6) is a conjugate in which a GS linker is bound to the C terminal of a wild-type hNRTN, HSA having the amino acid sequence of SEQ ID NO: 3 is further bound to the C terminal thereof, a linker having the amino acid sequence of SEQ ID NO: 196 is further bound to the C terminal thereof, and VHH having the amino acid sequence of SEQ ID NO: 186 is further bound to the C terminal thereof. A preferable example of (7) is a conjugate in which a linker having the amino acid sequence of SEQ ID NO: 196 is bound to the C terminal of a wild-type hNRTN, VHH having the amino acid sequence of SEQ ID NO: 186 is further bound to the C terminal thereof, a GS linker is further bound to the C terminal thereof, and HSA having the amino acid sequence of SEQ ID NO: 3 is further bound to the C terminal thereof.

Examples of preferable embodiments of the conjugate of an antibody, HSA, and hARTN include the following (1) to (10):
(1) a molecule containing: a conjugate in which a fusion protein of HSA and hARTN is bound to the C terminal of a heavy chain of an antibody directly or via a linker; and a light chain of an antibody;
(2) a molecule containing: a conjugate in which a fusion protein of HSA and hARTN is bound to the N terminal of a heavy chain of an antibody directly or via a linker; and a light chain of an antibody;
(3) a molecule containing: a conjugate in which a fusion protein of HSA and hARTN is bound to the C terminal of a light chain of an antibody directly or via a linker; and a heavy chain of an antibody;
(4) a molecule containing: a conjugate in which a fusion protein of HSA and hARTN is bound to the N terminal of a light chain of an antibody directly or via a linker; and a heavy chain of an antibody;
(5) a conjugate in which a single chain antibody or VHH is bound to the C terminal of HSA directly or via a linker, and hARTN is further bound to the C terminal thereof;
(6) a conjugate in which a single chain antibody or VHH is bound to the C terminal of hARTN directly or via a linker, and HSA is further bound to the C terminal thereof;
(7) a conjugate in which HSA is bound to the C terminal of hARTN directly or via a linker, and a single chain antibody or VHH is further bound to the C terminal thereof;
(8) a conjugate in which hARTN is bound to the C terminal of HSA directly or via a linker, and a single chain antibody or VHH is further bound to the C terminal thereof;
(9) a conjugate in which HSA is bound to the C terminal of a single chain antibody or VHH directly or via a linker, and hARTN is further bound to the C terminal thereof; and
(10) a conjugate in which hARTN is bound to the C terminal of a single chain antibody or VHH directly or via a linker, and HSA is further bound to the C terminal thereof.

A preferable example of (6) is a conjugate in which a GS linker is bound to the C terminal of a wild-type hARTN, HSA having the amino acid sequence of SEQ ID NO: 3 is further bound to the C terminal thereof, a linker having the amino acid sequence of SEQ ID NO: 196 is further bound to the C terminal thereof, and VHH having the amino acid sequence of SEQ ID NO: 186 is further bound to the C terminal thereof. A preferable example of (7) is a conjugate in which a linker having the amino acid sequence of SEQ ID NO: 196 is bound to the C terminal of a wild-type hARTN, VHH having the amino acid sequence of SEQ ID NO: 186 is further bound to the C terminal thereof, and HSA having the amino acid sequence of SEQ ID NO: 3 is further bound to the C terminal thereof.

Examples of preferable embodiments of the conjugate of an antibody, HSA, and hPSPN include the following (1) to (10):
(1) a molecule containing: a conjugate in which a fusion protein of HSA and hPSPN is bound to the C terminal of a heavy chain of an antibody directly or via a linker; and a light chain of an antibody;
(2) a molecule containing: a conjugate in which a fusion protein of HSA and hPSPN is bound to the N terminal of a heavy chain of an antibody directly or via a linker; and a light chain of an antibody;
(3) a molecule containing: a conjugate in which a fusion protein of HSA and hPSPN is bound to the C terminal of a light chain of an antibody directly or via a linker; and a heavy chain of an antibody;
(4) a molecule containing: a conjugate in which a fusion protein of HSA and hPSPN is bound to the N terminal of a light chain of an antibody directly or via a linker; and a heavy chain of an antibody;
(5) a conjugate in which a single chain antibody or VHH is bound to the C terminal of HSA directly or via a linker, and hPSPN is further bound to the C terminal thereof;
(6) a conjugate in which a single chain antibody or VHH is bound to the C terminal of hPSPN directly or via a linker, and HSA is further bound to the C terminal thereof;
(7) a conjugate in which HSA is bound to the C terminal of hPSPN directly or via a linker, and a single chain antibody or VHH is further bound to the C terminal thereof;
(8) a conjugate in which hPSPN is bound to the C terminal of HSA directly or via a linker, and a single chain antibody or VHH is further bound to the C terminal thereof;
(9) a conjugate in which HSA is bound to the C terminal of a single chain antibody or VHH directly or via a linker, and hPSPN is further bound to the C terminal thereof; and
(10) a conjugate in which hPSPN is bound to the C terminal of a single chain antibody or VHH directly or via a linker, and HSA is further bound to the C terminal thereof.

A preferable example of (6) is a conjugate in which a GS linker is bound to the C terminal of a wild-type hPSPN, HSA having the amino acid sequence of SEQ ID NO: 3 is further bound to the C terminal thereof, a linker having the amino acid sequence of SEQ ID NO: 196 is further bound to the C terminal thereof, and VHH having the amino acid sequence of SEQ ID NO: 186 is further bound to the C terminal thereof. A preferable example of (7) is a conjugate in which a linker having the amino acid sequence of SEQ ID NO: 196 is bound to the C terminal of a wild-type hPSPN, VHH having the amino acid sequence of SEQ ID NO: 186 is further bound to the C terminal thereof, a GS linker is further bound to the C terminal thereof, and HSA having the amino acid sequence of SEQ ID NO: 3 is further bound to the C terminal thereof.

Linkers for binding a fusion protein of SA and a lysosome enzyme, a fusion protein of SA and a cytokine, or a fusion protein of SA and a neurotrophic factor to an antibody via a linker are described in detail below. Those linkers are preferable as linkers particularly for binding a fusion protein of HSA and hGALC, a fusion protein of HSA and hGBA, a fusion protein of HSA and hIL-10, a fusion protein of HSA and hBDNF, a fusion protein of HSA and hNGF, a fusion protein of HSA and hNT-3, a fusion protein of HSA and hNT-4, a fusion protein of HSA and hCDNF, a fusion protein of HSA and hGDNF, a fusion protein of HSA and hNRTN, a fusion protein of HSA and hARTN, or a fusion protein of HSA and hPSPN to an antibody.

The linker disposed between an antibody or ligand and a fusion protein is a peptide chain constituted of preferably 1 to 60 or 1 to 50, more preferably 1 to 17, further preferably 1 to 10, further more preferably 1 to 5 amino acids, and the number of amino acids constituting the linker can appropriately be adjusted to, for example, 1, 2, 3, 1 to 17, 1 to 10, 10 to 40, 20 to 34, 23 to 31, 25 to 29, or 27. The amino acid sequence of the linker is not limited as long as the antibody linked thereby retains the affinity for a receptor on a cerebrovascular endothelial cell and the fusion protein linked by the linker is capable of exerting the physiological activity of the fusion protein under physiological conditions, and preferably constituted of glycine and serine. Examples of the amino acid sequence include an amino acid sequence consisting of either one amino acid of glycine or serine, amino acid sequence Gly-Ser, amino acid sequence Ser-Ser, amino acid sequence Gly-Gly-Ser, amino acid sequence Gly-Gly-Gly-Gly-Ser (SEQ ID NO: 9), amino acid sequence Gly-Gly-Gly-Gly-Gly-Ser (SEQ ID NO: 10), amino acid sequence Ser-Gly-Gly-Gly-Gly (SEQ ID NO: 11), and an amino acid sequence containing a sequence formed by sequentially connecting 1 to 10 or 2 to 5 of these amino acid sequences. For example, the linker has a sequence consisting of 1 to 50 amino acids or a sequence consisting of 2 to 17, 2 to 10, 10 to 40, 20 to 34, 23 to 31, 25 to 29, or 27 amino acids. For example, a linker containing amino acid sequence Gly-Ser can be suitably used as the linker. Also, a linker containing a sequence formed by sequentially connecting five repeats of amino acid sequence Gly-Gly-Gly-Gly-Ser (SEQ ID NO: 9) subsequently to amino acid sequence Gly-Ser, 27 amino acid sequences in total, can be suitably used as the linker. Further, a linker containing a sequence formed by sequentially connecting five repeats of amino acid sequence Gly-Gly-Gly-Gly-Ser (SEQ ID NO: 9), 25 amino acid sequences in total, can be suitably used as the linker.

Note that, in a case where a plurality of linkers is contained in one peptide chain in the present invention, the linkers are named first linker, second linker, and so on in order from the N terminal side for convenience.

Specific examples of the antibody to be bound to a fusion protein of SA and a lysosome enzyme, a fusion protein of SA and a cytokine, or a fusion protein of SA and a neurotrophic factor include the following antihuman transferrin receptor antibodies (anti-hTfR antibodies) :
(1) an antibody in which the variable region of the light chain contains the amino acid sequence of SEQ ID NO: 96 as CDR1, the amino acid sequence of SEQ ID NO: 97 as CDR2, and the amino acid sequence of SEQ ID NO: 98 as CDR3, and the variable region of the heavy chain contains the amino acid sequence of SEQ ID NO: 99 as CDR1, the amino acid sequence of SEQ ID NO: 100 as CDR2, and the amino acid sequence of SEQ ID NO: 101 as CDR3; and
(2) an antibody in which the variable region of the light chain contains the amino acid sequence of SEQ ID NO: 96 as CDR1, the amino acid sequence of SEQ ID NO: 97 as CDR2, and the amino acid sequence of SEQ ID NO: 98 as CDR3, and the variable region of the heavy chain contains the amino acid sequence of SEQ ID NO: 102 as CDR1, the amino acid sequence of SEQ ID NO: 103 as CDR2, and the amino acid sequence of SEQ ID NO: 104 as CDR3.

However, the antibody is not limited to these, and a mutation such as a substitution, a deletion, and an addition can be appropriately introduced to the amino acid sequences. Note that, the anti-hTfR antibodies of (1) and (2) are humanized anti-hTfR antibodies. Also, the anti-hTfR antibodies of (1) and (2) may be each Fab.

The anti-hTfR antibodies of (1) and (2) are preferable particularly as an antibody to be bound to a fusion protein of HSA and hGALC, a fusion protein of HSA and hGBA, a fusion protein of HSA and hIL-10, a fusion protein of HSA and hBDNF, a fusion protein of HSA and hNGF, a fusion protein of HSA and hNT-3, or a fusion protein of HSA and hNT-4.

An antibody obtained, for example, by adding a substitution, an addition, or a deletion to any of the anti-hTfR antibodies of (1) and (2) can also be used as an antibody to constitute a conjugate as long as the antibody retains the affinity for a human transferrin receptor.

In a case where an amino acid of the amino acid sequence of a light chain of any of the anti-hTfR antibodies of (1) and (2) is substituted with another amino acid, the number of amino acids to be substituted is preferably 1 to 10, more preferably 1 to 5, further preferably 1 to 3, and further more preferably 1 or 2. In a case where an amino acid in the amino acid sequence of a light chain is deleted, the number of amino acids to be deleted is preferably 1 to 10, more preferably 1 to 5, further preferably 1 to 3, and further more preferably 1 or 2. Also, a mutation having the substitution and deletion of amino acids in combination can be introduced.

In a case where an amino acid is added to the amino acid sequence of a light chain of any of the anti-hTfR antibodies of (1) and (2), preferably 1 to 10, more preferably 1 to 5, further preferably 1 to 3, or further more preferably 1 or 2 amino acids are added in the amino acid sequence of a light chain or added to the N terminal side or C terminal side thereof. A mutation having the addition, substitution, and deletion of amino acids in combination can be introduced. The amino acid sequence of a light chain having a mutation introduced thereto has an identity of preferably 80% or more, exhibits an identity of more preferably 90% or more, further preferably 95% or more, to the amino acid sequence of the original light chain.

In a case where an amino acid of the amino acid sequence of a heavy chain of any of the anti-hTfR antibodies of (1) and (2) is substituted with another amino acid, the number of amino acids to be substituted is preferably 1 to 10, more preferably 1 to 5, further preferably 1 to 3, and further more preferably 1 or 2. In a case where an amino acid in the amino acid sequence of a heavy chain is deleted, the number of amino acids to be deleted is preferably 1 to 10, more preferably 1 to 5, further preferably 1 to 3, and further more preferably 1 or 2. Also, a mutation having the substitution and deletion of amino acids in combination can be introduced.

In a case where an amino acid is added to the amino acid sequence of a heavy chain of any of the anti-hTfR antibodies of (1) and (2), preferably 1 to 10, more preferably 1 to 5, further preferably 1 to 3, or further more preferably 1 or 2 amino acids are added in the amino acid sequence of a heavy chain or added to the N terminal side or C terminal side thereof. A mutation having the addition, substitution, and deletion of amino acids in combination can be introduced. The amino acid sequence of a heavy chain having a mutation introduced thereto has an identity of preferably 80% or more, exhibits an identity of more preferably 90% or more, further preferably 95% or more, to the amino acid sequence of the original heavy chain.

Also, specific examples of the antibody to be bound to a fusion protein of SA and a lysosome enzyme, a fusion protein of SA and a cytokine, or a fusion protein of SA and a neurotrophic factor include the following anti-hTfR heavy chain antibody variable region (VHH) peptides (hTfR-binding peptides):
(3) an hTfR-binding peptide containing the amino acid sequence of SEQ ID NO: 105 or SEQ ID NO: 106 as CDR1, the amino acid sequence of SEQ ID NO: 107 or SEQ ID NO: 108 as CDR2, and the amino acid sequence of SEQ ID NO: 109 or SEQ ID NO: 110 as CDR3, and
(4) an hTfR-binding peptide containing the amino acid sequence of SEQ ID NO: 105 or SEQ ID NO: 106 as CDR1, the amino acid sequence of SEQ ID NO: 111 or SEQ ID NO: 112 as CDR2, and the amino acid sequence of SEQ ID NO: 109 or SEQ ID NO: 110 as CDR3.

However, the antibody is not limited to these, and a mutation such as a substitution, a deletion, and an addition can be appropriately introduced to the amino acid sequences. Note that, the hTfR-binding peptides of (3) and (4) are included in antibodies defined in the specification.

The hTfR-binding peptides of (3) and (4) are preferable particularly as an antibody to be bound to a fusion protein of HSA and hGALC, a fusion protein of HSA and hGBA, a fusion protein of HSA and hIL-10, a fusion protein of HSA and hBDNF, a fusion protein of HSA and hNGF, a fusion protein of HSA and hNT-3, or a fusion protein of HSA and hNT-4.

A peptide obtained, for example, by adding a substitution, an addition, or a deletion to any of the hTfR-binding peptides of (3) and (4) can also be used as an antibody to constitute a conjugate as long as the peptide retains the affinity for a human transferrin receptor.

In a case where an amino acid of the amino acid sequence of any of the hTfR-binding peptides of (3) and (4) is substituted with another amino acid, the number of amino acids to be substituted is preferably 1 to 10, more preferably 1 to 5, further preferably 1 to 3, and further more preferably 1 or 2. In a case where an amino acid in the amino acid sequence of any of the hTfR-binding peptides is deleted, the number of amino acids to be deleted is preferably 1 to 10, more preferably 1 to 5, further preferably 1 to 3, and further more preferably 1 or 2. Also, a mutation having the substitution and deletion of amino acids in combination can be introduced.

In a case where an amino acid is added to the amino acid sequence of any of the hTfR-binding peptides of (3) and (4), preferably 1 to 10, more preferably 1 to 5, further preferably 1 to 3, or further more preferably 1 or 2 amino acids are added in the amino acid sequence of a light chain or added to the N terminal side or C terminal side thereof. A mutation having the addition, substitution, and deletion of amino acids in combination can be introduced. The amino acid sequence of a light chain having a mutation introduced thereto has an identity of preferably 80% or more, exhibits an identity of more preferably 90% or more, further preferably 95% or more, to the amino acid sequence of the original hTfR-binding peptide.

A fusion protein of HSA and hGALC in the form of a conjugate obtained by being bound with an antibody or a ligand for a receptor on a cerebrovascular endothelial cell can pass through the blood-brain barrier and exert a function thereof in the brain. Accordingly, the conjugate can be used for production of a drug for intravascular administration for treating a central nervous system disease caused by deletion of GALC, such as Krabbe disease. Also, the conjugate can be used in a treatment method for a central nervous system disease caused by deletion of GALC, such as Krabbe disease, the treatment method including intravascularly administering (including intravenous injection such as intravenous infusion) a therapeutically effective amount to a target patient. The conjugate intravascularly administered can arrive in the brain and also in other organs expressing GALC. In addition, the drug can be used for preventing the onset of the disease. The same applies to a fusion protein of HSA and hGBA, a fusion protein of HSA and hIL-10, a fusion protein of HSA and hBDNF, a fusion protein of HSA and hNGF, a fusion protein of HSA and hNT-3, a fusion protein of HSA and hNT-4, a fusion protein of HSA and hCDNF, a fusion protein of HSA and hGDNF, a fusion protein of HSA and hNRTN, a fusion protein of HSA and hARTN, and a fusion protein of HSA and hPSPN.

When being bound to an antibody or ligand for a receptor on a cerebrovascular endothelial cell, a fusion protein of HSA and hGALC, a fusion protein of HSA and hGBA, a fusion protein of HSA and hIL-10, a fusion protein of HSA and hBDNF, a fusion protein of HSA and hNGF, a fusion protein of HSA and hNT-3, and a fusion protein of HSA and hNT-4 can be each used as a drug to be intravascularly administered and exert the drug action in the central nervous system (CNS). While the drug is typically administered to a patient through intravenous injection by intravenous infusion or the like, subcutaneous injection, or intramuscular injection, the route of administration is not particularly limited.

In a conjugate of a fusion protein of HSA and hGALC with an antibody, the phrase that hGALC has a function as an hGALC means that hGALC has a specific activity of preferably 10% or more, more preferably 20% or more, further preferably 50% or more, further more preferably 80% or more, for example, 90% or more, or 95% or more when the specific activity of a normal wild-type hGALC is regarded as 100%. Also, in a conjugate of a fusion protein of HSA and hGALC with a non-antibody protein, the phrase that hGALC has a function as an hGALC means that hGALC has a specific activity of preferably 10% or more, more preferably 20% or more, further preferably 50% or more, further more preferably 80% or more, for example, 90% or more, or 95% or more when the specific activity of a normal wild-type hGALC is regarded as 100%. Note that, the specific activity of the hGALC in the conjugate herein is computationally obtained by multiplying the enzyme activity (µM/h/mg protein) of an hGALC per unit mass of the conjugate by (the molecular weight of the conjugate/the molecular weight of the moiety corresponding to the hGALC in the conjugate). The same applies to a conjugate of a fusion protein of HSA and hGBA with an antibody. Although the same applies to a conjugate of a fusion protein of HSA and hIL-10 with an antibody, a conjugate of a fusion protein of HSA and hBDNF with an antibody, a conjugate of a fusion protein of HSA and hNGF with an antibody, a conjugate of a fusion protein of HSA and hNT-3 with an antibody, and a conjugate of a fusion protein of HSA and hNT-4 with an antibody, each of hIL-10, hBDNF, hNGF, hNT-3, and hNT-4 is not an enzyme anyway, and hence the specific activity is based not on the enzyme activity but on the physiological activity of the protein hIL-10, hBDNF, hNGF, hNT-3, or hNT-4. The same applies to hCDNF, hGDNF, hNRTN, hARTN, and hPSPN.

A fusion protein of SA and a lysosome enzyme, a fusion protein of SA and a cytokine, or a fusion protein of SA and a neurotrophic factor in an embodiment of the present invention can form a conjugate with a ligand. Here, the SA, lysosome enzyme, cytokine, and neurotrophic factor may be each derived from a human or from a non-human animal species. Examples of the fusion protein of SA and a lysosome enzyme include a fusion protein of HSA and hGALC and a fusion protein of HSA and hGBA; examples of the fusion protein of SA and a cytokine include a fusion protein of HSA and hIL-10; and examples of the fusion protein of SA and a neurotrophic factor include a fusion protein of HSA and hBDNF, a fusion protein of HSA and hNGF, a fusion protein of HSA and hNT-3, and a fusion protein of HSA and hNT-4.

Here, the ligand is capable of, for example, specifically binding to a receptor on a cerebrovascular endothelial cell, such as an insulin receptor, a transferrin receptor, a leptin receptor, a lipoprotein receptor, and an IGF receptor. The ligands corresponding to the insulin receptor, transferrin receptor, leptin receptor, lipoprotein receptor, and IGF receptor are insulin, leptin, lipoprotein, and IGF (IGF-1, IGF-2), respectively. The ligand may be a wild-type ligand, or a ligand having a mutation introduced thereto as long as the ligand is capable of specifically binding to the target receptor. The ligand may be a ligand fragment as long as the ligand fragment is capable of specifically binding to the target receptor. These ligands are preferable particularly as a ligand to be bound to a fusion protein of HSA and hGALC, a fusion protein of HSA and hGBA, a fusion protein of HSA and hIL-10, a fusion protein of HSA and hBDNF, a fusion protein of HSA and hNGF, a fusion protein of HSA and hNT-3, or a fusion protein of HSA and hNT-4.

In a case where the ligand is transferrin (Tf), for example, the transferrin may be human Tf or Tf of a non-human animal species, but is preferably human Tf. The transferrin may be a wild-type transferrin, or a transferrin having a mutation introduced therein as long as the transferrin has affinity for a transferrin receptor (TfR). The mutation is, for example, substitution, addition, or deletion of 1 or 2 amino acids. Also, the transferrin may be full-length transferrin, or a fragment thereof as long as the fragment has affinity for a transferrin receptor (TfR). The same applies to insulin, leptin, lipoprotein, and IGF (IGF-1, IGF-2).

A conjugate of a fusion protein of SA and a lysosome enzyme, a fusion protein of SA and a cytokine, or a fusion protein of SA and a neurotrophic factor with a ligand in an example of the present invention can be produced with the following method of (1) or (2):
(1) forming a conjugate by binding a fusion protein and a ligand via a non-peptide linker or a peptide linker; and
(2) producing a conjugate as a recombinant protein in which a fusion protein and a ligand are bound, via a linker sequence or directly, by a peptide bond.

For the non-peptide linker in the case of (1) mentioned above, any of polyethylene glycol, polypropylene glycol, copolymer of ethylene glycol and propylene glycol, polyoxyethylated polyol, polyvinyl alcohol, polysaccharide, dextran, polyvinyl ether, biodegradable polymers, lipid polymers, chitin, and hyaluronic acid, or a derivative of any of them, or a combination of any of them can be used. The peptide linker is a peptide chain constituted of 1 to 50 amino acids bound by peptide bonds or a derivative thereof, and the N terminal and the C terminal of the linker each form a covalent bond with either a fusion protein or a ligand to bind the fusion protein and the ligand. The fusion protein and the ligand are each produced as a recombinant protein.

In the case of (2) mentioned above, the fusion protein and ligand are produced as a recombinant protein in which the N terminal of the ligand is bound to the C terminal of the fusion protein, via a linker sequence or directly, or as a recombinant protein in which the N terminal of the fusion protein is bound to the C terminal of the ligand, via a linker sequence or directly. The expression vector, host cell, and medium that are used for producing the fusion protein of HSA and hGALC as mentioned above can be applied to those for producing the recombinant protein.

A fusion protein of SA and a lysosome enzyme, a fusion protein of SA and a cytokine, or a fusion protein of SA and a neurotrophic factor in an embodiment of the present invention can form a conjugate not only with an antibody and a ligand but also with another protein (A). Here, the SA, lysosome enzyme, cytokine, and neurotrophic factor may be each derived from a human or from a non-human animal species. Examples of the fusion protein of SA and a lysosome enzyme include a fusion protein of HSA and hGALC and a fusion protein of HSA and hGBA; examples of the fusion protein of SA and a cytokine include a fusion protein of HSA and hIL-10; and examples of the fusion protein of SA and a neurotrophic factor include a fusion protein of HSA and hBDNF, a fusion protein of HSA and hNGF, a fusion protein of HSA and hNT-3, and a fusion protein of HSA and hNT-4.

A conjugate of a fusion protein of SA and a lysosome enzyme, a fusion protein of SA and a cytokine, or a fusion protein of SA and a neurotrophic factor with protein (A) in an example of the present invention can be produced by the following method of (1) or (2):
(1) forming a conjugate by binding a fusion protein and protein (A) via a non-peptide linker or a peptide linker; and
(2) producing a conjugate as a recombinant protein in which a fusion protein and protein (A) are bound, via a linker sequence or directly by a peptide bond.

For the non-peptide linker in the case of (1) mentioned above, any of polyethylene glycol, polypropylene glycol, copolymer of ethylene glycol and propylene glycol, polyoxyethylated polyol, polyvinyl alcohol, polysaccharide, dextran, polyvinyl ether, biodegradable polymers, lipid polymers, chitin, and hyaluronic acid, or a derivative of any of them, or a combination of any of them can be used. The peptide linker is a peptide chain constituted of 1 to 50 amino acids bound by peptide bonds or a derivative thereof, and the N terminal and the C terminal of the linker each form a covalent bond with either a fusion protein or protein (A) to bind the fusion protein and protein (A). The fusion protein and protein (A) are each produced as a recombinant protein.

In the case of (2) mentioned above, the fusion protein and the ligand are produced as a recombinant protein in which the N terminal of the ligand is bound to the C terminal of the fusion protein, via a linker sequence or directly, or as a recombinant protein in which the N terminal of the fusion protein is bound to the C terminal of the ligand, via a linker sequence or directly. The expression vector, host cell, and medium that are used for producing the fusion protein of HSA and hGALC can be applied to those for producing the recombinant protein.

A conjugate of a fusion protein of HSA and hGALC with an antibody, and a conjugate of a fusion protein of HSA and hGALC with a ligand or protein (A) can be each used as a pharmaceutical composition targeted to Krabbe disease (galactosylceramide lipidosis, or globoid cell leukodystrophy) .

A conjugate of a fusion protein of HSA and hGBA with an antibody, and a conjugate of a fusion protein of HSA and hGBA with a ligand or protein (A) can be each used as a pharmaceutical composition targeted to Gaucher's disease.

A conjugate of a fusion protein of HSA and hIL-10 with an antibody, and a conjugate of a fusion protein of HSA and hIL-10 with a ligand or protein (A) can be each used as a pharmaceutical composition targeted to, for example, inflammatory diseases, neuropathic pain, multiple sclerosis, spinal cord injury, ALS, neuroinflammation, arthritis, and symptoms associated with other diseases of the joint, and autoimmune diseases.

A conjugate of a fusion protein of HSA and hBDNF with an antibody, and a conjugate of a fusion protein of HSA and hBDNF with a ligand or protein (A) can be each used as a pharmaceutical composition targeted to, for example, neurodegenerative diseases such as Alzheimer's disease, Parkinson's disease, and Huntington's disease, spinal degenerative diseases such as amyotrophic lateral sclerosis, developmental impairments such as diabetic neuropathy, ischemic cerebral disease, and Rett syndrome, schizophrenia, depression, and Rett syndrome.

A conjugate of a fusion protein of HSA and hNGF with an antibody, and a conjugate of a fusion protein of HSA and hNGF with a ligand or protein (A) can be each used as a pharmaceutical composition targeted to, for example, neurodegenerative diseases such as Alzheimer's disease, Parkinson's disease, and Huntington's disease.

A conjugate of a fusion protein of HSA and hNT-3 with an antibody, and a conjugate of a fusion protein of HSA and hNT-3 with a ligand or protein (A) can be each used as a pharmaceutical composition targeted to, for example, neurodegenerative diseases.

A conjugate of a fusion protein of HSA and hNT-4 with an antibody, and a conjugate of a fusion protein of HSA and hNT-4 with a ligand or protein (A) can be each used as a pharmaceutical composition targeted to, for example, neurodegenerative diseases.

A pharmaceutical composition containing a conjugate of a fusion protein of HSA and hGALC with an antibody, or a conjugate of a fusion protein of HSA and hGALC with a ligand or protein (A) as an active ingredient can be administered intravenously, intramuscularly, intraperitoneally, subcutaneously or intracerebroventricularly as an injection. Such an injection can be supplied as a lyophilized preparation or an aqueous liquid. The aqueous liquid may be in a form it is filled in a vial or supplied as a prefilled preparation in a syringe. The lyophilized preparation is dissolved and restored in an aqueous medium before use and then used. In a conjugate of a fusion protein of HSA and hGALC with an antibody contained in an aqueous liquid, the ratio (monomer (mass)/total protein (mass) × 100 (%)) of a monomer in the total protein is preferably 70% or more, more preferably 80% or more, further preferably 90% or more, for example, 95% or more, or 95% or more. The same applies to a solution prepared by dissolving and restoring a lyophilized preparation in an aqueous medium. The described matters can also be applied to a pharmaceutical composition containing a conjugate of a fusion protein of HSA and hGBA with an antibody, or a conjugate of a fusion protein of HSA and hGBA with a ligand or protein (A) as an active ingredient, a pharmaceutical composition containing a conjugate of a fusion protein of HSA and hIL-10 with an antibody, or a conjugate of a fusion protein of HSA and hIL-10 with a ligand or protein (A) as an active ingredient, a pharmaceutical composition containing a conjugate of a fusion protein of HSA and hBDNF with an antibody, or a conjugate of a fusion protein of HSA and hBDNF with a ligand or protein (A) as an active ingredient, a pharmaceutical composition containing a conjugate of a fusion protein of HSA and hNGF with an antibody, or a conjugate of a fusion protein of HSA and hNGF with a ligand or protein (A) as an active ingredient, a pharmaceutical composition containing a conjugate of a fusion protein of HSA and NT-3 with an antibody, or a conjugate of a fusion protein of HSA and hNT-3 with a ligand or protein (A) as an active ingredient, and a pharmaceutical composition containing a conjugate of a fusion protein of HSA and NT-4 with an antibody, or a conjugate of a fusion protein of HSA and hNT-4 with a ligand or protein (A) as an active ingredient.

When being expressed as a recombinant of protein, the wild-type hGALC is obtained as a multimer such as a dimer and a tetramer. In contrast to this, when a fusion protein of HSA and hGALC is expressed as a recombinant of protein, most of the fusion protein is obtained as a monomer. In a fusion protein of HSA and hGALC produced as a recombinant of protein, the ratio (monomer (mass)/total protein (mass) × 100 (%)) of a monomer in the total protein is preferably 70% or more, more preferably 80% or more, further preferably 90% or more, for example, 95% or more, or 95% or more.

When a fusion protein of HSA and hGALC such as HSA-hGALC and hGALC-HSA is expressed as a recombinant protein, the fusion protein can be obtained as a homogenous product of a monomer, and hence the method for producing hGALC, wherein hGALC is produced as a fusion protein with HSA, can be said to be superior as a method for producing hGALC also in terms of production management.

Also, when a conjugate of a fusion protein of HSA and hGALC with an antibody is expressed as a recombinant of protein, most of the conjugate is obtained as a monomer. In the conjugate produced as a recombinant of protein, the ratio (monomer (mass)/total protein (mass) × 100 (%)) of a monomer in the total protein is preferably 70% or more, more preferably 80% or more, further preferably 90% or more, for example, 95% or more, or 95% or more.

When a conjugate of a fusion protein of HSA and hGALC with an antibody is expressed as a recombinant protein, the conjugate can be obtained as a homogenous product of a monomer, and hence the method for producing hGALC as a fusion protein with HSA and an antibody can be said to be superior as a method for producing hGALC also in terms of production management.

A fusion protein of SA and a lysosome enzyme, a fusion protein of SA and a cytokine, or a fusion protein of SA and a neurotrophic factor, and a nucleic acid molecule containing a gene encoding the conjugate can be used for gene therapy. Also, a nucleic acid molecule containing a gene encoding a conjugate of any of the fusion proteins with an antibody, a nucleic acid molecule containing a gene encoding a conjugate of any of the fusion proteins with a ligand, and a nucleic acid molecule containing a gene encoding a conjugate of any of the fusion proteins with protein (A) can be used for gene therapy.

Here, the fusion protein is, for example, a fusion protein of HSA and hGALC, a fusion protein of HSA and hGBA, a fusion protein of HSA and hIL-10, a fusion protein of HSA and hBDNF, a fusion protein of HSA and hNGF, a fusion protein of HSA and hNT-3, or a fusion protein of HSA and hNT-4. The antibody is, for example, an anti-transferrin receptor antibody. The ligand is, for example, transferrin or a fragment thereof.

Examples of nucleic acid molecules that can be used for gene therapy in an embodiment of the present invention include those containing any of nucleotide sequences shown in the following:
(1) a nucleotide sequence containing a first inverted terminal repeat (ITR) or a functional equivalent thereof, a nucleotide sequence containing a gene expression regulatory site in the downstream thereof, a nucleotide sequence encoding a fusion protein in the further downstream, and a nucleotide sequence containing a second inverted terminal repeat (ITR) or a functional equivalent thereof in the further downstream;
(2) a nucleotide sequence containing a first long terminal repeat (LTR) or a functional equivalent thereof, a nucleotide sequence containing a gene expression regulatory site in the downstream thereof, a nucleotide sequence encoding a fusion protein in the further downstream, and a nucleotide sequence containing a second long terminal repeat (LTR) or a functional equivalent thereof in the further downstream;
(3) a nucleotide sequence containing a leader or a functional equivalent thereof, a nucleotide sequence containing a gene expression regulatory site in the downstream thereof, a nucleotide sequence encoding a fusion protein in the further downstream, and a nucleotide sequence containing a trailer or a functional equivalent thereof in the further downstream;
(4) a nucleotide sequence containing a first inverted terminal repeat (ITR) or a functional equivalent thereof, a nucleotide sequence containing a gene expression regulatory site in the downstream thereof, a nucleotide sequence encoding a conjugate of a fusion protein with an antibody in the further downstream, and a nucleotide sequence containing a second inverted terminal repeat (ITR) or a functional equivalent thereof in the further downstream;
(5) a nucleotide sequence containing a first long terminal repeat (LTR) or a functional equivalent thereof, a nucleotide sequence containing a gene expression regulatory site in the downstream thereof, a nucleotide sequence encoding a conjugate of a fusion protein with an antibody in the further downstream, and a nucleotide sequence containing a second long terminal repeat (LTR) or a functional equivalent thereof in the further downstream;
(6) a nucleotide sequence containing a leader or a functional equivalent thereof, a nucleotide sequence containing a gene expression regulatory site in the downstream thereof, a nucleotide sequence encoding a conjugate of a fusion protein with an antibody in the further downstream, and a nucleotide sequence containing a trailer or a functional equivalent thereof in the further downstream;
(7) a nucleotide sequence containing a first inverted terminal repeat (ITR) or a functional equivalent thereof, a nucleotide sequence containing a gene expression regulatory site in the downstream thereof, a nucleotide sequence encoding a conjugate of a fusion protein with a ligand in the further downstream, and a nucleotide sequence containing a second inverted terminal repeat (ITR) or a functional equivalent thereof in the further downstream;
(8) a nucleotide sequence containing a first long terminal repeat (LTR) or a functional equivalent thereof, a nucleotide sequence containing a gene expression regulatory site in the downstream thereof, a nucleotide sequence encoding a conjugate of a fusion protein with a ligand in the further downstream, and a nucleotide sequence containing a second long terminal repeat (LTR) or a functional equivalent thereof in the further downstream;
(9) a nucleotide sequence containing a leader or a functional equivalent thereof, a nucleotide sequence containing a gene expression regulatory site in the downstream thereof, a nucleotide sequence encoding a conjugate of a fusion protein with a ligand in the further downstream, and a nucleotide sequence containing a trailer or a functional equivalent thereof in the further downstream;
(10) a nucleotide sequence containing a first inverted terminal repeat (ITR) or a functional equivalent thereof, a nucleotide sequence containing a gene expression regulatory site in the downstream thereof, a nucleotide sequence encoding a conjugate of a fusion protein with protein (A) in the further downstream, and a nucleotide sequence containing a second inverted terminal repeat (ITR) or a functional equivalent thereof in the further downstream;
(11) a nucleotide sequence containing a first long terminal repeat (LTR) or a functional equivalent thereof, a nucleotide sequence containing a gene expression regulatory site in the downstream thereof, a nucleotide sequence encoding a conjugate of a fusion protein with protein (A) in the further downstream, and a nucleotide sequence containing a second long terminal repeat (LTR) or a functional equivalent thereof in the further downstream; and
(12) a nucleotide sequence containing a leader or a functional equivalent thereof, a nucleotide sequence containing a gene expression regulatory site in the downstream thereof, a nucleotide sequence encoding a conjugate of a fusion protein with protein (A) in the further downstream, and a nucleotide sequence containing a trailer or a functional equivalent thereof in the further downstream.

In the present invention, the term "nucleic acid molecule" mainly refers to either a DNA, in which deoxyribonucleotides are polymerized through phosphodiester bonding, or an RNA, in which ribonucleotides are polymerized through phosphodiester bonding.

In a case where a "nucleic acid molecule" is a DNA, the DNA may be single-stranded (single chain) or doublestranded with complementary strands. In a case where a DNA is single-stranded, the DNA may be a (+) strand or a (-) strand. Individual deoxyribonucleotides constituting a DNA may be each a naturally occurring deoxyribonucleotide or a deoxyribonucleotide obtained by introducing modification to a naturally occurring deoxyribonucleotide as long as a protein-coding gene contained in the DNA can be translated into an mRNA in a cell of a mammal (particularly a human). In an embodiment of the present invention, individual deoxyribonucleotides constituting a DNA may be each a naturally occurring deoxyribonucleotide or a deoxyribonucleotide obtained by introducing modification to a naturally occurring deoxyribonucleotide as long as a protein-coding gene contained in the DNA can be translated into an mRNA in a cell of a mammal (particularly a human) and the whole or part of the DNA can be replicated.

In a case where a "nucleic acid molecule" is an RNA, the RNA may be single-stranded (single chain) or doublestranded with complementary strands. In a case where an RNA is a single chain, the RNA may be a (+) strand or a (-) strand. In an embodiment of the present invention, individual ribonucleotides constituting an RNA may be each a naturally occurring ribonucleotide or a ribonucleotide obtained by introducing modification to a naturally occurring ribonucleotide as long as a protein-coding gene contained in the RNA can be reverse-transcribed into a DNA in a cell of a mammal (particularly a human). Also, in an embodiment of the present invention, individual ribonucleotides constituting an RNA may be each a naturally occurring ribonucleotide or a ribonucleotide obtained by introducing modification to a naturally occurring ribonucleotide as long as a protein-coding gene contained in the RNA can be translated into a protein in a cell of a mammal (particularly a human). Modification of a ribonucleotide is performed, for example, for suppressing the decomposition of an RNA by RNase to increase the intracellular stability of the RNA.

In an embodiment of the present invention, the term inverted terminal repeat (ITR) refers to a nucleotide sequence present as repeats of the same sequence at a terminal of a viral genome. An ITR derived from adeno-associated virus and an ITR derived from adenovirus can be suitably used. For example, the ITR of adeno-associated virus is a region that has a chain length of about 145 nucleotides and functions as, for example, an origin of replication. In an embodiment of the present invention, two inverted terminal repeats (ITRs) are present in a nucleic acid molecule, and they are referred to as a first inverted terminal repeat (ITR) and a second inverted terminal repeat (ITR). When a gene encoding a fusion protein is disposed between two ITRs, the ITR positioned on the 5' side is referred to as a first inverted terminal repeat (ITR), and the ITR positioned on the 3' side is referred to as a second inverted terminal repeat (ITR). Each inverted terminal repeat (ITR) may be derived from any virus as long as the ITR has at least one of the functions of the original ITR such as a function as an origin of replication and gene insertion into a host cell, and the ITR of adeno-associated virus is one of preferable ITRs.

In a case where an ITR is derived from adeno-associated virus, the serotype of the AAV is not particularly limited, and may be any of the serotypes 1, 2, 3, 4, 5, 6, 7, 8, 9, 10, and 11. For inverted terminal repeats (ITRs) of AAV serotype 2, for example, the first ITR contains the nucleotide sequence represented by SEQ ID NO: 113, and the second ITR contains the nucleotide sequence represented by SEQ ID NO: 114.

The ITRs are not limited to wild-type ITRs, and each may be an ITR obtained by introducing modification such as substitution, deletion, and addition to the nucleotide sequence of a wild-type ITR. In a case where a nucleotide of the nucleotide sequence of a wild-type ITR is substituted with another nucleotide, the number of nucleotides to be substituted is preferably 1 to 20, more preferably 1 to 10, and further preferably 1 to 3. In a case where deletion is added to the nucleotide sequence of a wild-type ITR, the number of nucleotides to be deleted is preferably 1 to 20, more preferably 1 to 10, and further preferably 1 to 3. Also, a mutation having the substitution and deletion of nucleotides in combination can be introduced. In a case where a nucleotide is added to a wild-type ITR, preferably 1 to 20, more preferably 1 to 10, or further preferably 1 to 3 nucleotides are added in the nucleotide sequence of the ITR or added to the 5' terminal or 3' terminal thereof.

A mutation having the addition, substitution, and deletion of nucleotides in combination can be introduced. The nucleotide sequence of an ITR having a mutation introduced thereto exhibits an identity of preferably 80% or more, more preferably 85% or more, further preferably 90% or more, further preferably 95% or more, or further preferably 98% or more to the nucleotide sequence of the wild-type ITR.

A functional equivalent of an ITR is a product that can be used in place of an ITR in terms of functions. Also, an ITR artificially constructed on the basis of an ITR is a functional equivalent of an ITR as long as the artificially constructed product can substitute for the ITR.

A functional equivalent of an AAV ITR is a product that can be used in place of an AAV ITR in terms of functions. Also, an ITR artificially constructed on the basis of an AAV ITR is a functional equivalent of an AAV ITR as long as the artificially constructed product can substitute for the AAV ITR.

Examples of functional equivalents of an artificially constructed first AAV inverted terminal repeat (first AAV-ITR) include that having the nucleotide sequence represented by SEQ ID NO: 115 (a functional equivalent of the first AAV-ITR). A product obtained by introducing substitution, deletion, or mutation to the nucleotide sequence represented by SEQ ID NO: 115 is also included in functional equivalents of the first AAV-ITR as long as the product can be used in place of the first AAV ITR in terms of functions. In a case where a nucleotide in the nucleotide sequence represented by SEQ ID NO: 115 is substituted with another nucleotide, the number of nucleotides to be substituted is preferably 1 to 20, more preferably 1 to 10, further preferably 1 to 5, and further preferably 1 to 3. In a case where a nucleotide in the nucleotide sequence represented by SEQ ID NO: 115 is deleted, the number of nucleotides to be deleted is preferably 1 to 20, more preferably 1 to 10, further preferably 1 to 5, and further preferably 1 to 3. Also, a mutant obtained by introducing a mutation having the substitution and deletion of nucleotides in combination to the ITR is a functional equivalent of the first AAV-ITR. In a case where a nucleotide is added to the nucleotide sequence represented by SEQ ID NO: 115, preferably 1 to 20, more preferably 1 to 10, further preferably 1 to 5, or further preferably 1 to 3 nucleotides are added in the nucleotide sequence or added to the 5' terminal or 3' terminal thereof. A mutant obtained by introducing a mutation having the addition, substitution, and deletion of nucleotides in combination to the ITR is also included in functional equivalents of the first AAV-ITR. The nucleotide sequence of an ITR having a mutation introduced thereto exhibits an identity of preferably 80% or more, more preferably 85% or more, further preferably 90% or more, further preferably 95% or more, or further more preferably 98% or more to the nucleotide sequence represented by SEQ ID NO: 115.

Examples of functional equivalents of an artificially constructed second inverted terminal repeat (second AAV-ITR) include that having the nucleotide sequence represented by SEQ ID NO: 116 (a functional equivalent of the second AAV-ITR). A product obtained by introducing substitution, deletion, or mutation to the nucleotide sequence represented by SEQ ID NO: 116 is also included in functional equivalents of the second AAV-ITR as long as the product can be used in place of the second AAV ITR in terms of functions. In a case where a nucleotide in the nucleotide sequence represented by SEQ ID NO: 116 is substituted with another nucleotide, the number of nucleotides to be substituted is preferably 1 to 20, more preferably 1 to 10, further preferably 1 to 5, and further preferably 1 to 3. In a case where a nucleotide in the nucleotide sequence represented by SEQ ID NO: 116 is deleted, the number of nucleotides to be deleted is preferably 1 to 20, more preferably 1 to 10, further preferably 1 to 5, and further preferably 1 to 3. Also, a mutant obtained by introducing a mutation having the substitution and deletion of nucleotides in combination to the ITR is a functional equivalent of the second AAV-ITR. In a case where a nucleotide is added to the nucleotide sequence represented by SEQ ID NO: 116, preferably 1 to 20, more preferably 1 to 10, further preferably 1 to 5, or further preferably 1 to 3 nucleotides are added in the nucleotide sequence or added to the 5' terminal or 3' terminal thereof. A mutant obtained by introducing a mutation having the addition, substitution, and deletion of nucleotides in combination to the ITR is also included in functional equivalents of the second AAV-ITR. The nucleotide sequence of an ITR having a mutation introduced thereto exhibits an identity of preferably 85% or more, more preferably 85% or more, further preferably 90% or more, further preferably 95% or more, or further more preferably 98% or more to the nucleotide sequence represented by SEQ ID NO: 116.

In an embodiment of the present invention, the term long terminal repeat (LTR) refers to a nucleotide sequence present as several hundred to several thousand repeats of the same sequence at a terminal of, for example, retrotransposon of a eukaryote, a retroviral genome, or a lentiviral genome. In an embodiment of the present invention, two long terminal repeats (LTRs) are present in a nucleic acid molecule, and they are referred to as a first long terminal repeat (LTR) and a second long terminal repeat (LTR). When a gene encoding a fusion protein is disposed between two LTRs, the LTR positioned on the 5' side is referred to as a first long terminal repeat (LTR), and the LTR positioned on the 3' side is referred to as a second long terminal repeat (LTR). Each long terminal repeat (LTR) may be derived from any virus as long as the LTR has at least one of the functions of the original LTR such as a function as an origin of replication and gene insertion into a host cell, and preferable LTRs include LTRs of a retroviral genome and LTRs of lentivirus. The LTRs are not limited to wild-type LTRs, and each may be an LTR obtained by introducing modification such as substitution, deletion, and addition to the nucleotide sequence of a wild-type LTR.

In a case where a nucleotide of the nucleotide sequence of a wild-type LTR is substituted with another nucleotide, the number of nucleotides to be substituted is preferably 1 to 20, more preferably 1 to 10, and further preferably 1 to 3. In a case where deletion is introduced to the nucleotide sequence of a wild-type LTR, the number of nucleotides to be deleted is preferably 1 to 20, more preferably 1 to 10, and further preferably 1 to 3. Also, a mutation having the substitution and deletion of nucleotides in combination can be introduced. In a case where a nucleotide is added to a wild-type LTR, preferably 1 to 20, more preferably 1 to 10, or further preferably 1 to 3 nucleotides are added in the nucleotide sequence of the LTR or added to the 5' terminal or 3' terminal thereof. A mutation having the addition, substitution, and deletion of nucleotides in combination can be introduced. The nucleotide sequence of an LTR having a mutation introduced thereto exhibits an identity of preferably 80% or more, more preferably 85% or more, further preferably 90% or more, further preferably 95% or more, or further more preferably 98% or more to the nucleotide sequence of the wild-type LTR.

A functional equivalent of an LTR is a product that can be used in place of an LTR in terms of functions. Also, an LTR artificially constructed on the basis of an LTR is a functional equivalent of an LTR as long as the artificially constructed product can substitute for the LTR.

A functional equivalent of a lentivirus LTR is a product that can be used in place of a lentivirus LTR in terms of functions. Also, an LTR artificially constructed on the basis of a lentivirus LTR is a functional equivalent of a lentivirus LTR as long as the artificially constructed product can substitute for the lentivirus LTR.

A functional equivalent of a retrovirus LTR is a product that can be used in place of a retrovirus LTR in terms of functions. Also, an LTR artificially constructed on the basis of a retrovirus LTR is a functional equivalent of a retrovirus LTR as long as the artificially constructed product can substitute for the retrovirus LTR.

Examples of functional equivalents of a first LTR include that having the nucleotide sequence represented by SEQ ID NO: 117. A product obtained by introducing substitution, deletion, or mutation to the nucleotide sequence represented by SEQ ID NO: 117 is also included in functional equivalents of the first LTR as long as the product can be used as the first LTR in terms of functions. In a case where a nucleotide in the nucleotide sequence represented by SEQ ID NO: 117 is substituted with another nucleotide, the number of nucleotides to be substituted is preferably 1 to 20, more preferably 1 to 20, further preferably 1 to 5, and further preferably 1 to 3. In a case where a nucleotide in the nucleotide sequence represented by SEQ ID NO: 117 is deleted, the number of nucleotides to be deleted is preferably 1 to 20, more preferably 1 to 10, further preferably 1 to 5, and further preferably 1 to 3. Also, a mutant obtained by introducing a mutation having the substitution and deletion of nucleotides in combination to the LTR is a functional equivalent of the first LTR. In a case where a nucleotide is added to the nucleotide sequence represented by SEQ ID NO: 117, preferably 1 to 20, more preferably 1 to 10, further preferably 1 to 5, or further preferably 1 to 3 nucleotides are added in the nucleotide sequence or added to the 5' terminal or 3° terminal thereof. A mutant obtained by introducing a mutation having the addition, substitution, and deletion of nucleotides in combination to the LTR is also included in functional equivalents of the first LTR. The nucleotide sequence of an LTR having a mutation introduced thereto exhibits an identity of preferably 80% or more, more preferably 85% or more, further preferably 90% or more, further preferably 95% or more, or further more preferably 98% or more to the nucleotide sequence represented by SEQ ID NO: 117.

Examples of functional equivalents of a second LTR include that having the nucleotide sequence represented by SEQ ID NO: 118. A product obtained by introducing substitution, deletion, or mutation to the nucleotide sequence represented by SEQ ID NO: 118 is also included in functional equivalents of the second LTR as long as the product can be used as the second LTR in terms of functions. In a case where a nucleotide in the nucleotide sequence represented by SEQ ID NO: 118 is substituted with another nucleotide, the number of nucleotides to be substituted is preferably 1 to 20, more preferably 1 to 10, further preferably 1 to 5, and further preferably 1 to 3. In a case where a nucleotide in the nucleotide sequence represented by SEQ ID NO: 118 is deleted, the number of nucleotides to be deleted is preferably 1 to 20, more preferably 1 to 10, further preferably 1 to 5, and further preferably 1 to 3. Also, a mutant obtained by introducing a mutation having the substitution and deletion of nucleotides in combination to the LTR is a functional equivalent of the second LTR. In a case where a nucleotide is added to the nucleotide sequence represented by SEQ ID NO: 118, preferably 1 to 20, more preferably 1 to 10, further preferably 1 to 5, or further preferably 1 to 3 nucleotides are added in the nucleotide sequence or added to the 5' terminal or 3' terminal thereof. A mutant obtained by introducing a mutation having the addition, substitution, and deletion of nucleotides in combination to the LTR is also included in functional equivalents of the second LTR. The nucleotide sequence of an LTR having a mutation introduced thereto exhibits an identity of preferably 80% or more, more preferably 85% or more, further preferably 90% or more, further preferably 95% or more, or further more preferably 98% or more to the nucleotide sequence represented by SEQ ID NO: 118.

In an embodiment of the present invention, the terms leader and trailer refer to nucleotide sequences partially complementary to each other that are each present at a terminal of a viral genome. A leader and trailer derived from Sendai virus can be suitably used. The leader and trailer of Sendai virus are each a region having a chain length of approximately 50 nucleotides. Normally, the leader is positioned on the 5' side, and the trailer is positioned on the 3' side.

In an embodiment of the present invention, for example, a Sendai virus-derived leader having the nucleotide sequence represented by SEQ ID NO: 119 and a Sendai virus-derived trailer having the nucleotide sequence represented by SEQ ID NO: 120 are suitably used.

Also, a leader and trailer obtained by introducing a mutation to a wild-type Sendai virus-derived leader and trailer can be suitably used in the present invention. In a case where a nucleotide of the nucleotide sequence is substituted with another nucleotide, the number of nucleotides to be substituted is preferably 1 to 20, more preferably 1 to 10, and further preferably 1 to 3. In a case where deletion is introduced to the nucleotide sequence(s) of a wild-type leader or/and trailer, the number of nucleotides to be deleted is preferably 1 to 20, more preferably 1 to 10, and further preferably 1 to 3. Also, a mutation having the substitution and deletion of nucleotides in combination can be introduced. In a case where a nucleotide is added, preferably 1 to 20, more preferably 1 to 10, or further preferably 1 to 3 nucleotides are added in the nucleotide sequence(s) of a leader or/and trailer or added to the 5' terminal or 3' terminal thereof. A mutation having the addition, substitution, and deletion of nucleotides in combination can be introduced. The nucleotide sequence having a mutation introduced thereto exhibits an identity of preferably 80% or more, more preferably 85% or more, further preferably 90% or more, further preferably 95% or more, or further more preferably 98% or more to the wild-type nucleotide sequence.

In an embodiment of the present invention, the nucleotide sequence that can be used as a nucleotide sequence containing a gene expression regulatory site that regulates the expression of a gene for a fusion protein is not particularly limited as long as the nucleotide sequence can express the fusion protein in a cell, tissue, or living body of a mammal (particularly a human) to which the gene encoding the fusion protein is introduced, and a nucleotide sequence containing a cytomegalovirus-derived promoter (optionally containing an enhancer), an SV40 early promoter, a human elongation factor-1α (EF-1α) promoter, a human ubiquitin C promoter, an LTR promoter of Rous sarcoma virus, which is a retrovirus, a dihydrofolate reductase promoter, a β-actin promoter, a phosphoglycerate kinase (PGK) promoter, a mouse albumin promoter, a human albumin promoter, and a human α-1 antitrypsin promoter is preferable. For example, a synthetic promoter containing a mouse albumin promoter downstream of a mouse α fetoprotein enhancer (mouse α fetoprotein enhancer/mouse albumin promoter) and having the nucleotide sequence represented by SEQ ID NO: 121 can be suitably used as a gene expression regulatory site. A chick β actin/MVM chimeric intron having the nucleotide sequence represented by SEQ ID NO: 122 may be disposed downstream of the mouse α fetoprotein enhancer/mouse albumin promoter. The expression level of the protein regulated by the gene expression regulatory site can be increased by disposing such an intron. Note that, in the nucleotide sequence represented by SEQ ID NO: 121, the nucleotide sequence from the 1st to the 219th corresponds to the mouse α fetoprotein enhancer, and the nucleotide sequence from the 241st to the 549th corresponds to the mouse albumin promoter.

The gene regulatory site may be a promoter for a gene that is organ-specifically or cell-type-specifically expressed. Use of an organ-specific expression promoter or cell-type-specific expression promoter allows a gene encoding a fusion protein and integrated in a nucleic acid molecule to be specifically expressed in a desired organ or cell.

A nucleic acid molecule in an embodiment of the present invention is that containing a gene encoding a conjugate of a fusion protein with an antibody between a first inverted terminal repeat (ITR) and a second inverted terminal repeat (ITR). Here, the fusion protein is, for example, a fusion protein of HSA and hGALC, a fusion protein of HSA and hGBA, a fusion protein of HSA and hIL-10, a fusion protein of HSA and hBDNF, a fusion protein of HSA and hNGF, a fusion protein of HSA and hNT-3, or a fusion protein of HSA and hNT-4, and the antibody is, for example, an anti-transferrin receptor antibody. The following (1) to (4) are examples of the nucleic acid molecule:
(1) a nucleic acid molecule containing a gene encoding a conjugate of a fusion protein with an antibody between a first inverted terminal repeat (ITR) and a second inverted terminal repeat (ITR), wherein the gene contains a nucleotide sequence encoding an internal ribosome entry site downstream of a gene encoding a light chain of the antibody and contains downstream thereof a nucleotide sequence encoding the conjugate in which the fusion protein is bound to the C terminal side or N terminal side of a heavy chain of the antibody, directly or via a linker;
(2) a nucleic acid molecule containing a gene encoding a conjugate of a fusion protein with an antibody between a first inverted terminal repeat (ITR) and a second inverted terminal repeat (ITR), wherein the gene contains a nucleotide sequence encoding an internal ribosome entry site downstream of a gene encoding the conjugate in which the fusion protein is bound to the C terminal side or N terminal side of a heavy chain of the antibody, directly or via a linker, and contains downstream thereof a nucleotide sequence encoding a light chain of the antibody;
(3) a nucleic acid molecule containing a gene encoding a conjugate of a fusion protein with an antibody between a first inverted terminal repeat (ITR) and a second inverted terminal repeat (ITR), wherein the gene contains a nucleotide sequence encoding an internal ribosome entry site downstream of a gene encoding a heavy chain of the antibody and contains downstream thereof a nucleotide sequence encoding the conjugate in which the fusion protein is bound to the C terminal side or N terminal side of a light chain of the antibody, directly or via a linker; and
(4) a nucleic acid molecule containing a gene encoding a conjugate of a fusion protein with an antibody between a first inverted terminal repeat (ITR) and a second inverted terminal repeat (ITR), wherein the gene contains a nucleotide sequence encoding an internal ribosome entry site downstream of a gene encoding the conjugate in which the fusion protein is bound to the C terminal side or N terminal side of a light chain of the antibody, directly or via a linker, and contains downstream thereof a nucleotide sequence encoding a heavy chain of the antibody.

In any of (1) to (4) in the above, the nucleic acid molecule may further contain a nucleotide sequence containing a gene expression regulatory site between the first inverted terminal repeat (ITR) and the gene encoding the conjugate of a fusion protein with an antibody. Also, in any of (1) to (4) in the above, the nucleotide sequence encoding an internal ribosome entry site may be replaced with a nucleotide sequence containing a gene expression regulatory site. Note that, the configuration is not limited to this, and in a case where the nucleic acid molecule has two gene expression regulatory sites, they are referred to as a first gene expression regulatory site and a first gene expression regulatory site in order from the first inverted terminal repeat (ITR) side for convenience. Also, in any of (1) to (4) in the above, the nucleotide sequence encoding an internal ribosome entry site may be replaced with a nucleotide sequence encoding 2A self-cleaving peptide. Porcine teschovirus-derived 2A peptide is a preferable example of 2A self-cleaving peptide.

A nucleic acid molecule in an embodiment of the present invention is that containing a gene encoding a conjugate of a fusion protein with an antibody between a first long terminal repeat (LTR) and a second long terminal repeat (LTR). Here, the fusion protein is, for example, a fusion protein of HSA and hGALC, a fusion protein of HSA and hGBA, a fusion protein of HSA and hIL-10, a fusion protein of HSA and hBDNF, a fusion protein of HSA and hNGF, a fusion protein of HSA and hNT-3, or a fusion protein of HSA and hNT-4, and the antibody is, for example, an anti-transferrin receptor antibody. The following (1) to (4) are examples of the nucleic acid molecule:
(1) a nucleic acid molecule containing a gene encoding a conjugate of a fusion protein with an antibody between a first long terminal repeat (LTR) and a second long terminal repeat (LTR), wherein the gene contains a nucleotide sequence encoding an internal ribosome entry site downstream of a gene encoding a light chain of the antibody and contains downstream thereof a nucleotide sequence encoding the conjugate in which the fusion protein is bound to the C terminal side or N terminal side of a heavy chain of the antibody, directly or via a linker;
(2) a nucleic acid molecule containing a gene encoding a conjugate of a fusion protein with an antibody between a first long terminal repeat (LTR) and a second long terminal repeat (LTR), wherein the gene contains a gene encoding an internal ribosome entry site downstream of a gene encoding the conjugate in which the fusion protein is bound to the C terminal side or N terminal side of a heavy chain of the antibody, directly or via a linker, and contains downstream thereof a nucleotide sequence encoding a light chain of the antibody;
(3) a nucleic acid molecule containing a gene encoding a conjugate of a fusion protein with an antibody between a first long terminal repeat (LTR) and a second long terminal repeat (LTR), wherein the gene contains a nucleotide sequence encoding an internal ribosome entry site downstream of a gene encoding a heavy chain of the antibody and contains downstream thereof a nucleotide sequence encoding the conjugate in which the fusion protein is bound to the C terminal side or N terminal side of a light chain of the antibody, directly or via a linker; and
(4) a nucleic acid molecule containing a gene encoding a conjugate of a fusion protein with an antibody between a first long terminal repeat (LTR) and a second long terminal repeat (LTR), wherein the gene contains a nucleotide sequence encoding an internal ribosome entry site downstream of a gene encoding the conjugate in which the fusion protein is bound to the C terminal side or N terminal side of a light chain of the antibody, directly or via a linker, and contains downstream thereof a nucleotide sequence encoding a heavy chain of the antibody.

In any of (1) to (4) in the above, the nucleic acid molecule may further contain a nucleotide sequence containing a gene expression regulatory site between the first long terminal repeat (LTR) and the gene encoding the conjugate of a fusion protein with an antibody. Also, in any of (1) to (4) in the above, the nucleotide sequence encoding an internal ribosome entry site may be replaced with a nucleotide sequence containing a gene expression regulatory site. Note that, the configuration is not limited to this, and in a case where the nucleic acid molecule has two gene expression regulatory sites, they are referred to as a first gene expression regulatory site and a first gene expression regulatory site in order from the first inverted terminal repeat (ITR) side for convenience. Also, in any of (1) to (4) in the above, the nucleotide sequence encoding an internal ribosome entry site may be replaced with a nucleotide sequence encoding 2A self-cleaving peptide. Porcine teschovirus-derived 2A peptide is a preferable example of 2A self-cleaving peptide.

In the nucleic acid molecule containing a nucleotide sequence encoding an internal ribosome entry site mentioned above, expression of one peptide chain constituting the fusion protein is regulated by the gene expression regulatory site and expression of the other peptide chain is regulated by the nucleotide sequence encoding an internal ribosome entry site. The two peptide chains pair in a cell and form a conjugate of a fusion protein with an antibody.

A nucleic acid molecule in an embodiment of the present invention can be used in an AAV vector system. When wild-type AAV singly infects a human cell as a host cell, the viral genome is integrated in chromosome 19 in a site-specific manner via the inverted terminal repeats (ITRs) present both ends of the viral genome. Genes in the viral genome integrated in the genome of the host cell are hardly expressed, but when the cell is infected with a helper virus, AAV is cleaved from the host genome, and replication of the infectious virus is initiated. In a case where the helper virus is adenovirus, the genes responsible for the helper action are E1A, E1B, E2A, VA1, and E4. In a case where the host cell is an HEK293 cell, which is a human embryonic kidney tissue-derived cell, transformed with E1A and E1B of adenovirus, the E1A and E1B genes are originally expressed in the host cell.

The wild-type AAV genome contains two genes: rep and cap. rep proteins (rep78, rep68, rep52, and rep40) produced from the rep gene are essential for capsid formation, and involved in chromosomal integration of the viral genome. The cap gene is responsible for producing three capsid proteins (VP1, VP2, and VP3).

In the genome of wild-type AAV, an ITR is present at each end, and the rep gene and the cap gene are present between the ITRs. This genome is encapsulated in a capsid to form an AAV virion. A recombinant AAV virion (rAAV virion) is such a virion that a region containing the rep gene and the cap gene is replaced with a gene encoding an exogenous protein and the resultant is encapsulated in a capsid.

rAAV virions are used for treatment as a vector to deliver an exogenous gene to cells.

For producing recombinant AAV virions that are used for introducing an exogenous gene into a cell, tissue, or living body, three plasmids are typically used: (1) a plasmid having a structure containing a nucleotide sequence containing a first inverted terminal repeat (ITR) derived from a virus such as AAV, a nucleotide sequences containing a second inverted terminal repeat (ITR), and a gene encoding a protein of interest disposed between the two ITRs (plasmid 1), (2) a plasmid containing an AAV Rep gene, which has functions necessary for integrating the nucleotide sequence of a region sandwiched between the ITR sequences (including the ITR sequences) in the genome of a host cell, and a gene encoding capsid protein of AAV (plasmid 2); and (3) a plasmid containing an E2A region, E4 region, and VA1 RNA region of adenovirus (plasmid 3). However, the configuration is not limited to this, and two plasmids, specifically, a plasmid obtained by linking two of plasmids 1 to 3 to integrate into one plasmid and the residual plasmid can be used. Further, a plasmid obtained by linking plasmids 1 to 3 to integrate into one plasmid can be used. A protein of interest in an embodiment of the present invention is a conjugate of a fusion protein with an antibody. Here, the fusion protein is, for example, a fusion protein of HSA and hGALC, a fusion protein of HSA and hGBA, a fusion protein of HSA and hIL-10, a fusion protein of HSA and hBDNF, a fusion protein of HSA and hNGF, a fusion protein of HSA and hNT-3, or a fusion protein of HSA and hNT-4, and the antibody is, for example, an anti-transferrin receptor antibody.

In typical production of recombinant AAV virions, those three plasmids are first introduced to a host cell, such as an HEK293 cell, having the E1a gene and Elb gene of adenovirus integrated in the genome with a common transfection technique. Then, a region containing a nucleotide sequence containing a first inverted terminal repeat (ITR), a nucleotide sequence containing a second inverted terminal repeat (ITR), and a gene encoding a protein of interest and disposed between the two ITRs is replicated in the host cell, the resulting single-stranded DNA is packaged in capsid protein of AAV, and as a result a recombinant virion is formed. Having infectivity, this recombinant AAV virion can be used for introducing an exogenous gene into a cell, tissue, or living body. An exogenous gene in an embodiment of the present invention is a gene encoding a conjugate of a fusion protein with an antibody. In a cell or the like to which the gene has been introduced, the conjugate comes to be expressed from the gene. Here, the fusion protein is, for example, a fusion protein of HSA and hGALC, a fusion protein of HSA and hGBA, a fusion protein of HSA and hIL-10, a fusion protein of HSA and hBDNF, a fusion protein of HSA and hNGF, a fusion protein of HSA and hNT-3, or a fusion protein of HSA and hNT-4, and the antibody is, for example, an anti-transferrin receptor antibody.

Rep protein of adeno-associated virus (AAV) is encoded by the rep gene of AAV. The Rep protein has functions, for example, necessary for integrating the AAV genome into the genome of a host cell via ITRs present in the AAV genome. There is a plurality of subtypes for the Rep protein, and two subtypes, Rep68 and Rep78, are needed for integrating the AAV genome in the genome of a host cell. Rep68 and Rep78 are translation products from two types of mRNA given by transcription from the same gene through selective splicing. The phrase of Rep protein of AAV is meant to include at least the two proteins: Rep68 and Rep78.

In an embodiment of the present invention, the phrase of a nucleotide sequence encoding Rep protein of adeno-associated virus (the nucleotide sequence of the Rep region) means a nucleotide sequence encoding at least Rep68 and Rep78, or a nucleotide sequence obtained by introducing a mutation thereto. The Rep protein is preferably that of AAV serotype 2, but is not limited thereto, and may be that of any of serotypes 1, 3, 4, 5, 6, 7, 8, 9, 10, and 11. A preferable example of the nucleotide sequence encoding Rep protein of wild-type AAV serotype 2 is that having the nucleotide sequence represented by SEQ ID NO: 123.

The Rep68 may be a mutant obtained by introducing modification such as substitution, deletion, and addition to the amino acid sequence of wild-type Rep68 of AAV of any of serotypes 1, 2, 3, 4, 5, 6, 7, 8, 9, 10, and 11, as long as the mutant exerts the functions of Rep68. Also, such a Rep68 mutant obtained by introducing a mutation to the Rep68 is included in the Rep68.

In a case where an amino acid in the amino acid sequence of wild-type Rep68 is substituted with another amino acid, the number of amino acids to be substituted is preferably 1 to 10, more preferably 1 to 5, and further preferably 1 to 3. In a case where an amino acid in the amino acid sequence of wild-type Rep68 is deleted, the number of amino acids to be deleted is preferably 1 to 10, more preferably 1 to 5, and further preferably 1 to 3. Also, a mutant obtained by introducing a mutation having the substitution and deletion of amino acids in combination to the Rep68 is regarded as Rep68. In a case where an amino acid is added, preferably 1 to 10, more preferably 1 to 5, or further preferably 1 to 3 amino acids are added in the amino acid sequence of wild-type Rep68 or added to the N terminal or C terminal thereof. A mutant obtained by introducing a mutation having the addition, substitution, and deletion of amino acids in combination to the Rep68 is also included in the Rep68. The amino acid sequence of Rep68 having a mutation introduced thereto exhibits an identity of preferably 80% or more, more preferably 85% or more, further preferably 90% or more, further preferably 95% or more, or further preferably 98% or more to the amino acid sequence of wild-type Rep68.

The Rep78 may be a mutant obtained by introducing modification such as substitution, deletion, and addition to the amino acid sequence of wild-type Rep78 of AAV of any of serotypes 1, 2, 3, 4, 5, 6, 7, 8, 9, 10, and 11, as long as the mutant exerts the functions of Rep78. Also, such a Rep78 mutant obtained by introducing a mutation to the Rep78 is included in the Rep78.

In a case where an amino acid in the amino acid sequence of wild-type Rep78 is substituted with another amino acid, the number of amino acids to be substituted is preferably 1 to 10, more preferably 1 to 5, and further preferably 1 to 3. In a case where an amino acid in the amino acid sequence of wild-type Rep78 is deleted, the number of amino acids to be deleted is preferably 1 to 10, more preferably 1 to 5, and further preferably 1 to 3. Also, a mutant obtained by introducing a mutation having the substitution and deletion of amino acids in combination to the Rep78 is regarded as Rep78. In a case where an amino acid is added, preferably 1 to 10, more preferably 1 to 5, or further preferably 1 to 3 amino acids are added in the amino acid sequence of wild-type Rep78 or added to the N terminal or C terminal thereof. A mutant obtained by introducing a mutation having the addition, substitution, and deletion of amino acids in combination to the Rep78 is also included in the Rep78. The amino acid sequence of Rep78 having a mutation introduced thereto exhibits an identity of preferably 80% or more, more preferably 85% or more, further preferably 90% or more, further preferably 95% or more, or further preferably 98% or more to the amino acid sequence of wild-type Rep78.

A functional equivalent of Rep protein of AAV is, for Rep68, a product that can be used in place of Rep68 in terms of functions, and, for Rep78, is a product that can be used in place of Rep78 in terms of functions. A functional equivalent of Rep protein may be a mutant of wild-type Rep protein.

Modification such as substitution, deletion, and addition can be introduced to the nucleotide sequence represented by SEQ ID NO: 123 as long as the resulting nucleotide sequence encodes functional Rep68 and Rep78. In a case where a nucleotide in the nucleotide sequence represented by SEQ ID NO: 123 is substituted with another nucleotide, the number of nucleotides to be substituted is preferably 1 to 20, more preferably 1 to 10, and further preferably 1 to 3. In a case where a nucleotide in the nucleotide sequence represented by SEQ ID NO: 123 is deleted, the number of nucleotides to be deleted is preferably 1 to 20, more preferably 1 to 10, and further preferably 1 to 3. Also, a mutant obtained by introducing a mutation having the substitution and deletion of nucleotides in combination can be used as a nucleotide sequence encoding Rep protein. In a case where a nucleotide is added, preferably 1 to 20, more preferably 1 to 10, or further preferably 1 to 3 nucleotides are added in the nucleotide sequence represented by SEQ ID NO: 123 or added to the 5' terminal or 3' terminal thereof. A mutant obtained by introducing a mutation having the addition, substitution, and deletion of nucleotides in combination can also be used as a nucleic acid molecule encoding Rep protein. The nucleotide sequence having a mutation introduced hereto exhibits an identity of preferably 80% or more, more preferably 85% or more, further preferably 90% or more, further preferably 95% or more, or further preferably 98% or more to the nucleotide sequence represented by SEQ ID NO: 123. It should be noted that, in a case where such a mutation is introduced, the start codon of the gene encoding the Rep proteins Rep68 and Rep78 is preferably ACG.

In an embodiment of the present invention, the phrase of a nucleotide sequence encoding Cap protein of adeno-associated virus (the nucleotide sequence of the Cap region) means a nucleotide sequence containing a nucleotide sequence at least encoding VP1, which is one of proteins constituting the capsid of AAV, or containing a nucleotide sequence obtained by introducing a mutation thereto. The VP1 is preferably that of AAV serotype 8, but is not limited thereto, and may be that of any of serotypes 1, 2, 3, 4, 5, 6, 7, 8, 9, 10, and 11. Preferable examples of the nucleotide sequence of the Cap region of serotype 8 include the nucleotide sequence represented by SEQ ID NO: 124.

The VP1 may be a mutant obtained by introducing modification such as substitution, deletion, and addition to the amino acid sequence of wild-type VP1 of AAV of any of serotypes 1, 2, 3, 4, 5, 6, 7, 8, 9, 10, and 11, as long as the mutant exerts the functions of VP1. In an embodiment of the present invention, such a VP1 mutant obtained by introducing a mutation to the VP1 is also included in the VP1.

In a case where a nucleotide in the nucleotide sequence represented by SEQ ID NO: 124 is substituted with another nucleotide, the number of nucleotides to be substituted is preferably 1 to 20, more preferably 1 to 10, and further preferably 1 to 3. In a case where a nucleotide in the nucleotide sequence represented by SEQ ID NO: 124 is deleted, the number of nucleotides to be deleted is preferably 1 to 20, more preferably 1 to 10, and further preferably 1 to 3. Also, a mutant obtained by introducing a mutation having the substitution and deletion of nucleotides in combination can be used as a nucleotide sequence encoding Cap protein. In a case where a nucleotide is added, preferably 1 to 20, more preferably 1 to 10, or further preferably 1 to 3 nucleotides are added in the nucleotide sequence represented by SEQ ID NO: 124 or added to the 5' terminal or 3' terminal thereof. A mutant obtained by introducing a mutation having the addition, substitution, and deletion of nucleotides in combination can also be used as a nucleic acid molecule encoding Cap protein. The nucleotide sequence having a mutation introduced thereto exhibits an identity of preferably 80% or more, more preferably 85% or more, further preferably 90% or more, further more preferably 95% or more, or further preferably 98% or more to the nucleotide sequence represented by SEQ ID NO: 124.

With an AAV vector, a recombinant AAV virion having a packaged nucleic acid molecule containing an exogenous gene between a first ITR and a second ITR can be obtained. Having infectivity, the recombinant AAV virion can be used for introducing an exogenous gene into a cell, tissue, or living body. An exogenous gene in an embodiment of the present invention is a gene encoding a conjugate of a fusion protein with an antibody. Here, the fusion protein is, for example, a fusion protein of HSA and hGALC, a fusion protein of HSA and hGBA, a fusion protein of HSA and hIL-10, a fusion protein of HSA and hBDNF, a fusion protein of HSA and hNGF, a fusion protein of HSA and hNT-3, or a fusion protein of HSA and hNT-4, and the antibody is, for example, an anti-transferrin receptor antibody. In a cell or the like to which the gene has been introduced, the fusion protein comes to be expressed from the gene. Recombinant virions having a packaged nucleic acid molecule containing an exogenous gene between a first ITR and a second ITR and being applicable for introducing a gene to a cell or the like include not only those with an AAV vector system but also those with an adenovirus vector system. With the adenovirus vector system, a recombinant adenovirus virion having a packaged nucleic acid molecule containing an exogenous gene between a first ITR and a second ITR is obtained. Having infectivity, the recombinant adenovirus virion can be used for introducing an exogenous gene into a cell, tissue, or living body.

Lentivirus is a virus belonging to the genus *lentivirus* in the subfamily Orthoretrovirinae, which is one of the subfamilies of the family Retroviridae, and has a single-chain (+)-strand RNA genome (ssRNA). The genome of lentivirus contains gag (a region encoding structural proteins including capsid protein), pol (a region encoding a group of enzymes including reverse transcriptase), and env (a region encoding envelope protein necessary for binding to a host cell) as essential genes, and these are sandwiched between two LTRs (5' LTR and 3' LTR). In addition to them, the genome of lentivirus contains, as auxiliary genes, Rev (a region encoding a protein having a function to bind to RRE (rev responsive element) present in viral RNA to transport the viral RNA from the nucleus to the cytoplasm), tat (a region encoding a protein having a function to bind to TAR in 5' LTR to increase the promoter activity of the LTR), and vif, vpr, vpu, nef, and so on.

Lentivirus is an envelope virus, and infects a cell through the fusion of the envelope with the cell membrane. In addition, lentivirus is an RNA virus, and reverse transcriptase is present in the virion. After infection with lentivirus, a single-chain positive-strand DNA is replicated from the (+)-strand RNA genome by the action of the reverse transcriptase, and a double-stranded DNA is further synthesized. From this double-stranded DNA, a protein as a constituent component of the virion is expressed, and the (+)-strand RNA genome is packaged therein; thus, the virion proliferates. In an embodiment of the present invention, the lentivirus vector is a product developed on the basis of the genome of HIV-1, which is one of lentiviruses, but is not limited thereto.

The first-generation lentivirus vector consists of three plasmids: a packaging plasmid, an Env plasmid, and an introduction plasmid. The packaging plasmid has genes of gag and pol under regulation by a CMV promoter and the like. The Env plasmid has an env gene under regulation by a CMV promoter. The introduction plasmid has a 5' LTR, RRE, a gene encoding a protein of interest under regulation by a CMV promoter, and a 3' LTR. By introducing these to a host cell with a common transfection technique, a recombinant virion having a nucleic acid molecule containing an exogenous gene between the first LTR and the second LTR and packaged in capsid protein is obtained. The packaging plasmid of the first-generation lentivirus vector contains virus-derived auxiliary genes: rev, tat, vif, vpr, vpu, and nef. Here, a protein of interest in an embodiment of the present invention is a conjugate of a fusion protein with an antibody. Here, the fusion protein is, for example, a fusion protein of HSA and hGALC, a fusion protein of HSA and hGBA, a fusion protein of HSA and hIL-10, a fusion protein of HSA and hBDNF, a fusion protein of HSA and hNGF, a fusion protein of HSA and hNT-3, or a fusion protein of HSA and hNT-4, and the antibody is, for example, an anti-transferrin receptor antibody. Note that, promoters other than a CMV promoter, including an SV40 early promoter, a human elongation factor-1α (EF-1α) promoter, a human ubiquitin C promoter, an LTR promoter of Rous sarcoma virus, which is a retrovirus, a dihydrofolate reductase promoter, a β-actin promoter, a phosphoglycerate kinase (PGK) promoter, a mouse albumin promoter, a human albumin promoter, and human α-1 antitrypsin promoter, are preferable as the promoter to regulate the gene encoding the protein of interest. For example, a synthetic promoter having the nucleotide sequence represented by SEQ ID NO: 121, containing a mouse albumin promoter downstream of a mouse α fetoprotein enhancer (mouse α fetoprotein enhancer/mouse albumin promoter), can be used.

As with the case of the first-generation, the second-generation lentivirus vector consists of three plasmids: a packaging plasmid, an Env plasmid (envelope plasmid), and an introduction plasmid. However, the auxiliary genes vif, vpr, vpu, and nef, which are not essential genes, have been deleted from the packaging plasmid.

The third-generation lentivirus vector consists of four plasmids: a packaging plasmid, an Env plasmid (envelope plasmid), a Rev plasmid, and an introduction plasmid. In the third-generation, rev, which is present in the packaging plasmid in the second-generation, has been replaced with the Rev plasmid as an independent existence. In addition, tat has been deleted from the packaging plasmid. Further, TAR in the 5' LTR in the introduction plasmid has been replaced with a CMV promoter.

Having infectivity, the recombinant virion can be used for introducing an exogenous gene into a cell, tissue, or living body. An exogenous gene in an embodiment of the present invention is a gene encoding a conjugate of a fusion protein with an antibody. In a cell or the like to which the gene has been introduced, the conjugate comes to be expressed from the gene. Here, the fusion protein is, for example, a fusion protein of HSA and hGALC, a fusion protein of HSA and hGBA, a fusion protein of HSA and hIL-10, a fusion protein of HSA and hBDNF, a fusion protein of HSA and hNGF, a fusion protein of HSA and hNT-3, or a fusion protein of HSA and hNT-4, and the antibody is, for example, an anti-transferrin receptor antibody.

Retrovirus has a single-chain (+)-strand RNA genome (ssRNA). In the viral genome, genes of gag (encoding structural proteins including capsid protein), pol (encoding a group of enzymes including reverse transcriptase), env (encoding envelope protein necessary for binding to a host cell), and a packaging signal (Ψ) are encoded, and these are sandwiched between two long terminal repeats (LTRs, 5' LTR and 3' LTR). When retrovirus infects a host cell, the (+)-strand RNA is translocated together with reverse transcriptase into the cell, and reverse-transcribed into a duplex DNA.

Retrovirus vectors have been developed mainly on the basis of mouse leukemia virus, and, for the purpose of eliminating the pathogenicity for increased safety, their viral genomes have been divided to delete the self-replication competence with the infectivity kept. The first-generation retrovirus vector consists of a packaging plasmid (the viral genome with a packaging signal excluded therefrom) and an introduction plasmid (a packaging signal, part of gag, and an exogeneous gene with the ends sandwiched between a 5' LTR and a 3' LTR). Accordingly, the occurrence of homologous recombination in the gag sequence parts that are present in the packaging plasmid and the introduction plasmid in common leads to the appearance of retrovirus capable of self-replication, that is, an RC (replication-competent) virus. In the second-generation retrovirus vector, the LTR on the 3' side of the first-generation packaging plasmid has been replaced with a polyA addition signal. Because of this, the simultaneous occurrence of homologous recombination at two positions, in the gag sequence and upstream of the polyA addition signal, is needed for the generation of an RC virus, and the probability of the generation is very low, and thus increased safety has been achieved. The third-generation is constituted of three plasmids, in which the second-generation packaging plasmid has been further divided into a plasmid encoding gag/pol and a plasmid encoding env. Because of this, the simultaneous occurrence of homologous recombination at three positions is needed for the generation of an RC virus, and the probability of the generation is extremely low, and thus further increased safety has been achieved.

In typical production of recombinant retrovirus virions, those packaging plasmid and introduction plasmid are first introduced to a host cell with a common transfection technique. Then, a region containing a 5' LTR, a 3' LTR, and a gene encoding a protein of interest and disposed between the two LTRs is replicated in the host cell, the resulting single-chain (+)-strand RNA is packaged in capsid protein of retrovirus, and as a result a recombinant retrovirus virion is formed. Having infectivity, this recombinant retrovirus virion can be used for introducing an exogenous gene into a cell, tissue, or living body. An exogenous gene in an embodiment of the present invention is a gene encoding a conjugate of a fusion protein with an antibody. In a cell or the like to which the gene has been introduced, the conjugate comes to be expressed from the gene. Here, the fusion protein is, for example, a fusion protein of HSA and hGALC, a fusion protein of HSA and hGBA, a fusion protein of HSA and hIL-10, a fusion protein of HSA and hBDNF, a fusion protein of HSA and hNGF, a fusion protein of HSA and hNT-3, or a fusion protein of HSA and hNT-4, and the antibody is, for example, an anti-transferrin receptor antibody.

In an embodiment of the present invention, the nucleic acid molecule can be in an encapsulated form in a liposome, a lipid nanoparticle (LNP), or the like. The term liposome refers to a spherical vesicle having a lipid bilayer, and a liposome is mainly constituted of phospholipid, particularly of phosphatidylcholine. However, the liposome is not limited to this, and may be a product obtained by adding another lipid such as yolk phosphatidylethanolamine to a liposome as long as a lipid bilayer is formed. Since the cell membrane is mainly constituted of a phospholipid bilayer membrane, liposomes have an advantage of superior biocompatibility. The term lipid nanoparticle refers to a particle having a diameter of 10 nm to 1000 nm, typically approximately less than 200 nm, and containing lipid as a main component, and a lipid nanoparticle can encapsulate a hydrophobic (lipophilic) molecule and contains a biocompatible lipid such as triglyceride, diglyceride, monoglyceride, fatty acid, and steroid as a main constituent component. When a gene encapsulated in a liposome or lipid nanoparticle is administered into a living body, the gene is inferred to be incorporated into a cell, for example, through direct fusion with the cell membrane of the cell or endocytosis, and then localized into the nucleus, thus introduced into the cell. The gene transfer method with a liposome or lipid nanoparticle is superior to gene transfer with a viral vector with respect to the absence of limitation to the size of a gene to be introduced and high safety. A liposome, lipid nanoparticle, or the like encapsulating the nucleic acid molecule of the present invention can be used for introducing a gene for a conjugate of a fusion protein with an antibody into a cell, tissue, or living body. In a cell or the like to which the gene has been introduced, a fusion protein comes to be expressed from the gene. In the specification, the phrase "a liposome, lipid nanoparticle, or the like" is meant to include not only the liposomes and lipid nanoparticles mentioned above, but also polymer nanoparticles, micelles, emulsions, nanoemulsions, microspheres, nanospheres, microcapsules, nanocapsules, dendrimers, nanogels, metal nanoparticles, and all kinds of nano/microparticles that can be used as a drug delivery system (DDS).

The behavior of a nucleic acid molecule that is in the form of a plasmid, in an encapsulated form in a recombinant virus virion, or in an encapsulated form in a liposome, lipid nanoparticle, or the like and has been introduced into a cell, tissue, or living body in an embodiment of the present invention is shown below in (1) to (6) as examples. However, the behavior of the nucleic acid molecule is not limited to them.
(1) In an embodiment, the nucleic acid molecule is a single-chain (+)-strand RNA; when being introduced into a cell, a gene encoding a conjugate of a fusion protein with an antibody and contained in the nucleic acid molecule is translated, and the conjugate is expressed.
(2) In an embodiment, the nucleic acid molecule is a single-chain (+)-strand RNA; when being introduced into a cell, the nucleic acid molecule is reverse-transcribed to form a single-chain (+)-strand DNA, this DNA is then transcribed and translated, and the conjugate is expressed.
(3) In an embodiment, the nucleic acid molecule is a single-chain (+)-strand RNA or (-)-strand RNA; when being introduced into a cell, the nucleic acid molecule is reverse-transcribed to form a double-stranded DNA, this DNA is then transcribed and translated, and the conjugate is expressed.
(4) In an embodiment, the nucleic acid molecule is a single-chain (+)-strand or (-) RNA; when being introduced into a cell, the nucleic acid molecule is reverse-transcribed to form a double-stranded DNA, this DNA then undergoes random recombination or homologous recombination with the genome of the host cell to be integrated into the genome, the integrated DNA is transcribed and translated, and the conjugate is expressed.
(5) In an embodiment, the nucleic acid molecule is a single-chain (+)-strand DNA; when being introduced into a cell, the nucleic acid molecule is transcribed and translated, and the conjugate is expressed.
(6) In an embodiment, the nucleic acid molecule is a double-stranded DNA; when being introduced into a cell, the nucleic acid molecule is transcribed and translated, and the conjugate is expressed.

The nucleic acid molecule that is in the form of a plasmid, in an encapsulated form in a recombinant virus virion, or in an encapsulated form in a liposome, lipid nanoparticle, or the like can be introduced into a cell, tissue, or living body.

In a case where the destination of introduction of the nucleic acid molecule is the inside of a living body, the nucleic acid molecule is administered in the form of a plasmid, in an encapsulated form in a recombinant virus virion, or in an encapsulated form in a liposome, lipid nanoparticle, or the like by a parenteral means such as subcutaneous injection, intramuscular injection, and intravenous injection.

In a case where the destination of introduction of the nucleic acid molecule is a cell, the type of cell is not particularly limited, and, for example, any of a mesenchymal stem cell, a dental pulp-derived stem cell, a hematopoietic stem cell, an embryonic stem cell, an endothelial stem cell, a mammary stem cell, an intestinal stem cell, a hepatic stem cell, a pancreatic stem cell, a neural stem cell, and an iPS cell.

Since a cell having the nucleic acid molecule introduced therein comes to express a conjugate of a fusion protein with an antibody, this can be transplanted to a patient for treatment.

### Examples

The present invention is described in more detail below with reference to Examples, but it is not intended that the present invention be limited to Examples.

Examples 1 to 13 below are results of an experiment on a fusion protein of HSA and hGALC.

### [Example 1] Production of Wild-Type hGALC Expression Plasmid, HSA-hGALC Expression Plasmid, and hGALC-HSA Expression Plasmid

A DNA fragment having the nucleotide sequence represented by SEQ ID NO: 2, which contains a gene encoding wild-type hGALC having an amino acid sequence represented by SEQ ID NO: 1, was synthesized. The DNA fragment was subjected to restriction enzyme treatment with MluI and NotI (Takara Bio Inc.) and separated by agarose gel electrophoresis. After EtBr staining, a band containing the target DNA fragment was excised under UV irradiation to extract DNA from the gel using QIAEX II Gel Extraction Kit (QIAGEN). Similarly, pCI-neo vector (Promega Corporation) was subjected to restriction enzyme treatment with MluI and NotI, followed by gel extraction and purification. Each DNA fragment treated with the restriction enzymes was mixed with each vector treated with the restriction enzymes, and ligation reaction was performed at 16°C for 30 to 60 minutes using Ligation high Ver.2 (Toyobo Co., Ltd.) to incorporate the DNA fragment into a pCI vector. A DNA fragment containing the nucleotide sequence represented by SEQ ID NO: 6, which contains a gene encoding HSA-hGALC represented by SEQ ID NO: 5, a fusion protein formed by linking the C terminal of wild-type HSA (SEQ ID NO: 3) and the N terminal of wild-type hGALC (SEQ ID NO: 1) via a linker sequence Gly-Ser, and a DNA fragment containing the nucleotide sequence represented by SEQ ID NO: 8, which contains a gene encoding hGALC-HSA represented by SEQ ID NO: 7, a fusion protein formed by linking the C terminal of wild-type hGALC (SEQ ID NO: 1) and the N terminal of wild-type HSA (SEQ ID NO: 3) via a linker sequence Gly-Ser, were each similarly subjected to restriction enzyme treatment, extraction, purification, and then ligation reaction to incorporate each DNA fragment into a pCI vector.

Next, each ligation reaction solution was used to transform *Escherichia coli* (*E. coli* DH5α Competent Cells, Takara Bio Inc.). To confirm whether the obtained transformant retained the target plasmid DNA, a single colony was cultured overnight in an LB liquid medium (LB Broth, Sigma-Aldrich), and plasmid DNA was purified from the bacterial cells using FastGene Plasmid Mini Kit (NIPPON Genetics Co, Ltd.). The purified plasmid DNA was subjected to restriction enzyme treatment with MluI and NotI and separated by agarose gel electrophoresis to confirm insertion of the target insert DNA. Each plasmid confirmed to incorporate wild-type hGALC, HSA-hGALC, and hGALC-HSA was purified by a conventional method.

### [Example 2] Transient Expression of Wild-Type hGALC, HSA-hGALC, and hGALC-HSA

Transient expression of wild-type hGALC, HSA-hGALC, and hGALC-HSA was performed using a plasmid incorporating a gene encoding each of wild-type hGALC, HSA-hGALC, and hGALC-HSA into the purified pCI-neo vector obtained in Example 1.

According to the high titer protocol of ExpiCHO(TM) Expression System (Thermo Fisher Scientific Inc.), ExpiCHO cells were transformed with a plasmid incorporating the gene encoding each of wild-type hGALC, HSA-hGALC, and hGALC-HSA. After transformation, the cells were cultured for 8 days to express each of wild-type hGALC, HSA-hGALC, and hGALC-HSA in a culture supernatant. After 6, 7, and 8 days from the start of culture, the culture solution was centrifuged to recover the culture supernatant.

### [Example 3] Confirmation of Expression Level of Wild-Type hGALC, HSA-hGALC, and hGALC-HSA by Transient Expression (SDS Page Electrophoresis)

Each 10 µL of the culture supernatant obtained in Example 2 was mixed with 8 µL of 2× Sample Buffer (Bio-Rad Laboratories, Inc.) and 2 µL of 2-mercaptoethanol and heat-denatured under reducing conditions by heating at 100°C for 3 minutes. The heat-denatured solution was added in 5 µL portions to each well of a 5 to 20% polyacrylamide gel placed in 50 mM Tris buffer/380 mM glycine buffer (pH 8.3) containing 0.1% SDS, followed by electrophoresis at a constant current of 25 mA. After electrophoresis, the gel was immersed in Oriole fluorescent gel stain (Bio-Rad Laboratories, Inc.) and shaken at room temperature for 90 minutes. After washing the gel with pure water, an electrophoresis image of the band corresponding to each protein visualized was obtained using a lumino image analyzer (Amersham Imager 600RGB, Cytiva).

### [Example 4] Confirmation of Expression Level of Wild-Type hGALC, HSA-hGALC, and hGALC-HSA by Transient Expression (Western Blotting)

Electrophoresis was performed in the same manner as in Example 3, a nitrocellulose membrane and the gel after electrophoresis were sandwiched between blotting paper immersed in 25 mM Tris buffer/192 mM glycine buffer containing 20% methanol, and the protein was transferred to the nitrocellulose membrane by energizing a current of 1.0 A and 25 V for 10 minutes in a blotting device. The transferred nitrocellulose membrane was immersed in PBST containing 5% skim milk and shaken for 1 hour, then immersed in an Anti-GALC antibody (Rabbit polyclonal to GALC, abcam) diluted to 0.4 µg/mL and shaken for 1 hour. After washing the membrane with PBST, the membrane was immersed in a solution of Anti-mouse IgG (H+L), HRP Conjugate (Promega Corporation) diluted to 0.4 µg/mL, shaken for 30 minutes, and washed again with PBST. An HRP detection reagent (Bio-Rad Laboratories, Inc.) was added dropwise to the transfer surface of the membrane and allowed to react for 5 minutes, thereby obtaining a Western blotting image of the band corresponding to each protein visualized using a lumino image analyzer (Amersham Imager 600RGB, Cytiva).

### [Example 5] Confirmation of Expression Level of Wild-Type hGALC, HSA-hGALC, and hGALC-HSA by Transient Expression (Enzyme Activity Measurement)

A sample solution was prepared by diluting the culture supernatant obtained in Example 2 100-fold with citrate buffer (pH 4.5) containing 0.5% Triton X-100. The sample solution was further appropriately diluted as needed for the measurement. A standard solution was prepared by serially diluting 4-MU (4-methylumbelliferone, Sigma-Aldrich) from 75 to 4.39 µM with 100 mM citrate buffer (pH 4.2) containing 0.1% BSA. A substrate solution was prepared by diluting 4-methylumbelliferyl-β-D-galactopyranoside (Sigma-Aldrich), an artificial substrate of hGALC, to 1 mmol/L with citrate buffer (pH 4.5) containing 0.5% Triton X-100. The sample solution or standard solution was added to a microplate at 25 µL/well, and then 25 µL of the substrate solution was added to each well and mixed by stirring with a plate shaker. The plate was allowed to stand at 37°C for 1 hour, and then 150 µL of 200 mmol/L glycine-NaOH buffer (pH 10.6) was added to each well to stop the reaction. The fluorescence intensity of each well was then measured with a fluorescence plate reader (Gemini XPS, Molecular Devices, LLC.) (excitation wavelength: 365 nm, fluorescence wavelength: 460 nm). The fluorescence intensity is proportional to the concentration of 4-MU (4-methylumbelliferone) contained in the solution in each well. A calibration curve was created based on the measurement results of the standard solution, and the measurement value of each sample solution was interpolated to determine enzyme activity levels (µM/h). Note that the unit of enzyme activity levels is the amount of substrate decomposed per hour.

### [Example 6] Confirmation of Expression Level of Wild-Type hGALC, HSA-hGALC, and hGALC-HSA by Transient Expression

### (Result)

Figure 15(a) shows results of enzyme activity measurement of wild-type hGALC, HSA-hGALC, and hGALC-HSA by transient expression measured in Example 5. In addition, the relative amounts of wild-type hGALC, HSA-hGALC, and hGALC-HSA (relative amounts when the expression level of wild-type hGALC on each measurement day was defined as 1.0) based on the results of enzyme activity measurement are shown in Table 1.

**[Table 1]**

| **Table 1. Expression level of wild-type hGALC and fusion protein by transient expression (relative amount to expression level of wild-type hGALC on each measurement day defined as 1)** | | |
|---|---|---|
| **Days after start of culture** | **Expression protein** | **Expression level (average)** |
| **Day6** | **Wild-type hGALC** | **1.0** |
| | **HSA-HGALC** | **3.2** |
| | **hGALC-HSA** | **1.5** |
| **Day7** | **Wild-type hGALC** | **1.0** |
| | **HSA-hGALC** | **2.9** |
| | **hGALC-HSA** | **2.0** |
| **Day8** | **Wild-type hGALC** | **1.0** |
| | **HSA-hGALC** | **3.7** |
| | **hGALC-HSA** | **2.1** |

These results showed that the expression levels of HSA-hGALC and hGALC-HSA were 3.2 and 1.5 times higher, respectively, than that of wild-type hGALC, when the expression levels (converted to enzyme activity levels) were examined 6 days after the start of culture and revealed that hGALC, which has low expression levels and is difficult to produce in large quantities when expressed as a recombinant in its wild-type form, could be efficiently expressed in the form of HSA-hGALC or hGALC-HSA to express more hGALC as a recombinant protein. The same applies to 7 days and 8 days after the start of culture.

The electrophoresis image and the Western blotting image obtained in Example 3 are shown in Figures 15(b) and (c), respectively. From the Western blotting image, bands corresponding to wild-type hGALC, HSA-hGALC, and hGALC-HSA on the electrophoresis image were identified. The expression levels (in terms of the number of molecules) of the wild-type hGALC, the portion corresponding to hGALC of HSA-hGALC, and the portion corresponding to hGALC of hGALC-HSA, which were estimated from the intensity of the band of the Western blotting image, were substantially correlated with the expression levels (in terms of the enzyme activity levels) shown in Figure 15(a) and Table 1. That is, the relative value of the expression level shown as the expression level (in terms of enzyme activity levels) in Table 1 can be regarded as the expression level (in terms of the number of molecules) of the wild-type hGALC, the portion corresponding to hGALC of HSA-hGALC, and the portion corresponding to hGALC of hGALC-HSA as it is. These results indicate that hGALC, which is difficult to produce in large quantities as a recombinant in the wild type form, can be expressed in a larger number of molecules in the form of a fusion protein with HSA without decreasing the specific activity. Note that the specific activity of hGALC in the fusion protein is calculated by multiplying the enzyme activity of hGALC per unit mass of the fusion protein (µM/hour/mg protein) by (molecular weight of the fusion protein/molecular weight of the portion corresponding to hGALC in the fusion protein).

### [Example 7] Survival Rate of Cells in Transient Expression of Wild-Type hGALC, HSA-hGALC, and hGALC-HSA, etc.

In the transient expression of wild-type hGALC, HSA-hGALC, and hGALC-HSA carried out in Example 2, the viable cell density and cell survival rate in the culture solution after 6, 7, and 8 days from the start of the culture of transformed ExpiCHO cells were measured using EVE Automated Cell Counter (NanoEntek). Table 2 shows results of the viable cell density and cell survival rate in the culture solution after 6, 7, and 8 days from the start of culture for each cell expressing wild-type hGALC, HSA-hGALC, and hGALC-HSA.

**[Table 2]**

| **Table 2. Viable cell density and survival rate of cell in transient expression** | | | | |
|---|---|---|---|---|
| **Days after start of culture** | | **Day6** | **Day7** | **Day8** |
| **Wild-type hGALC** | **Viable cell density (× 10⁶ cell/mL)** | **7.0** | **6.0** | **6.0** |
| | **Survival rate (%)** | **80** | **66** | **60** |
| **HSA-hGALC** | **Viable cell density (cell/mL)** | **6.2** | **6.4** | **5.8** |
| | **Survival rate (%)** | **88** | **85** | **89** |
| **hGALC-HSA** | **Viable cell density (cell/mL)** | **7.6** | **7.2** | **6.0** |
| | **Survival rate (%)** | **85** | **80** | **71** |

No significant difference in viable cell density was observed between cells expressing each of wild-type hGALC, HSA-hGALC, and hGALC-HSA. However, as for the cell survival rate, the cells expressing HSA-hGALC maintained a high survival rate of 80% or more from 6 days to 8 days after the start of culture, and the cells expressing hGALC-HSA maintained a cell survival rate of 70% or more while gradually decreasing from 6 days to 8 days after the start of culture, whereas the cells expressing wild-type hGALC greatly decreased the cell survival rate from 6 days to 8 days after the start of culture and declined to 60% 8 days after the start of culture. Assuming that the survival rate of the wild-type hGALC expression cells on each measurement day was 1.0, the relative ratios of the survival rates were 1.1 and 1.1 for the HSA-hGALC and hGALC-HSA expression cells, respectively, after 6 days from the start of culture, 1.3 and 1.2 for the HSA-hGALC and hGALC-HSA expression cells, respectively, after 7 days from the start of culture, and 1.5 and 1.2 for the HSA-hGALC and hGALC-HSA expression cells, respectively, after 8 days from the start of culture.

These results are considered to show that the wild-type hGALC, when expressed as a recombinant protein, gives stress to a host cell to decrease a survival rate thereof, and that the expression of wild-type hGALC as a fusion protein formed by binding HSA to the wild-type hGALC can reduce the stress given to the host cell by the wild-type hGALC, thereby suppressing a decrease in the survival rate. That is, one of the reasons for an increase in the expression level of HSA-hGALC or hGALC-HSA when expressed as a recombinant protein as compared with the wild-type hGALC shown in Example 6 is considered to be that the stress applied to the host cells when the wild-type hGALC was expressed was reduced, thereby suppressing the decrease in the survival rate of the cells.

### [Example 8] Summary

The above results indicate that, when hGALC is produced as a recombinant protein, hGALC can be produced in the form of a fusion protein of HSA-hGALC or hGALC-HSA to obtain hGALC with about 1.5 to 3.6 times the number of molecules without decreasing the specific activity of hGALC. That is, a method for producing recombinant hGALC by expressing hGALC in the form of a fusion protein of HSA-hGALC or hGALC-HSA can be concluded to be a very effective method for producing a large quantity of recombinant hGALC. In addition, since the contents flow out from dead cells and become contaminants, an increase in the number of dead cells during culture may make purification of the expressed fusion protein difficult. Therefore, expression of hGALC in the form of a fusion protein of HSA-hGALC or hGALC-HSA can suppress cell death during culture and facilitate purification of the expressed fusion protein. Thus, purification efficiency at the time of purification can be enhanced.

### [Example 9] SE-HPLC Analysis of Wild-Type hGALC, HSA-hGALC, and hGALC-HSA by Transient Expression

To evaluate the physical properties of wild-type hGALC, HSA-hGALC, and hGALC-HSA obtained by transient expression, the culture supernatant of each protein was subjected to a purification step, followed by SE-HPLC analysis.

### [Purification step]

To the culture supernatant on day 8 containing each of wild-type hGALC, HSA-hGALC, and hGALC-HSA by transient expression obtained in Example 2 was added 25 mM MES buffer (pH 6.5) containing an equal volume of 50 mM NaCl. This solution was loaded at a constant flow rate onto a Hitrap Q column (cytiva), a strong anion-exchange column equilibrated with 25 mM MES buffer (pH 6.5) containing 50 mM NaCl. The column was then washed with five column volumes of the same buffer at the same flow rate. Then, 25 mM MES buffer (pH 6.5) containing 500 mM NaCl was used to elute wild-type hGALC, HSA-hGALC and hGALC-HSA adsorbed onto the strong anion-exchange column with a salt concentration gradient.

### [SE-HPLC step]

TSKgel G3000SWXL 5 µm column, a size exclusion column chromatography column, (7.8 mm in diameter × 30 cm in length, TOSOH CORPORATION) was set on Shimadzu HPLC system LC-20A (Shimadzu Corporation). An absorptiometer was installed downstream of the column to measure the absorbance (measurement wavelength: 215 nm) of the effluent from the column continuously. After the column was equilibrated with 0.2 M aqueous sodium phosphate buffer solution containing 10% ethanol, eluates containing each of wild-type hGALC, HSA-hGALC and hGALC-HSA obtained in the purification step were loaded on the column, and the 0.2 M aqueous sodium phosphate buffer solution containing 10% ethanol was further allowed to flow at a flow rate of 0.5 mL/min. During this time, an elution profile was obtained by measuring the absorbance (Measurement wavelength: 215 nm) of the effluent from the column.

### [Evaluation of Physical Properties]

The elution profile is shown in Figure 16. The wild-type hGALC has two main peaks, which are considered to correspond to a tetramer of hGALC and a dimer of hGALC in this order from left to right on the elution profile. On the other hand, both HSA-hGALC and hGALC-HSA have one main peak, which is considered to be a peak of a monomer of HSA-hGALC or hGALC-HSA, respectively. This indicates that molecules with hGALC activity are stably obtained as monomers when the fusion protein of HSA and hGALC is expressed, compared with when wild-type hGALC is expressed in cells. In each of HSA-hGALC and hGALC-HSA, the mass ratio of the monomer to the total expression level is 90% or more. Since HSA-hGALC and hGALC-HSA can be obtained as homogeneous monomers when expressed as recombinant proteins, the method for producing hGALC, in which hGALC is produced as a fusion protein with HSA, can be concluded to be excellent also from the viewpoint of production management.

### [Example 10] Production of Plasmid Expressing Conjugate of Anti-hTfR Antibody and Fusion Protein of HSA and hGALC

Plasmids expressing the following four proteins were each produced as conjugates of an anti-hTfR antibody (Fab) consisting of a heavy chain having an amino acid sequence represented by SEQ ID NO: 25 and a light chain having an amino acid sequence represented by SEQ ID NO: 23, and a fusion protein of HSA and hGALC:
(1) a conjugate consisting of a fusion protein obtained by binding the N terminal of HSA-hGALC to the C terminal of the heavy chain of the anti-hTfR antibody (Fab) via a linker, and the light chain of the anti-hTfR antibody (Fab) (referred to as Fab-HSA-hGALC);
(2) a conjugate consisting of a fusion protein obtained by binding the C terminal of HSA-hGALC to the N terminal of the heavy chain of the anti-hTfR antibody (Fab) via a linker, and the light chain of the anti-hTfR antibody (Fab) (referred to as HSA-hGALC-Fab);
(3) a conjugate consisting of a fusion protein obtained by binding the N terminal of hGALC-HSA to the C terminal of the heavy chain of the anti-hTfR antibody (Fab) via a linker, and the light chain of the anti-hTfR antibody (Fab) (referred to as Fab-hGALC-HSA); and
(4) a conjugate consisting of a fusion protein obtained by binding the C terminal of hGALC-HSA to the N terminal of the heavy chain of the anti-hTfR antibody (Fab) via a linker, and the light chain of the anti-hTfR antibody (Fab) (referred to as hGALC-HSA-Fab).

### [Production of Dual (+)pCI-neo Vector]

A primer (pCI insert F3 primer) having the nucleotide sequence represented by SEQ ID NO: 26, which contains, from the 5' side, an NheI site, an AscI site, an NruI site, an AfeI site, and a NotI site into which a NotI inactivation sequence was inserted, was synthesized. In addition, a primer (pCI insert R3 primer) having the nucleotide sequence represented by SEQ ID NO: 27, which contains a sequence complementary thereto, was synthesized. These two primers were annealed to obtain a DNA fragment having protruding terminals complementary to NheI- and NotI-digested DNA at both ends.

A pCI vector (Promega Corporation) was digested with NheI and NotI (Takara Bio Inc.), into which the above-described DNA fragment was inserted by a ligation reaction. The resulting plasmid was designated as pCI MCS-modified vector (Figure 17). The pCI MCS-modified vector was digested with BamHI and BglII (Takara Bio Inc.), and a DNA fragment containing a CMV immediate-early enhancer/promoter region, a multicloning site, and a polyA sequence was excised. The pCI-neo vector (Promega Corporation) was subjected to BamHI digestion and CIAP (Takara Bio Inc.) treatment, and the excised DNA fragment was inserted by a ligation reaction. The resulting plasmid was designated as Dual(+)pCI-neo vector (Figure 18).

### [Production of Each Conjugate Expression Plasmid]

A DNA fragment containing the nucleotide sequence represented by SEQ ID NO: 29, which contains a gene encoding a conjugate (anti-hTfR antibody Fab heavy chain-HSA-hGALC) obtained by binding the N terminal of HSA-hGALC to the C terminal of the heavy chain of the anti-hTfR antibody (Fab) having an amino acid sequence represented by SEQ ID NO: 28 via a linker, was digested with restriction enzymes MluI and NotI (Takara Bio Inc.) and inserted between the MluI and NotI sites of the Dual(+)pCI-neo vector. Furthermore, the Dual(+)pCI-neo vector, into which the gene encoding the anti-hTfR antibody Fab heavy chain-HSA-hGALC had been inserted, was digested with restriction enzymes AscI and AfeI (New England BioLabs, Inc.), and a DNA fragment containing the nucleotide sequence represented by SEQ ID NO: 36, which contains the gene encoding the light chain of the anti-hTfR antibody (Fab) having an amino acid sequence represented by SEQ ID NO: 23 which had been digested with AscI and AfeI, was inserted into the vector. The resulting plasmid was designated as a Fab-HSA-hGALC-expression plasmid.

Similarly, an HSA-hGALC-Fab expression plasmid was produced using a DNA fragment containing the nucleotide sequence represented by SEQ ID NO: 31, which contains a gene encoding a conjugate obtained by binding the C terminal of HSA-hGALC to the N terminal of the heavy chain of the anti-hTfR antibody (Fab) having an amino acid sequence represented by SEQ ID NO: 30 via a linker, and a DNA fragment containing the nucleotide sequence represented by SEQ ID NO: 36, which contains a gene encoding the light chain of the anti-hTfR antibody (Fab) having an amino acid sequence represented by SEQ ID NO: 23.

Similarly, a Fab-hGALC-HSA expression plasmid was produced using a DNA fragment containing the nucleotide sequence represented by SEQ ID NO: 33, which contains a gene encoding a conjugate obtained by binding the N terminal of hGALC-HSA to the C terminal of the heavy chain of the anti-hTfR antibody (Fab) having an amino acid sequence represented by SEQ ID NO: 32 via a linker, and a DNA fragment containing the nucleotide sequence represented by SEQ ID NO: 36, which contains a gene encoding the light chain of the anti-hTfR antibody (Fab) having an amino acid sequence represented by SEQ ID NO: 23.

Similarly, an hGALC-HSA-Fab expression plasmid was produced using a DNA fragment containing the nucleotide sequence represented by SEQ ID NO: 35, which contains a gene encoding a conjugate obtained by binding the C terminal of hGALC-HSA to the N terminal of the heavy chain of the anti-hTfR antibody (Fab) having an amino acid sequence represented by SEQ ID NO: 34 via a linker, and a DNA fragment containing the nucleotide sequence represented by SEQ ID NO: 36, which contains a gene encoding the light chain of the anti-hTfR antibody (Fab) having an amino acid sequence represented by SEQ ID NO: 23.

### [Example 11] Transient Expression of Each Conjugate

According to the high titer protocol of ExpiCHO^{™} Expression System (Thermo Fisher Scientific Inc.), ExpiCHO cells were each transformed with each of the conjugate expression plasmids produced in Example 10. After transformation, the cells were cultured for 8 days to express each of Fab-HSA-hGALC, HSA-hGALC-Fab, Fab-hGALC-HSA, and hGALC-HSA-Fab in a culture supernatant. After 8 days from the start of culture, each culture solution was centrifuged to recover the culture supernatant.

### [Example 12] Confirmation of Expression Level of Fab-HSA-hGALC, HSA-hGALC-Fab, Fab-hGALC-HSA, and hGALC-HSA-Fab by Transient Expression (Enzyme Activity Measurement)

Using the culture supernatant obtained in Example 11 as a sample solution, the expression levels of Fab-HSA-hGALC, HSA-hGALC-Fab, Fab-hGALC-HSA, and hGALC-HSA-Fab by transient expression were confirmed by enzyme activity measurement in the same manner as in the method described in Example 5.

Figure 19 shows results of enzyme activity measurement of wild-type hGALC, Fab-HSA-hGALC, HSA-hGALC-Fab, Fab-hGALC-HSA, and hGALC-HSA-Fab. Note that the value of the result of the enzyme activity measurement of wild-type hGALC was the value of the measurement described in Example 2 obtained by using the culture supernatant of cells expressing wild-type hGALC 8 days after the start of culture as the sample solution. In addition, the relative amounts of wild-type hGALC, Fab-HSA-hGALC, HSA-hGALC-Fab, Fab-hGALC-HSA, and hGALC-HSA-Fab (relative amounts when the expression level of wild-type hGALC after 8 days from the start of culture was defined as 1.0) based on the results of enzyme activity measurement are shown in Table 3.

**[Table 3]**

| Table 3. Expression level of wild-type hGALC and each conjugate by transient expression (relative amount to expression level of wild-type hGALC 8 days after start of culture defined as 1) | | |
|---|---|---|
| Days after start of culture | Expression protein | Expression level (average) |
| Day8 | Wild-type hGALC | 1.0 |
| | Fab-HSA-hGALC | 3.8 |
| | HSA-hGALC-Fab | 2.3 |
| | Fab-hGALC-HSA | 3.0 |
| | hGALC-HSA-Fab | 1.7 |

These results showed that the expression levels of Fab-HSA-hGALC, HSA-hGALC-Fab, Fab-hGALC-HSA, and hGALC-HSA-Fab were about 3.8, 2.3, 3.0, and 1.7 times higher, respectively, than that of wild-type hGALC, according to the expression levels (converted to enzyme activity levels) 8 days after the start of culture, and revealed that more hGALC could be expressed as recombinant proteins by converting wild-type hGALC to HSA-hGALC or hGALC-HSA, even in the form of conjugates in which these are further bound to antibodies. Since all of Fab-HSA-hGALC, HSA-hGALC-Fab, Fab-hGALC-HSA, and hGALC-HSA-Fab, which are conjugates of an anti-hTfR antibody and a fusion protein of HSA and hGALC, with such a complex structure and a huge molecular weight as compared with the wild-type hGALC, were expected to be reduced in expression level as compared with the wild-type hGALC when expressed as recombinant proteins, the above results were unexpected.

### [Example 13] SE-HPLC Analysis of Fab-HSA-hGALC, HSA-hGALC-Fab, Fab-hGALC-HSA, and hGALC-HSA-Fab by Transient Expression

To evaluate the physical properties of Fab-HSA-hGALC, HSA-hGALC-Fab, Fab-hGALC-HSA, and hGALC-HSA-Fab obtained by transient expression, the culture supernatant of each protein was subjected to a purification step, followed by SE-HPLC analysis.

### [Purification Step]

To the culture supernatant on day 8 containing each of Fab-HSA-hGALC, HSA-hGALC-Fab, Fab-hGALC-HSA, and hGALC-HSA-Fab by transient expression obtained in Example 11 was added 0.1-fold volume of 2 M arginine solution (pH 6.5). This solution was loaded onto a Capture Select(TM) CH1-XL column (Thermo Fisher Scientific Inc.) equilibrated with 25 mM MES buffer (pH 6.5) containing five column volumes of 200 mM arginine to adsorb each conjugate onto the column. The Capture Select(TM) CH1-XL column is an affinity column in which a ligand with a property of specifically binding to the CH1 domain of an IgG antibody is immobilized on a carrier. The column was then washed with five column volumes of the same buffer. The column was then further washed with three column volumes of 25 mM MES buffer (pH 6.5). Each of the conjugates adsorbed onto the column was then eluted with five column volumes of 20 mM acetate buffer (pH 4.0). The eluate was immediately neutralized by placing it in a vessel containing 250 mM MES buffer (pH 6.0) and 2 M NaCl solution in advance.

### [SE-HPLC step]

TSKgel G3000SWXL 5 µm column, a size exclusion column chromatography column, (7.8 mm in diameter × 30 cm in length, TOSOH CORPORATION) was set on Shimadzu HPLC system LC-20A (Shimadzu Corporation). An absorptiometer was installed downstream of the column to measure the absorbance (measurement wavelength: 215 nm) of the effluent from the column continuously. After the column was equilibrated with 0.2 M aqueous sodium phosphate buffer solution containing 10% ethanol, eluates containing each of Fab-HSA-hGALC, HSA-hGALC-Fab, Fab-hGALC-HSA, and hGALC-HSA-Fab obtained in the purification step were loaded on the column, and the 0.2 M aqueous sodium phosphate buffer solution containing 10% ethanol was further allowed to flow at a flow rate of 0.5 mL/min. During this time, an elution profile was obtained by measuring the absorbance (Measurement wavelength: 215 nm) of the effluent from the column.

### [Evaluation of physical properties]

The elution profile is shown in Figure 20. All of Fab-HSA-hGALC, HSA-hGALC-Fab, Fab-hGALC-HSA, and hGALC-HSA-Fab have one main peak, which is considered to be a peak of a monomer of Fab-HSA-hGALC, HSA-hGALC-Fab, Fab-hGALC-HSA, or hGALC-HSA-Fab, respectively. In comparison with the elution profile of the wild-type hGALC obtained in Example 9 (Figure 16), in which two main peaks were observed, it can be seen that molecules with hGALC activity were stably obtained as monomers even when a conjugate of the fusion protein of HSA and hGALC and an antibody was expressed, compared with when wild-type hGALC was expressed in cells. That is, it was shown that even when the fusion protein of HSA and hGALC is further formed into a conjugate with an antibody, the fusion of HSA to hGALC contributes to stabilization of hGALC. In each of the conjugates, the mass ratio of the monomer to the total expression level is 90% or more. Since the conjugates can be obtained as homogeneous monomers when expressed as recombinant proteins, the method for producing hGALC, in which hGALC is produced as a conjugate of HSA and an antibody (Fab), can be concluded to be excellent also from the viewpoint of production management.

Examples 14 to 20 below are results of an experiment on a fusion protein of HSA and hGBA.

### [Example 14] Production of Wild-Type hGBA Expression Plasmid

The pE-neo7 vector and pCAGIPuro vector (Miyahara M. et. al., J. Biol. Chem. 275, 613-618 (2000)) were subjected to restriction enzyme treatment with BamHI and NotI (Takara Bio Inc.), respectively, and separated by agarose gel electrophoresis. Note that the pE-neo7 vector was produced by the method described in International Publication No. WO 2012/101998. After EtBr staining, a band containing the target DNA fragment was excised under UV irradiation to extract DNA from the gel using QIAEX II Gel Extraction Kit (QIAGEN). Ligation reaction was performed at 16°C for 60 minutes using Ligation high Ver. 2 to complete the pEI-puro vector.

A DNA fragment having the nucleotide sequence represented by SEQ ID NO: 38, which contains a gene encoding wild-type hGBA (SEQ ID NO: 37), was synthesized. The DNA fragment was subjected to restriction enzyme treatment with MluI and NotI (Takara Bio Inc.) and separated by agarose gel electrophoresis. After EtBr staining, a band containing the target DNA fragment was excised under UV irradiation to extract DNA from the gel using QIAEX II Gel Extraction Kit (QIAGEN).

The DNA fragment and the pEI-puro vector subjected to restriction enzyme treatment with MluI and NotI were mixed, and ligation reaction was performed at 16°C for 60 minutes using Ligation high Ver. 2 to complete the pEI-puro-hGBA vector.

pEI-puro-hGBA vector and pE-mIRES-GS vector were subjected to restriction enzyme treatment with MluI and NotI, followed by gel extraction and purification. Note that the pE-mIRES-GS vector was produced by the method described in International Publication No. WO 2013/161958. Each vector treated with the restriction enzymes was mixed, and ligation reaction was performed at 16°C for 60 minutes using Ligation high Ver. 2 to complete the pEmIGS-hGBA vector (Figure 21).

pEmIGS-hGBA vector and pCIneo vector (Promega Corporation) were subjected to restriction enzyme treatment with MluI and NotI, followed by gel extraction and purification. Then, each vector treated with restriction enzymes was mixed, and ligation reaction was performed at 16°C for 60 minutes using Ligation high Ver. 2 to complete the pCIneo-hGBA vector, a wild-type hGBA expression plasmid (Figure 22).

### [Example 15] Production of HSA-hGBA Expression Plasmid and hGBA-HSA Expression Plasmid

A DNA fragment having the nucleotide sequence represented by SEQ ID NO: 40, which contains a gene encoding HSA-hGBA represented by SEQ ID NO: 39, a fusion protein formed by linking the C terminal of wild-type HSA (SEQ ID NO: 3) and the N terminal of wild-type hGBA (SEQ ID NO: 37) via a linker sequence represented by SEQ ID NO: 9, was synthesized. The DNA fragment was subjected to restriction enzyme treatment with MluI and NotI and separated by agarose gel electrophoresis. After EtBr staining, a band containing the target DNA fragment was excised under UV irradiation to extract DNA from the gel using QIAEX II Gel Extraction Kit. Similarly, pCI-neo vector (Promega Corporation) was subjected to restriction enzyme treatment with MluI and NotI, followed by gel extraction and purification. The vector and DNA fragment treated with the restriction enzymes were mixed together, and ligation reaction was performed at 16°C for 60 minutes using Ligation high Ver. 2 to complete the pCIneo-HSA-hGBA vector, an HSA-hGBA expression plasmid (Figure 23). A DNA fragment having the nucleotide sequence represented by SEQ ID NO: 42, which contains a gene encoding hGBA-HSA represented by SEQ ID NO: 41, a fusion protein formed by linking the C terminal of wild-type hGBA (SEQ ID NO: 37) and the N terminal of wild-type HSA (SEQ ID NO: 3) via a linker sequence represented by SEQ ID NO: 9, was also subjected to a ligation reaction in the same manner to complete pCIneo-hGBA-HSA vector, an hGBA-HSA expression plasmid.

### [Example 16] Confirmation and Purification of Each Plasmid

Each ligation reaction solution containing each of pCIneo-hGBA vector, pCIneo-HSA-hGBA vector, and pCIneo-hGBA-HSA vector obtained in Example 14 and Example 15 was used to transform Escherichia coli (E. coli DH5α Competent Cells, Takara Bio Inc.). From the obtained transformant, each plasmid incorporating wild-type hGBA, HSA-hGBA, and hGBA-HSA was purified in the same manner as described in Example 1.

### [Example 17] Transient Expression of Wild-Type hGBA, HSA-hGBA, and hGBA-HSA

Purified pCIneo-hGBA vector, pCIneo-HSA-hGBA vector, and pCIneo-hGBA-HSA vector obtained in Example 16 were used for transient expression of wild-type hGBA, HSA-hGBA, and hGBA-HSA.

According to the high titer protocol of ExpiCHO(TM) Expression System (Thermo Fisher Scientific Inc.), ExpiCHO cells were transformed with pCIneo-hGBA vector, pCIneo-HSA-hGBA vector, and pCIneo-hGBA-HSA vector. After transformation, the cells were cultured for 8 days to express each of wild-type hGBA, HSA-hGBA, and hGBA-HSA in a culture supernatant. After 8 days, each culture solution was centrifuged to recover the culture supernatant.

### [Example 18] Confirmation of Expression Level of Wild-Type hGBA, HSA-hGBA, and hGBA-HSA by Transient Expression (Enzyme Activity Measurement)

As a sample solution, the culture supernatant obtained in Example 17 was appropriately diluted with 0.4 M KH₂PO₄/0.4 M K₂HPO₄/0.125% Na-taurocholate/0.15% TrionX-100/0.1% BSA solution and subjected to measurement. A standard solution was prepared by serially diluting 4-MU (4-methylumbelliferone, Sigma-Aldrich) from 200 to 3.13 µM with 0.4 M KH₂PO₄/0.4 M K₂HPO₄/0.125% Na-taurocholate/0.15% TrionX-100/0.1% BSA solution. A substrate solution was prepared by diluting 4-methylumbelliferyl-β-D-glucopyranoside (Sigma-Aldrich), an artificial substrate of hGBA, to 4 mmol/L with 0.4 M KH₂PO₄/0.4 M K₂HPO₄/0.125% Na-taurocholate/0.15% TrionX-100/0.1% BSA solution. The sample solution or standard solution was added to a microplate at 10 µL/well, and then 70 µL of the substrate solution was added to each well and mixed by stirring with a plate shaker. The plate was allowed to stand at 37°C for 1 hour, and then 200 µL of 200 mM glycine/50 mM Na2CO3 buffer (pH 10.7) was added to each well to stop the reaction. The fluorescence intensity of each well was then measured with a fluorescence plate reader (Gemini XPS, Molecular Devices, LLC.) (excitation wavelength: 365 nm, fluorescence wavelength: 460 nm). The fluorescence intensity is proportional to the concentration of 4-MU (4-methylumbelliferone) contained in the solution in each well. A calibration curve was created based on the measurement results of the standard solution, and the measurement value of each sample solution was interpolated to determine enzyme activity levels (µM/h). Note that the unit of enzyme activity levels is the amount of substrate decomposed per hour.

### [Example 19] Confirmation of Expression Level of Wild-Type hGBA, HSA-hGBA, and hGBA-HSA by Transient Expression (SDS Page Electrophoresis)

Each 10 µL of the culture supernatant obtained in Example 17 was mixed with 10 µL of 2× Sample Buffer (Bio-Rad Laboratories, Inc.), to which DTT had been added to give a final concentration of 0.4 M, and heat-denatured under reducing conditions by heating at 100°C for 5 minutes. The heat-denatured solution was applied in 15 µL portions to each well of a 5 to 20% polyacrylamide gel placed in 50 mM Tris buffer/380 mM glycine buffer (pH 8.3) containing 0.1% SDS, followed by electrophoresis at a constant current of 25 mA. After electrophoresis, the gel was immersed in Oriole fluorescent gel stain (Bio-Rad Laboratories, Inc.) and shaken at room temperature for 60 minutes. After washing the gel with pure water, an electrophoresis image of the band corresponding to each protein visualized was obtained using a lumino image analyzer (Amersham Imager 600RGB, Cytiva).

### [Example 20] Confirmation of Expression Level of Wild-type hGBA, HSA-hGBA, and hGBA-HSA by Transient Expression

### (Result)

Figure 24 shows results of enzyme activity measurement of wild-type hGBA, HSA-hGBA, and hGBA-HSA by transient expression measured in Example 18. In addition, the relative amounts of wild-type hGBA, HSA-hGBA, and hGBA-HSA based on the results of enzyme activity measurement are shown in [T1].

**[Table 4]**

| Table 4. Expression level of wild-type hGBA and fusion protein by transient expression (relative amount to expression level of wild-type hGBA defined as 1) | |
|---|---|
| Expression protein | Expression level |
| Wild-type hGBA | 1.0 |
| HSA-hGBA | 22 |
| hGBA-HSA | 18 |

The above results showed that the expression levels of HSA-hGBA and hGBA-HSA were 22 and 18 times higher, respectively, than that of wild-type hGBA, according to the expression levels (converted to enzyme activity levels) 8 days after the start of culture, and revealed that hGBA, which has low expression levels and is difficult to produce in large quantities when expressed as a recombinant in its wild-type form, could be efficiently expressed in the form of HSA-hGBA or hGBA-HSA to express more hGBA as a recombinant protein.

Next, the electrophoresis image of SDS-PAGE obtained in Example 19 is shown in Figure 25. The results of SDS-PAGE showed that the expression levels of HSA-hGBA and hGBA-HSA were 10 to 20 times or more higher than that of wild-type hGBA from the comparison of band intensities, and were substantially correlated with the expression levels (converted to enzyme activity levels) shown in Figure 24 and Table 4. In other words, it was found that hGBA, which has low expression levels and is difficult to produce in large quantities when expressed as a recombinant in its wild-type form, could be efficiently expressed in the form of HSA-hGBA or hGBA-HSA to express more hCBA as a recombinant protein.

The above results indicate that, when hGBA is produced as a recombinant protein, hGBA can be produced in the form of a fusion protein of HSA-hGBA or hGBA-HSA to obtain hGBA with about 20 times the number of molecules. That is, a method for producing a recombinant hGBA by expressing hGBA in the form of a fusion protein of HSA-hGBA or hGBA-HSA can be concluded to be a very effective method for producing a large quantity of recombinant hGBA.

Hereinafter, Examples 21 to 26 relate to fusion proteins of mouse serum albumin (MSA) and mouse IL-10 (mIL-10). In Examples 21 to 26, MSA used was a mutant MSA (MSA-A320T, SEQ ID NO: 125) in which alanine at amino acid position 320 of the wild-type MSA having an amino acid sequence represented by SEQ ID NO: 16 was substituted with threonine.

### [Example 21] Production of Wild-Type mIL-10 Expression Plasmid, mIL-10-MSA Expression Plasmid, and MSA-mIL-10 Expression Plasmid

A DNA fragment having the nucleotide sequence represented by SEQ ID NO: 127, which contains a gene encoding wild-type mIL-10 (SEQ ID NO: 126); a DNA fragment having the nucleotide sequence represented by SEQ ID NO: 129, which contains a gene encoding mIL-10-MSA represented by SEQ ID NO: 128, a fusion protein in which the C terminal of the wild-type mIL-10 (SEQ ID NO: 126) and the N terminal of the mutant MSA (SEQ ID NO: 125) were bound directly; and a DNA fragment having the nucleotide sequence represented by SEQ ID NO: 131, which contains a gene encoding MSA-mIL-10 represented by SEQ ID NO: 130, a fusion protein in which the C terminal of the mutant MSA (SEQ ID NO: 125) and the N terminal of the wild-type mIL-10 (SEQ ID NO: 126) are bound directly, were synthesized. The DNA fragments were subjected to restriction enzyme treatment with MluI and NotI (Takara Bio Inc.) and separated by agarose gel electrophoresis. After EtBr staining, a band containing the target DNA fragment was excised under UV irradiation to extract DNA from the gel using QIAEX II Gel Extraction Kit (QIAGEN). Similarly, pCI-neo vector (Promega Corporation) was subjected to restriction enzyme treatment with MluI and NotI, followed by gel extraction and purification. Each DNA fragment treated with the restriction enzymes was mixed with the vector treated with the restriction enzymes, and ligation reaction was performed at 16°C for 60 minutes using DNA Ligation Kit Mighty Mix (Takara Bio Inc.).

Next, each ligation reaction solution was used to transform Escherichia coli (One Shot MAX Efficiency DH10B T1 Phage-Resistant Cells, Thermo Fisher Scientific Inc.). From the obtained transformant, each plasmid incorporating the target gene was purified in the same manner as described in Example 1.

### [Example 22] Transient Expression of Wild-Type mIL-10, mIL-10-MSA, and MSA-mIL-10

According to the high titer protocol of ExpiCHO Expression System (Thermo Fisher Scientific Inc.), ExpiCHO cells were transformed with a plasmid incorporating the gene encoding each of wild-type mIL-10, mIL-10-MSA, and MSA-mIL-10. After transformation, the cells were cultured for 8 days to express each of wild-type mIL-10, mIL-10-MSA, and MSA-mIL-10 in a culture supernatant. After culture, the culture solution was centrifuged to recover the culture supernatant.

### [Example 23] Confirmation of Expression Level of Wild-Type mIL-10, mIL-10-MSA, and MSA-mIL-10 by Transient Expression (SDS-Page Electrophoresis)

Then, 6 µL of the culture supernatant obtained in Example 22 was mixed with 2 µL of 4× Sample Buffer (Bio-Rad Laboratories, Inc.), to which (±)-dithiothreitol (FUJIFILM Wako Pure Chemical Corporation) had been added to reach 40 mg/mL, and heat-denatured under reducing conditions by heating at 100°C for 3 minutes. The heat-denatured sample was applied in 5 µL portions to each well of a 5 to 20% polyacrylamide gel (FUJIFILM Wako Pure Chemical Corporation) placed in 50 mM Tris buffer/380 mM glycine buffer (pH 8.3) containing 0.1% SDS, followed by electrophoresis at a constant current of 25 mA. After electrophoresis, the gel was immersed in EZFluor UV 1-step Fluorescent Protein Gel Stain (Merck) and shaken at room temperature for 60 minutes. After washing the gel with pure water, a band of the protein was detected using a lumino image analyzer (Amersham ImageQuan 800, Cytiva).

### [Example 24] Confirmation of Expression Level of Wild-Type mIL-10, mIL-10-MSA, and MSA-mIL-10 by Transient Expression (ELISA)

ELISA was performed using Mouse IL-10 ELISA Kit (Proteintech Group, Inc.). The culture supernatant obtained in Example 22 was diluted 50,000 times with Sample Diluent attached to the kit and used as a sample solution. The standard solution for wild-type mIL-10 was prepared by diluting the Standard attached to the kit with Sample Diluent, and the standard solutions for mIL-10-MSA and MSA-mIL-10 were prepared by diluting the purified products obtained by the method described in Example 25 below with Sample Diluent. To each well of a 96-well plate on which an anti-mouse IL-10 antibody was immobilized, 100 µL of the sample solution or standard solution was added and incubated at 37°C for 100 minutes. Next, each well was washed four times with 300 µL of Wash Buffer attached to the kit, and then 100 µL of mouse IL-10 detection antibody was added to each well and incubated at 37°C for 60 minutes. Each well was washed four times with 300 µL of Wash Buffer, and then 100 µL of Streptavidin-HRP was added to each well and incubated at 37°C for 40 minutes. After each well was washed four times with 300 µL of Wash Buffer, 100 µL of TMB substrate was added to each well, followed by a color development reaction at 37°C. After sufficient color development was achieved, 100 µL of TMB stop solution was added to each well to stop the reaction. The absorbance at a wavelength of 450 nm was measured with a plate reader (SpectraMax iD3, Molecular Devices, LLC.), followed by the creation of a calibration curve using analysis software (SoftMax Pro 7.1, Molecular Devices, LLC.), into which the measurement value of each sample solution was interpolated to calculate the concentration (converted to the amount of substance) in each culture supernatant.

### [Example 25] Acquisition of Purified Products of mIL-10-MSA and MSA-mIL-10

The purified products of mIL-10-MSA and MSA-mIL-10 used for standard solution preparation in Example 24 were obtained according to the following method.

### [Production of mIL-10-MSA Expression Plasmid and MSA-mIL-10 Expression Plasmid]

The mIL-10-MSA expression plasmid and the MSA-mIL-10 expression plasmid produced in Example 21 were subjected to restriction enzyme treatment with Mlu I and Not I (Takara Bio Inc.), and a DNA fragment containing the nucleotide sequence of the mIL-10-MSA gene or the MSA-mIL-10 gene was separated by agarose gel electrophoresis. After EtBr staining, a band containing the target DNA fragment was excised under UV irradiation to extract DNA from the gel using FastGene Gel/PCR Extraction Kit (NIPPON Genetics Co., Ltd.). Similarly, pE-mIRES-GS-puro vector was subjected to restriction enzyme treatment with MluI and NotI, followed by gel extraction and purification. Note that the mIRES-GS-puro vector was constructed by the method described in WO 2013/161958. Each DNA fragment treated with the restriction enzymes was mixed with the vector treated with the restriction enzymes to perform ligation reaction at 16°C for 30 to 60 minutes using Ligation high Ver. 2 (Toyobo Co., Ltd.).

Next, each ligation reaction solution was used to transform Escherichia coli (E. coli DH5α Competent Cells, Takara Bio Inc.). From the obtained transformant, each plasmid incorporating the target gene was purified in the same manner as described in Example 1.

### [Production of mIL-10-MSA Expression Bulk Cell and MSA-mIL-10 Expression Bulk Cell]

An expression plasmid incorporating the gene encoding mIL-10-MSA or MSA-mIL-10 obtained above was introduced into a serum-free conditioned strain of CHO-K1 cells using a gene transfection device (Super Electroporator NEPA21, Nepa Gene Co., Ltd.), and selective culture was carried out in CD OptiCHO medium (Thermo Fisher Scientific Inc.) containing methionine sulfoximine (Sigma-Aldrich) and puromycin (Thermo Fisher Scientific Inc.). In the selective culture, the concentrations of methionine sulfoximine and puromycin were increased stepwise until the final concentrations of methionine sulfoximine and puromycin reached 300 µM and 10 µg/mL, respectively. The culture solution volume was sequentially expanded with repeated medium changes every 3 or 4 days, and cells were recovered when the survival rate of cells in the culture exceeded 90% and were designated as mIL-10-MSA expression bulk cells and MSA-mIL-10 expression bulk cells.

### [Culture of mIL-10-MSA Expression Bulk Cell and MSA-mIL-10 Expression Bulk Cell]

In CD OptiCHO medium containing 300 µM methionine sulfoximine and 10 µg/mL puromycin, the mIL-10-MSA expression bulk cells and MSA-mIL-10 expression bulk cells obtained above were each seeded at a cell density of 2 × 10⁵ cells/mL and cultured by shaking in the presence of 5% CO² at 37°C. The MSA-mIL-10 expression bulk cells were cultured under conditions of 32°C from the third day of culture onward. After 10 days from the start of culture, the culture supernatant was recovered by centrifugation.

### [Purification of mIL-10-MSA and MSA-mIL-10]

The culture supernatant of the mIL-10-MSA expression bulk cells recovered above was filtered through a Rapid-Flow PES membrane filter unit (pore size: 0.2 µm, Thermo Fisher Scientific Inc.) and passed through a HiTrap Q HP column (Cytiva) equilibrated with 25 mM HEPES buffer (pH 7.4) containing 100 mM NaCl to adsorb mIL-10-MSA to the resin. Subsequently, the column was washed with 25 mM HEPES buffer (pH 7.4) containing 100 mM NaCl, and the mIL-10-MSA was eluted from the resin with 25 mM HEPES buffer (pH 7.4) containing 350 mM NaCl.

The eluate from the HiTrap Q HP column containing mIL-10-MSA was diluted 6-fold with 25 mM MES buffer (pH 6.8) containing 5 mM phosphoric acid and was passed through a CHT TypeI column (Bio-Rad Laboratories, Inc.) equilibrated with 25 mM MES buffer (pH 6.8) containing 5 mM phosphoric acid to adsorb mIL-10-MSA to the resin. Subsequently, the column was washed with 25 mM MES buffer (pH 6.8) containing 5 mM phosphoric acid, and mIL-10-MSA was eluted from the resin with 25 mM MES buffer (pH 6.8) containing 200 mM phosphoric acid, resulting in a mIL-10-MSA purified product. In the same manner, MSA-mIL-10 was purified to obtain an MSA-mIL-10 purified product.

### [Example 26] Confirmation of Expression Level of Wild-Type mIL-10, mIL-10-MSA, and MSA-mIL-10 by Transient Expression (Result)

Figure 26 shows results of ELISA concentration measurement of wild-type mIL-10, mIL-10-MSA, and MSA-mIL-10 by transient expression as measured in Example 24. The relative amounts of wild-type mIL-10, mIL-10-MSA, and MSA-mIL-10 based on the results of the ELISA concentration measurement are shown in [T2].

**[Table 5]**

| Table 5. Expression level of wild-type mIL-10 and fusion protein by transient expression (relative amount to expression level of wild-type mIL-10 defined as 1) | |
|---|---|
| Expression protein | Expression level (average) |
| Wild-type mIL-10 | 1.0 |
| mIL-10-MSA | 4.5 |
| MSA-mIL-10 | 4.2 |

The above results revealed that the expression levels of mIL-10-MSA and MSA-mIL-10 were 4.5 and 4.2 times higher, respectively, than that of wild-type mIL-10, according to the expression levels (concentration) 8 days after the start of culture, and revealed that IL-10, which has low expression levels and is difficult to produce in large quantities when expressed as a recombinant in its wild-type form, could be efficiently expressed in the form of IL-10-SA or SA-IL-10 to express more IL-10 as a recombinant protein.

Next, the electrophoresis image of SDS-PAGE obtained in Example 23 is shown in Figure 27. The results of SDS-PAGE showed that the expression levels of mIL-10-MSA and MSA-mIL-10 were higher than that of wild-type mIL-10 from the comparison of band intensities, which corresponded to the measurement values by ELISA shown in Figure 26 and Table 5. It was found that IL-10, which has low expression levels and is difficult to produce in large quantities when expressed as a recombinant in its wild-type form, could be efficiently expressed in the form of IL-10-SA or SA-IL-10 to express more IL-10 as a recombinant protein.

The above results indicate that when mIL-10 is produced as a recombinant protein, mIL-10 with about 4 times the number of molecules can be obtained by producing mIL-10 in the form of a fusion protein of mIL-10-MSA or MSA-mIL-10, and further indicate that when hIL-10 is produced as a recombinant protein, hIL-10 with a larger number of molecules can be obtained by producing hIL-10 in the form of a fusion protein of hIL-10-HSA or HSA-hIL-10. That is, a method for producing recombinant IL-10 by expressing IL-10 in the form of a fusion protein of IL-10-SA or SA-IL-10 can be concluded to be a very effective method for producing a large quantity of recombinant IL-10.

Hereinafter, Examples 27 to 33 relate to a fusion protein of HSA and human neurotrophic factor (hBDNF, hNGF, hNT-3, or hNT-4).

### [Example 27] Production of Wild-Type Human Neurotrophic Factor Expression Plasmid

The following DNA fragments (1) to (4) were synthesized:
(1) a DNA fragment having the nucleotide sequence represented by SEQ ID NO: 133, which contains a gene encoding a wild-type hBDNF (SEQ ID NO: 132) with an amino acid sequence represented by Gly-Ser (hereinafter referred to as GS) and a histidine tag having an amino acid sequence of SEQ ID NO: 195 consisting of 6 histidines (hereinafter referred to as His6) added in this order to the C terminal;
(2) a DNA fragment having the nucleotide sequence represented by SEQ ID NO: 135, which contains a gene encoding wild-type hNGF (SEQ ID NO: 134) with His6 added to the C terminal;
(3) a DNA fragment having the nucleotide sequence represented by SEQ ID NO: 137, which contains a gene encoding wild-type hNT-4 (SEQ ID NO: 136) with His6 added to the C terminal; and
(4) a DNA fragment having the nucleotide sequence represented by SEQ ID NO: 139, which contains a gene encoding wild-type hNT-3 (SEQ ID NO: 138) with His6 added to the C terminal.

The DNA fragments (1) to (4) above were subjected to restriction enzyme treatment with MluI and NotI (Takara Bio Inc.) and separated by agarose gel electrophoresis. After EtBr staining, a band containing the target DNA fragment was excised under UV irradiation to extract DNA from the gel using QIAEX II Gel Extraction Kit (QIAGEN). Similarly, pCI-neo vector (Promega Corporation) was subjected to restriction enzyme treatment with MluI and NotI, followed by gel extraction and purification. Each DNA fragment treated with the restriction enzymes was mixed with the vector treated with the restriction enzymes, and ligation reaction was performed at 16°C for 30 to 60 minutes using Ligation Mix (Takara Bio Inc.).

Next, each ligation reaction solution was used to transform Escherichia coli (ECOS(TM) X Competent E. coli DH5α, NIPPON GENE CO., LTD.). From the obtained transformant, each plasmid incorporating the target gene was purified in the same manner as described in Example 1. Each purified vector was used as a wild-type hBDNF expression vector, a wild-type hNGF expression vector, a wild-type hNT-4 expression vector, and a wild-type hNT-3 expression vector in the following experiments.

### [Example 28] Production of Plasmid Expressing Fusion Protein of HSA and Human Neurotrophic Factor

The DNA fragments (1) to (4) synthesized in Example 27 were subjected to restriction enzyme treatment with MluI and BamHI (Takara Bio Inc.) and separated by agarose gel electrophoresis. After EtBr staining, a band containing the target DNA fragment was excised under UV irradiation, and the DNA fragment was extracted from the gel using QIAEX II Gel Extraction Kit (QIAGEN) and purified. The purified DNA fragment was designated as insert DNA1.

The following DNA fragments (1) to (5) were synthesized:
(1) a DNA fragment having the nucleotide sequence represented by SEQ ID NO: 141, which contains a gene encoding wild-type HSA (SEQ ID NO: 140) with His6 added to the C terminal;
(2) a DNA fragment having the nucleotide sequence represented by SEQ ID NO: 133, which contains a gene encoding wild-type hBDN (SEQ ID NO: 132) with His6 added to the C terminal;
(3) a DNA fragment having the nucleotide sequence represented by SEQ ID NO: 135, which contains a gene encoding wild-type hNGF (SEQ ID NO: 134) with His6 added to the C terminal;
(4) a DNA fragment having the nucleotide sequence represented by SEQ ID NO: 137, which contains a gene encoding wild-type hNT-4 (SEQ ID NO: 136) with His6 added to the C terminal; and
(5) a DNA fragment having the nucleotide sequence represented by SEQ ID NO: 139, which contains a gene encoding wild-type hNT-3 (SEQ ID NO: 138) with His6 added to the C terminal.

Using the above DNA fragments (1) to (5) as templates, PCR reactions of Nos. 1 to 5 were carried out with the forward primer and reverse primer shown in [T3]. Hereafter, for convenience, wild-type HSA with His6 added to the C terminal is also referred to as wild-type HSA. The same applies to wild-type hBDNF, wild-type hNGF, wild-type hNT-4, and wild-type hNT-3.

**[Table 6]**

| Table 6. Combination of template and primer in each PCR reaction | | | |
|---|---|---|---|
| PCR reaction No. | Template | Forward primer | Reverse primer |
| 1 | SEQ ID NO: | SEQ ID NO: | SEQ ID NO: |
| | 141 | 142 | 147 |
| 2 | SEQ ID NO: | SEQ ID NO: | SEQ ID NO: |
| | 133 | 143 | 147 |
| 3 | SEQ ID NO: | SEQ ID NO: | SEQ ID NO: |
| | 135 | 144 | 147 |
| 4 | SEQ ID NO: | SEQ ID NO: | SEQ ID NO: |
| | 137 | 145 | 147 |
| 5 | SEQ ID NO: | SEQ ID NO: | SEQ ID NO: |
| | 139 | 146 | 147 |

DNA fragments obtained by PCR of Nos. 1 to 5 above were subjected to restriction enzyme treatment with BglII and NotI (Takara Bio Inc.), followed by gel extraction and purification. The purified DNA fragment was designated as insert DNA2.

pCI-neo vector (Promega Corporation) was subjected to restriction enzyme treatment with MluI and NotI, followed by gel extraction and purification. The insert DNA1 and insert DNA2 described above were each mixed with the vector treated with the restriction enzymes in the combination shown in Table 7, and ligation reaction was performed at 16°C for 30 to 60 minutes using Ligation Mix (Takara Bio Inc.). This reaction inserts into the pCI-neo vector a DNA fragment in which insert DNA2 is in-frame connected to the 3' end of insert DNA1.

Table 7 shows the target fusion proteins encoded by the resulting genes for each combination of insert DNA1 and insert DNA2. The names and structures of the fusion proteins in this example are as shown in (1) to (8) below:
(1) hBDNF-HSA: a fusion protein having an amino acid sequence represented by SEQ ID NO: 148, in which wild-type HSA (SEQ ID NO: 3) is bound to the C terminal of wild-type hBDNF (SEQ ID NO: 60) via a linker represented by an amino acid sequence Gly-Ser, with His6 further bound to the C terminal thereof;
(2) hNGF-HSA: a fusion protein having an amino acid sequence represented by SEQ ID NO: 149, in which wild-type HSA (SEQ ID NO: 3) is bound to the C terminal of wild-type hNGF (SEQ ID NO: 62) via a linker represented by an amino acid sequence Gly-Ser, with His6 further bound to the C terminal thereof;
(3) hNT-4-HSA: a fusion protein having an amino acid sequence represented by SEQ ID NO: 150, in which wild-type HSA (SEQ ID NO: 3) is bound to the C terminal of wild-type hNT-4 (SEQ ID NO: 66) via a linker represented by an amino acid sequence Gly-Ser, with His6 further bound to the C terminal thereof;
(4) hNT-3-HSA: a fusion protein having an amino acid sequence represented by SEQ ID NO: 151, in which wild-type HSA (SEQ ID NO: 3) is bound to the C terminal of wild-type hNT-3 (SEQ ID NO: 64) via a linker represented by an amino acid sequence Gly-Ser, with His6 further bound to the C terminal thereof;
(5) HSA-hBDNF: a fusion protein having an amino acid sequence represented by SEQ ID NO: 152, in which a pro-form of wild-type hBDNF (SEQ ID NO: 84) is bound to the C terminal of wild-type HSA (SEQ ID NO: 3) via a linker represented by an amino acid sequence Gly-Ser, with His6 further bound to the C terminal thereof;
(6) HSA-hNGF: a fusion protein having an amino acid sequence represented by SEQ ID NO: 153, in which a pro-form of wild-type hNGF (SEQ ID NO: 87) is bound to the C terminal of wild-type HSA (SEQ ID NO: 3) via a linker represented by an amino acid sequence Gly-Ser, with His6 further bound to the C terminal thereof;
(7) HSA-hNT-4: a fusion protein having an amino acid sequence represented by SEQ ID NO: 154, in which a pro-form of wild-type hNT-4 (SEQ ID NO: 93) is bound to the C terminal of wild-type HSA (SEQ ID NO: 3) via a linker represented by an amino acid sequence Gly-Ser, with His6 further bound to the C terminal thereof; and
(8) HSA-hNT-3: a fusion protein having an amino acid sequence represented by SEQ ID NO: 155, in which a pro-form of wild-type hNT-3 (SEQ ID NO: 90) is bound to the C terminal of wild-type HSA (SEQ ID NO: 3) via a linker represented by an amino acid sequence Gly-Ser, with His6 further bound to the C terminal thereof.

Since for all of HSA-hBDNF, HSA-hNGF, HSA-hNT-4 and HSA-hNT-3, pro-forms thereof are used as human neurotrophic factors (hBDNF, hNGF, hNT-4 or hNT-3) to be fused to the C terminal side of HSA, when these proteins are expressed as recombinant proteins, a part or all of the molecules of the fusion proteins may be cleaved and finally obtained as a single wild-type human neurotrophic factor (hBDNF, hNGF, hNT-4 or hNT-3), but all of them are treated as being expressed as fusion proteins except when the results of SDS-PAGE and Western blotting obtained in Example 30 and Example 31 are observed.

**[Table 7]**

| Table 7. Combination of target fusion protein and DNA fragment | | |
|---|---|---|
| Fusion protein | Insert DNA1 (DNA fragment subjected to MIuI/BamHI treatment) | Insert DNA2 (DNA fragment subjected to BbIII/NotI treatment) |
| hBDNF-HSA (SEQ ID NO: 148) | SEQ ID NO: 133 | PCR product of No. 1 |
| hNGF-HSA (SEQ ID NO: 149) | SEQ ID NO: 135 | PCR product of No. 1 |
| hNT-4-HSA (SEQ ID NO: 150) | SEQ ID NO: 137 | PCR product of No. 1 |
| hNT-3-HSA (SEQ ID NO: 151) | SEQ ID NO: 139 | PCR product of No. 1 |
| HSA-hBDNF (SEQ ID NO: 152) | SEQ ID NO: 141 | PCR product of No. 2 |
| HSA-hNGF (SEQ ID NO: 153) | SEQ ID NO: 141 | PCR product of No. 3 |
| HSA-hNT-4 (SEQ ID NO: 154) | SEQ ID NO: 141 | PCR product of No. 4 |
| HSA-hNT-3 (SEQ ID NO: 155) | SEQ ID NO: 141 | PCR product of No. 5 |

Next, each ligation reaction solution was used to transform Escherichia coli (ECOSTM X Competent E. coli DH5α, NIPPON GENE CO., LTD.) with expression vectors encoding the above fusion proteins (1) to (8), respectively. From the obtained transformant, each plasmid incorporating the target gene was purified in the same manner as described in Example 1.

### [Example 29] Transient Expression of Wild-Type Human Neurotrophic Factor and Fusion Protein of HSA and Human Neurotrophic Factor

According to the high titer protocol of ExpiCHO Expression System (Thermo Fisher Scientific Inc.), ExpiCHO(TM) cells were transformed with a plasmid incorporating the gene encoding wild-type human neurotrophic factor or a fusion protein of HSA and human neurotrophic factor obtained in Examples 27 and 28, respectively. After transformation, the cells were cultured for 8 days to express each of wild-type human neurotrophic factor and a fusion protein of HSA and human neurotrophic factor in a culture supernatant. After culture, the culture solution was centrifuged to recover the culture supernatant.

### [Example 30] Confirmation of Expression Level of Wild-Type Human Neurotrophic Factor and Fusion Protein of HSA and Human Neurotrophic Factor by Transient Expression (SDS-PAGE)

Then, 10 µL of the culture supernatant obtained in Example 29 was mixed with 8 µL of 2× Sample Buffer (Bio-Rad Laboratories, Inc.) and 2 µL of 2-mercaptoethanol (Bio-Rad Laboratories, Iinc.) and heat-denatured under reducing conditions by heating at 100°C for 3 minutes. The heat-denatured sample was applied in 5 µL portions to each well of a 10 to 20% polyacrylamide gel (FUJIFILM Wako Pure Chemical Corporation) placed in 50 mM Tris buffer/380 mM glycine buffer (pH 8.3) containing 0.1% SDS, followed by electrophoresis at a constant current of 25 mA. After electrophoresis, the gel was immersed in Oriole fluorescent gel stain (Bio-Rad Laboratories, Inc.) and shaken at room temperature for 90 minutes. After washing the gel with pure water, a band of the protein was detected with a lumino image analyzer (Amersham Imager 600RGB, Cytiva).

### [Example 31] Confirmation of Expression Level of Wild-Type Human Neurotrophic Factor and Fusion Protein of HSA and Human Neurotrophic Factor by Transient Expression (Western Blotting)

Electrophoresis was performed in the same manner as in Example 30, a nitrocellulose membrane and the gel after electrophoresis were sandwiched between blotting papers immersed in 25 mM Tris buffer/192 mM glycine buffer containing 20% methanol, and the protein was transferred to the nitrocellulose membrane by energizing a current of 1.0 A and 25 V for 10 minutes in a blotting device (Trans-Blot Turbo Transfer System, Bio-Rad Laboratories, Inc.). The transferred nitrocellulose membrane was immersed in TBST containing 5% skim milk and shaken for 1 hour, then immersed in a Mouse anti-His tag mAb (MEDICAL & BIOLOGICAL LABORATORIES CO., LTD.) solution diluted to 0.4 µg/mL and shaken for 1 hour. After washing the membrane with TBST, the membrane was immersed in a solution of Anti-mouse IgG (H+L), HRP Conjugate (Promega Corporation) diluted to 0.2 µg/mL, shaken for 60 minutes, and washed again with TBST. An HRP detection reagent (Bio-Rad Laboratories, Inc.) was added dropwise to the transfer surface of the membrane and allowed to react for 5 minutes, followed by detection with a lumino image analyzer.

### [Example 32] Confirmation of Expression Level of Wild-Type Human Neurotrophic Factor and Fusion Protein of HSA and Human Neurotrophic Factor by Transient Expression (ELISA)

To compare the expression levels of wild-type human neurotrophic factor and fusion protein of HSA and human neurotrophic factor by transient expression, ELISA measurement was made using Human Multi-Neurotrophin Rapid Screening ELISA Kit (Biosensis Pty Ltd.). The culture supernatant obtained in Example 29 was appropriately diluted with Assay diluent A attached to the kit as a sample solution, and the concentration of the wild-type human neurotrophic factor or the fusion protein of HSA and human neurotrophic factor in the culture supernatant was calculated. Each type of wild-type human neurotrophic factor alone or human neurotrophic factor fused to HSA is described in detail below.

### [Measurement of Wild-Type hBDNF and Fusion Protein of HSA and hBDNF]

As a sample solution, an ExpiCHO culture supernatant expressing wild-type hBDNF, hBDNF-HSA, or HSA-hBDNF obtained in Example 29 was diluted 100,000-fold with Assay diluent A. As a standard solution, 1000 pg/mL BDNF solution attached to the kit was serially diluted with Assay diluent A to prepare each of 500, 250, 125, 63, 31, 16, and 8 pg/mL solutions. To each well of a 96-well plate on which anti-BDNF antibody was immobilized, 100 µL of the sample solution or standard solution was added and shaken with a plate shaker at room temperature for 90 minutes. Next, each well was washed five times with 200 µL of Wash buffer, and then 100 µL of BDNF detection antibody (biotin-labeled anti-BDNF antibody) was added to each well and shaken with a plate shaker at room temperature for 30 minutes. Subsequently, each well was washed five times with 200 µL of Wash buffer, and then 100 µL of Streptavidin-HRP was added to each well and shaken with a plate shaker at room temperature for 30 minutes. After each well was washed five times with 200 µL of Wash buffer, 100 µL of TMB substrate was added to each well and allowed to stand at room temperature for color development reaction. After sufficient color development was achieved, 100 µL of TMB stop solution was added to each well to stop the reaction. The absorbance at a wavelength of 450 nm was measured with a microplate reader (Spectramax 190 EXT, Molecular Devices, LLC.), followed by the creation of a calibration curve using analysis software (SoftMax Pro, Molecular Devices, LLC.), into which the measurement value of each sample solution was interpolated to calculate the concentration (converted to the amount of substance) in each culture supernatant.

### [Measurement of Wild-Type hNGF and Fusion Protein of HSA and hNGF]

As a sample solution, an ExpiCHO culture supernatant expressing wild-type hNGF, hNGF-HSA, or HSA-hNGF obtained in Example 29 was diluted 100,000-fold with Assay diluent A. As a standard solution, 500 pg/mL NGF solution attached to the kit was serially diluted with Assay diluent A to prepare each of 250, 125, 63, 31, 16, 8, and 4 pg/mL solutions. To each well of a 96-well plate on which anti-NGF antibody was immobilized, 100 µL of the sample solution or standard solution was added and shaken with a plate shaker at room temperature for 90 minutes. Next, each well was washed five times with 200 µL of Wash buffer, and then 100 µL of NGF detection antibody (biotin-labeled anti-NGF antibody) was added to each well and shaken with a plate shaker at room temperature for 30 minutes. Subsequently, each well was washed five times with 200 µL of Wash buffer, and then 100 µL of Streptavidin-HRP was added to each well and shaken with a plate shaker at room temperature for 30 minutes. After each well was washed five times with 200 µL of Wash buffer, 100 µL of TMB substrate was added to each well and allowed to stand at room temperature for color development reaction. After sufficient color development was achieved, 100 µL of TMB stop solution was added to each well to stop the reaction. The absorbance at a wavelength of 450 nm was measured with a microplate reader (Spectramax 190 EXT, Molecular Devices, LLC.), followed by the creation of a calibration curve using analysis software (SoftMax Pro, Molecular Devices, LLC.), into which the measurement value of each sample solution was interpolated to calculate the concentration (converted to the amount of substance) in each culture supernatant.

### [Measurement of Wild-Type hNT-4 and Fusion Protein of HSA and hNT-4]

A sample solution was prepared by diluting ExpiCHO culture supernatants expressing wild-type hNT-4, hNT-4-HSA, and HSA-hNT-4 obtained in Example 29 100,000-fold with Assay diluent A. As a standard solution, 2000 pg/mL NT-4 solution attached to the kit was serially diluted with Assay diluent A to prepare each of 1000, 500, 250, 125, 63, 31, and 16 pg/mL solutions. To each well of a 96-well plate on which anti-NT-4 antibody was immobilized, 100 µL of the sample solution or standard solution was added and shaken with a plate shaker at room temperature for 90 minutes. Next, each well was washed five times with 200 µL of Wash buffer, and then 100 µL of NT-4 detection antibody (biotin-labeled anti-NGF antibody) was added to each well and shaken with a plate shaker at room temperature for 30 minutes. Subsequently, each well was washed five times with 200 µL of Wash buffer, and then 100 µL of Streptavidin-HRP was added to each well and shaken with a plate shaker at room temperature for 30 minutes. After each well was washed five times with 200 µL of Wash buffer, 100 µL of TMB substrate was added to each well and allowed to stand at room temperature for color development reaction. After sufficient color development was achieved, 100 µL of TMB stop solution was added to each well to stop the reaction. The absorbance at a wavelength of 450 nm was measured with a microplate reader (Spectramax 190 EXT, Molecular Devices, LLC.), followed by the creation of a calibration curve using analysis software (SoftMax Pro, Molecular Devices, LLC.), into which the measurement value of each sample solution was interpolated to calculate the concentration (converted to the amount of substance, with one monomer as one molecule) in each culture supernatant.

### [Measurement of Wild-Type hNT-3 and Fusion Protein of HSA and hNT-3]

A sample solution was prepared by diluting ExpiCHO culture supernatants expressing wild-type hNT-3, hNT-3-HSA, and HSA-hNT-3 obtained in Example 29 100,000-fold with Assay diluent A. As a standard solution, 2000 pg/mL NT-3 solution attached to the kit was serially diluted with Assay diluent A to prepare each of 1000, 500, 250, 125, 63, 31, and 16 pg/mL solutions. To each well of a 96-well plate on which anti-NT-3 antibody was immobilized, 100 µL of the sample solution or standard solution was added and shaken with a plate shaker at room temperature for 90 minutes. Next, each well was washed five times with 200 µL of Wash buffer, and then 100 µL of NT-3 detection antibody (biotin-labeled anti-NGF antibody) was added to each well and shaken with a plate shaker at room temperature for 30 minutes. Subsequently, each well was washed five times with 200 µL of Wash buffer, and then 100 µL of Streptavidin-HRP was added to each well and shaken with a plate shaker at room temperature for 30 minutes. After each well was washed five times with 200 µL of Wash buffer, 100 µL of TMB substrate was added to each well and allowed to stand at room temperature for color development reaction. After sufficient color development was achieved, 100 µL of TMB stop solution was added to each well to stop the reaction. The absorbance at a wavelength of 450 nm was measured with a microplate reader (Spectramax 190 EXT, Molecular Devices, LLC.), followed by the creation of a calibration curve using analysis software (SoftMax Pro, Molecular Devices, LLC.), into which the measurement value of each sample solution was interpolated to calculate the concentration (converted to the amount of substance, with one monomer as one molecule) in each culture supernatant.

### [Example 33] Confirmation of Expression Level of Wild-Type Human Neurotrophic Factor and Fusion Protein of HSA and Human Neurotrophic Factor by Transient Expression (Result)

Figure 28 shows results of ELISA concentration measurement of wild-type human neurotrophic factor and a fusion protein of HSA and human neurotrophic factor by transient expression as measured in Example 32. The relative amounts of the wild-type human neurotrophic factor and the fusion protein of HSA and human neurotrophic factor based on the results of the ELISA concentration measurement are shown in [T4].

**[Table 8]**

| Table 8. Expression level of wild-type human neurotrophic factor and fusion protein by transient expression (relative amount to expression level of each wild-type human neurotrophic factor defined as 1) | | |
|---|---|---|
| Human neurotrophic factor | Expression protein | Expression level (average) |
| hBDNF | Wild-type hBDNF | 1.0 |
| | hBDNF-HSA | 29 |
| | HSA-hBDNF | 3.0 |
| hNGF | Wild-type hNGF | 1.0 |
| | hNGF-HSA | 1.6 |
| | HSA-HNGF | 1.3 |
| hNT-4 | Wild-type hNT-4 | 1.0 |
| | hNT-4-HSA | 4.1 |
| | HSA-hNT-4 | 1.8 |
| hNT-3 | Wild-type hNT-3 | 1.0 |
| | hNT-3-HSA | 12 |
| | HSA-hNT-3 | 1.0 |

The above results showed that the expression levels of hBDNF-HSA and HSA-hBDNF were 29 and 3.0 times higher, respectively, than that of wild-type hBDNF, according to the expression levels (concentration) 8 days after the start of culture, and revealed that hBDNF, which has low expression levels and is difficult to produce in large quantities when expressed as a recombinant in its wild-type form, could be efficiently expressed in the form of a fusion protein of hBDNF and HSA to express more hBDNF as a recombinant protein.

The expression levels of hNGF-HSA and HSA-hNGF were 1.6 and 1.3 times higher, respectively, than that of wild-type hNGF, and it was found that hNGF, which has low expression levels and is difficult to produce in large quantities when expressed as a recombinant in its wild-type form, could be efficiently expressed in the form of a fusion protein of hNGF and HSA to express more hNGF as a recombinant protein.

The expression levels of hNT-4-HSA and HSA-hNT-4 were 4.1 and 1.8 times higher, respectively, than that of wild-type hNT-4, and it was found that hNT-4, which has low expression levels and is difficult to produce in large quantities when expressed as a recombinant in its wild-type form, could be efficiently expressed in the form of a fusion protein of hNT-4 and HSA to express more hNT-4 as a recombinant protein.

The expression level of hNT-3-HSA was 12 times higher than that of wild-type hNT-3, and it was found that hNT-3, which has low expression levels and is difficult to produce in large quantities when expressed as a recombinant in its wild-type form, could be efficiently expressed in the form of a fusion protein of hNT-3 and HSA to express more hNT-3 as a recombinant protein.

Next, the electrophoresis image of SDS-PAGE obtained in Example 30 is shown in Figure 29. The results of SDS-PAGE showed that hBDNF-HSA and HSA-hBDNF had higher expression levels than wild-type hBDNF from the comparison of band intensities, hNGF-HSA and HSA-hNGF had higher expression levels than wild-type hNGF, hNT-4-HSA and HSA-hNT-4 had higher expression levels than wild-type hNT-4, and hNT-3-HSA and HSA-hNT-3 had higher expression levels than wild-type hNT-3, all of which corresponded to the expression levels (concentrations) shown in Figure 28 and Table 8. It was found that a human neurotrophic factor, which has low expression levels and is difficult to produce in large quantities when expressed as a recombinant in its wild-type form, could be efficiently expressed in the form of a fusion protein of HSA and human neurotrophic factor to express more human neurotrophic factors as recombinant proteins.

Next, the electrophoresis image of Western blotting obtained in Example 31 is shown in Figure 30. The results of Western blotting showed that hBDNF-HSA and HSA-hBDNF had higher expression levels than wild-type hBDNF from the comparison of band intensities, hNGF-HSA and HSA-hNGF had higher expression levels than wild-type hNGF, hNT-4-HSA and HSA-hNT-4 had higher expression levels than wild-type hNT-4, and hNT-3-HSA and HSA-hNT-3 had higher expression levels than wild-type hNT-3, all of which corresponded to the expression levels (concentrations) shown in Figure 28 and Table 8. However, HSA-hBDNF, HSA-hNGF, HSA-hNT-4, and HSA-hNT-3 were also observed together with a band that was cleaved in the middle and detected as a single mature human neurotrophic factor. It was found that a human neurotrophic factor, which has low expression levels and is difficult to produce in large quantities when expressed as a recombinant in its wild-type form, could be efficiently expressed in the form of a fusion protein of HSA and human neurotrophic factor to express more human neurotrophic factors as recombinant proteins.

The above results indicate that when human neurotrophic factor is produced as a recombinant protein, human neurotrophic factor in the form of a fusion protein of human neurotrophic factor and HSA can be produced to obtain a larger number of molecules of human neurotrophic factor. That is, a method for producing a recombinant human neurotrophic factor by expressing human neurotrophic factor in the form of a fusion protein of human neurotrophic factor and HSA can be concluded to be a very effective method for producing a large quantity of recombinant human neurotrophic factor.

### [Example 34] Production of Wild-Type hCDNF Expression Plasmid, hCDNF-HSA Expression Plasmid, and HSA-hCDNF Expression Plasmid

A DNA fragment having the nucleotide sequence represented by SEQ ID NO: 157, which contains a gene encoding wild-type hCDNF (SEQ ID NO: 156) with His6 added to the C terminal; a DNA fragment having the nucleotide sequence represented by SEQ ID NO: 159, which contains a gene encoding hCDNF-HSA represented by SEQ ID NO: 158, a fusion protein formed by linking the C terminal of wild-type hCDNF and the N terminal of HSA (SEQ ID NO: 3) via a GS linker, with His6 further added to the C terminal thereof; and a DNA fragment having the nucleotide sequence represented by SEQ ID NO: 161, which contains a gene encoding HSA-hCDNF represented by SEQ ID NO: 160, a fusion protein formed by linking the C terminal of HSA (SEQ ID NO: 3) and the N terminal of wild-type hCDNF via a GS linker, with His6 further added to the C terminal thereof, were synthesized. The DNA fragments were subjected to restriction enzyme treatment with MluI and NotI (Takara Bio Inc.) and separated by agarose gel electrophoresis. After EtBr staining, a band containing the target DNA fragment was excised under UV irradiation to extract DNA from the gel using QIAEX II Gel Extraction Kit (QIAGEN). Similarly, pCI-neo vector (Promega Corporation) was subjected to restriction enzyme treatment with MluI and NotI, followed by gel extraction and purification. Each DNA fragment treated with the restriction enzymes was mixed with the vector treated with the restriction enzymes, and ligation reaction was performed at 16°C for 60 minutes using DNA Ligation Kit Mighty Mix (Takara Bio Inc.). Hereafter, for convenience, wild-type hCDNF with His6 added to the C terminal is also referred to as wild-type hCDNF.

Next, each ligation reaction solution was used to transform Escherichia coli (One Shot MAX Efficiency DH10B T1 Phage-Resistant Cells, Thermo Fisher Scientific Inc.). To confirm whether the obtained transformant retained the target plasmid DNA, a single colony was cultured overnight in an LB liquid medium (Difco LB Broth, Lennox, BD) to purify a small amount of plasmid DNA using QIAprep Spin Miniprep Kit (QIAGEN). The purified plasmid DNA was subjected to restriction enzyme treatment with MluI and NotI and separated by agarose gel electrophoresis to confirm insertion of the target insert DNA. Each plasmid confirmed to incorporate the target gene was purified by a conventional method.

### [Example 35] Transient Expression of Wild-Type hCDNF, hCDNF-HSA, and HSA-hCDNF

According to the high titer protocol of ExpiCHO Expression System (Thermo Fisher Scientific Inc.), ExpiCHO cells were transformed with a plasmid incorporating the gene encoding each of wild-type hCDNF, hCDNF-HSA, and HSA-hCDNF. After transformation, the cells were cultured for 8 days to express each of wild-type hCDNF, hCDNF-HSA, and HSA-hCDNF in a culture supernatant. After culture, the culture solution was centrifuged to recover the culture supernatant.

### [Example 36] Confirmation of Expression Level of Wild-Type hCDNF, hCDNF-HSA, and HSA-hCDNF by Transient Expression (SDS-Page Electrophoresis)

Then, 6 µL of the culture supernatant obtained in Example 35 was mixed with 2 µL of 4× Sample Buffer (Bio-Rad Laboratories, Inc.), to which (±)-dithiothreitol (FUJIFILM Wako Pure Chemical Corporation) had been added to reach 40 mg/mL, and heat-denatured under reducing conditions by heating at 100°C for 3 minutes. The heat-denatured sample was applied in 5 µL portions to each well of a 5 to 20% polyacrylamide gel (FUJIFILM Wako Pure Chemical Corporation) placed in 50 mM Tris buffer/380 mM glycine buffer (pH 8.3) containing 0.1% SDS, followed by electrophoresis at a constant current of 25 mA. After electrophoresis, the gel was immersed in EZFluor UV 1-step Fluorescent Protein Gel Stain (Merck) and shaken at room temperature for 60 minutes. After washing the gel with pure water, a band of the protein was detected using a lumino image analyzer (Amersham ImageQuan 800, Cytiva).

The electrophoresis image of SDS-PAGE is shown in Figure 31(a). The results of SDS-PAGE shows that the expression levels of hCDNF-HSA and HSA-hCDNF are at least two and five times higher than that of wild-type hCDNF, respectively, from the comparison of band intensities. It is understood that hCDNF, which has low expression levels and is difficult to produce in large quantities when expressed as a recombinant in its wild-type form, can be efficiently expressed in the form of hCDNF-SA or SA-hCDNF to express more hCDNF as a recombinant protein. The results of Western blotting also provided a similar understanding (Figure 31 (b)).

### [Example 37] Production of Wild-Type hGFL Expression Plasmid, hGFL-HSA Expression Plasmid, and HSA-hGFL Expression Plasmid

A DNA fragment having the nucleotide sequence represented by SEQ ID NO: 163, which contains a gene encoding wild-type hGDNF (SEQ ID NO: 162) with His6 added to the C terminal; a DNA fragment having the nucleotide sequence represented by SEQ ID NO: 165, which contains a gene encoding hGDNF-HSA having an amino acid sequence represented by SEQ ID NO: 164, a fusion protein formed by linking the C terminal of wild-type hGDNF and the N terminal of HSA via a GS linker, with His6 further added to the C terminal thereof; and a DNA fragment having the nucleotide sequence represented by SEQ ID NO: 167, which contains a gene encoding HSA-hGDNF having an amino acid sequence represented by SEQ ID NO: 166, a fusion protein formed by linking the C terminal of HSA and the N terminal of a pro-form of wild-type hGDNF via a GS linker, with His6 further added to the C terminal thereof, were synthesized. Hereafter, for convenience, wild-type hGDNF with His6 added to the C terminal is also referred to as wild-type hGDNF.

A DNA fragment having the nucleotide sequence represented by SEQ ID NO: 169, which contains a gene encoding wild-type hNRTN (SEQ ID NO: 168) with His6 added to the C terminal; a DNA fragment having the nucleotide sequence represented by SEQ ID NO: 171, which contains a gene encoding hNRTN-HSA having an amino acid sequence represented by SEQ ID NO: 170, a fusion protein formed by linking the C terminal of wild-type hNRTN and the N terminal of HSA via a GS linker, with His6 further added to the C terminal thereof; and a DNA fragment having the nucleotide sequence represented by SEQ ID NO: 173, which contains a gene encoding HSA--hNRTN-HTS6 having an amino acid sequence represented by SEQ ID NO: 172, a fusion protein formed by linking the C terminal of HSA and the N terminal of a pro-form of wild-type hNRTN via a GS linker, with His6 further added to the C terminal thereof, were synthesized. Hereafter, for convenience, wild-type hNRTN with His6 added to the C terminal is also referred to as wild-type hNRTN.

A DNA fragment having the nucleotide sequence represented by SEQ ID NO: 175, which contains a gene encoding wild-type hARTN (SEQ ID NO: 174) with His6 added to the C terminal; a DNA fragment having the nucleotide sequence represented by SEQ ID NO: 177, which contains a gene encoding hARTN-HSA having an amino acid sequence represented by SEQ ID NO: 176, a fusion protein formed by linking the C terminal of wild-type hARTN and the N terminal of HSA via a GS linker, with His6 further added to the C terminal thereof; and a DNA fragment having the nucleotide sequence represented by SEQ ID NO: 179, which contains a gene encoding HSA-hARTN-HIS6 having an amino acid sequence represented by SEQ ID NO: 178, a fusion protein formed by linking the C terminal of HSA and the N terminal of a pro-form of wild-type hARTN via a GS linker, with His6 further added to the C terminal thereof, were synthesized. Hereafter, for convenience, wild-type hARTN with His6 added to the C terminal is also referred to as wild-type hARTN.

A DNA fragment having the nucleotide sequence represented by SEQ ID NO: 181, which contains a gene encoding wild-type hPSPN (SEQ ID NO: 180) with His6 added to the C terminal; a DNA fragment having the nucleotide sequence represented by SEQ ID NO: 183, which contains a gene encoding hPSPN-HSA having an amino acid sequence represented by SEQ ID NO: 182, a fusion protein formed by linking the C terminal of wild-type hPSPN and the N terminal of HSA via a GS linker, with His6 further added to the C terminal thereof; and a DNA fragment having the nucleotide sequence represented by SEQ ID NO: 185, which contains a gene encoding HSA-hPSPN having an amino acid sequence represented by SEQ ID NO: 184, a fusion protein formed by linking the C terminal of HSA and the N terminal of a pro-form of wild-type hPSPN via a GS linker, with His6 further added to the C terminal thereof, were synthesized. Hereafter, for convenience, wild-type hPSPN with His6 added to the C terminal is also referred to as wild-type hPSPN.

The synthesized DNA fragment above were subjected to restriction enzyme treatment with MluI and NotI (Takara Bio Inc.) and separated by agarose gel electrophoresis. After EtBr staining, a band containing the target DNA fragment was excised under UV irradiation to extract DNA from the gel using QIAEX II Gel Extraction Kit (QIAGEN). Similarly, pCI-neo vector (Promega Corporation) was subjected to restriction enzyme treatment with MluI and NotI, followed by gel extraction and purification. Each DNA fragment treated with the restriction enzymes was mixed with the vector treated with the restriction enzymes, and ligation reaction was performed at 16°C for 60 minutes using DNA Ligation Kit Mighty Mix (Takara Bio Inc.).

Next, each ligation reaction solution was used to transform Escherichia coli (One Shot MAX Efficiency DH10B T1 Phage-Resistant Cells, Thermo Fisher Scientific Inc.). From the obtained transformant, each plasmid incorporating the target gene was purified in the same manner as described in Example 1.

### [Example 38] Transient Expression of hGFL

According to the high titer protocol of ExpiCHO Expression System (Thermo Fisher Scientific Inc.), ExpiCHO cells were transformed with each of the plasmids prepared in Example 37. After transformation, the cells were cultured for 8 days to express each of wild-type hGDNF, hGDNF-HSA, HSA-hGDNF, wild-type hNRTN, hNRTN-HSA, HSA-hNRTN, wild-type hARTN, hARTN-HSA, HSA-hARTN, wild-type hPSPN, hPSPN-HSA, and HSA-hPSPN in a culture supernatant. After culture, the culture solution was centrifuged to recover the culture supernatant.

### [Example 39] Confirmation of Expression Level of hGFL by Transient Expression (SDS-Page Electrophoresis)

Then, 6 µL of the culture supernatant obtained in Example 38 was mixed with 2 µL of 4× Sample Buffer (Bio-Rad Laboratories, Inc.), to which (±)-dithiothreitol (FUJIFILM Wako Pure Chemical Corporation) had been added to reach 40 mg/mL, and heat-denatured under reducing conditions by heating at 100°C for 3 minutes. The heat-denatured sample was applied in 5 µL portions to each well of a 5 to 20% polyacrylamide gel (FUJIFILM Wako Pure Chemical Corporation) placed in 50 mM Tris buffer/380 mM glycine buffer (pH 8.3) containing 0.1% SDS, followed by electrophoresis at a constant current of 25 mA. After electrophoresis, the gel was immersed in EZFluor UV 1-step Fluorescent Protein Gel Stain (Merck) and shaken at room temperature for 60 minutes. After washing the gel with pure water, a band of the protein was detected using a lumino image analyzer (Amersham ImageQuan 800, Cytiva).

The electrophoresis image of SDS-PAGE and the results of Western blotting are shown in Figure 32 (a) and Figure 32 (b), respectively.

The comparison of band intensities in the electrophoresis image of SDS-PAGE on hGDNF suggests that the expression level of hGDNF-HSA is at least five times higher than that of wild-type hGDNF. The same is true for Western blotting. Although it is difficult to compare HSA-hGDNF in the electrophoresis image of SDS-PAGE because the pro-form hGDNF is decomposed after expression, the comparison of band intensities detected by Western blotting suggests that the expression level of HSA-hGDNF is at least two times higher than that of wild-type hGDNF.

Regarding hNRTN, wild-type hNRTN is hardly detected in any of the electrophoresis image of SDS-PAGE and Western blotting. On the other hand, hNRTN-HSA is strongly detected in both electrophoresis image and Western blotting. HSA-hNRTN is not clearly detected in the electrophoresis image of SDS-PAGE because the pro-form hNRTN is decomposed, but detected by Western blotting.

Regarding hARTN, wild-type hARTN is hardly detected in any of the electrophoresis image of SDS-PAGE and Western blotting. On the other hand, hARTN-HSA is strongly detected in both electrophoresis image of SDS-PAGE and Western blotting. Although HSA-hARTN is weaker than hARTN-HSA, hARTN-HSA is also strongly detected in both electrophoresis image of SDS-PAGE and Western blotting.

Regarding hPSPN, wild-type hPSPN is hardly detected in any of the electrophoresis image of SDS-PAGE and Western blotting. On the other hand, hPSPN-HSA is strongly detected in both electrophoresis image of SDS-PAGE and Western blotting. Although HSA-hPSPN is weaker than hPSPN-HSA, hPSPN-HSA is also strongly detected in both electrophoresis image of SDS-PAGE and Western blotting.

From the above results, the following can be stated.

Although it is difficult to express wild-type hGDNF as a recombinant protein using CHO cells, hGDNF can be efficiently expressed as a recombinant protein by expressing it in the form of hGDNF-SA or SA-hGDNF. That is, hGDNF can be produced as a recombinant protein in large quantities by expressing it in the form of hGDNF-SA or SA-hGDNF. In particular, since hGDNF-SA enables particularly efficient expression, expression in the form of hGDNF-SA can be concluded to be more effective method for producing a large quantity of recombinant hGDNF.

Wild-type hNRTN is difficult to express as a recombinant protein. However, since hNRTN can be expressed as a recombinant protein by expressing it in the form of hNRTN-SA or SA-hNRTN, hNRTN can be produced as a recombinant protein by expressing it in such a form. In particular, since the form of hNRTN-SA enables particularly efficient expression of recombinant protein, expression in the form of hNRTN-SA can be concluded to be more effective method for producing a large quantity of recombinant hNRTN.

Wild-type hARTN is difficult to express as a recombinant protein using CHO cells. However, since hARTN can be efficiently expressed as a recombinant protein by expressing it in the form of hARTN-SA or SA-hARTN, hARTN can be efficiently produced as a recombinant protein by expressing it in such a form. In particular, since hARTN-SA enables particularly efficient expression, expression in the form of hARTN-SA can be concluded to be more effective method for producing a large quantity of recombinant hARTN.

Wild-type hPSPN is difficult to express as a recombinant protein using CHO cells. However, since hPSPN can be efficiently expressed as a recombinant protein by expressing it in the form of hPSPN-SA or SA-hPSPN, hPSPN can be efficiently produced as a recombinant protein by expressing it in such a form. In particular, since the form of hPSPN-SA enables particularly efficient expression of recombinant protein, expression in the form of PSPN-SA can be concluded to be more effective method for producing a large quantity of recombinant hPSPN.

### [Example 40] Production of hBDNF-VHH Expression Plasmid

The plasmid obtained in Example 27 incorporating the gene encoding wild-type hBDNF (SEQ ID NO: 132) with His6 added was subjected to restriction enzyme treatment with MluI and BamHI (Takara Bio Inc.), followed by gel extraction and purification. The purified DNA fragment was designated as insert DNA1.

A DNA fragment having the nucleotide sequence represented by SEQ ID NO: 187, which contains a gene encoding TfR-binding VHH (SEQ ID NO: 186), was synthesized. The DNA fragment was subjected to restriction enzyme treatment with BglII and NotI (Takara Bio Inc.), followed by gel extraction and purification. The purified DNA fragment was designated as insert DNA2.

pCI-neo vector (Promega Corporation) was subjected to restriction enzyme treatment with MluI and NotI (Takara Bio Inc.), followed by gel extraction and purification. The insert DNA1 and the insert DNA2 described above were mixed with the vector treated with the restriction enzymes to perform ligation reaction at 16°C for 60 minutes using Ligation Mix (Takara Bio Inc.). This reaction inserts a DNA fragment in which insert DNA2 is in-frame connected to the 3' end of insert DNA1.

Next, a ligation reaction solution was used to transform Escherichia coli (ECOS X Competent E. coli DH5a, NIPPON GENE CO., LTD.). To confirm whether the obtained transformant retained the target plasmid DNA, a single colony was cultured overnight in an LB liquid medium (LB Broth, Sigma-Aldrich) to purify a small amount of plasmid DNA using FastGene Plasmid Mini Kit (NIPPON Genetics Co., Ltd.). The purified plasmid DNA was subjected to restriction enzyme treatment with Mlu I and Not I and separated by agarose gel electrophoresis to confirm insertion of the target gene. This plasmid was purified by a conventional method.

### [Example 41] Production of hBDNF-HSA-VHH Expression Plasmid

The plasmid obtained in Example 28 incorporating the gene encoding hBDNF-HSA (SEQ ID NO: 148) with a histidine tag added was subjected to restriction enzyme treatment with MluI and BamHI (Takara Bio Inc.), followed by gel extraction and purification. The purified DNA fragment was designated as insert 3.

The insert DNA3 described above and the insert DNA2 obtained in Example 40 were mixed with the pCI-neo vector treated with the restriction enzymes obtained in Example 40 to perform ligation reaction at 16°C for 60 minutes using Ligation Mix (Takara Bio Inc.). This reaction inserts a DNA fragment in which insert DNA2 is in-frame connected to the 3' end of insert DNA3.

Next, a ligation reaction solution was used to transform Escherichia coli (ECOS X Competent E. coli DH5a, NIPPON GENE CO., LTD.). To confirm whether the obtained transformant retained the target plasmid DNA, a single colony was cultured overnight in an LB liquid medium (LB Broth, Sigma-Aldrich) to purify a small amount of plasmid DNA using FastGene Plasmid Mini Kit (NIPPON Genetics Co., Ltd.). The purified plasmid DNA was subjected to restriction enzyme treatment with MluI and NotI and separated by agarose gel electrophoresis to confirm insertion of the target gene. This plasmid was purified by a conventional method.

### [Example 42] Production of hBDNF-VHH-HSA Expression Plasmid

Using the hBDNF-VHH expression plasmid obtained in Example 40 as a template, a PCR reaction was carried out with a forward primer represented by SEQ ID NO: 188 (SF158) and a reverse primer represented by SEQ ID NO: 189 (SF211). The DNA fragment obtained by the PCR was subjected to restriction enzyme treatment with MluI and BamHI (Takara Bio Inc.), followed by gel extraction and purification. The purified DNA fragment was designated as insert 4.

Using a DNA fragment having the nucleotide sequence represented by SEQ ID NO: 141, which contains a gene encoding wild-type HSA (SEQ ID NO: 140) synthesized in Example 28, as a template, a PCR reaction was carried out with a forward primer represented by SEQ ID NO: 142 and a reverse primer represented by SEQ ID NO: 190 (SF149). The DNA fragment obtained by the PCR was subjected to restriction enzyme treatment with BgIII and NotI (Takara Bio Inc.), followed by gel extraction and purification. The purified DNA fragment was designated as insert 5.

The insert DNA4 and the insert DNA5 described above were mixed with the pCI-neo vector treated with the restriction enzymes obtained in Example 40 to perform ligation reaction at 16°C for 60 minutes using Ligation Mix (Takara Bio Inc.). This reaction inserts a DNA fragment in which insert DNA5 is in-frame connected to the 3' end of insert DNA4.

Next, a ligation reaction solution was used to transform Escherichia coli (ECOS X Competent E. coli DH5a, NIPPON GENE CO., LTD.). To confirm whether the obtained transformant retained the target plasmid DNA, a single colony was cultured overnight in an LB liquid medium (LB Broth, Sigma-Aldrich) to purify a small amount of plasmid DNA using FastGene Plasmid Mini Kit (NIPPON Genetics Co., Ltd.). The purified plasmid DNA was subjected to restriction enzyme treatment with MluI and NotI and separated by agarose gel electrophoresis to confirm insertion of the target gene. This plasmid was purified by a conventional method.

### [Example 43] Transient Expression of hBDNF-VHH, hBDNF-HSA-VHH, and hBDNF-VHH-HSA

According to the high titer protocol of ExpiCHO(TM) Expression System (Thermo Fisher Scientific Inc.), ExpiCHO cells were transformed with a plasmid incorporating the gene encoding each of hBDNF-VHH, hBDNF-HSA-VHH, and hBDNF-VHH-HSA obtained in Examples 40, 41, and 42. After transformation, the cells were cultured for 8 days to express each of hBDNF-VHH, hBDNF-HSA-VHH, and hBDNF-VHH-HSA in a culture supernatant. After culture, the culture solution was centrifuged to recover the culture supernatant. Note that hBDNF-VHH expressed here has the amino acid sequence of SEQ ID NO: 191, hBDNF-HSA-VHH has the amino acid sequence of SEQ ID NO: 192, and hBDNF-VHH-HSA has the amino acid sequence of SEQ ID NO: 193.

### [Example 44] Confirmation of Expression Level of hBDNF-VHH, hBDNF-HSA-VHH, and hBDNF-VHH-HSA by Transient Expression (SDS-PAGE)

Then, 10 µL of the culture supernatant obtained in Example 43 was mixed with 8 µL of 2× Sample Buffer (Bio-Rad Laboratories, Inc.) and 2 µL of 2-mercaptoethanol (Bio-Rad Laboratories, Inc.) and heat-denatured under reducing conditions by heating at 100°C for 3 minutes. The heat-denatured sample was applied in 5 µL portions to each well of a 5 to 20% polyacrylamide gel (FUJIFILM Wako Pure Chemical Corporation) placed in 50 mM Tris buffer/380 mM glycine buffer (pH 8.3) containing 0.1% SDS, followed by electrophoresis at a constant current of 25 mA. After electrophoresis, the gel was immersed in Oriole fluorescent gel stain (Bio-Rad Laboratories, Inc.) and shaken at room temperature for 90 minutes. After washing the gel with pure water, a band of the protein was detected with a lumino image analyzer (Amersham Imager 600RGB, Cytiva).

The electrophoresis image of SDS-PAGE is shown in Figure 33. In hBDNF-HSA-VHH and hBDNF-VHH-HSA, there is a strong band at the location (A) corresponding to these molecular weights, and it is possible to confirm their abundant expression. On the other hand, in hBDNF-VHH, there is no clear band at the location (B) corresponding to the molecular weight of hBDNF-VHH, indicating that the expression level is low.

### [Example 45] Purification of hBDNF-HSA

To the culture supernatant on day 8 containing hBDNF-HSA by transient expression obtained in Example 29 was added 50 mM HEPES buffer (pH 7.4) containing four volumes of 500 mM NaCl. This solution was loaded at a constant flow rate onto a HisTrap HP column (Cytiva) equilibrated with 50 mM HEPES buffer (pH 7.4) containing 500 mM NaCl. The column was then washed with five column volumes of the same buffer at the same flow rate. Then, 50 mM HEPES buffer (pH 7.4) containing 500 mM NaCl and 500 mM imidazole was used to elute hBDNF-HSA adsorbed onto the column with an imidazole concentration gradient.

To the solution containing hBDNF-HS obtained through HisTrap HP purification was added 50 mM HEPES buffer (pH 7.4) containing nine volumes of 50 mM NaCl. This solution was loaded at a constant flow rate onto a HiTrap CM FF column (Cytiva) equilibrated with 50 mM HEPES buffer (pH 7.4) containing 50 mM NaCl. The column was then washed with five column volumes of the same buffer at the same flow rate. Then, 50 mM HEPES buffer (pH 7.4) containing 500 mM NaCl was used to elute hBDNF-HSA adsorbed onto the column with a salt concentration gradient. The solution containing the hBDNF-HSA was designated as an hBDNF-HSA purified product.

### [Example 46] Purification of hBDNF-HSA-VHH and hBDNF-VHH-HSA

To the culture supernatant on day 8 containing each of hBDNF-HSA-VHH and hBDNF-VHH-HSA by transient expression obtained in Example 43 was added 20 mM HEPES buffer (pH 7.4) containing an equal volume of 150 mM NaCl. This solution was loaded onto a MabSelect Xtra column (Cytiva) equilibrated with 20 mM HEPES buffer (pH 7.4) containing 150 mM NaCl to adsorb each protein onto the column. The column was then washed with five column volumes of the same buffer. Each protein adsorbed onto the column was then eluted with 10 mM acetate buffer (pH 3.5) containing eight column volumes of 100 mM NaCl. The eluate was immediately neutralized by placing it in a vessel containing 1 M HEPES buffer (pH 8.0) in advance.

To the solution containing each of hBDNF-HSA-VHH and hBDNF-VHH-HSA obtained through MabSelect Xtra purification was added 50 mM MES buffer (pH 6.0) containing four volumes of 50 mM NaCl. This solution was loaded at a constant flow rate onto a HiTrap SP HP column (Cytiva) equilibrated with 50 mM MES buffer (pH 6.0) containing 50 mM NaCl. The column was then washed with five column volumes of the same buffer at the same flow rate. Then, 50 mM MES buffer (pH 6.0) containing 500 mM NaCl was used to elute each of hBDNF-HSA-VHH and hBDNF-VHH-HSA adsorbed onto the column with a salt concentration gradient. The solutions containing the hBDNF-HSA-VHH and the hBDNF-VHH-HSA were designated as an hBDNF-HSA-VHH purified product and an hBDNF-VHH-HSA purified product, respectively.

### [Example 47] SE-HPLC Analysis on hBDNF-HSA, hBDNF-HSA-VHH, and hBDNF-VHH-HSA Purified Products

The molecular sizes of hBDNF-HSA, hBDNF-HSA-VHH, and hBDNF-VHH-HSA purified products were evaluated using SE-HPLC. Superdex 200 Increase 10/300 GL (Cytiva) was set in Shimadzu HPLC system LC-20A (Shimadzu Corporation), and an absorptiometer was installed downstream thereof to measure the absorbance (measurement wavelength: 215 nm) of the effluent from the column continuously. After the column was equilibrated with 0.2 M sodium phosphate buffer, the hBDNF-HSA purified product obtained in Example 45, the hBDNF-HSA-VHH purified product obtained in Example 46, and the hBDNF-VHH-HSA purified product were each loaded onto the column, and the 0.2 M sodium phosphate buffer was allowed to flow at a constant flow rate. During this time, an elution profile was obtained by measuring the absorbance (Measurement wavelength: 215 nm) of the effluent from the column.

The elution profile is shown in Figure 34. All of hBDNF-HSA, hBDNF-HSA-VHH, and hBDNF-VHH-HSA have one main peak, which is considered to be a peak of a monomer of hBDNF-HSA, hBDNF-HSA-VHH, and hBDNF-VHH-HSA, respectively. The peak area ratios indicate that 97.6%, 99.0%, and 98.1% of hBDNF-HSA, hBDNF-HSA-VHH, and hBDNF-VHH-HSA exist as monomers, respectively.

### [Example 48] Binding Affinity Evaluation of hBDNF-HSA-VHH and hBDNF-VHH-HSA for Human and Mouse TfR

The binding affinity of hBDNF-HSA-VHH and hBDNF-VHH-HSA for human or mouse TfR was measured with Octet RED96e (ForteBio), a biomolecule interaction analysis system using biolayer interferometry. Human TfR (hTfR) used was a recombinant containing the amino acid sequence of the extracellular domain of hTfR from the 89th cysteine residue from the N terminal side to the phenylalanine at the C terminal in the amino acid sequence of hTfR represented by SEQ ID NO: 22. Mouse TfR (mTfR) used was, a recombinant of the extracellular domain of mTfR corresponding to the extracellular domain of hTfR.

Each of the hBDNF-HSA-VHH purified product and the hBDNF-VHH-HSA purified product obtained in Example 46 was serially diluted with HBS-P + buffer (Cytiva) containing 1% BSA (FUJIFILM Wako Pure Chemical Corporation) to prepare solutions of 80, 40, 20 nM. This was used as an analyte solution. Human TfR and mouse TfR were each diluted in HBS-P + buffer containing 1% BSA to prepare a 20 µg/mL solution. This was used as a ligand solution.

The sample solution and the ligand solution were added to predetermined wells of a black 96-well plate for Octet RED96e (Greiner) in 200 µL each. To the wells for baseline, dissociation and washing was added 200 µL of HBS-P + buffer containing 1% BSA. To the wells for regeneration was added 200 µL of 10 mM glycine buffer (pH 1.7). To the wells for activation was added 200 µL of 0.5 mM NiCl₂ solution. This 96-well plate and a Ni-NTA biosensor (ForteBio) were placed in place in an Octet RED96e instrument. After data acquisition by operating the Octet RED96e under the conditions shown in Table 9 below, the association rate constant (kₒₙ) and dissociation rate constant (k_{off}) in the 1:1 binding model were calculated using the software attached to Octet RED96e, and the dissociation constant (Kd) was calculated. The measurement was carried out at a temperature of 25 to 30°C.

Table 10 shows the measurement results of the binding affinity for hTfR and mTfR. It can be seen that both hBDNF-HSA-VHH and hBDNF-VHH-HSA have high binding affinity for hTfR. It can also be seen that both of them have high binding affinity for mTfR, although they are slightly weaker than those for hTfR.

**[Table 9]**

| **Table 9. Operating condition of Octet RED96e** | | | | |
|---|---|---|---|---|
| | **Step** | **Contact time (see)** | **Rotational speed (rpm)** | **Threshold** |
| **1** | **Regeneration** | **5** | **1000** | **-** |
| **2** | **Washing** | **5** | **1000** | **-** |
| **Steps 1 and 2 are repeated 6 times.** | | | | |
| **3** | **Activation** | **60** | **1000** | **-** |
| **4** | **Baseline 1** | **600** | **1000** | **-** |
| **5** | **Loading** | **600** | **1000** | **1.0** |
| **6** | **Baseline 2** | **150** | **1000** | **-** |
| **7** | **Binding** | **120** | **1000** | **-** |
| **8** | **Dissociation** | **200** | **1000** | **-** |
| **Steps 1 to 8 are repeated until all the samples are measured.** | | | | |

**[Table 10]**

| Table 10. Measurement results of binding affinity of hBDNF-HSA-VHH and hBDNF-VHH-HSA for hTFR and mTFR | | | | |
|---|---|---|---|---|
| | | *k*ₒₙ (1/Ms) | *k*_{off} *(1*/*s)* | *K*_{D} (M) |
| BDNF-HSA-VHH | human TfR | 3.18 × 10⁵ | 1.46 × 10⁻⁴ | 4.60 × 10-10 |
| BDNF-VHH-HSA | | 2.36 × 10⁵ | 2.13 × 10⁻⁴ | 9.04 × 10⁻¹⁰ |
| BDNF-HSA-VHH | mouse TfR | 3.26 × 10⁵ | 6.64 × 10⁻⁴ | 2.04 × 10⁻⁹ |
| BDNF-VHH-HSA | | 1.88 × 10⁵ | 5.30 × 10⁻⁴ | 2.82 × 10⁻⁹ |

### [Example 49] Production of Retroviral Vector Plasmid with TrkB Receptor Chimeric Gene Inserted

A DNA fragment having the nucleotide sequence represented by SEQ ID NO: 194, which contains a gene encoding a chimeric receptor consisting of extracellular domain of BDNF receptor TrkB and transmembrane domain and cytoplasmic domain of thrombopoietin receptor c-Mpl, was synthesized. This fragment was subjected to restriction enzyme treatment with MluI and NotI (Takara Bio Inc.) and separated by agarose gel electrophoresis. After EtBr staining, a band containing the target DNA fragment was excised under UV irradiation, and the DNA fragment was extracted from the gel using QIAEX II Gel Extraction Kit (QIAGEN) and purified. Similarly, retroviral vector pMX-II was subjected to restriction enzyme treatment with MluI and NotI, followed by gel extraction and purification. The DNA fragment treated with the restriction enzymes was mixed with the vector treated with the restriction enzymes to perform ligation reaction using Ligation Mix (Takara Bio Inc.).

Next, a ligation reaction solution was used to transform Escherichia coli (ECOS X Competent E. coli DH5a, NIPPON GENE CO., LTD.). From the obtained transformant, a plasmid with the target gene inserted therein was purified in the same manner as described in Example 1.

### [Example 50] Generation of Retrovirus with TrkB Receptor Chimeric Gene Inserted

HEK293 cells were cultured in DMEM medium (Thermo Fisher Scientific Inc.) containing 10% fetal bovine serum (Cytiva). HEK293 cells in the exponential growth phase were seeded at a density of 5 × 10⁵ cells/mL and cultured overnight at 37°C in 5% CO₂. Next, the HEK293 cells were transfected using Lipofectamine 3000 Transfection Reagent (Thermo Fisher Scientific Inc.). To 250 µL of Opti-MEMI medium (Thermo Fisher Scientific Inc.), 5.5 µg of the plasmid produced in Example 49, 5.5 µg of packaging plasmid pCL-Eco (Promega Corporation), and 22 µL of P3000 Reagent were added and mixed to prepare a DNA solution. In addition, 16.5 µL of Lipofectamine 3000 was added to 250 µL of the Opti-MEMI medium and mixed to prepare a lipid solution. The DNA solution and the lipid solution were mixed and allowed to stand at room temperature for 10 minutes, then added directly to the culture solution of HEK293 cells cultured overnight, and cultured under conditions of 37°C and 5% CO₂. After 24 hours, the culture supernatant was removed, the HEK293 cells were washed with DPBS (Thermo Fisher Scientific Inc.), DMEM medium containing 10% fetal bovine serum was added to culture the cells under conditions of 37°C and 5% CO₂, thereby expressing recombinant Moloney murine leukemia retrovirus (MoMLV) incorporating TrkB receptor chimeric genes in the culture supernatant. After 24 hours, the culture supernatant containing the recombinant MoMLV was recovered, and the supernatant obtained by centrifugation at 3000 rpm for 5 minutes was used as a retrovirus solution.

### [Example 51] Construction of BDNF-Dependent Cell BaF/TrkB·c-Mpl Chimera Cell

To use the WEHI-3 cell culture supernatant as a source of mIL-3 for the preparation of BaF3 cells to be used for transformation, the cells were cultured in RPMI1640 medium (Thermo Fisher Scientific Inc.) containing 10% fetal bovine serum (Cytiva), and the culture supernatant was fractionated. The resulting supernatant was filtered through a membrane (Nalgene) having a pore size of 0.22 µm. The BaF3 cells were cultured in the RPMI1640 medium containing 10% fetal bovine serum and 10% WEHI-3 cell culture supernatant.

A cell culture solution of 2 × 10⁶ BaF3 cells in an exponential growth phase was transferred to a centrifuge tube and centrifuged at 1000 rpm for 5 minutes, followed by removal of the supernatant. To 1.5 mL of the retrovirus solution prepared in Example 50, 0.3 mL of the WEHI-3 cell culture supernatant and 1.2 mL of the RPMI1640 medium containing 10% fetal bovine serum were added and mixed, and the mixture was added to the previously collected BaF3 cells and suspended. An additional 3 µL of 10 mg/mL polybrene was added, mixed, transferred to a culture flask, and cultured under conditions of 37°C and 5% CO₂. After 8 hours, 0.3 mL of the WEHI-3 cell culture supernatant and 2.7 mL of the RPMI1640 medium containing 10% fetal bovine serum were added, and the cells were further cultured for 16 hours. The whole cell culture solution was collected and centrifuged at 1000 rpm for 5 minutes to remove the supernatant. The cell pellet was suspended in 5 mL of DPBS and centrifuged at 1000 rpm for 5 minutes to remove the supernatant. This operation was repeated three times to wash the cells. The washed cells were suspended in RPMI1640 medium containing 100 ng/mL hBDNF and 10% fetal bovine serum to start selective culture. The cells were subcultured every 2 to 3 days to obtain cells that acquired hBDNF-dependent proliferation ability. These cells were designated as BaF/TrkB·c-Mpl chimera cells.

### [Example 52] Evaluation of BDNF Activity of hBDNF-HSA, hBDNF-HSA-VHH, and hBDNF-VHH-HSA Purified Products Using BaF/TrkB·c-Mpl Chimera Cell

The biological activity of each fusion protein was evaluated by a cell proliferation assay using cells having a BDNF-dependent cell proliferation ability. The cell culture solution of 2.4 × 10⁶ BaF/TrkB·c-Mpl chimera cells constructed in Example 51 was transferred to a centrifuge tube and centrifuged at 1200 rpm for 2 minutes to remove the supernatant. The cell pellet was suspended in 5 mL of the RPMI1640 medium containing 10% fetal bovine serum and centrifuged at 1200 rpm for 2 minutes to remove the supernatant. This operation was repeated twice to remove hBDNF in the medium. The cell cycle was synchronized by suspending the cell pellet in 6 mL of the RPMI1640 medium containing 10% fetal bovine serum, transferring the whole volume to a culture flask, and culturing under conditions of 37°C and 5% CO₂ for 22 hours. The cell culture solution was diluted in the same medium to a volume of 2 × 10⁵ cells/mL, and 50 µL was plated in each well of a 96-well plate. Subsequently, recombinant wild-type hBDNF (Abcam), the purified product of hBDNF-HSA obtained in Example 45 and the purified products of hBDNF-HSA-VHH and hBDNF-VHH-HSA obtained in Example 46 were serially diluted in the same medium to a concentration of 200,000, 40,000, 8,000, 1,600, 320, 64, 12.8, 2.56, 0.512 pmol/L. Each of these dilutions was added to each well of a 96-well plate containing BaF/TrkB·c-Mpl chimera cells at a volume of 50 µL, shaken with a plate mixer, and then cultured under conditions of 37°C and 5% CO₂. After 48 hours, 20 µL of CellTiter 96 AQueous One Solution Cell Proliferation Assay (MTS) reagent (Promega Corporation) was added to each well of the 96-well plate, shaken with a plate mixer, and then cultured under conditions of 37°C and 5% CO₂ for 2 hours. Next, 25 µL of 10% SDS solution was added to each well of the 96-well plate and mixed with a plate mixer to stop the reaction, and the absorbance at 490 nm was measured with an absorption plate reader. Based on the measurement results, a 4-parameter logistic curve plot was prepared using SoftMax Pro software (Molecular Devices, LLC.) to obtain a dose-growth curve. Note that all measurements were performed in duplicate.

Figure 35 shows results of BDNF activity evaluation. All molecules are found to have the activity of promoting cell proliferation of BaF/TrkB·c-Mpl chimera cells. The EC50 of wild-type hBDNF is 1.19 nmol/L, and hBDNF-HSA, hBDNF-HSA-VHH, and hBDNF-VHH-HSA are 1.65, 3.32, 5.53, respectively.

### [Example 53] Summary of Experimental Result on hBDNF-VHH, hBDNF-HSA-VHH, and hBDNF-VHH-HSA

From the experimental results obtained in Examples 44 to 52, the following can be concluded.

As shown in Example 33, hBDNF, which is difficult to be expressed as a recombinant protein using CHO cells, can be efficiently expressed as a recombinant protein by forming it into a fusion protein with HSA. The same applies to a fusion protein formed by binding hBDNF and VHH, and this fusion protein can be efficiently expressed as a recombinant protein by further binding HSA thereto. In addition, a fusion protein formed by binding hBDNF and VHH, to which HAS is bound, has activity as hBDNF and can bind to hTfR and mTfR. Thus, it can be concluded that further binding of HSA to the fusion protein formed by binding hBDNF and VHH is an effective method for producing a large quantity of such a fusion protein as a recombinant protein having a desired activity. Since most of the fusion protein formed by binding hBDNF and VHH, to which HSA is bound, is a monomer when expressed as a recombinant protein, such a measure can be considered excellent from the viewpoint of product management.

### [Example 54] Evaluation of Expression Level of hBDNF-HSA Using BaF/TrkB·c-Mpl Chimera Cell

A cell proliferation assay was performed as described in Example 52 to measure hBDNF content in a culture supernatant. The culture supernatant on day 8 containing each of wild-type hBDNF and hBDNF-HSA by transient expression obtained in Example 29 was diluted 1,000-fold with RPMI1640 medium containing 10% fetal bovine serum, and 50 µL of the dilution was added to each well of a 96-well plate containing BaF/TrkB·c-Mpl chimera cells to perform a cell proliferation assay. Using the dose-growth curve of wild-type hBDNF as a calibration curve, the hBDNF content in the culture supernatant was calculated. In addition, the culture supernatant on day 8 containing hBDNF-HSA by transient expression obtained in Example 29 was diluted 10,000-fold with RPMI1640 medium containing 10% fetal bovine serum, and 50 µL of the dilution was added to each well of a 96-well plate containing BaF/TrkB·c-Mpl chimera cells to perform a cell proliferation assay. Using the dose-growth curve of a hBDNF-HSA purified product as a calibration curve, the hBDNF-HSA content in the culture supernatant was calculated.

The results are shown in Figure 36. It can be seen that hBDNF-HSA has almost the same BDNF activity per molecule as that of wild-type hBDNF. That is, the binding of HSA to the C-terminal of hBDNF has little effect on the activity of hBDNF. Based on the measurement values, the concentrations of hBDNF-HSA and wild-type hBDNF contained in the culture supernatant obtained in Example 29 were calculated based on the activity of BDNF. As a result, the expression level of hBDNF-HSA was calculated to be about 26 times that of the wild-type hBDNF. The expression level of HSA-hBDNF was calculated to be about three times that of the wild-type hBDNF. This value is generally consistent with the ELISA measurement value shown in Table 8.

### [Industrial Applicability]

The present invention enables efficient and industrial production of, for example, a protein that is challenging to produce as a recombinant protein by expressing the protein as a recombinant protein in the form of a fusion protein with SA.

### Sequence Listing Free Text

SEQ ID NO: 1: Example of amino acid sequence of wild-type hGALC
SEQ ID NO: 2: Example of nucleotide sequence encoding wild-type hGALC, synthetic sequence
SEQ ID NO: 3: Example of amino acid sequence of wild-type HSA
SEQ ID NO: 4: Example of nucleotide sequence encoding wild-type HSA, synthetic sequence
SEQ ID NO: 5: Example of amino acid sequence of HSA-hGALC
SEQ ID NO: 6: Example of nucleotide sequence encoding HSA-hGALC, synthetic sequence
SEQ ID NO: 7: Example of amino acid sequence of hGALC-HSA
SEQ ID NO: 8: Example of nucleotide sequence encoding hGALC-HSA, synthetic sequence
SEQ ID NO: 9: Example 1 of amino acid sequence of linker
SEQ ID NO: 10: Example 2 of amino acid sequence of linker
SEQ ID NO: 11: Example 3 of amino acid sequence of linker
SEQ ID NO: 12: Amino acid sequence of human serum albumin Redhill
SEQ ID NO: 13: Amino acid sequence of mutant human serum albumin (HSA-A320T)
SEQ ID NO: 14: Example of amino acid sequence of wild-type mGALC
SEQ ID NO: 15: Example of nucleotide sequence encoding wild-type mGALC, synthetic sequence
SEQ ID NO: 16: Example of amino acid sequence of wild-type MSA
SEQ ID NO: 17: Example of nucleotide sequence encoding wild-type MSA, synthetic sequence
SEQ ID NO: 18: Example of amino acid sequence of MSA-mGALC
SEQ ID NO: 19: Example of nucleotide sequence encoding MSA-mGALC, synthetic sequence
SEQ ID NO: 20: Example of amino acid sequence of mGALC-MSA
SEQ ID NO: 21: Example of nucleotide sequence encoding mGALC-MSA, synthetic sequence
SEQ ID NO: 22: Amino acid sequence of human transferrin receptor
SEQ ID NO: 23: Amino acid sequence of light chain of anti-hTfR antibody
SEQ ID NO: 24: Example of amino acid sequence of wild-type hIL-10
SEQ ID NO: 25: Amino acid sequence of Fab heavy chain of anti-hTfR antibody
SEQ ID NO: 26: pCI Insert F3 primer, synthetic sequence
SEQ ID NO: 27: pCI Insert R3 primer, synthetic sequence
SEQ ID NO: 28: Amino acid sequence of anti-hTfR antibody Fab heavy chain-HSA-hGALC
SEQ ID NO: 29: Nucleotide sequence encoding anti-hTfR antibody Fab heavy chain-HSA-hGALC, synthetic sequence
SEQ ID NO: 30: Amino acid sequence of HSA-hGALC-anti-hTfR antibody Fab heavy chain
SEQ ID NO: 31: Nucleotide sequence encoding HSA-hGALC-anti-hTfR antibody Fab heavy chain, synthetic sequence
SEQ ID NO: 32: Amino acid sequence of anti-hTfR antibody Fab heavy chain-hGALC-HSA
SEQ ID NO: 33: Nucleotide sequence encoding anti-hTfR antibody Fab heavy chain-hGALC-HSA, synthetic sequence
SEQ ID NO: 34: Amino acid sequence of hGALC-HSA-anti-hTfR antibody Fab heavy chain
SEQ ID NO: 35: Nucleotide sequence encoding hGALC-HSA-anti-hTfR antibody Fab heavy chain, synthetic sequence
SEQ ID NO: 36: Nucleotide sequence encoding light chain of anti-hTfR antibody, synthetic sequence
SEQ ID NO: 37: Example of amino acid sequence of wild-type hGBA
SEQ ID NO: 38: Example of nucleotide sequence encoding wild-type hGBA
SEQ ID NO: 39: Example of amino acid sequence of HSA-hGBA
SEQ ID NO: 40: Example of nucleotide sequence encoding HSA-hGBA, synthetic sequence
SEQ ID NO: 41: Example of amino acid sequence of hGBA-HSA
SEQ ID NO: 42: Example of nucleotide sequence encoding hGBA-HSA, synthetic sequence
SEQ ID NO: 43: Example of amino acid sequence of wild-type mGBA
SEQ ID NO: 44: Example of nucleotide sequence encoding wild-type mGBA
SEQ ID NO: 45: Example of amino acid sequence of MSA-mGBA
SEQ ID NO: 46: Example of nucleotide sequence encoding MSA-mGBA, synthetic sequence
SEQ ID NO: 47: Example of amino acid sequence of mGBA-MSA
SEQ ID NO: 48: Example of nucleotide sequence encoding mGBA-MSA, synthetic sequence
SEQ ID NO: 49: Example of nucleotide sequence encoding wild-type hIL-10, synthetic sequence
SEQ ID NO: 50: Example of amino acid sequence of HSA-hIL-10
SEQ ID NO: 51: Example of nucleotide sequence encoding HSA-hIL-10, synthetic sequence
SEQ ID NO: 52: Example of amino acid sequence of hIL-10-HSA
SEQ ID NO: 53: Example of nucleotide sequence encoding hIL-10-HSA, synthetic sequence
SEQ ID NO: 54: Example 2 of amino acid sequence of HSA-hIL-10
SEQ ID NO: 55: Example 2 of amino acid sequence of hIL-10-HSA
SEQ ID NO: 56: Example 3 of amino acid sequence of HSA-hIL-10
SEQ ID NO: 57: Example 3 of amino acid sequence of hIL-10-HSA
SEQ ID NO: 58: Example 4 of amino acid sequence of HSA-hIL-10
SEQ ID NO: 59: Example 4 of amino acid sequence of hIL-10-HSA
SEQ ID NO: 60: Example of amino acid sequence of wild-type hBDNF
SEQ ID NO: 61: Example of nucleotide sequence encoding wild-type hBDNF, synthetic sequence
SEQ ID NO: 62: Example of amino acid sequence of wild-type hNGF
SEQ ID NO: 63: Example of nucleotide sequence encoding wild-type hNGF, synthetic sequence
SEQ ID NO: 64: Example of amino acid sequence of wild-type hNT-3
SEQ ID NO: 65: Example of nucleotide sequence encoding wild-type hNT-3, synthetic sequence
SEQ ID NO: 66: Example of amino acid sequence of wild-type hNT-4
SEQ ID NO: 67: Example of nucleotide sequence encoding wild-type hNT-4, synthetic sequence
SEQ ID NO: 68: Example of amino acid sequence of HSA-hBDNF
SEQ ID NO: 69: Example of nucleotide sequence encoding HSA-hBDNF, synthetic sequence
SEQ ID NO: 70: Example of amino acid sequence of HSA-hNGF
SEQ ID NO: 71: Example of nucleotide sequence encoding HSA-hNGF, synthetic sequence
SEQ ID NO: 72: Example of amino acid sequence of HSA-hNT-3
SEQ ID NO: 73: Example of nucleotide sequence encoding HSA-hNT-3, synthetic sequence
SEQ ID NO: 74: Example of amino acid sequence of HSA-hNT-4
SEQ ID NO: 75: Example of nucleotide sequence encoding HSA-hNT-4, synthetic sequence
SEQ ID NO: 76: Example of amino acid sequence of hBDNF-HSA
SEQ ID NO: 77: Example of nucleotide sequence encoding hBDNF-HSA, synthetic sequence
SEQ ID NO: 78: Example of amino acid sequence of hNGF-HSA
SEQ ID NO: 79: Example of nucleotide sequence encoding hNGF-HSA, synthetic sequence
SEQ ID NO: 80: Example of amino acid sequence of hNT-3-HSA
SEQ ID NO: 81: Example of nucleotide sequence encoding hNT-3-HSA, synthetic sequence
SEQ ID NO: 82: Example of amino acid sequence of hNT-4-HSA
SEQ ID NO: 83: Example of nucleotide sequence encoding hNT-4-HSA, synthetic sequence
SEQ ID NO: 84: Example of amino acid sequence of wild-type hBDNF (pro-form)
SEQ ID NO: 85: Example 2 of amino acid sequence of HSA-hBDNF
SEQ ID NO: 86: Example 2 of amino acid sequence of hBDNF-HSA
SEQ ID NO: 87: Example of amino acid sequence of wild-type hNGF (pro-form)
SEQ ID NO: 88: Example 2 of amino acid sequence of HSA-hNGF
SEQ ID NO: 89: Example 2 of amino acid sequence of hNGF-HSA
SEQ ID NO: 90: Example of amino acid sequence of wild-type hNT-3 (pro-form)
SEQ ID NO: 91: Example 2 of amino acid sequence of HSA-hNT-3
SEQ ID NO: 92: Example 2 of amino acid sequence of hNT-3-HSA
SEQ ID NO: 93: Example of amino acid sequence of wild-type hNT-4 (pro-form)
SEQ ID NO: 94: Example 2 of amino acid sequence of HSA-hNT-4
SEQ ID NO: 95: Example 2 of amino acid sequence of hNT-4-HSA
SEQ ID NO: 96: Amino acid sequence of light chain CDR1 of anti-hTfR antibody
SEQ ID NO: 97: Amino acid sequence of light chain CDR2 of anti-hTfR antibody
SEQ ID NO: 98: Amino acid sequence of light chain CDR3 of anti-hTfR antibody
SEQ ID NO: 99: Amino acid sequence 1 of heavy chain CDR1 of anti-hTfR antibody
SEQ ID NO: 100: Amino acid sequence 1 of heavy chain CDR2 of anti-hTfR antibody
SEQ ID NO: 101: Amino acid sequence 1 of heavy chain CDR3 of anti-hTfR antibody
SEQ ID NO: 102: Amino acid sequence 2 of heavy chain CDR1 of anti-hTfR antibody
SEQ ID NO: 103: Amino acid sequence 2 of heavy chain CDR2 of anti-hTfR antibody
SEQ ID NO: 104: Amino acid sequence 2 of heavy chain CDR3 of anti-hTfR antibody
SEQ ID NO: 105: Amino acid sequence 1 of CDR1 of hTfR-affinity peptide
SEQ ID NO: 106: Amino acid sequence 2 of CDR1 of hTfR-affinity peptide
SEQ ID NO: 107: Amino acid sequence 1 of CDR2 of hTfR-affinity peptide
SEQ ID NO: 108: Amino acid sequence 2 of CDR2 of hTfR-affinity peptide
SEQ ID NO: 109: Amino acid sequence 1 of CDR3 of hTfR-affinity peptide
SEQ ID NO: 110: Amino acid sequence 2 of CDR3 of hTfR-affinity peptide
SEQ ID NO: 111: Amino acid sequence 3 of CDR2 of hTfR-affinity peptide
SEQ ID NO: 112: Amino acid sequence 4 of CDR2 of hTfR-affinity peptide
SEQ ID NO: 113: Nucleotide sequence of first inverted terminal repeat (first ITR) in AAV of serotype 2, wild-type
SEQ ID NO: 114: Nucleotide sequence of first inverted terminal repeat (second ITR) in AAV of serotype 2, wild-type
SEQ ID NO: 115: Preferred example of nucleotide sequence of first inverted terminal repeat (first ITR), synthetic sequence
SEQ ID NO: 116: Preferred example of nucleotide sequence of second inverted terminal repeat (second ITR), synthetic sequence
SEQ ID NO: 117: Preferred example of nucleotide sequence of first long terminal repeat (first LTR), synthetic sequence
SEQ ID NO: 118: Preferred example of nucleotide sequence of second long terminal repeat (second LTR), synthetic sequence
SEQ ID NO: 119: Nucleotide sequence of leader of Sendai virus genome
SEQ ID NO: 120: Nucleotide sequence of trailer of Sendai virus genome
SEQ ID NO: 121: Nucleotide sequence of mouse α-fetoprotein enhancer/mouse albumin promoter, synthetic sequence
SEQ ID NO: 122: Nucleotide sequence of chicken β actin/MVM chimeric intron, synthetic sequence
SEQ ID NO: 123: Nucleotide sequence including Rep region of AAV2, synthetic sequence
SEQ ID NO: 124: Nucleotide sequence of Cap region of AAV8
SEQ ID NO: 125: Amino acid sequence of mutant mouse serum albumin (MSA-A320T)
SEQ ID NO: 126: Example of amino acid sequence of wild-type mIL-10
SEQ ID NO: 127: Example of nucleotide sequence encoding wild-type mIL-10, synthetic sequence
SEQ ID NO: 128: Example of amino acid sequence of mIL-10-MSA
SEQ ID NO: 129: Example of nucleotide sequence encoding mIL-10-MSA, synthetic sequence
SEQ ID NO: 130: Example of amino acid sequence of MSA-mIL-10
SEQ ID NO: 131: Example of nucleotide sequence encoding MSA-mIL-10, synthetic sequence
SEQ ID NO: 132: Example of amino acid sequence of wild-type hBDNF attached with His6
SEQ ID NO: 133: Example of nucleotide sequence encoding wild-type hBDNF attached with His6, synthetic sequence
SEQ ID NO: 134: Example of amino acid sequence of wild-type hNGF attached with His6
SEQ ID NO: 135: Example of nucleotide sequence encoding wild-type hNGF attached with His6, synthetic sequence
SEQ ID NO: 136: Example of amino acid sequence of wild-type hNT-4 attached with His6
SEQ ID NO: 137: Example of nucleotide sequence encoding wild-type hNT-4 attached with His6, synthetic sequence
SEQ ID NO: 138: Example of amino acid sequence of wild-type hNT-3 attached with His6
SEQ ID NO: 139: Example of nucleotide sequence encoding wild-type hNT-3 attached with His6, synthetic sequence
SEQ ID NO: 140: Example of amino acid sequence of wild-type HSA attached with His6
SEQ ID NO: 141: Example of nucleotide sequence encoding wild-type HSA attached with His6, synthetic sequence
SEQ ID NO: 142: Forward primer 1, synthetic sequence
SEQ ID NO: 143: Forward primer 2, synthetic sequence
SEQ ID NO: 144: Forward primer 3, synthetic sequence
SEQ ID NO: 145: Forward primer 4, synthetic sequence
SEQ ID NO: 146: Forward primer 5, synthetic sequence
SEQ ID NO: 147: Reverse primer, synthetic sequence
SEQ ID NO: 148: Amino acid sequence of hBDNF-HSA
SEQ ID NO: 149: Amino acid sequence of hNGF-HSA
SEQ ID NO: 150: Amino acid sequence of hNT-4-HSA
SEQ ID NO: 151: Amino acid sequence of hNT-3-HSA
SEQ ID NO: 152: Amino acid sequence of HSA-hBDNF
SEQ ID NO: 153: Amino acid sequence of HSA-hNGF
SEQ ID NO: 154: Amino acid sequence of HSA-hNT-4
SEQ ID NO: 155: Amino acid sequence of HSA-hNT-3
SEQ ID NO: 156: Example of amino acid sequence of wild-type hCDNF attached with His6
SEQ ID NO: 157: Example of nucleotide sequence encoding wild-type hCDNF attached with His6, synthetic sequence
SEQ ID NO: 158: Amino acid sequence of hCDNF-HSA
SEQ ID NO: 159: Nucleotide sequence encoding hCDNF-HSA, synthetic sequence
SEQ ID NO: 160: Amino acid sequence of HSA-hCDNF
SEQ ID NO: 161: Nucleotide sequence encoding HSA-hCDNF, synthetic sequence
SEQ ID NO: 162: Example of amino acid sequence of wild-type hGDNF attached with His6
SEQ ID NO: 163: Example of nucleotide sequence encoding wild-type hGDNF attached with His6, synthetic sequence
SEQ ID NO: 164: Amino acid sequence of hGDNF-HSA
SEQ ID NO: 165: Nucleotide sequence encoding hGDNF-HSA, synthetic sequence
SEQ ID NO: 166: Amino acid sequence of HSA-hGDNF
SEQ ID NO: 167: Nucleotide sequence encoding HSA-hGDNF, synthetic sequence
SEQ ID NO: 168: Example of amino acid sequence of wild-type hNRTN attached with His6
SEQ ID NO: 169: Example of nucleotide sequence encoding wild-type hNRTN attached with His6, synthetic sequence
SEQ ID NO: 170: Amino acid sequence of hNRTN-HSA
SEQ ID NO: 171: Nucleotide sequence encoding hNRTN-HSA, synthetic sequence
SEQ ID NO: 172: Amino acid sequence of HSA-hNRTN
SEQ ID NO: 173: Nucleotide sequence encoding HSA-hNRTN, synthetic sequence
SEQ ID NO: 174: Example of amino acid sequence of wild-type hARTN attached with His6
SEQ ID NO: 175: Example of nucleotide sequence encoding wild-type hARTN attached with His6, synthetic sequence
SEQ ID NO: 176: Amino acid sequence of hARTN-HSA
SEQ ID NO: 177: Nucleotide sequence encoding hARTN-HSA, synthetic sequence
SEQ ID NO: 178: Amino acid sequence of HSA-hARTN
SEQ ID NO: 179: Nucleotide sequence encoding HSA-hARTN6, synthetic sequence
SEQ ID NO: 180: Example of amino acid sequence of wild-type hPSPN attached with His6
SEQ ID NO: 181: Example of nucleotide sequence encoding wild-type hPSPN attached with His6, synthetic sequence
SEQ ID NO: 182: Amino acid sequence of hPSPN-HSA
SEQ ID NO: 183: Nucleotide sequence encoding hPSPN-HSA, synthetic sequence
SEQ ID NO: 184: Amino acid sequence of HSA-hPSPN
SEQ ID NO: 185: Nucleotide sequence encoding HSA-hPSPN, synthetic sequence
SEQ ID NO: 186: Amino acid sequence of TfR-binding VHH
SEQ ID NO: 187: Nucleotide sequence encoding TfR-binding VHH, synthetic sequence
SEQ ID NO: 188: Nucleotide sequence of forward primer (SF158), synthetic sequence
SEQ ID NO: 189: Nucleotide sequence of reverse primer (SF211), synthetic sequence
SEQ ID NO: 190: Nucleotide sequence of reverse primer (SF149), synthetic sequence
SEQ ID NO: 191: Amino acid sequence of hBDNF-VHH
SEQ ID NO: 192: Amino acid sequence of hBDNF-HSA-VHH
SEQ ID NO: 193: Amino acid sequence of hBDNF-VHH-HSA
SEQ ID NO: 194: Nucleotide sequence encoding chimeric receptor consisting of extracellular domain of BDNF receptor TrkB and transmembrane domain and cytoplasmic domain of thrombopoietin receptor c-Mpl, synthetic sequence
SEQ ID NO: 195: Amino acid sequence of histidine tag
SEQ ID NO: 196: Example 4 of amino acid sequence of linker

## Claims

1. A fusion protein comprising a neurotrophic factor and serum albumin (SA).

2. The fusion protein according to claim 1, wherein the neurotrophic factor is a human neurotrophic factor.

3. The fusion protein according to claim 1 or 2, wherein the SA is human serum albumin (HSA).

4. The fusion protein according to any one of claims 1 to 3, wherein the neurotrophic factor is a human brain-derived neurotrophic factor (hBDNF) having an identity of 80% or more to wild-type human brain-derived neurotrophic factor having an amino acid sequence represented by SEQ ID NO: 60, and the SA is a human serum albumin (HSA) having an identity of 80% or more to wild-type human serum albumin having an amino acid sequence represented by SEQ ID NO: 3.

5. The fusion protein according to claim 4, wherein the hBDNF has an identity of 90% or more to the wild-type hBDNF having the amino acid sequence represented by SEQ ID NO: 60, and the HSA has an identity of 90% or more to the wild-type HSA having the amino acid sequence represented by SEQ ID NO: 3.

6. The fusion protein according to claim 4, wherein the hBDNF comprises an amino acid sequence having a substitution, deletion or/and addition of 1 to 10 amino acids in the amino acid sequence of the wild-type hBDNF represented by SEQ ID NO: 60.

7. The fusion protein according to claim 4, wherein the hBDNF comprises an amino acid sequence having a substitution, deletion or/and addition of 1 to 5 amino acids in the amino acid sequence of the wild-type hBDNF represented by SEQ ID NO: 60.

8. The fusion protein according to claim 4, wherein the hBDNF comprises an amino acid sequence having a substitution, deletion or/and addition of 1 to 3 amino acids in the amino acid sequence of the wild-type hBDNF represented by SEQ ID NO: 60.

9. The fusion protein according to claim 4, wherein the hBDNF comprises an amino acid sequence having a single amino acid substitution in the amino acid sequence of the wild-type hBDNF represented by SEQ ID NO: 60.

10. The fusion protein according to claim 9, wherein the amino acid substitution is a substitution within a family of amino acids having a side chain to be possibly hydroxylated.

11. The fusion protein according to any one of claims 4 to 10, wherein the HSA comprises an amino acid sequence having a substitution, deletion or/and addition of 1 to 10 amino acids in the amino acid sequence of the wild-type HSA represented by SEQ ID NO: 3.

12. The fusion protein according to any one of claims 4 to 10, wherein the HSA comprises an amino acid sequence having a substitution, deletion or/and addition of 1 to 5 amino acids in the amino acid sequence of the wild-type HSA represented by SEQ ID NO: 3.

13. The fusion protein according to any one of claims 4 to 10, wherein the HSA comprises an amino acid sequence having a substitution, deletion or/and addition of 1 to 3 amino acids in the amino acid sequence of the wild-type HSA represented by SEQ ID NO: 3.

14. The fusion protein according to claim 4, wherein the hBDNF comprises the amino acid sequence of the wild-type hBDNF represented by SEQ ID NO: 60, and the HSA comprises the amino acid sequence of the wild-type human serum albumin represented by SEQ ID NO: 3.

15. The fusion protein according to claim 4, wherein the hBDNF comprises the amino acid sequence of the wild-type hBDNF represented by SEQ ID NO: 60, and the HSA comprises an amino acid sequence of a wild-type human serum albumin represented by SEQ ID NO: 12.

16. The fusion protein according to claim 4, wherein the hBDNF comprises the amino acid sequence of the wild-type hBDNF represented by SEQ ID NO: 60, and the HSA comprises an amino acid sequence of a wild-type human serum albumin represented by SEQ ID NO: 13.

17. The fusion protein according to any one of claims 4 to 16, wherein the HSA is bound to a C terminal of the hBDNF directly or via a linker.

18. The fusion protein according to any one of claims 4 to 16, wherein the hBDNF is bound to a C terminal of the HSA directly or via a linker.

19. The fusion protein according to claim 17 or 18,
wherein the linker is a peptide chain consisting of 1 to 150 amino acids.

20. The fusion protein according to claim 19, wherein the linker consists of an amino acid sequence selected from the group consisting of the following (a) to (g):
(a) Gly;
(b) Ser;
(c) Gly Ser;
(d) Gly Gly Ser;
(e) an amino acid sequence represented by SEQ ID NO: 9;
(f) an amino acid sequence represented by SEQ ID NO: 10; and
(g) an amino acid sequence represented by SEQ ID NO: 11.

21. The fusion protein according to claim 19, wherein the linker consists of 2 to 10 repeats of an amino acid sequence selected from the group consisting of the following (a) to (g):
(a) Gly;
(b) Ser;
(c) Gly Ser;
(d) Gly Gly Ser;
(e) the amino acid sequence represented by SEQ ID NO: 9;
(f) the amino acid sequence represented by SEQ ID NO: 10; and
(g) the amino acid sequence represented by SEQ ID NO: 11.

22. The fusion protein according to claim 19, wherein the linker consists of 2 to 6 repeats of an amino acid sequence selected from the group consisting of the following (a) to (g):
(a) Gly;
(b) Ser;
(c) Gly Ser;
(d) Gly Gly Ser;
(e) the amino acid sequence represented by SEQ ID NO: 9;
(f) the amino acid sequence represented by SEQ ID NO: 10; and
(g) the amino acid sequence represented by SEQ ID NO: 11.

23. The fusion protein according to claim 19, wherein the linker consists of 3 to 5 repeats of an amino acid sequence selected from the group consisting of the following (a) to (g):
(a) Gly;
(b) Ser;
(c) Gly Ser;
(d) Gly Gly Ser;
(e) the amino acid sequence represented by SEQ ID NO: 9;
(f) the amino acid sequence represented by SEQ ID NO: 10; and
(g) the amino acid sequence represented by SEQ ID NO: 11.

24. The fusion protein according to claim 19, wherein the linker consists of the amino acid sequence represented by Gly Ser.

25. The fusion protein according to claim 18, wherein the fusion protein comprises an amino acid sequence having an identity of 80% or more to an amino acid sequence represented by SEQ ID NO: 85.

26. The fusion protein according to claim 18, wherein the fusion protein comprises an amino acid sequence having an identity of 90% or more to an amino acid sequence represented by SEQ ID NO: 85.

27. The fusion protein according to claim 26, wherein the fusion protein comprises an amino acid sequence having a substitution, deletion or/and addition of 1 to 10 amino acids in the amino acid sequence represented by SEQ ID NO: 85.

28. The fusion protein according to claim 26, wherein the fusion protein comprises an amino acid sequence having a substitution, deletion or/and addition of 1 to 5 amino acids in the amino acid sequence represented by SEQ ID NO: 85.

29. The fusion protein according to claim 26, wherein the fusion protein comprises an amino acid sequence having a substitution, deletion or/and addition of 1 to 3 amino acids in the amino acid sequence represented by SEQ ID NO: 85.

30. The fusion protein according to claim 18, wherein the fusion protein comprises an amino acid sequence represented by SEQ ID NO: 85.

31. The fusion protein according to claim 17, wherein the fusion protein comprises an amino acid sequence having an identity of 80% or more to an amino acid sequence represented by SEQ ID NO: 76.

32. The fusion protein according to claim 17, wherein the fusion protein comprises an amino acid sequence having an identity of 90% or more to an amino acid sequence represented by SEQ ID NO: 76.

33. The fusion protein according to claim 32, wherein the fusion protein comprises an amino acid sequence having a substitution, deletion or/and addition of 1 to 10 amino acids in the amino acid sequence represented by SEQ ID NO: 76.

34. The fusion protein according to claim 32, wherein the fusion protein comprises an amino acid sequence having a substitution, deletion or/and addition of 1 to 5 amino acids in the amino acid sequence represented by SEQ ID NO: 76.

35. The fusion protein according to claim 32, wherein the fusion protein comprises an amino acid sequence having a substitution, deletion or/and addition of 1 to 3 amino acids in the amino acid sequence represented by SEQ ID NO: 76.

36. The fusion protein according to claim 17, wherein the fusion protein comprises an amino acid sequence represented by SEQ ID NO: 76.

37. The fusion protein according to any one of claims 4 to 36, wherein the fusion protein has a specific activity of 10% or more compared to a specific activity of a normal wild-type hBDNF.

38. The fusion protein according to any one of claims 1 to 3, wherein the neurotrophic factor is a human nerve growth factor (hNGF) having an identity of 80% or more to wild-type human nerve growth factor having an amino acid sequence represented by SEQ ID NO: 62, and the SA is a human serum albumin (HSA) having an identity of 80% or more to wild-type human serum albumin having an amino acid sequence represented by SEQ ID NO: 3.

39. The fusion protein according to claim 38, wherein the hNGF has an identity of 90% or more to the wild-type hNGF having the amino acid sequence represented by SEQ ID NO: 62, and the HSA has an identity of 90% or more to the wild-type HSA having the amino acid sequence represented by SEQ ID NO: 3.

40. The fusion protein according to claim 38, wherein the hNGF comprises an amino acid sequence having a substitution, deletion or/and addition of 1 to 10 amino acids in the amino acid sequence of the wild-type hNGF represented by SEQ ID NO: 62.

41. The fusion protein according to claim 38, wherein the hNGF comprises an amino acid sequence having a substitution, deletion or/and addition of 1 to 5 amino acids in the amino acid sequence of the wild-type hNGF represented by SEQ ID NO: 62.

42. The fusion protein according to claim 38, wherein the hNGF comprises an amino acid sequence having a substitution, deletion or/and addition of 1 to 3 amino acids in the amino acid sequence of the wild-type hNGF represented by SEQ ID NO: 62.

43. The fusion protein according to claim 38, wherein the hNGF comprises an amino acid sequence having a single amino acid substitution in the amino acid sequence of the wild-type hNGF represented by SEQ ID NO: 62.

44. The fusion protein according to claim 43, wherein the amino acid substitution is a substitution within a family of amino acids having a side chain to be possibly hydroxylated.

45. The fusion protein according to any one of claims 38 to 44, wherein the HSA comprises an amino acid sequence having a substitution, deletion or/and addition of 1 to 10 amino acids in the amino acid sequence of the wild-type HSA represented by SEQ ID NO: 3.

46. The fusion protein according to any one of claims 38 to 44, wherein the HSA comprises an amino acid sequence having a substitution, deletion or/and addition of 1 to 5 amino acids in the amino acid sequence of the wild-type HSA represented by SEQ ID NO: 3.

47. The fusion protein according to any one of claims 38 to 44, wherein the HSA comprises an amino acid sequence having a substitution, deletion or/and addition of 1 to 3 amino acids in the amino acid sequence of the wild-type HSA represented by SEQ ID NO: 3.

48. The fusion protein according to claim 38, wherein the hNGF comprises the amino acid sequence of the wild-type hNGF represented by SEQ ID NO: 62, and the HSA comprises the amino acid sequence of the wild-type HSA represented by SEQ ID NO: 3.

49. The fusion protein according to claim 38, wherein the hNGF comprises the amino acid sequence of the wild-type hNGF represented by SEQ ID NO: 62, and the HSA comprises the amino acid sequence of the wild-type HSA represented by SEQ ID NO: 12.

50. The fusion protein according to claim 38, wherein the hNGF comprises the amino acid sequence of the wild-type hNGF represented by SEQ ID NO: 62, and the HSA comprises the amino acid sequence of the wild-type HSA represented by SEQ ID NO: 13.

51. The fusion protein according to any one of claims 38 to 50, wherein the HSA is bound to a C terminal of the hNGF directly or via a linker.

52. The fusion protein according to any one of claims 38 to 50, wherein the hNGF is bound to the C terminal of the HSA directly or via a linker.

53. The fusion protein according to claim 51 or 52,
wherein the linker is a peptide chain consisting of 1 to 150 amino acids.

54. The fusion protein according to claim 53, wherein the linker consists of an amino acid sequence selected from the group consisting of the following (a) to (g):
(a) Gly;
(b) Ser;
(c) Gly Ser;
(d) Gly Gly Ser;
(e) an amino acid sequence represented by SEQ ID NO: 9;
(f) an amino acid sequence represented by SEQ ID NO: 10; and
(g) an amino acid sequence represented by SEQ ID NO: 11.

55. The fusion protein according to claim 53, wherein the linker consists of 2 to 10 repeats of an amino acid sequence selected from the group consisting of the following (a) to (g):
(a) Gly;
(b) Ser;
(c) Gly Ser;
(d) Gly Gly Ser;
(e) the amino acid sequence represented by SEQ ID NO: 9;
(f) the amino acid sequence represented by SEQ ID NO: 10; and
(g) the amino acid sequence represented by SEQ ID NO: 11.

56. The fusion protein according to claim 53, wherein the linker consists of 2 to 6 repeats of an amino acid sequence selected from the group consisting of the following (a) to (g):
(a) Gly;
(b) Ser;
(c) Gly Ser;
(d) Gly Gly Ser;
(e) the amino acid sequence represented by SEQ ID NO: 9;
(f) the amino acid sequence represented by SEQ ID NO: 10; and
(g) the amino acid sequence represented by SEQ ID NO: 11.

57. The fusion protein according to claim 53, wherein the linker consists of 3 to 5 repeats of an amino acid sequence selected from the group consisting of the following (a) to (g):
(a) Gly;
(b) Ser;
(c) Gly Ser;
(d) Gly Gly Ser;
(e) the amino acid sequence represented by SEQ ID NO: 9;
(f) the amino acid sequence represented by SEQ ID NO: 10; and
(g) the amino acid sequence represented by SEQ ID NO: 11.

58. The fusion protein according to claim 53, wherein the linker consists of the amino acid sequence represented by Gly Ser.

59. The fusion protein according to claim 52, wherein the fusion protein comprises an amino acid sequence having an identity of 80% or more to an amino acid sequence represented by SEQ ID NO: 88.

60. The fusion protein according to claim 52, wherein the fusion protein comprises an amino acid sequence having an identity of 90% or more to an amino acid sequence represented by SEQ ID NO: 88.

61. The fusion protein according to claim 60, wherein the fusion protein comprises an amino acid sequence having a substitution, deletion or/and addition of 1 to 10 amino acids in the amino acid sequence represented by SEQ ID NO: 88.

62. The fusion protein according to claim 60, wherein the fusion protein comprises an amino acid sequence having a substitution, deletion or/and addition of 1 to 5 amino acids in the amino acid sequence represented by SEQ ID NO: 88.

63. The fusion protein according to claim 60, wherein the fusion protein comprises an amino acid sequence having a substitution, deletion or/and addition of 1 to 3 amino acids in the amino acid sequence represented by SEQ ID NO: 88.

64. The fusion protein according to claim 51, wherein the fusion protein comprises an amino acid sequence represented by SEQ ID NO: 88.

65. The fusion protein according to claim 51, wherein the fusion protein comprises an amino acid sequence having an identity of 80% or more to an amino acid sequence represented by SEQ ID NO: 78.

66. The fusion protein according to claim 51, wherein the fusion protein comprises an amino acid sequence having an identity of 90% or more to an amino acid sequence represented by SEQ ID NO: 78.

67. The fusion protein according to claim 66, wherein the fusion protein comprises an amino acid sequence having a substitution, deletion or/and addition of 1 to 10 amino acids in the amino acid sequence represented by SEQ ID NO: 78.

68. The fusion protein according to claim 66, wherein the fusion protein comprises an amino acid sequence having a substitution, deletion or/and addition of 1 to 5 amino acids in the amino acid sequence represented by SEQ ID NO: 78.

69. The fusion protein according to claim 66, wherein the fusion protein comprises an amino acid sequence having a substitution, deletion or/and addition of 1 to 3 amino acids in the amino acid sequence represented by SEQ ID NO: 78.

70. The fusion protein according to claim 51, wherein the fusion protein comprises an amino acid sequence represented by SEQ ID NO: 78.

71. The fusion protein according to any one of claims 38 to 70, wherein the fusion protein has a specific activity of 10% or more compared to a specific activity of a normal wild-type hNGF.

72. The fusion protein according to any one of claims 1 to 3, wherein the neurotrophic factor is human neurotrophin-3 (hNT-3) having an identity of 80% or more to wild-type human neurotrophin-3 having an amino acid sequence represented by SEQ ID NO: 64, and the SA is human serum albumin (HSA) having an identity of 80% or more to wild-type human serum albumin having an amino acid sequence represented by SEQ ID NO: 3.

73. The fusion protein according to claim 72, wherein the hNT-3 has an identity of 90% or more to wild-type hNT-3 having the amino acid sequence represented by SEQ ID NO: 64, and the HSA has an identity of 90% or more to the wild-type HSA having the amino acid sequence represented by SEQ ID NO: 3.

74. The fusion protein according to claim 72, wherein the hNT-3 comprises an amino acid sequence having a substitution, deletion or/and addition of 1 to 10 amino acids in the amino acid sequence of the wild-type hNT-3 represented by SEQ ID NO: 64.

75. The fusion protein according to claim 72, wherein the hNT-3 comprises an amino acid sequence having a substitution, deletion or/and addition of 1 to 5 amino acids in the amino acid sequence of the wild-type hNT-3 represented by SEQ ID NO: 64.

76. The fusion protein according to claim 72, wherein the hNT-3 comprises an amino acid sequence having a substitution, deletion or/and addition of 1 to 3 amino acids in the amino acid sequence of the wild-type hNT-3 represented by SEQ ID NO: 64.

77. The fusion protein according to claim 72, wherein the hNT-3 comprises an amino acid sequence having a single amino acid substitution in the amino acid sequence of the wild-type hNT-3 represented by SEQ ID NO: 64.

78. The fusion protein according to claim 77, wherein the amino acid substitution is a substitution within a family of amino acids having a side chain to be possibly hydroxylated.

79. The fusion protein according to any one of claims 72 to 78, wherein the HSA comprises an amino acid sequence having a substitution, deletion or/and addition of 1 to 10 amino acids in the amino acid sequence of the wild-type HSA represented by SEQ ID NO: 3.

80. The fusion protein according to any one of claims 72 to 78, wherein the HSA comprises an amino acid sequence having a substitution, deletion or/and addition of 1 to 5 amino acids in the amino acid sequence of the wild-type HSA represented by SEQ ID NO: 3.

81. The fusion protein according to any one of claims 72 to 78, wherein the HSA comprises an amino acid sequence having a substitution, deletion or/and addition of 1 to 3 amino acids in the amino acid sequence of the wild-type HSA represented by SEQ ID NO: 3.

82. The fusion protein according to claim 72, wherein the hNT-3 comprises the amino acid sequence of the wild-type hNT-3 represented by SEQ ID NO: 64, and the HSA comprises the amino acid sequence of the wild-type human serum albumin represented by SEQ ID NO: 3.

83. The fusion protein according to claim 72, wherein the hNT-3 comprises the amino acid sequence of the wild-type hNT-3 represented by SEQ ID NO: 64, and the HSA comprises the amino acid sequence of the wild-type human serum albumin represented by SEQ ID NO: 12.

84. The fusion protein according to claim 72, wherein the hNT-3 comprises the amino acid sequence of the wild-type hNT-3 represented by SEQ ID NO: 64 and the HSA comprises the amino acid sequence of the wild-type human serum albumin represented by SEQ ID NO: 13.

85. The fusion protein according to any one of claims 72 to 84, wherein the HSA is bound to a C terminal of the hNT-3 directly or via a linker.

86. The fusion protein according to any one of claims 72 to 84, wherein the hNT-3 is bound to the C terminal of the HSA directly or via a linker.

87. The fusion protein according to claim 85 or 86,
wherein the linker is a peptide chain consisting of 1 to 150 amino acids.

88. The fusion protein according to claim 87, wherein the linker consists of an amino acid sequence selected from the group consisting of the following (a) to (g):
(a) Gly;
(b) Ser;
(c) Gly Ser;
(d) Gly Gly Ser;
(e) an amino acid sequence represented by SEQ ID NO: 9;
(f) an amino acid sequence represented by SEQ ID NO: 10; and
(g) an amino acid sequence represented by SEQ ID NO: 11.

89. The fusion protein according to claim 87, wherein the linker consists of 2 to 10 repeats of an amino acid sequence selected from the group consisting of the following (a) to (g):
(a) Gly;
(b) Ser;
(c) Gly Ser;
(d) Gly Gly Ser;
(e) the amino acid sequence represented by SEQ ID NO: 9;
(f) the amino acid sequence represented by SEQ ID NO: 10; and
(g) the amino acid sequence represented by SEQ ID NO: 11.

90. The fusion protein according to claim 87, wherein the linker consists of 2 to 6 repeats of an amino acid sequence selected from the group consisting of the following (a) to (g):
(a) Gly;
(b) Ser;
(c) Gly Ser;
(d) Gly Gly Ser;
(e) the amino acid sequence represented by SEQ ID NO: 9;
(f) the amino acid sequence represented by SEQ ID NO: 10; and
(g) the amino acid sequence represented by SEQ ID NO: 11.

91. The fusion protein according to claim 87, wherein the linker consists of 3 to 5 repeats of an amino acid sequence selected from the group consisting of the following (a) to (g):
(a) Gly;
(b) Ser;
(c) Gly Ser;
(d) Gly Gly Ser;
(e) the amino acid sequence represented by SEQ ID NO: 9;
(f) the amino acid sequence represented by SEQ ID NO: 10; and
(g) the amino acid sequence represented by SEQ ID NO: 11.

92. The fusion protein according to claim 87, wherein the linker consists of the amino acid sequence represented by Gly Ser.

93. The fusion protein according to claim 86, wherein the fusion protein comprises an amino acid sequence having an identity of 80% or more to an amino acid sequence represented by SEQ ID NO: 91.

94. The fusion protein according to claim 86, wherein the fusion protein comprises an amino acid sequence having an identity of 90% or more to an amino acid sequence represented by SEQ ID NO: 91.

95. The fusion protein according to claim 94, wherein the fusion protein comprises an amino acid sequence having a substitution, deletion or/and addition of 1 to 10 amino acids in the amino acid sequence represented by SEQ ID NO: 91.

96. The fusion protein according to claim 94, wherein the fusion protein comprises an amino acid sequence having a substitution, deletion or/and addition of 1 to 5 amino acids in the amino acid sequence represented by SEQ ID NO: 91.

97. The fusion protein according to claim 94, wherein the fusion protein comprises an amino acid sequence having a substitution, deletion or/and addition of 1 to 3 amino acids in the amino acid sequence represented by SEQ ID NO: 91.

98. The fusion protein according to claim 86, wherein the fusion protein comprises an amino acid sequence represented by SEQ ID NO: 91.

99. The fusion protein according to claim 85, wherein the fusion protein comprises an amino acid sequence having an identity of 80% or more to an amino acid sequence represented by SEQ ID NO: 80.

100. The fusion protein according to claim 85, wherein the fusion protein comprises an amino acid sequence having an identity of 90% or more to an amino acid sequence represented by SEQ ID NO: 80.

101. The fusion protein according to claim 100, wherein the fusion protein comprises an amino acid sequence having a substitution, deletion or/and addition of 1 to 10 amino acids in the amino acid sequence represented by SEQ ID NO: 80.

102. The fusion protein according to claim 100, wherein the fusion protein comprises an amino acid sequence having a substitution, deletion or/and addition of 1 to 5 amino acids in the amino acid sequence represented by SEQ ID NO: 80.

103. The fusion protein according to claim 100, wherein the fusion protein comprises an amino acid sequence having a substitution, deletion or/and addition of 1 to 3 amino acids in the amino acid sequence represented by SEQ ID NO: 80.

104. The fusion protein according to claim 85, wherein the fusion protein comprises an amino acid sequence represented by SEQ ID NO: 80.

105. The fusion protein according to any one of claims 72 to 104, wherein the fusion protein has a specific activity of 10% or more compared to a specific activity of a normal wild-type hNT-3.

106. The fusion protein according to any one of claims 1 to 3, wherein the neurotrophic factor is human neurotrophin-4 (hNT-4) having an identity of 80% or more to wild-type human neurotrophin-4 having an amino acid sequence represented by SEQ ID NO: 66, and the SA is human serum albumin (HSA) having an identity of 80% or more to wild-type human serum albumin having an amino acid sequence represented by SEQ ID NO: 3.

107. The fusion protein according to claim 106, wherein the hNT-4 has an identity of 90% or more to wild-type hNT-4 having an amino acid sequence represented by SEQ ID NO: 66, and the HSA has an identity of 90% or more to the wild-type HSA having the amino acid sequence represented by SEQ ID NO: 3.

108. The fusion protein according to claim 106, wherein the hNT-4 comprises an amino acid sequence having a substitution, deletion or/and addition of 1 to 10 amino acids in the amino acid sequence of the wild-type hNT-4 represented by SEQ ID NO: 66.

109. The fusion protein according to claim 106, wherein the hNT-4 comprises an amino acid sequence having a substitution, deletion or/and addition of 1 to 5 amino acids in the amino acid sequence of the wild-type hNT-4 represented by SEQ ID NO: 66.

110. The fusion protein according to claim 106, wherein the hNT-4 comprises an amino acid sequence having a substitution, deletion or/and addition of 1 to 3 amino acids in the amino acid sequence of the wild-type hNT-4 represented by SEQ ID NO: 66.

111. The fusion protein according to claim 106, wherein the hNT-4 comprises an amino acid sequence having a single amino acid substitution in the amino acid sequence of the wild-type hNT-4 represented by SEQ ID NO: 66.

112. The fusion protein according to claim 111, wherein the amino acid substitution is a substitution within a family of amino acids having a side chain to be possibly hydroxylated.

113. The fusion protein according to any one of claims 106 to 112, wherein the HSA comprises an amino acid sequence having a substitution, deletion or/and addition of 1 to 10 amino acids in the amino acid sequence of the wild-type HSA represented by SEQ ID NO: 3.

114. The fusion protein according to any one of claims 106 to 112, wherein the HSA comprises an amino acid sequence having a substitution, deletion or/and addition of 1 to 5 amino acids in the amino acid sequence of the wild-type HSA represented by SEQ ID NO: 3.

115. The fusion protein according to any one of claims 106 to 112, wherein the HSA comprises an amino acid sequence having a substitution, deletion or/and addition of 1 to 3 amino acids in the amino acid sequence of the wild-type HSA represented by SEQ ID NO: 3.

116. The fusion protein according to claim 106, wherein the hNT-4 comprises the amino acid sequence of the wild-type hNT-4 represented by SEQ ID NO: 66, and the HSA comprises the amino acid sequence of the wild-type HSA represented by SEQ ID NO: 3.

117. The fusion protein according to claim 106, wherein the hNT-4 comprises the amino acid sequence of the wild-type hNT-4 represented by SEQ ID NO: 66, and the HSA comprises the amino acid sequence of the wild-type HSA represented by SEQ ID NO: 12.

118. The fusion protein according to claim 106, wherein the hNT-4 comprises the amino acid sequence of the wild-type hNT-4 represented by SEQ ID NO: 66, and the HSA comprises the amino acid sequence of the wild-type HSA represented by SEQ ID NO: 13.

119. The fusion protein according to any one of claims 106 to 118, wherein the HSA is bound to a C terminal of the hNT-4 directly or via a linker.

120. The fusion protein according to any one of claims 106 to 118, wherein the hNT-4 is bound to the C terminal of the HSA directly or via a linker.

121. The fusion protein according to claim 119 or 120,
wherein the linker is a peptide chain consisting of 1 to 150 amino acids.

122. The fusion protein according to claim 121, wherein the linker consists of an amino acid sequence selected from the group consisting of the following (a) to (g):
(a) Gly;
(b) Ser;
(c) Gly Ser;
(d) Gly Gly Ser;
(e) an amino acid sequence represented by SEQ ID NO: 9;
(f) an amino acid sequence represented by SEQ ID NO: 10; and
(g) an amino acid sequence represented by SEQ ID NO: 11.

123. The fusion protein according to claim 121, wherein the linker consists of 2 to 10 repeats of an amino acid sequence selected from the group consisting of the following (a) to (g):
(a) Gly;
(b) Ser;
(c) Gly Ser;
(d) Gly Gly Ser;
(e) the amino acid sequence represented by SEQ ID NO: 9;
(f) the amino acid sequence represented by SEQ ID NO: 10; and
(g) the amino acid sequence represented by SEQ ID NO: 11.

124. The fusion protein according to claim 121, wherein the linker consists of 2 to 6 repeats of an amino acid sequence selected from the group consisting of the following (a) to (g):
(a) Gly;
(b) Ser;
(c) Gly Ser;
(d) Gly Gly Ser;
(e) the amino acid sequence represented by SEQ ID NO: 9;
(f) the amino acid sequence represented by SEQ ID NO: 10; and
(g) the amino acid sequence represented by SEQ ID NO: 11.

125. The fusion protein according to claim 121, wherein the linker consists of 3 to 5 repeats of an amino acid sequence selected from the group consisting of the following (a) to (g):
(a) Gly;
(b) Ser;
(c) Gly Ser;
(d) Gly Gly Ser;
(e) the amino acid sequence represented by SEQ ID NO: 9;
(f) the amino acid sequence represented by SEQ ID NO: 10; and
(g) the amino acid sequence represented by SEQ ID NO: 11.

126. The fusion protein according to claim 121, wherein the linker consists of the amino acid sequence represented by Gly Ser.

127. The fusion protein according to claim 120, wherein the fusion protein comprises an amino acid sequence having an identity of 80% or more to an amino acid sequence represented by SEQ ID NO: 94.

128. The fusion protein according to claim 120, wherein the fusion protein comprises an amino acid sequence having an identity of 90% or more to an amino acid sequence represented by SEQ ID NO: 94.

129. The fusion protein according to claim 128, wherein the fusion protein comprises an amino acid sequence having a substitution, deletion or/and addition of 1 to 10 amino acids in the amino acid sequence represented by SEQ ID NO: 94.

130. The fusion protein according to claim 128, wherein the fusion protein comprises an amino acid sequence having a substitution, deletion or/and addition of 1 to 5 amino acids in the amino acid sequence represented by SEQ ID NO: 94.

131. The fusion protein according to claim 128, wherein the fusion protein comprises an amino acid sequence having a substitution, deletion or/and addition of 1 to 3 amino acids in the amino acid sequence represented by SEQ ID NO: 94.

132. The fusion protein according to claim 119, wherein the fusion protein comprises an amino acid sequence represented by SEQ ID NO: 94.

133. The fusion protein according to claim 119, wherein the fusion protein comprises an amino acid sequence having an identity of 80% or more to an amino acid sequence represented by SEQ ID NO: 82.

134. The fusion protein according to claim 119, wherein the fusion protein comprises an amino acid sequence having an identity of 90% or more to an amino acid sequence represented by SEQ ID NO: 82.

135. The fusion protein according to claim 134, wherein the fusion protein comprises an amino acid sequence having a substitution, deletion or/and addition of 1 to 10 amino acids in the amino acid sequence represented by SEQ ID NO: 82.

136. The fusion protein according to claim 134, wherein the fusion protein comprises an amino acid sequence having a substitution, deletion or/and addition of 1 to 5 amino acids in the amino acid sequence represented by SEQ ID NO: 82.

137. The fusion protein according to claim 134, wherein the fusion protein comprises an amino acid sequence having a substitution, deletion or/and addition of 1 to 3 amino acids in the amino acid sequence represented by SEQ ID NO: 82.

138. The fusion protein according to claim 134, wherein the fusion protein comprises an amino acid sequence represented by SEQ ID NO: 82.

139. The fusion protein according to any one of claims 106 to 138, wherein the fusion protein has a specific activity of 10% or more compared to a specific activity of a normal wild-type hNT-4.

140. DNA comprising a gene encoding the fusion protein according to any one of claims 1 to 139.

141. An expression vector comprising the DNA according to claim 140.

142. A mammalian cell transformed with the expression vector according to claim 141.

143. A method for producing a fusion protein, comprising a step of culturing the mammalian cell according to claim 142 in a serum-free medium.

144. A conjugate of the fusion protein according to any one of claims 1 to 139 with an antibody, wherein the antibody binds to a receptor on a cerebrovascular endothelial cell to allow the fusion protein to pass through blood-brain barrier (BBB).

145. The conjugate according to claim 144, wherein the receptor on a cerebrovascular endothelial cell is selected from the group consisting of an insulin receptor, a transferrin receptor, a leptin receptor, a lipoprotein receptor, and an IGF receptor.

146. The conjugate according to claim 144, wherein the receptor on a cerebrovascular endothelial cell is a transferrin receptor.

147. The conjugate according to any one of claims 144 to 146, wherein the antibody is a Fab antibody, a F(ab')₂ antibody, a F(ab') antibody, a single domain antibody, a single chain antibody, or an Fc antibody.

148. The conjugate according to any one of claims 144 to 147, wherein the fusion protein is bound to either a C terminal side or N terminal side of a light chain of the antibody.

149. The conjugate according to any one of claims 144 to 147, wherein the fusion protein is bound to either a C terminal side or N terminal side of a heavy chain of the antibody.

150. The conjugate according to any one of claims 144 to 149, wherein the fusion protein is bound to either a C terminal side or N terminal side of a light chain of the antibody or to either a C terminal side or N terminal side of a heavy chain thereof via a linker sequence.

151. The conjugate according to claim 150, wherein the linker sequence consists of 1 to 50 amino acid residues.

152. The conjugate according to claim 151, wherein the linker sequence comprises a single glycine, a single serine or an amino acid sequence selected from the group consisting of amino acid sequence Gly-Ser, amino acid sequence Ser-Ser, amino acid sequence Gly-Gly-Ser, the amino acid sequence of SEQ ID NO: 9, the amino acid sequence of SEQ ID NO: 10, the amino acid sequence of SEQ ID NO: 11, and an amino acid sequence formed by sequentially connecting 1 to 10 of these amino acid sequences.

153. DNA comprising a gene encoding the conjugate according to any one of claims 144 to 152.

154. An expression vector comprising the DNA according to claim 153.

155. A mammalian cell transformed with the expression vector according to claim 154.

156. A method for producing a conjugate of a fusion protein of a protein having physiological activity and SA with an antibody, comprising a step of culturing the mammalian cell according to claim 155 in a serum-free medium.
